(19) Europäisches Patentamt
European Patent Office
Office européen des brevets

(11) EP 0 851 913 B1

(12) **EUROPEAN PATENT SPECIFICATION**

(45) Date of publication and mention of the grant of the patent:
**19.05.2004 Bulletin 2004/21**

(21) Application number: **96926323.5**

(22) Date of filing: **12.08.1996**

(51) Int Cl.[7]: **C12N 9/20**, C11D 3/386

(86) International application number:
**PCT/DK1996/000341**

(87) International publication number:
**WO 1997/007202 (27.02.1997 Gazette 1997/10)**

(54) **NOVEL LIPOLYTIC ENZYMES**

NEUARTIGE LIPOLYTISCHE ENZYME

NOUVELLES ENZYMES LIPOLYTIQUES

(84) Designated Contracting States:
**AT BE CH DE DK ES FI FR GB GR IE IT LI NL PT SE**

(30) Priority:
**11.08.1995 DK 90595**
**29.09.1995 DK 109695**
**14.02.1996 US 11627 P**
**01.04.1996 DK 37496**
**07.05.1996 US 16754 P**

(43) Date of publication of application:
**08.07.1998 Bulletin 1998/28**

(73) Proprietor: **Novozymes A/S**
**2880 Bagsvaerd (DK)**

(72) Inventors:
- **OKKELS, Jens, Sigurd**
**DK-2880 Bagsvaerd (DK)**
- **SVENDSEN, Allan**
**DK-2880 Bagsvaerd (DK)**
- **BORCH, Kim**
**DK-2880 Bagsvaerd (DK)**
- **THELLERSEN, Marianne**
**DK-2880 Bagsvaerd (DK)**
- **PATKAR, Shamkant, Anant**
**DK-2880 Bagsvaerd (DK)**
- **PETERSEN, Dorte, Aaby**
**DK-2880 Bagsvaerd (DK)**
- **ROYER, John, C.**
**Davis, CA 95616 (US)**
- **KRETZSCHMAR, Titus**
**DK-2880 Bagsvaerd (DK)**

(56) References cited:
**EP-B- 0 305 216**
**WO-A-92/05249**
**WO-A-93/01285**
**WO-A-94/03578**
**WO-A-94/14964**

- **DIALOG INFORMATION SERVICES, File 351, Dialog Accession No. 009892827, WPI Accession No. 94-172743/21, ASAHI KASEI KOGYO KK: "Modified Lipase Comprising Hydrophobic Peptide at N-terminal - has Increased Activity and is Useful to Hydrolyse Lipid into Glycerine and Fatty Acid"; & JP,A,06 113 845, 26-04-94, 9421 (Basic).**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 851 913 B1

## Description

FIELD OF THE INVENTION

The present invention relates to novel lipolytic enzymes which are capable of removing a substantial amount of fatty matter during a one cycle wash.

BACKGROUND OF THE INVENTION

For a number of years lipolytic enzymes have been used as detergent enzymes, i. e. to remove lipid or fatty stains from clothes and other textiles. For instance, various microbial lipases have been suggested as detergent enzymes. Examples of such lipases include a *Humicola lanuginosa* lipase, e.g. described in EP 258 068 and EP 305 216, a *Rhizomucor miehei* lipase, e.g. as described in EP 238 023 and Boel, et al., Lipids 23, 701-706, 1988, , *Absidia* sp. lipolytic enzymes (WO 96/13578), a *Candida* lipase, such as a *C. antarctica* lipase, e.g. the *C. antarctica* lipase A or B described in EP 214 761, a *Pseudomonas* lipase such as a *Ps. alcaligenes* and *Ps. pseudoalcaligenes* lipase, e. g. as described in EP 218 272, a *Ps. cepacia* lipase, e.g. as described in EP 331 376, a *Pseudomonas* sp. lipase as disclosed in WO95/14783, a *Bacillus* lipase, e.g. a *B. subtilis* lipase (Dartois et al., 1993), a *B. stearothermophilus* lipase (JP 64/744992) and a *B. pumilus* lipase (EP 91 00664).

Furthermore, a number of cloned lipases have been described, including the *Penicillium camembertii* lipase described by Yamaguchi, S. et al., 1991, the *Geotricum candidum* lipase (Schimada, Y. et al., 1989, Schrag, J.D et al (1991) Nature 351, p. 761), and various *Rhizopus* lipases such as a *R. delemar* lipase (R.D. Joerger and M.J.Hass (1993), Lipids 28 p. 81-88), a *R. niveus* lipase (W. Kugimiya et al. (1992), Biosci. Biotech. Biochem. 5, p. 716-719), *R. javinicus* (W. Uyttenbroeck et al. (1993) Biol. chem. Hoppe-Seyler 374, p. 245-254) and a *R. oryzae* (Haas, M.J., Allen, J. and Berka, T.R. (1991) Gene 109, p.107-113) which has a substantially identical sequence to the other *Rhizopus* lipases.

Other types of lipolytic enzymes having been suggested as detergent enzymes include socalled cutinases, e.g. derived from *Pseudomonas mendocina* as described in WO 88/09367, or a cutinase derived from *Fusarium solani pisi* (e.g. described in WO 90/09446).

In recent years attempts have been made to prepare lipase variants having improved properties for detergent purposes. For instance, WO 92/05249 discloses lipase variants with improved properties, in which certain characteristics of wild-type lipase enzymes have been changed by specific, i.e. site-directed modifications of their amino acid sequences. More specifically, lipase variants are described, in which one or more amino acid residues of the so-called lipid contact zone of the parent lipase has been modified.

WO 94/01541 describes lipase variants with improved properties, in which an amino acid residue occupying a critical position vis a vis the active site of the lipase has been modified.

EP 407 225 discloses lipase variants with improved resistance towards proteolytic enzymes, which have been prepared by specifically defined amino acid modifications.

EP 260 105 describes hydrolases in which an amino acid residue within 15 Å from the active site has been substituted.

WO 95/35381 discloses *Pseudomonas* sp. lipase variants, in particular *P. glumae* and *P. pseudoalcaligenes* lipase variants which have been modified so as to increase the hydrophobicity at the surface of the enzyme.

WO 96/00292 discloses *Pseudomonas* sp. lipase variants, in particular *P. glumae* and *P. pseudoalcaligenes* lipase variants which have been modified so as to improve the enzyme's compatibility to anionic surfactants,

WO 95/30744 discloses mutant lipases such as *Pseudomonas* sp. lipases which have been modified to an increased surfactant resistance

WO 94/25578 discloses mutant lipases comprising at least a substitution of the methionine corresponding to position 21 in the *P. pseudoalcaligenes* lipase, in particular to leucine, serine or alanine.

All of the above mentioned lipase variants have been constructed by use of site-directed mutagenesis resulting in a modification of specific amino acid residues which have been chosen either on the basis of their type or on the basis of their location in the secondary or tertiary structure of the parent lipase.

An alternative approach for constructing mutants or variants of a given protein has been based on random mutagenesis. For instance, US 4,898,331 and WO 93/01285 disclose such techniques.

WO 95/22615 discloses variants of lipolytic enzymes having an improved washing performance, the variants having been prepared by a method involving subjecting a DNA sequence encoding the parent lipolytic enzyme to random mutagenesis and screening for variants having a decreased dependence to calcium and/or an improved tolerance towards a detergent or one or more detergent components as compared to the parent lipolytic enzyme.

WO 95/09909 discloses, inter alia, chemically modified lipases or lipase mutants which has a higher pI than the corresponding modified enzyme.

A drawback of all detergent lipolytic enzymes described until now is that they exert the best fat removing effect after more than one wash cycle, presumably because the known lipolytic enzymes, when deposited on the fatty stain to be removed, are more active during a certain period of the drying process than during the wash process itself (Gormsen et al., in Proceedings of the 3rd World Conference on Detergents, AOCS press, 1993, pp 198-203). This has the practical consequence that at least two wash cycles (separated by a sufficient drying period) are required to obtain a substantial removal of fatty stains.

Some lipolytic enzymes have been described as allegedly being capable of removing fatty matter during the first wash cycle. Thus, WO 94/03578 discloses a detergent composition which in addition to various detergent components an enzyme which is alleged to be capable of exhibiting a substantial lipolytic activity during the main cycle of a wash process. Examples of lipolytic enzymes allegedly exhibiting the above activity include stem-specific cutinases such as the cutinase from *Fusarium solani pisi, Fusarium roseum culmorum, Rhizoctonia solani* and *Alternaria brassicicola.* However, when tested under realistic washing conditions none of these enzymes are capable of removing substantial amounts of a fatty stain during a one cycle wash process (cf the examples hereinafter).

Thus, a need exists for lipolytic enzymes which under realistic wash conditions are capable of removing substantial amounts of fatty matter during one wash cycle.

SUMMARY OF THE INVENTION

The present inventors have now surprisingly identified and constructed a novel class of lipolytic enzymes which are capable of removing substantial amounts of a fatty material during a one cycle wash performed under realistic washing conditions. The present invention is based on this finding.

Accordingly, in a first aspect the invention relates to a lipolytic enzyme which, when present in detergent composition A and/or B defined herein, is capable of removing at least 15 % more lard from a lard stained swatch than the same detergent composition without the enzyme, in a one cycle wash assay comprising subjecting 7 lard-stained cotton swatches (9 x 9 cm) per beaker to a one cycle wash in a thermostated Terg-O-to-Meter (TOM), each beaker containing 1000 ml of water comprising 3.2 mM $Ca^{2+}/Mg^{2+}$ (in a ratio of 5:1) and 5 g/l of said detergent composition, pH 10, and comprising 12500 LU/l of the lipolytic enzyme, the wash treatment being carried out for 20 minutes at a temperature of 30°C, followed by rinsing for 15 minutes in running tap water and overnight linedrying at room temperature, subsequent extraction and quantification of fatty matter on the swatches by Soxhlet extraction.

The Detergent Composition A and/or B and the one cycle wash assay are further described in the Materials and Methods section herein.

The present invention constitutes the first true demonstration of the surprising fact that it is possible to develop (identify and/or create) first wash lipolytic enzymes. Thus, what hitherto has been considered impossible (based on several years intensive research by a number of research teams throughout the world (as reflected by the number of hopeful patent applications filed in this field as mentioned above)) has now been shown to be possible.

The present inventors have developed very convenient and succesful methods for creating first wash lipolytic enzymes.

Accordingly, in a second important aspect the invention relates to a method of preparing a first wash mutated lipolytic enzyme, which method comprises at least the following steps:

(a) subjecting a DNA sequence encoding a parent lipolytic enzyme to mutagenesis, conveniently random mutagenesis to form a variety of mutated DNA sequences;
(b) expressing the mutated DNA sequences in host cells;
(c) screening for host cells expressing a mutated lipolytic enzyme which has a decreased dependence on calcium and/or an improved tolerance towards a detergent or a detergent component as compared to the parent lipolytic enzyme; and
(d) selecting a mutated lipolytic enzyme among those resulting from step (c) which, when present in the detergent composition A and/or B in a concentration of 12500 LU/l, is capable of removing at least 15 % more lard from a lard stained swatch, than the same detergent composition without the enzyme, in the one cycle wash assay described above.

In a further aspect the invention relates to a method of preparing a first wash mutated lipolytic enzyme which method comprises at least the following steps:

(a) constructing mutated DNA sequences by combining a DNA sequence encoding a first parent lipolytic enzyme and a DNA sequence encoding a second parent lipolytic enzyme and optionally further DNA sequences encoding a third (and optionally further) parent lipolytic enzymes, the DNA sequences being sufficiently homologous to allow for recombination between parts of or the entire DNA sequences to take place,

(b) expressing the resulting mutated DNA sequences in host cells, and

(c) selecting a mutated lipolytic enzyme encoded by a mutated DNA sequence which, when present in detergent composition A or B in a concentration of 12500 LU/l, is capable of removing at least 15% more lard from a lard stained swatch than the same detergent composition without the enzyme, in the one cycle wash described above.

In a preferred embodiment the methods according to the second and third aspects of the invention are combined, i.e. a mutated lipolytic enzyme resulting from the method of the second aspect is used as a parent enzyme in the method according to the third aspect.

In a further aspect the invention relates to a DNA construct comprising a DNA sequence encoding a first wash lipolytic enzyme as defined above.

In a still further aspect the invention relates to a recombinant expression vector carrying the DNA construct, a cell which is transformed with the DNA construct or the vector as well as a method of producing a first wash lipolytic enzyme by culturing said cell under conditions conducive to the production of the enzyme, after which the enzyme is recovered from the culture.

In final aspects the invention relates to the use of a first wash lipolytic enzyme as a detergent enzyme, in particular for washing or dishwashing, and to a detergent additive and a detergent composition comprising the enzyme.

**BRIEF DESCRIPTION OF THE DRAWING**

Fig.1 shows the plasmid pYESHL,

Fig. 2 the plasmid pAO1,

Fig. 3 the plasmid pAHL,

Figs 4 and 5 a graphical illustration of a PCR mutagenesis method,

Fig. 6 the plasmid pJSO37;

Fig. 7 the construction of the plasmid pDM177,

Figure 8 the construction of *Aspergillus* vector pCaHj485

Figure 9 shows the original sequence of the *Absidia reflexa* ATTC 44896 lipase. The trip-lett coding for the first amino acid serine of the mature NL127 as well as the stop codon are underlined.

Figure 10 shows the *Absidia reflexa* ATTC 44896 sequence in the context of the yeast expression vector pTiK05.

Figure 11 shows the mating factor $\alpha$1 signal sequence

DEFINITIONS AND BACKGROUND ON LIPASE STRUCTURE

**Definitions of terms used in the present application**

In the present context the term "lipolytic enzyme" is intended to indicate an enzyme classified under the Enzyme Classification number E.C. 3.1.1 (Carboxylic Ester Hydrolases) in accordance with the Recommendations (1992) of the International Union of Biochemistry and Molecular Biology (IUBMB)). Lipolytic enzymes thus exhibit hydrolytic activity towards at least one of the types of ester bonds present in at least one of the following lipids: mono-, di- and triglycerides, phospholipids (all classes), thioesters, cholesterol esters, wax-esters, cutin, suberin, synthetic esters, etc. (cf. the different types of esterbonds mentioned in the context of E.C. 3.1.1).

Thus, the lipolytic enzyme may, e.g., be what has conventionally been termed a lipase, a phospholipase, an esterase or a cutinase. The term "lipolytic enzyme" is intended to embrace naturally-occurring enzymes as well as enzymes, which as compared to a naturally-occurring enzyme, have been modified, e.g. by modification of one or more amino acid residues of the enzyme or by chemical modification. In the present context the latter type of a lipolytic enzyme is termed a variant.

In the present context the capability of the enzyme in removing a substantial amount of fatty matter during a

one cycle wash is also referred to as a first wash effect, a one cycle wash effect, a through-the-wash effect, and the like. Analogously, lipolytic enzymes of the invention, which are capable of effecting removal of a substantial amount of a fatty material during a one cycle wash, are called first wash lipolytic enzymes, through-the-wash lipolytic enzymes, one cycle wash lipolytic enzymes, and the like.
In the present context the term "Detergent Composition A and/or B" as used to define the lard removing capability of a given lipolytic enzyme of the invention is intended to indicate that the lipolytic enzyme has the indicated lard removing capability when present in either or both of Detergent Compositions A and B.

In the present context the term "parent enzyme" is intended to include a naturally-occurring enzyme as well as a variant of such enzyme. Accordingly, the term is used to identify the starting material to be modified in accordance with a method of the invention for preparing first wash lipolytic enzymes, irrespectively of whether said starting material is a naturally-occurring enzyme or a variant of such enzyme.

The term "a variety of mutated sequences" as used about the method according to the second aspect is intended to be understood to indicate that at least two, but preferably a much higher number of different sequences, such as at least 10, at least 50 at least 100, at least 1000 sequences have resulted from the mutagenesis.

The term "random mutagenesis" is intended to be understood in a conventional manner, i.e. to indicate an introduction of one or more mutations at random positions of the parent enzyme or introduction of random amino acid residues in selected positions or regions of the parent enzyme. The random mutagenesis is normally accompanied by a screening which allows the selection of mutated lipolytic enzymes which, as compared with the parent enzyme, have improved properties. Suitable techniques for introducing random mutations and screening for improved properties are discussed in detail below.

The term "satisfactory wash performance" as used about lipolytic enzymes disclosed herein is intended to indicate that the enzyme has an improved performance when tested in a suitable wash assay or a wash related assay (such as the assays described in the Materials and Methods and Example 5 below) as compared to the commercially available lipolytic enzymes (Lumafast and Lipomax from Genencor, Lipolase and Lipolase Ultra from Novo Nordisk and Liposam (from Showa Denko). The improved performance may be in terms of lipid stain removing capability and/ or a decreased calcium dependency, an improved tolerance towards a detergent or detergent component, an increased hydrophobicity, an interesting substrate specificity, or the like.

In the present context, the term "decreased dependence on calcium" as used in connection with the screening for mutated lipolytic enzymes, in particular lipolytic enzymes exhibiting enzymatic activity towards lipase substrates having hydrocarbon chains (ffa-part) of a length exceeding approx. 6-8 C-atomes, is intended to mean that the mutated lipolytic enzyme requires lower amounts of $Ca^{2+}$ for exhibiting the same degree of activity and/or stability as the parent enzyme when tested under similar conditions. In other words the stability and/or activity of the enzyme is/are increased in the absence of calcium as compared to that of the parent enzyme. The stability may, e.g., be assayed by a determination of residual activity upon preincubation under Ca-free conditions and/or DSC (Differential Scanning Calorimetry) in the absence/presence of free Ca2+. Preferably, the mutated lipolytic enzyme of the invention is substantially independent of the presence of calcium for exhibiting enzymatic activity, in particular at a pH higher than 8.

The term "improved tolerance towards a detergent or detergent component" as used in connection with the screening for mutated lipolytic enzymes is intended to mean that the mutated lipolytic enzyme is active at higher concentrations of the detergent or detergent component than the parent lipolytic enzyme.

In the present context the term "detergent" is intended to indicate a mixture of detergent ingredients normally used for washing or dishwashing. Analogously, a "detergent component" is intended to indicate a component or ingredient normally found in detergent or dishwashing compositions, specific examples of which are given in the section further below entitled "Detergent compositions".

## Background on lipolytic enzyme structure and definition of structure terminology

The 3D structure of a number of lipolytic enzymes has been determined. It has been found that the structures have a common motif in the core of the protein consisting of a central β-sheet, one of the strands ending in a nucleophil elbow including the active serine residue (Ollis et al, 1992). Lipolytic enzymes comprise a lipid contact zone which is a surface with increased surface hydrophobicity which interacts with the lipid substrate at or during hydrolysis. For lipolytic enzymes containing a lid the lipid contact zone is typically formed when the enzyme is activated by substrate (and the lid thereby displaced). For lipolytic enzymes which do not contain a lid there is generally little or no corresponding substantial movement leading to the creation of the lipid contact zone. The lipid substrate is a conglomerate of single lipid substrate molecules. The lipid contact zone contains a binding area to which a single lipid substrate molecule binds before hydrolysis. This binding area contains an acyl-binding hydrophobic cleft and a so-called hydrolysis pocket, which is situated around the active site Ser, and in which the hydrolysis of the lipid substrate is believed to take place. The lipid contact zone includes one or more protein secondary structure elements, i.e., loop sequences, the amino acid residues of which contact, bind to and/or interact with the substrate during hydrolysis when the lipolytic

enzyme is activated.

The lipid contact zone may be recognized, e.g. from a three-dimensional structure of the lipolytic enzyme in question created by suitable computer programs. The lipid contact zone may be identified by searching the structure for the relevant features defining the zone, including a zone positioned on top of the active site residues and containing a lid structure (for lipolytic enzymes containing a lid) which when opened creates a hydrophobic surface containing a narrow hydrophobic binding pocket. The conformation of the inactive and activated *H. lanuginosa* lipolytic enzyme, respectively, is shown in Figs. 1 and 2 of WO 92/05249.

In terms of amino acid residues the lipid contact zone of the *H. lanuginosa* lipolytic enzyme is defined by amino acid residues 21-25, 36-38, 56-62, 81-98, 110-116, 144-147, 172-174, 199-213 and 248-269. These residues have been identified on the basis of computer model simulations of the interaction between the lipolytic enzyme and a lipid substrate. For lipolytic enzymes having substantially the same structure as the *H. lanuginosa* lipolytic enzyme, e.g. the lipolytic enzymes produced by *Rhizomucor miehei,* by the *Rhizopus oryzae,* by *Penicillium camembertii* and by *Absidia* sp. (cf the "Background of the Invention" section above) the lipid contact zone is constituted by amino acid residues occupying homologous positions to those given above for the *H. lanuginosa* enzyme. The homologous positions may be identified by an alignment of the relevant amino acid sequences (e.g. using the UWGCG GAP programme) looking for groups of sequence similarity, but may more conveniently be done by comparing the structures or structure models of the relevant enzymes. More specifically, the lipid contact zone of these enzymes is constituted of the following residues (the numbering used refers to the amino acid residue in the mature enzyme, the sequence of which is apparent from the references disclosed in the Background of the Invention section above unless otherwise indicated):

*Penicillium camembertii*: 21-25, 36-38, 56-62, 81-98, 109-115, 143-146, 172-174, 198-212, 247-280;
*Rhizopus oryzae*: 29-33, 39-41, 57-62, 81-98, 109-115, 143-146, 175-177, 202-216, 245-269.
*Rhizomucor miehei:* 29-33, 39-41, 56-61, 80-97, 108-114, 142-145, 174-176, 201-215, 245-269;
*Absidia* sp. lipase: 29-33, 39-41, 56-61, 80-97, 108-114, 142-145, 171-173, 198-212, and 239-263, the numbering based on that the mature enzyme has the following N-terminal sequence: SSKQDYR. The entire sequence is apparent from (SEQ ID NO 98).

As an alternative or in addition to the homology based identification of the lipid contact zone, the lipid contact zone may be identified by

a) calculating the hydrophobic vector of the 3-D molecular structure of the activated enzyme;
b) making a cut perpendicular to the vector through the CA-atom (C$\alpha$-atom) of the second amino acid residue after the active site serine in the linear sequence;
c) including all residues with at least one atom on that side of the cut to which the vector points; and
d) selecting from those residues, those which have at least one atom within 5 Ångström of the surface of the protein.

The hydrophobic vector is calculated from the protein structure by summing up all residue vectors for residues having a surface accessibility (Lee et al., *Mol. Biol.* 55, pp. 379-400 (1971)) of at least 15%. The starting point of the residue vector is defined as the CA-atom of the residue and its direction is through the mass centre of the sidechain. The magnitude of each residue vector is defined as the residues relative free energy of transfer between water and a more hydrophobic solvent (see, e.g., Creighton, Protein, W. Freeman & Co., p. 151 (1984)). The surface accessibility of each residue is calculated using the Connolly program (Lee et al., *op. cit.)*.

Using the above method and/or the alignment of the various sequences, which is apparent from Svendsen et al, Biochimica et Biophysica Acta, 1259 (1995) 9-17, the following lipid contact zones of lipolytic enzymes isolated from various *Pseudomonas* sp. have been identified (the numbering used refers to the amino acid residues of the mature enzyme as presented in the above mentioned publication (Svendsen et al. (1995))):

*Pseudomonas cepacia* lipase: 15-36, 110-167, 209-266, 281-304;
*Pseudomonas pseudoalcaligenes* lipase: 15-35, 106-163, 200-232, 250-271;
*Pseudomonas glumae:* 15-36, 110-167, 209-266, 281-304;
*Pseudomonas mendocina (*SD702) lipase: 19-39, 111-166, 213-244, 258-279 (the sequence is apparent from WO 95/14783),
*Pseudomonas sp.*(Liposam®) lipase: 17-37, 109-161, 208-239, 253-274 (SEQ ID NO 99).
*Pseudomonas wisconsinensis* lipase: 13-34, 106,-161, 200-242, 250-270 (the sequence is apparent from WO96/12012)

The lipid contact zone for lipolytic enzymes which do not contain a lid structure may be determined from the topology of the core as evaluated in a structure or model of the three-dimensional structure of the lipolytic enzyme. In

this manner the lipid contact zone of the *Fusarium solani pisi* lipolytic enzyme has been determined to amino acid residues 40-50, 78-91, 119-121, 147-154, 171-193 (as evaluated on the basis of the mature enzyme).

Some lipolytic enzymes also comprise a surface loop structure, i.e., a lid, which is part of the lipid contact zone. The surface loop structure covers the active serine when the lipolytic enzyme is in inactive form. When the enzyme is activated, the surface loop structure shifts to expose the active serine residue. The surface loop structure has a predominantly hydrophobic inner surface facing the binding pocket and a predominantly hydrophilic outer surface.

Example of lipolytic enzymes which have a surface loop structure are those produced by *Humicola lanuginosa, Rhizomucor miehei, Rhizopus* sp., *Penicillium camembertii* and *Absidia* sp., a number of *Pseudomonas* sp., such as *Ps. cepacia, Ps. aeroginosa, Ps. fragi* (cf. the "Background of the Invention" section above), *Candida rugosa* (Grochulski.P et al (1993) J. Biol. Chem. 268, p. 12843) and the human pancreatic lipase described in Winkler et al., *Nancre* 343, pp. 771-74 (1990).

The surface loop structure of the lipolytic enzyme produced by *Humicola lanuginosa* DSM 4109 is defined by amino acid residues at positions 82-96. The surface loop structure of lipolytic enzymes with substantially the same three-dimensional structure (cf above) is defined by the amino acid residues occupying homologous positions to those of the *H. lanuginosa* lipolytic enzyme, i.e.81-98 (for the *Penicillium camembertii* lipase), 82-99 (for *Rhizopus oryzae*), 80-97 (for *Rhizomucor miehei*), 80-97 (for *Absidae sp* lipase).

The surface loop structure of a representative number of lipolytic enzymes produced by *Pseudomonas* sp. are: *Ps. glumae*: 135-155, *Ps. cepacia* 135-155, *Ps pseudoalcaligenes* 132-152), *Pseudomonas* sp lipase (SD705) (Liposam®) 129-149. shown in SEQ ID NO 99.

## DETAILED DESCRIPTION OF THE INVENTION

### The first wash lipolytic enzyme of the invention

Preferably, the lipolytic enzyme of the invention is capable of effecting an even higher lard removing capability than that stated above. Accordingly, in a preferred embodiment Detergent Composition A and/or B comprising the lipolytic enzyme of the invention is capable of removing at least 15%, such as at least 20% more lard, than Detergent Composition A and/or B, respectively, which does not comprise the lipolytic enzyme, when tested in the one cycle wash assay described herein in a concentration of 12500 LU/l. In a more preferred embodiment the lipolytic enzyme is one, which, when present in Detergent Composition A and/or B allows the detergent composition to remove at least 25 % such as at least 30% or 35 % or 40 % or 50 % more lard than Detergent Composition A and/or B without the lipolytic enzyme, when tested in the one cycle wash assay as described herein.

The concentration of lipolytic enzyme used in the above described one cycle wash assay (i.e. 12500 LU/l) may be considered high for practical applications, but has been chosen for assay purposes in order to minimize the analytical variation. A more realistic concentration is 1250 LU/l which in an alternative embodiment may be used to define the lard removing capability of a lipolytic enzyme of the invention. Accordingly, in a further embodiment the lipolytic enzyme is one which is capable of removing at least 15%, such as at least 20 % more lard, than Detergent Composition A and/or Detergent Composition B which does not comprise the lipolytic enzyme, when used in the one cycle wash assay described herein in a concentration of 1250 LU/l. In an even more preferred embodiment the lipolytic enzyme, when present in Detergent Composition A and/or B in a concentration of 1250 LU/l, allows the detergent composition to remove at least 25 % such as at least 30% or 35 % more lard than Detergent Composition A and/or B without the lipolytic enzyme, when used in a one cycle wash assay as described herein.

In preferred embodiments the first wash lipolytic enzyme of the invention is capable of removing:

- when present in Detergent composition A in a concentration of 1250 LU/l at least 15 % more lard from a lard stained swatch than Detergent composition A without the enzyme,
- when present in Detergent A in a concentration of 12500 LU/l at least 40% more lard from a lard stained swatchthan Detergent Composition A without the enzyme,
- when present in Detergent composition B in a concentration of 1250 LU/l at least 15% more lard from a lard stained swatch than Detergent composition B without the enzyme,
- when present in Detergent B in a concentration of 12500 LU/l at least 15 % more lard from a lard stained swatchthan Detergent Composition B without the enzyme,

when tested in a one cycle wash assay as described herein.

In Example 5 herein a comparison is shown between the fat removing capability of lipolytic enzymes of the invention and that of lipolytic enzymes described in WO 94/03578 alleged to have a through-the-wash-effect. It is seen that the enzymes of the invention removed substantially more lard in a one cycle wash than the prior art enzymes. The comparison between the enzymes has been done by use of the same assay.

While the lipolytic enzyme of the invention may be of any of the above mentioned types of lipolytic enzymes such as a hydrolase exhibiting activity towards ester and/or phospholipid bonds, it is particularly preferred that the enzyme is a lipolytic enzyme which exhibits activity towards esterbonds in mono-, di- and/or tri-glycerides and/or which exhibits activity towards cutin. Such enzymes are generally considered to be of high interest as detergent enzymes.

In the Materials and Methods section and in Example 5 low suitable assays for identifying first wash lipolytic enzymes are given. These assay may be used to identify naturally-occurring first wash lipolytic enzymes. More specifically, in order to identify a naturally-occurring first wash lipolytic enzyme according to the invention candidate enzymes are recovered from suitable organisms expected to produce lipolytic enzymes, such as organisms which are taxonomically related to the ones given in the "Background of the Invention" section above or discussed later on in the "Parent Lipolytic Enzymes"section, or organisms which are found in an environment which require the organism to produce lipolytic enzymes in order to prevail. Subsequently, the recovered enzymes are subjected to the first wash lipolytic enzyme assays disclosed herein.

Although the first wash lipolytic enzyme of the invention may be a novel naturally-occurring enzyme (identified on the basis of its first wash performance) it is presently preferred that the enzyme is a modified enzyme, i.e an enzyme which has been prepared by subjecting a parent lipolytic enzyme to mutagenesis and/or to chemical modification so as to result in a modified lipolytic enzyme which has a first wash activity. The parent lipolytic enzyme may be one which has a first wash activity (which may thus be improved by the mutagenesis or chemical modification) or may be without any first wash activity as defined herein. In one embodiment it is considered advantageous that the parent enzyme has a satisfactory wash performance itself or even a first wash performance, the latter property then being improved by the mutation(s). Parent enzymes with a satisfactory (but not necessarily a first wash performance) may be selected using the assay described in Example 6 hereinafter.

The chemical modification of amino acid residues of the parent enzyme may e.g. be performed in accordance with the principles disclosed in WO 95/09909 the content of which is incorporated herein by reference. For instance, the chemical modification may be accomplished by coupling an amine ligand (such as an aminated sugar, aminated alcohol or aminated glucosamine or isomeric forms thereof) to the carboxyl group of glutamic acid or aspartic acid residues in the enzyme. The chemical modification may be performed by methods known in the art, such as those described in WO 95/09909. The chemical modification may be done on acid groups so as to remove negative charges.

The mutagenesis of the parent lipolytic enzyme is preferably done so as to improve the substrate binding affinity of the parent enzyme. More specifically, it has been found that an improved substrate binding affinity may result in a first wash activity being obtained. It is presently contemplated that an improved substrate binding affinity may be achieved by making the surface of the parent enzyme less negative. Accordingly, the mutagenesis may be performed so as to replace at least one neutral amino acid residue located at the surface of the parent enzyme with a positively charged amino acid residue, deleting a negatively charged amino acid residue located at the surface of the parent enzyme or replacing a negatively charged amino acid residue located at the surface of the parent enzyme with a neutral (including hydrophobic) or positively charged amino acid residue. Amino acid residues located at the surface of the enzyme may be identified by use of the Conolly program referred to in the Definitions section above. In a preferred embodiment the mutagenesis is performed so as to remove the amino acid residue D and/or E, and/or to insert, conveniently by replacement, of R, K, W, F, Y, I, L. A suitable test for an improved substrate binding affinity is described in example 11 hereinafter.

The 1st wash effect of the above changes from negative towards positive surface may be improved and/or stabilized by introduction of exchanges optimizing the structure or stability. Thus, for instance introduction of a proline residue into the enzyme surface may lead to an increased proteolytic and/or thermal stability; introduction of hydrophilic amino acid residues, e.g. Glu and/or Asp, may increase the anionic detergent stability, and the introduction of hydrophobic amino acid residues may increase the adsorption/affinity of the enzyme. The introduction of the above type of amino acid residues may either be accomplished by simply inserting the amino acid residues into a suitable location at the surface of the enzyme or by replacing amino acid residue(s) located at such position(s).

It is presently believed that a first wash lipolytic enzyme of the invention is a variant of a parent lipolytic enzyme which comprises at least one mutation, but typically more mutations, preferably located at the surface of the enzyme. The variant may comprise more mutations such as at least 2, 3, 4 or 5 mutations, e.g. in the range of 1-20, 1-15, 1-12, 1-10, 1-9, 1-8, 1-7, 1-6, 1-5 or 1-4 mutations, or any number of mutations which does not impair the enzymatic activity of the enzyme.

It has been found that mutations within as well as outside the lipid contact zone of the parent *H. lanuginosa* lipase disclosed herein may be of importance for achieving a first wash activity. Accordingly, the first wash lipolytic enzyme of the invention carrying a mutation may be constructed from a parent lipolytic enzyme by modification of at least one amino acid residue outside the lipid contact zone of the parent enzyme and/or by addition of at least one amino acid residue outside said zone, and/or by modification of at least one amino acid residue within the lipid contact zone of the parent enzyme and/or by addition of at least one amino acid residue within said zone.

Accordingly, in another embodiment the lipolytic enzyme of the invention is one, which has been prepared

from the parent enzyme by modification, deletion or substitution of at least one amino acid residue in the lipid contact zone of the parent enzyme or addition of at least one amino acid residue to said zone. In a still further embodiment the lipolytic enzyme is one which has been prepared from the parent enzyme by modification, deletion or substituion of at least one amino acid residue outside the lipid contact zone or addition of at least one amino acid residue to said zone, the amino acid residue preferably being located at the surface of the parent enzyme. The mutations within or outside the lipid contact zone are preferably conducted to as to improve the substrate binding affinity of the resulting modified enzyme, conveniently be removal of negative charges as described above.

Although site-directed mutagenesis following the above principles (and combined with testing of the resulting enzyme variants for first wash activity) may be used for the creation of first wash lipolytic enzymes it is presently preferred to use other methods of creating first wash lipolytic enzymes. Random mutagenesis, in particular localized random mutagenesis, as well as in vivo recombination of homologous genes have been found to be of particular interest for that purpose - these methods are described in detail further below.

*First wash lipolytic enzyme modified in a non-structural part of its C- or N-terminus*

It has surprisingly been found that it is possible to confer a first wash effect to a parent lipolytic enzyme or to significantly enhance the first wash effect of a parent lipolytic enzyme by applying at least one N-terminal and/or C-terminal peptide addition at or within a non-structural part of the parent enzyme in its mature form or by introducing other changes in a non-structural part of the C-terminal and/or N-terminal end of the parent mature enzyme.

Accordingly, in a further highly preferred embodiment the first wash lipolytic enzyme of the invention is a variant of a parent lipolytic enzyme which, as compared to the parent enzyme, has been modified at or within a non-structural part of the N- and/or C-terminal end of the parent enzyme.

In the present context the term "modified" is intended to indicate that i) an appropriate peptide addition has been applied to the parent enzyme or ii) one or more amino acid residues within the non-structural part of the C-terminal and/or N-terminal part of the parent mature enzyme has/have been deleted or replaced by other amino acid residues, or iii) the parent enzyme has been modified by a combination of i) or ii). In the present context first wash lipolytic enzymes of the invention which have been modified in this way may be termed a modified enzyme of the invention.

In the present context the term "peptide addition" is intended to indicate that a stretch of one or more consecutive amino acid residues has been added to either or both of the N- and/or C-terminal end(s) of the parent enzyme (i.e. fused to the first and/or last amino acid residue of the parent enzyme) or inserted within the non-structural part of the N- and/or C-terminal end(s) of the parent enzyme. The modified enzyme may comprise a peptide addition at either the N-terminal or the C-terminal end or both in the N- and the C-terminal ends of the parent lipolytic enzyme. If a peptide addition is applied to both the N- and the C-terminus of the parent enzyme, the peptide additon at either terminus may have the same amino acid sequence or different amino acid sequence. Multiple copies of the same or different peptide additions may be inserted or added.

The term "an appropriate peptide addition" is used to indicate that the peptide addition to be used is one which is capable of effecting a first wash performance or an improved first wash performance. The "appropriateness" of the peptide addition may be checked by a comparative analysis of the first wash performance of a modified enzyme to which the peptide addition has been applied and of the corresponding parent enzyme, respectively. The wash performance may, e.g., be determined by the one cycle wash assay described in the Materials and Methods section.

It is presently contemplated that the improved first wash performance effected by the peptide addition is, at least in part, due to an increased affinity of the modified lipolytic enzyme towards its lipid substrate (although this may not be the only reason). Accordingly, in a preferred embodiment the peptide addition is one which confer an increased affinity of the modified enzyme towards its lipid substrate.

The term "mature enzyme" is used in its conventional meaning, i.e. to indicate the active form of the enzyme resulting after expression and posttranslational processing (to remove pro and/or pre-sequences) by the producer organism in question. When the enzyme is a secreted enzyme, the mature enzyme will normally be the form of the enzyme resulting after secretion. More specifically this means that the pre- and pro-peptide sequences, if present, have been removed from the initially translated enzyme, *i. e.* the unprocessed enzyme

The term "non-structural part" is intended to indicate the part of the N- and C-terminal end, respectively, which is outside the first or last, respectively, structural element, such as an α-helix or β-sheet structure, of the folded mature enzyme. The non-structural part may easily be identified in a three-dimensional structure or model of the enzyme in question. Typically, the non-structural part comprises the first or the last about 1-20 amino acid residues of the amino acid sequence constituting the enzyme. For enzymes having a similar three-dimensional structure to that of the *H. lanuginosa* lipolytic enzyme the insertion may be made in the part of said enzyme which corresponds to a "non-structural part" of the *H. lanuginosa* lipolytic enzyme.

The non-structural part of the *H. lanuginosa* lipolytic enzyme normally comprises the first or the last about 1-20 amino acid residues of the mature enzyme.

It is presently believed that the capability of the peptide addition of providing the desired first wash effect depends on, e.g., the identity of the parent enzyme to be modified, the structure (including length) of the peptide addition, the impact of the peptide addition on the structure of the entire lipolytic enzyme, the nature or functionality of amino acid residues of the peptide addition, etc. A prerequisite for the peptide addition being capable of providing the desired effect is, of course, that the modified enzyme containing the peptide addition is expressible in a suitable host organism. The following general considerations may be of relevance for the design of a suitable peptide addition:

Length of peptide addition: It has been found that peptide additions containing varying numbers of amino acid residues are capable of providing the desired first wash effect and thus, it is not possible to specify an exact number of amino acid residues to be present in the peptide addition to be used in accordance with the present invention. It is contemplated that the upper limit of the number of amino acid residues is determined, inter alia, on the impact of the peptide addition on the expression, the structure and/or the activity of the resulting modified enzyme. It is believed that the peptide addition may comprise a substantial number of amino acid residues, however, without all of these amino acid residues need to contribute to the desired first wash effect (even if the peptide addition contains a substantial number of amino acid residues only a small number of these need to provide the desired function, this small number may be termed the functional part of the peptide addition). The main consideration in relation to the lower limit of the number of amino acid residues of the peptide addition will normally be that the number should be sufficient to provide the desired first wash effect.

The peptide addition may thus comprise a single amino acid residue or an amino acid chain of from 2 and 500 amino acids, such as from 1 to 200, or from 2 to 100, preferably from 2 to 50, such as 3 to 50, even more preferably from 7-45 and still more preferably between 1 and 15, such as between 1 and 10 or 1 and 7, especially between 4 and 10, such as 4 and 7 amino acids.

Stability: The peptide addition should preferably be chosen so as to provide a modified lipolytic enzyme with a stable peptide addition and an acceptable structural stability of the parent enzyme. For instance, a peptide addition which in itself forms a structural element, such as an $\alpha$-helix or a $\beta$-sheet, may stabilize the resulting modified enzyme and thus be used in the context of the present invention. Peptide sequences capable of forming such structures are known in the art. Alternatively, an improved structural stability may be provided by introduction of cystein bridges in the modified lipolytic enzyme of the invention. For instance, a cystein bridge between the peptide addition and the mature part of the enzyme may be established if at least one of the amino acid residues of the peptide addition is a cystein residue which is located so as to be able to form a covalent binding to a cystein residue in the mature part of the enzyme. If no suitable cystein is present in the mature enzyme, a cystein may be inserted at a suitable location of said parent enzyme, conveniently by replacing an amino acid of the parent enzyme, which is considered unimportant for the activity.

In addition, it may be desirable that at least one of the amino acid residues of the peptide addition is chosen so as to make the peptide addition less susceptibility to proteolytic degradation by proteolytic enzymes of the host cell used for expressing the modified lipolytic enzyme. For instance, the peptide addition may comprise at least one, and preferably at least two proline residues. Preferably, the peptide addition comprises 1-5, such as 1-4 or 1-3 or two or one proline residues. The proline residue(s) is(are) preferably placed at the proteolytic cleavage site or close thereto. Alternatively, the peptide addition may be one which provides a protease stable loop to the modified lipase, e.g. as described in EP 407 225 or WO 93/11254.

Nature of amino acid residues of the peptide addition: As stated above and without being limited to any theory, it is presently believed that the improved performance may at least partly be due to an increased affinity of the modified lipolytic enzyme toward the substrate provided by the peptide addition. In particular, it is believed that favourable electrostatic interactions may be obtained between the negatively charged lipid surface and positively charged and/or hydrophobic amino acid residues present in the modified enzyme. Accordingly, it is particularly preferred that the modified enzyme of the invention comprises a peptide addition with at least one positive charge, such as at least 2, 3, 4 or more positive charges or expressed differently, in which a substantial number of the amino acid residues of the peptide addition is positively charged and/or hydrophobic.

Analogously, and in order to reduce the negative charge in a non-structural end of the parent enzyme it is preferred to remove at least one such as two or more negatively charged amino acid residues from a non-structural N-terminal or C-terminal part of the parent enzyme of choice, in particular from the part of the parent lipase being constructed of the 1-5 first or last N-terminal or C-terminal amino acid residues, such as 1-4, or 1-3 or 1-2. The negatively charged amino acid residue may either be removed or replaced by a neutral, a positively charged or a hydrophobic amino acid residue. For instance, the negatively charged amino acid residue to be removed may be an E or D which may be replaced with either of the positively charged amino acid residues R, K or H, the neutral amino acid residues S, T, G or Q, or the hydrophobic amino acid residues A, I, W F or L. Similarly, a neutral amino acid residue of a non-structural N-terminal or C-terminal part of the parent enzyme may be replaced with a positively charged or hydrophobic amino acid residue as defined above.

Accordingly, the modified lipolytic enzyme of the invention in addition or as an alternative to a N-terminal and/or C-terminal extension may comprise a mutation in the non-structural C-terminal and/or N-terminal end of the parent enzyme, which mutation has involved deleting or replacing a negatively charged amino acid residue of said non-structural part with a positively charged or neutral amino acid residue or with a hydrophobic amino acid residue.

If a peptide addition is present in both the N- and the C-terminal of the parent enzyme, the peptide addition at or within each of the terminals may have the same or a different amino acid sequence.

Test of suitability of peptide addition: the effect of using a given peptide addition, e.g., designed on the basis of the above principles may be tested by constructing a modified lipolytic enzyme containing the peptide addition and testing the properties of the resulting enzyme for first wash activity.

The peptide addition can be generalised in the following way.

The first residue (counted from the outer residue) is named "a", the second is named "b", the third "c" etc. Thus, in case of an N-terminal addition the first amino acid residue is termed "a", in case of a C-terminal addition the last amino acid residue is termed "a".

In an important embodiment of the invention the peptide addition consists of from 1 to 7 amino acids. Such peptide addition, which can be applied to both the N- and/or C-terminal of the parent enzyme, can be referred to as:

a (one amino acid peptide addition)
a-b (two amino acids peptide addition)
a-b-c (three amino acids peptide addition)
a-b-c-d (four amino acids peptide addition)
a-b-c-d-e (five amino acids peptide addition)
a-b-c-d-e-f (six amino acids peptide addition)
a-b-c-d-e-f-g (seven amino acids peptide addition)

Each letter defines an amino acid residue.

a, b, c, d, e, f and g may independently be any amino acid including Alanine (A), Valine (V), Leucine (L), Isoleucine (I), Proline (P), Phenylalanine (F), Tryptophan (W), Methionine (M), Glycine (G), Serine (S), Threonine (T), Cysteine (C), Tyrosine (Y), Asparagine (N), Glutamine (Q), Aspartic acid (D), Glutamic acid (E), Lysine (K), Arginine (R), and Histidine (H).

In specific embodiments a, b, c, d, e, f, and g are independently one of the following amino acids:

a: Leu, Ile, Val, Trp, Phe, Ser, Arg, Cys, or Lys,
b: Leu, Ile, Val, Trp, Phe Ser, Pro, Arg, Lys, Cys or His,
c: Leu, Ile, Val, Trp, Phe, Ser, Pro, Arg, Cys, or Lys.
d: Leu, Ile, Val, Trp, Phe Ser, Pro, Arg, Cys, or Lys.
e: Leu, Ile, Val, Trp, Phe, Pro, Arg, Lys, Ala, Glu, Cys, or Asp,
f: Leu, Ile, Val, Trp, Phe, Pro, Arg, Lys, Ala, Glu, Cys, or Asp,
g: Leu, Ile, Val, Trp, Phe, Pro, Arg, Lys, Cys, or Met.

In a preferred embodiment at least one such as one, two, three or four of a, b, c, d, e, f, or g is a positively charged amino acid, *i.e.* Arg (R) or Lys (K) or a hydrophobic amino acid, *i.e.* Leu, Ile, Val, Trp or Phe.

As stated further above, and dependent on the host cell of choice it is generally believed that it is important that the peptide addition comprises at least one proline residue in order to protect the modified lipolytic enzyme against proteolytic degradation during the processing of the enzyme by the host cell of choice. It may be desirable that the proline residue occupies position two (i.e. b) and/or three (i.e. c) of the peptide addition or a position close to the desired cleavage point (i.e. the point where processing by the host cell in question is believed to occur). Accordingly, in one embodiment b and optionally c of the peptide addition is Pro

In another embodiment of the invention a-b is SP (Ser-Pro), A-P or Q-P. If the peptide addition contains more amino acid residues, e.g. between 4 and 7 amino acids the peptide addition has the general formula SPcd, SPcde, SPcdef, SPcdefg or APcd, APcde, APcdef, Apcdefg or QPcd, QPcde, QPcdef, QPcdefg. In each of these formulae c, d, e , f, and g may be any amino acid. However, preferred are the above mentioned group of amino acids.

In another embodiment a-b comprise at least one positive amino acids (*i.e.* Arg and Lys) or hydrophobic amino acid residue *(i.e.* Leu, Ile, Val, Trp and Phe).

Specifically, the peptide addition applied to the parent lipolytic enzyme may advantageously be one of the following amino acid residues or peptides:

Arg (R), or Lys (K), or Leu (L), or Ile (I), or
Val (V), or Trp (W) or Phe (F), or
Arg-Pro (RP), or

Lys-Lys (KK), or
Arg-Lys (RK), or
Lys-Arg (KR), or
Arg-Arg (RR), or
Arg-Arg-Pro (RRP), or
Arg-Pro-Val-Ser-Gln-Asp (RPVSQD)
Ser-Pro-Ile-Arg-Met (SPIRM), or
Ser-Pro-Ile-Arg-Ala-Arg (SPIRAR), or
Ser-Pro-Ile-Arg-Pro-Arg (SPIRPR) or
Ser-Pro-Ile-Arg-Glu-Arg (SPIRER), or
Ser-Pro-Ile-Arg-Lys (SPIRK), or
Ser-Pro-Ite-Lys-Lys (SPIKK), or
Ser-Pro-Ile-Arg-Arg-Pro (SPIRRP), or
Ser-Pro-Pro-Arg-Arg (SPPRR), or
Ser-Pro-Iso-Pro-Arg (SPIPR), or
Ser-Pro-Arg-Pro-Arg (SPRPR), or
Ser-Pro-Ile-Arg (SPIR), or
Ser-Pro-Ile-Arg-Arg (SPIRR), or
Ser-Cys-ile-Arg-Arg, (SCIRR), or
Ser-Pro-Ile-Arg-Pro-Arg-Pro (SPIRPRP), or
Ser-Cys-Ile-Arg-Pro-Arg-Pro (SCPIRPRP), or
Ser-Pro-Arg-Arg-Pro-Arg-Thr (SPRRPRT), or
Ser-Pro-Phe-Arg-Pro-Lys-Leu (SPFRPKL), or
Ser-Pro-Pro-Arg-Arg-Pro (SPPRRP), or
Ser-Pro-Ile-Arg-Arg-Glu (SPIRRE), or
Ser-Pro-Pro-Arg-Pro-Pro (SPPRPP), or
Ser-Pro-Pro-Arg-Pro-Arg (SPPRPR), or
Ser-Pro-Pro-Trp-Trp-Pro (SPPWWP), or
Ser-Pro-Pro-Trp-Arg-Pro (SPPWRP), or
Ser-Pro-Pro-Arg-Trp-Pro (SPPRWP), or
Ser-Pro-Pro-Arg-Trp-Pro (SPPRWP), or
Ser-His-Trp-Arg-Arg-Trp (SHWRRW), or
Ser-His-Trp-Arg-Lys (SHWRK), or
Ser-His-Trp-Arg-Arg (SHWRR), or
Thr-Ala-Ile-Arg-Pro-Arg-Lys (TAIRPRK),
Ser-Thr-Arg-Arg-Pro-Arg-Pro (STRRPRP),
Gly-Pro-Ile-Arg-Pro-Arg-Pro (GPIRPRP), or
Leu-Pro-Phe-Arg-Gln-Arg-Pro (LPFRQRP), or
Ser-Arg-Ser-Arg-His-Asn-Ala (SRSRHNA), or
Ile-Pro-Ile-Arg-Pro-Arg-Arg (IPIRPRR), or
Ser-Thr-Arg-Arg-Pro-Arg-Pro (STRRPRP), or
Thr-Ala-Ile-Arg-Pro-Arg-Lys (TAIRPRK), or
Trp-Arg-Trp-Arg-Trp-Arg (WRWRWR), or
Gln-Pro-Ile-Arg-Arg (QPIRR), or
Ser-His-Trp-Gln-Gln (SHWQQ), or
Ser-Ala-Leu-Arg-Pro-Arg-Lys (SALRPRK).

Also contemplated according to the invention is additions comprising more than 7 amino acids, such as from 8 to 15 amino acids.

Such peptides can be generalised as:

a-b-c-d-e-f-g-h (8 amino acid peptide)
a-b-c-d-e-f-g-h-i (9 amino acid peptide)
a-b-c-d-e-f-g-h-i-j (10 amino acid peptide)
a-b-c-d-e-f-g-h-i-j-k (11 amino acid peptide)
a-b-c-d-e-f-g-h-i-j-k-l (12 amino acid peptide)
a-b-c-d-e-f-g-h-i-j-k-l-m-(13 amino acid peptide)
a-b-c-d-e-f-g-h-i-j-k-l-m-n(14 amino acid peptide)

a-b-c-d-e-f-g-h-i-j-k-l-m-n-o (15 amino acid peptide).

a to o may be any of the twenty amino acids mentioned above.

The a-g stretch may be as defined above in relation to a peptide addition comprising 1 to 7 amino acid residues.

h, i, j, k, l, m, n, o may as mentioned above be any amino acid, preferably any of the following amino acids: Arg, Lys, Ala, Val, Trp, Ile, Phe, Ser or Pro.

Specific examples of such additions are listed below:

Arg-Pro-Arg-Pro-Arg-Pro-Arg-Pro (RPRPRPRP), or
Ser-Ser-Thr-Arg-Arg-Ala-Ser-Pro-Ile-Lys-Lys (SSTRRASPIKK), or
Ala-Trp-Trp-Pro-Ser-Pro-Ile-Arg-Pro-Arg-Pro (AWWPSPIRPRP), or
Ala-Pro-Pro-Pro-Arg-Pro-Arg-Pro-Arg-Pro-Arg-Pro (APPPRPRPRPRP), or
Ala-Pro-Pro-Pro-Arg-Thr-Arg-Pro-Arg-Pro-Arg-Ser (APPPRTRPRPRS), or
Ser-Pro-Lys-Arg-Lys-Pro-Arg-Pro (SPKRKPRP), or
Ser-Gln-Arg-Ile-Lys-Gln-Arg-Ile-Lys (SQRIKQRIK), or
Ser-Pro-Pro-Pro-Arg-Pro-Arg-Pro (SPPPRPRP), or
Ser-Pro-Ile-Arg-Pro-Arg-Pro-Arg-Pro-Arg SPIRPRPRPR, or
Ser-Pro-Ile-Arg-Lys-Ala-Trp-Trp-Pro (SPIRKAWWP), or
Ala-Pro-Pro-Pro-Lys-Ala-Ser-Pro-Arg-Gln-Arg-Pro (APPPKASPRQRP), or
Ser-Pro-Ile-Arg-Pro-Arg-Pro-Ser-Pro-Ile-Arg-Pro-Arg-Pro-Arg (SPIRPRPSPIRPRP), or
Ser-Pro-Pro-Arg-Trp-Pro-Arg-Arg (SPPRWPRR), or
Ser-Pro-Pro-Arg-Trp-Pro-Arg-Trp (SPPRWPRW), or
Ser-Pro-Pro-Arg-Trp-Pro-Trp-Arg (SPPRWPWR), or
Ser-Pro-Pro-Trp-Arg-Pro-Arg-Arg (SPPWRPRR), or
Ser-Pro-Pro-Trp-Trp-Pro-Arg-Trp (SPPWWPRW, or
Ser-Pro-Pro-Trp-Trp-Pro-Trp-Arg (SPPWWPWR), or
Ser-Pro-Pro-Trp-Trp-Pro-Trp-Trp (SPPWWPWW), or
Ser-Pro-Pro-Trp-Pro-Arg-pro-Arg-Pro (SPPWPRPRP), or
Ala-Pro-Pro-Pro-Arg-Pro-Arg-Leu-Leu-Pro-Ile-Ser (APPPRPRLLPIS), or
Ala-Pro-Pro-Pro-Thr-Arg-Gln-Arg-Gln-Ser-Pro (APPPTRQRQSP), or
Ala-Pro-Pro-Pro-Arg-Thr-Ile-Pro-Arg-Ser-Ser-Pro (APPPRTIPRSSP).

In any of the above specified peptide additions (whether comprising 1 to 7 or 1 to 15 amino acid residues) in which the position "a" is a Ser, Ala, Arg, Lys or Pro, the Ser may be replaced with an Ala, Arg, Lys or Pro, the Ala with a Ser, Arg, Lys or Pro and the Arg, Lys or Pro with a Ala or Ser.

It is to be emphasised that the above peptide addition may be at either the N-terminal and/or the C-terminal. Examples of modified lipolytic enzymes with both a N- and a C-terminal peptide addition include all combinations of the peptide additions specifically mentioned above. Two specific examples of such are the N-terminal addition SPIRPRP together with the C-terminal addition RRP or RR.

In addition to the above specified peptide additions it has been found that a suitable peptide addition may simply be constituted by or comprise a part of or the entire propeptide sequence normally associated with the parent lipolytic enzyme in question. Thus, for instance in relation to first wash *H. lanuginosa* lipolytic enzyme variants a suitable peptide addition may comprise or be constituted of SPIRR - i.e. part of the normal propeptide sequence of the *H. lanuginosa* lipolytic enzyme sequence.

If the peptide addition is inserted into the non-structural part of the parent enzyme, it may replace one or more of the amino acid residues of said non-structural part. For instance, the peptide addition may replace one or more amino acid residues occupying the first, *e.g.* 1-5, amino acid residues of the N-terminal end and/or the last, *e.g.* 1-5, amino acids of the enzyme (*i.e.* the 1-5 amino acid residues of the C-terminal end). For instance, the peptide addition may replace amino acid residue(s) 1 and/or 2 and/or 3 and/or 4, and/or 5, etc. from either end of the parent enzyme. When the parent enzyme is a variant of the *H. lanuginosa* lipase which comprises amino acid modifications in its structural, mature part it has been of particular interest to combine any of the above peptide additions (applied in the N-terminal) with a deletion of the first amino acid residue of the mature parent enzyme, *i.e.* the 1E.

*Methods of applying a peptide addition to a parent lipolytic enzyme*

Although a first wash lipolytic enzyme of the invention may be obtained by adding (fusing or inserting) a synthetically produced peptide addition into the parent lipolytic enzyme in question, it is presently preferred that the modified enzyme of the invention is prepared by i) modifying the nucleotide, preferably DNA, sequence encoding the

parent enzyme so as to encode the desired peptide addition applied to the N- and/or the C-terminal end(s) of the parent enzyme (*e.g.* by inserting a nucleic acid (preferably DNA) sequence encoding the peptide addition at the relevant location in the nucleic acid (preferably DNA) sequence encoding the parent enzyme), ii) expressing the resulting modified nucleic acid (preferably DNA) sequence in a suitable expression system, and iii) recovering the resulting modified enzyme.

In the present context, the term "applied to" is intended to indicate that the addition is fused to the N- and/or C-terminal end (*e.g.* to the first or last amino acid residue) of the mature enzyme or inserted into a non-structural part of the N-terminal and/or C-terminal end of the mature enzyme.

Many enzymes are expressed as "prepro-enzymes", i.e. as enzymes consisting of the mature enzyme, a secretory signal peptide (*i.e.* prepeptide) and a pro-peptide. The prepro-enzyme is processed intracellularly to be secreted into the fermentation medium, from which the mature enzyme can be isolated and/or purified. The peptide addition to the parent enzyme can be carried out by applying nucleic acid sequences encoding the desirable peptide additions upstream (for N-terminal peptide additions) and/or downstream (for C-terminal peptide additions) to the DNA sequence encoding the parent enzyme.

The insertion should be performed in such a way that the desired modified enzyme (i.e. having the desired peptide addition(s)) is expressed and secreted by the host cell after transcription, translation, and processing of the enzyme. The term "processing" means in this context removal of pre- and pro-peptides (except, of course, when the pro-peptide is identical to the desired peptide addition. This will be dealt with further below).

Downstream sequences (encoding a C-terminal addition) can be inserted between the DNA sequence encoding the parent enzyme and the terminating codon. However, if the unprocessed DNA sequence comprises a propeptide encoding DNA sequence at the C-terminal end the insertion/addition of the DNA sequence encoding the peptide addition can also take place between the DNA sequences encoding the pro-peptide and the mature enzyme, respectively.

In most cases it is possible to extend the parent enzyme upstream by inserting a DNA sequence encoding the peptide addition between the DNA sequence encoding the pro-peptide or the prepeptide (if no prosequence is present) and the DNA sequence encoding the mature enzyme.

The DNA sequence encoding the peptide addtion in question shall, of course, be chosen so as to match the codon preferences of the expression system intended for the production of the first wash lipolytic enzyme of the invention.

The insertion/addition of a DNA sequence encoding the peptide addition can be carried out by any standard techniques known by any skilled person in the field of molecular biology, cf., *e.g.* Sambrook et al., 1989). This include, *e.g.,* the polymerase chain reaction (PCR) using specific primers, for instance described in US patent 4,683,202 or R. K. Saiki et al., (1988), Science, 239, 487-491. How to provide for the expression and secretion of adjacent DNA sequence(s) will be described below.

In connection with the present invention it has been found that some host cells may be less suited for the production of a first wash lipolytic enzyme comprising a peptide addtion, in that part or all of the peptide addition(s) may be cut off during the posttranslational or other processesing performed by the host cell. Accordingly, the term "suitable expression system" is intended to indicate an expression system (host cell and optionally expression vector) which allows for at least a portion of the intact desired first wash lipolytic enzyme comprising the peptide addition to be produced, *i.e.* an expression system which does not, *e.g.* as part of the posttranslational or other processing by the host cell of choice, remove part or all of the peptide addition (and thereby produce the enzyme without the desired peptide addition). Expressed differently, the expression system (including the host cell, cultivation conditions and/or recovery conditions) are preferably selected so that at the most a partial processing of the pre, pro or prepro-form of the lipolytic enzyme occur resulting in that at least 5%, such as at least 10%, such as at least 15 %, such as at least 20 %, such as at least 25 %, such as at least 50 %, such as at least 75 % of the produced enzyme molecules comprise the desired peptide addition, e.g. the entire pro-sequence or a substantial part thereof. Typically, the expression system to be used is devoid of or reduced in one or more proteolytic activities exerting the undesired posttranslational processing.The choice of expression system and thus host cell will depend on the lipolytic enzyme to be produced as will be discussed in detail further below.

While care must be exerted to select a proper expression system for producing a first wash lipolytic enzyme of the invention which comprises a peptide addition at its N- and/or C-terminus (in particular when a modified DNA sequence is used for the production), it has been found that when the peptide addition is the propeptide or part thereof normally associated with the parent lipolytic enzyme in question, the peptide addition (thus constituting a part of or the entire propeptide sequence) may be applied by expressing a DNA sequence encoding the parent lipolytic enzyme in question in an expression system which is incapable of processing the translated polypeptide in the normal manner, and thereby results in the production of an enzyme which comprises a part of or the entire propeptide or a similar peptide sequence associated with the mature protein prior to its processing. In this case, the propeptide or similar peptide sequence constitutes the peptide addition. The pro-peptide or similar peptide sequence may be heterologous

or homologous to the parent enzyme and can be present in both the N- and C-terminal of the parent enzyme.

Accordingly, if a suitable stretch of amino acids is already encoded in the prepro form of the parent enzyme and this stretch of amino acids is cut off in the processing of the enzyme by a given expression system, the peptide addition can be applied by changing the expression host system to a system in which said processing of said stretch of amino acids does not occur or modify the gene sequence to eliminate the post-translation processing, e.g. by saturating the processing enzyme(s) with one or more copies of a pro-like peptide (such as one of the peptide additions shown herein) or by changing the pro-peptide sequence, e.g. to remove a posttranslational processing site. In such a case the secretory signal pre-peptide will be cut off during or after the secretion, resulting in a modified enzyme consisting of the parent enzyme comprising the pro-peptide or part thereof or a similar peptide sequence encoded by the corresponding DNA sequence, *i.e.* a lipolytic enzyme being extended at either its N-terminal or C-terminal end.

In other words, the first wash lipolytic enzyme of the invention which comprises a peptide addtion may be designed and/or produced by a method comprising:
cultivating a host cell transformed with a DNA sequence encoding the parent lipolytic enzyme including its (pre)pro sequence under conditions suitable for production of the enzyme comprising at least a part of the entire pro(pre)-sequence, the host cell being one which is incapable or inefficient in the processing of the pro-enzyme to be expressed into the mature enzyme, and recovering and optionally purifying the resulting modified enzyme.
The DNA sequence encoding the parent lipolytic enzyme may be present on an expression vector, when transformed into the host cell.

The host cell may be of a different origin than the parent enzyme, e.g. of another genus than the one from which the parent enzyme is derived, or may have another posttranslational processing machinery than the source of the parent enzyme. Yeast cells have been found of particular use for applying peptide additions (in the form of the propeptide or a part thereof) to parent filamentous fungal lipolytic enzymes, in particular to *H. lanuginosa* lipolytic enzyme variants, due to the different processesing system of the yeast cells as compared to the filamentous fungal cells. Examples of suitable yeast cells for said purpose are cells derived from a strain of *Saccharomyces* sp., in particular *Saccharomyces cerevisiae*, or a strain of *Han-senula* sp..

In an alternative and highly preferred embodiment the first wash lipolytic enzyme of the invention comprising a peptide addition has been designed and/or produced by a method comprising the following steps:

a) subjecting a DNA sequence encoding the parent lipolytic enzyme with a peptide addition to localized random mutagenesis in the peptide addition or in a non-structural part of the C-terminal or N-terminal end of the parent enzyme,
b) expressing the mutated DNA sequence obtained in step a) in a host cell,
c) screening for host cells expressing a mutated lipolytic enzyme which has an improved performance as compared to the parent lipolytic enzyme,
d) selecting a mutated lipolytic enzyme among those resulting from step c) which, when present in detergent composition A and/or B with 12500 LU/l detergent, is capable of removing at least 15 % more lard from a lard stained swatch, than the same detergent composition without the enzyme, in a one cycle wash assay as disclosed herein.

By this approach a number of highly advantageous first wash lipolytic enzymes with different peptide additions have been created. The peptide addition present on the DNA sequence to be mutagenized may be constituted by or comprise the prosequence or a part thereof normally associated with the parent lipolytic enzyme or may be any other peptide addition, e.g. one of the peptide additions exemplified above. Each of steps a)-d) may be carried out as described in the sections further below entitled "Random mutagenesis" and "Localized Random Mutagenesis".

**The parent lipolytic enzyme**

The parent lipolytic enzyme to be modified in accordance with the invention may be of any origin. Thus, the enzyme may be of mammalian, plant, vertebrate or any other origin. However, it is presently preferred that the enzyme is of microbial origin in that a number of microbial strains have been found to produce enzymes of particular use for detergent purposes.

More specifically, the parent lipolytic enzyme may be derived from a fungus, i.e. a yeast or a filamentous fungus. For instance, the enzyme may be derived from a filamentous fungus of the class of *Plectomycetes,* preferably the order of *Eurotiales* and more preferably the family like *Eremascaceae, Monoascaceae, Pseudoeurotiaceae* and *Trichocomaceae*, the latter containing genera like *Emericella, Aspergillus, Penicillium, Eupenicillium, Paecilomyces, Talaromyces, Thermoascus* and *Sclerocleista*. More specifically, the parent enzyme may be one which is derivable from a strain of a *Humicola* sp., e.g. *H. brevispora, H. lanuginosa, H. brevis var. thermoidea* and *H. insolens* (US 4,810,414), a strain of a *Rhizomucor* sp., e.g. *Rh. miehei* (EP 238023), a strain of a *Rhizopus* sp., e.g. *R. delemar*

(Hass et al., (1991), Gene 109,107-113), *R. niveus* (Kugimiya et al., (1992) Biosci.Biotech. Biochem 56, 716-719) or *R. oryzae*, a strain of a *Candida* sp., e.g. *C. cylindracea* (also called *C. rugosa*) or *C. antarctica* (WO 88/02775) or *C. antarctica* lipase A or B (EP 214 761), a strain of a *Fusarium* sp., e.g. *F. oxysporum* (EP 130,064) or *F. solani pisi* (WO 90/09446) or variants thereof (WO 94/14964), *F. solani pisi* (GB 2 296 011) a strain of a *Venturia* spp., e.g. *V. inaequalis*, a strain of a *Collerotrichum* spp., e.g. *C. gloeosporioides*, or *C. lagenarium*, a strain of *Geotricum, e.g. G. candidum* (Schimada et al., (1989), J.Biochem., 106, 383-388), a strain of *Aspergillus,* e.g. *A. niger,* or an *Aspergillus* sp. lipolytic enzyme variant (EP 167,309), or a strain of a *Penicillium* spp., e.g. *P. spinulosum* or *P. camembertii* (Yamaguchi et al., (1991), Gene 103, 61-67).

In the present context, "derivable from" is intended not only to indicate an enzyme produced by a strain of the organism in question, but also an enzyme encoded by a DNA sequence isolated from such strain and produced in a host organism transformed with said DNA sequence. Furthermore, the term is intended to indicate an enzyme which is encoded by a DNA sequence of synthetic and/or cDNA origin and which has the identifying characteristics of the enzyme in question. Finally, the term is intended to embrace variants of the enzyme, *e.g.* carrying one or more mutations as compared to the naturally occurring enzyme, or homologous enzymes which may be naturally-occurring enzymes produced by other strains or organisms, which, e.g. may be isolated by hybridization to oligonucleotide probes prepared on the basis of the amino acid or DNA sequence of any of the above enzymes (the hybridization conditions involving presoaking in 5xSSC and prehybridizing for 1 h at ~40°C in a solution of 20% formamide, 5xDenhardt's solution, 50 mM sodium phosphate, pH 6.8, and 50 g of denatured sonicated calf thymus DNA, followed by hybridization in the same solution supplemented with 100 M ATP for 18h at ~40°C, or other methods described by Sambrook et al., 1989) or which is immunologically cross-reactive with said enzymes (e.g. as determined by the method of Hudson et al., 1989).

Of particular interest as a parent lipolytic enzyme is one derivable from a strain of *H. lanuginosa*, *e.g*. the *H. lanuginosa* strain DSM 4109, e.g. the mature form of the enzyme described in EP 305 216 or a variant thereof as described in WO 92/05249, WO 94/01541, WO 94/14951, WO 94/25577, PCT/DK94/00079 (all from Novo Nordisk A/S), which are hereby incorporated by reference.

Throughout the present application the name *Humicola lanuginosa* has been used to identify one preferred parent enzyme, *i.e.* the one mentioned immediately above. However, in recent years *H. lanuginosa* has also been termed *Thermomyces lanuginosus* (a species introduced the first time by Tsiklinsky in 1989) since the fungus show morphological and physiological similarity to *Thermomyces lanuginosus*. Accordingly, it will be understood that whenever reference is made to *H. lanuginosa* this term could be replaced by *Thermomyces lanuginosus.* The DNA encoding part of the 18S ribosomal gene from *Thermomyces lanuginosus* (or *H. lanuginosa)* have been sequenced. The resulting 18S sequence was compared to other 18S sequences in the GenBank database and a phylogenetic analysis using parsimony (PAUP, Version3.1.1, Smithsonian Institution, 1993) have also been made. This clearly assigns *Thermomyces lanuginosus* to the class of *Plec tomycetes,* probably to the order of *Eurotiales*. According to the Entrez Browser at the NCBI (National Center for Biotechnology Information), this relates *Thermomyces lanuginosus* to families like *Eremascaceae, Monoascaceae*, *Pseudoeurotiaceae* and *Trichocomaceae,* the latter containing genera like *Emericella, Aspergillus, Penicillium, Eupenicillium, Paecilomyces, Talaromyces, Thermoascus* and *Sclerocleista.*

The parent lipolytic enzyme to be modified in accordance with the present invention may be derivable from a bacterium. For instance, the DNA sequence encoding the parent lipolytic enzyme may be derivable from a strain of *Pseudomonas* spp., such as *Ps. cepacia, Ps. alcaligenes, Ps. pseudoalcaligens, Ps. mendocina* (also termed *Ps. putida*), *Ps. syringae, Ps. aeroginosa, Ps. wisconsinensis* (WO 96/12012) or *Ps. fragi*, a strain of *Bacillus* spp., e.g. *B. subtilis* or *B. pumilus* or a strain of *Streptomyces* sp., e.g. *S. scabies.*

In connection with the *Pseudomonas sp.* lipases it has been found that lipases from the following organisms have a high degree of homology, such as at least 60 % homology, at least 80% homology or at least 90% homology, and thus are contemplated to belong to the same family of lipases: Ps. ATCC21808, *Pseudomonas* sp. lipase commercially available as Liposam®, *Ps. aeruginosa* EF2, *Ps. aeruginosa* PAC1R, *Ps. aeruginosa* PAO1, *Ps. aeruginosa* TE3285, *Ps. sp*. 109, *Ps. pseudoalcaligenes* M1, *Ps. glumae, Ps. cepacia* DSM3959, *Ps. cepacia* M-12-33, *Ps. sp*. KWI-56, *Ps. putida* IFO3458, *Ps. putida* IFO12049 (Gilbert, E. J., (1993), Pseudomonas lipases: Biochemical properties and molecular cloning. Enzyme Microb. Technol., 15, 634-645). The species *Pseudomonas cepacia* has recently been reclassified as *Burkholderia cepacia,* but is termed *Ps. cepacia* in the present application.

Specific examples hereof include a *Pseudomonas* lipolytic enzyme, e.g. *Ps. fragi, Ps. stutzeri, Ps. cepacia* and *Ps. fluorescens* (WO 89/04361), or *Ps. plantarii* or *Ps. gladioli* (US 4,950,417) or *Ps. alcaligenes* and *Ps. pseudoalcaligenes* (EP 218 272, EP 331 376, or WO 94/25578 (disclosing variants of the *Ps. pseudoalcaligenes* lipolytic enzyme with the mutation M21S, M21L or M21A), the *Pseudomonas* sp. variants disclosed in EP 407 225, or a *Pseudomonas* sp. lipolytic enzyme, such as the *Ps. mendocina lipolytic* enzyme described in WO 88/09367 and US 5,389,536 or variants thereof as described in US 5,352,594.

Other specific examples include a *Bacillus* lipolytic enzyme, such as the lipolytic enzyme from *B. subtilis* (Dartois et al., (1993) Biochemica et Biophysica acta 1131, 253-260) or *B. stearothermophilus* (JP 64/7744992) or *B.*

*pumilus* (WO 91/16422) and a *Chromobacterium* lipolytic enzyme (especially one derivable from *C. viscosum*).

Specific examples of readily available commercial lipolytic enzyme which may serve as parent lipolytic enzymes according to the invention include Lipolase®, Lipolase® Ultra (available from Novo Nordisk A/S).

Examples of other lipolytic enzymes specifically contemplated to be modifiable according to the invention are Lumafast®, i.e. a *Ps. mendocina* lipolytic enzyme and Lipomax®, i.e. *a Ps. alcaligenes* lipolytic enzyme, a *Fusarium solani* lipase (cutinase) from Unilever, a *Bacillus* sp. lipase from Solvay enzymes (US 5427936, EP 528828); and Liposam®, (a *Ps. mendocina* lipase from Showa Denko) and further the *Pseudomonas* sp. lipase described in WO 95/06720 which have been sequenced and found to have the amino acid sequence shown in SEQ ID NO 99.

It is to be emphasized that the parent lipolytic enzyme to be modified according to the invention may be any of the above mentioned lipolytic enzymes and any variant, modification, or truncation thereof. Examples of such parent enzymes which are specifically contemplated include the enzymes described in WO92/05249, WO 94/01541, WO 94/14951, WO 94/25577, WO 95/22615 and a protein engineered lipase variants as described in EP 407 225; a protein engineered *Ps. mendocina* lipase as described in US 5,352,594; a cutinase variant as described in WO 94/14964; a variant of an *Aspergillus* lipolytic enzyme as described in EP patent 167,309; and *Pseudomonas sp.* lipase described in WO 95/06720.

**Specific first wash H. lanuginosa lipolytic enzyme variants**

For ease of reference specific variants of the invention are described by use of the following nomenclature: Original amino acid(s):position(s):substituted amino acid(s)

According to this nomenclature, for instance the replacement of aspartic acid by valine in position 96 is shown as:

Asp 96 Val  or  D96V

a deletion of aspartic acid in the same position is shown as:

Asp 96 *  or  D96*

and insertion of an additional amino acid residue such as lysine is shown as:

Asp 96 ValLys  or  D96VK

Multiple mutations are separated by pluses, i.e.:

Asp 96 Val + Glu 87 Lys  or  D96V+E87K

representing mutations in positions 96 and 87 replacing aspartic acid and glutamic acid by valine and lysine, respectively.

When one or more alternative amino acid residues may be inserted in a given position it is indicated as
D96V,N or
D96V or D96N

Furthermore, when a position suitable for modification is identified herein without any specific modification being suggested, it is to be understood that any amino acid residue may be substituted for the amino acid residue present in the position. Thus, for instance, when a modification of an aspartic acid in position 96 is mentioned, but not specified, it is to be understood that the aspartic acid may be deleted or replaced by any other amino acid, i.e. any one of R,N,A,C,Q,E,G,H,I,L,K,M,F,P,S,T,W,Y,V, or a further amino acid residue inserted at that position.

Finally, when a mutation of the parent *H. lanuginosa* lipolytic enzyme is identified herein, it is intended to be understood to include a mutation of an amino acid residue occupying a homologous position in a lipolytic enzyme which has substantially the same structure as or a structure or amino acid sequence which can be aligned with that of the *H. lanuginosa* enzyme (e.g. *Rhizopus oryzae*, *Rhizomucor miehei, Absidia* sp. and *Penicillium camembertii* lipolytic enzymes mentioned herein). The homologous position can easily be identified by comparison between the structures.

The *H. lanuginosa* lipolytic enzyme variants may be characterized by being a combination of at least two different parent variants which indvidually have been found or indicated to have a good wash performance or otherwise interesting properties as described above. The good wash performance may e.g. be determined as described in Example 6. The combination between the parent variants may be random or specific.In connection with the present invention it has been found that particularly interesting results are obtained when the parent variants to be combined contains a mutation in at least one, but preferably more of the following positions: 1, 2, 3, 4, 5, 19, 49, 53, 56, 57, 59, 62, 83, 85, 90, 94, 96, 97, 99, 101, 102, 111, 116, 126, 127, 137, 167, 170, 181, 187, 210, 221, 225, 234, 239, 249, 252 256, 263, 264, 267, such as at least one or preferably more of the following mutations: E1K, E1S, V2G, S3T, Q4P, D5E, A19T, A49P ,Y53C, E56K, D57G, G59V, D62R, S83T, S85F, I90F, N94K, F95L, D96A, D96H, D96L, L97M, E99K, N101S, D102Y, D111N, S116P, Q126R, K127C, D137G, D167G, S170P, F181L, V187A, E210K, E210V, W221L, W221A, G225P, D234R, D234Y, E239C, Q249R, I252L, P256T, G263A, L264Q, T267R. It will be understood that the above mutations or mutated positions may be present on the same parent lipolytic enzyme, but preferably on various of the different parent lipolytic enzymes to be combined.

In particular, it has been found that a first wash *H. lanuginosa* lipolytic enzyme of the invention may be a combination

of at least two of the following parent *H. lanuginosa* lipolytic enzyme variants or parts of these variants:

(a) E56R+D57L+I90F+D96L+E99K
(b) E56R+D57L+V60M+D62N+S83T+D96P+D102E
(c) D57G+N94K+D96L+L97M
(d) E87K+G91A+D96R+I100V+E129K+K237M+I252L+P256T+G263A+L264Q
(e) E56R+D57G+S58F+D62C+T64R+E87G+G91A+F95L+D96P+K98I
(f) E210K
(g) S83T+N94K+D96N
(h) E87K+D96V
(i) N94K+D96A
(j) E87K+G91A+D96A
(k) D167G+E210V
(l) S83T+G91A+Q249R
(m) E87K+G91A
(n) S83T+E87K+G91A+N94K+D96N+D111N
(o) N73D+E87K+G91A+N94I+D96G
(p) L67P+176V+S83T+E87N+I90N+G91A+D96A+K98R
(q) S83T+E87K+G91A+N92H+N94K+D96M
(s) S85P+E87K+G91A+D96L+L97V
(t) E87K+I90N+G91A+N94S+D96N+I100T
(u) I34V+S54P+F80L+S85T+D96G+R108W+G109V+D111G+S116P+L124S+ V132M+V140Q+V141A+F142S+ H145R+N162T+I166V+F181P+F183S+R205G+A 243T+D254G+F262L,
(v) N94K, D96A, Q249R,
(w) E87K, G91A, D96W, D102N

Methods suitable for combining different parent variants are described below in the section in entitled "Combination of DNA sequences encoding lipolytic enzymes". A particular suitable method is the one described in Materials and Methods section herein.

In another embodiment the first wash lipolytic enzyme of the invention is a variant of the *H*. *lanuginosa* lipolytic enzyme (the amino acid sequence of which is shown in EP 305 216) which comprises a mutation in at least one, but preferably more of the following positions: 1, 2, 3, 4, 5, 19, 49, 53, 56, 57, 59, 62, 83, 85, 90, 94, 96, 97, 99, 101, 102, 111, 116, 126, 127, 137, 167, 170, 181, 187, 210, 221, 225, 234, 239, 249, 252 256, 263, 264, 267, such as at least one or preferably more of the following mutations, E1K, E1S, V2G, S3T, Q4P, D5E, A19T, A49P, Y53C, E56K, D57G, G59V, D62R, S83T, S85F, I90F, N94K, F95L, D96A, D96H, D96L, L97M, E99K, N101S, D102Y, D111N, S116P, Q126R, K127C, D137G, D167G, S170P, F181L, V187A, E210K, E210V, W221L, W221A, G225P, D234R, D234Y, E239C, Q249R, I252L, P256T, G263A, L264Q, T267R

In a more specific embodiment the first wash lipolytic enzyme of the invention is a variant of the *H*. *lanuginosa* lipolytic enzyme, in which at least one of the following amino acid residues has been replaced with another amino acid residue: A49, G59, S85, I90, S116, Q126, D137, S170 or W221.

Although the above identified amino acid residues may be replaced by any other of the 19 possible amino acid residues it is preferred that the amino acid residue is replaced as follows:

A49P, G59V, S85F, I90F, S116P, Q126R, D137G, S170P or W221L or by an amino acid residue belonging to the same charge group (cf the definition below) as that of the inserted amino acid residue, e.g. A49T instead of A49P. If a negatively charged amino acid residue is replaced, e.g. D137, it is preferred that it is replaced by an amino acid residue belonging to the positive charge group or the neutral group, e.g. D137G,N,K, as defined below:

Negative charge group: D,E
Positive charge group: K,R,H
Neutral group: I,C,S,T,P,W,M,G,A,P,N,Y,Q,L,V

It is contemplated that a variant comprising a mutation in the following positions is capable of exhibiting first wash activity or improved wash performance:

D57X+N94(K or R)+D96X+L97X+Q249(K or R)
N94(K or R)+D96X+L97X+Q249(K or R)
N94(K or R)+D96X+Q249(K or R)
D137X+D167X+E210X+W221X

D137X+D167X+E210X
I90X+D96X+E99X+V187X
I90X+D96X+E99X
I90(F or W or Y) + D96X + E99X
E56X+D57X+D62X+S85X+D96X+D102X+E210X N94(K or R)+F95L+D96X+D234X, in which X, may be any amino acid residue and may be identical, pairwise identical or different.

A *H. lanuginosa* lipolytic enzyme variant of the invention may comprise the following mutations:

D57G+N94K+D96L+Q249R
D57G+N94K+D96L+S116P+Q249R
D57G+G59V+N94K+D96L+Q249R
D57G+N94K+D96L+S116P+S170P+Q249R
D57G+G59V+N94K+D96L+S170P+Q249R
D57G+N94K+D96L+S170P+Q249R
D167G+E210V+Q249R
E56K+D167G+E210V
D137G+D167G+E210V+Q249R
D167G+E210V+W221L+Q249R
D57G+N94K+F95L+D96H,L+Q249R
D57G+N94K+D96L+E210K
D57G+G59V+N94K+D96L+S116P+S170P+Q249R
S3R+D137G+D167G+E210V+W221L
D137G+D167G+E210V+W221L+N233R
S3R+I90F+D96L+E99K+V187A+Q249R
I90F+D96L+E99K+V187A+D233R
I90F+D96L+E99K+V187A+D234Y
I90F+D96L+E99K+V187A+T231R
I90F+D96L+E99K+V187A
D62R+I90F+D96L+E99K+V187A
I90F+D96L+E99K+V187A+N200R+R209A
I90F+D96L+E99K+V187A+T199R+N200R+R209A
D57G+D62R+N94K+D96L+Q249R
D57G+N94K+D96L+N200R+R209A+Q249R
D57G+N94K+D96L+T199R+N200R+Q249R
I90F+D96L+E99K+V187A+T199R
D57G+N94K+D96L+T199R+R209A+Q249R
I90F+D96L+E99K+V187A+Q249R
I90F+D96L+E99K+V187A+P253R
I90F+D96L+E99K+D137G+D167G+V187A+Q249R
I90F+D96L+E99K+D137G+V187A+Q249R
D96L+E99K+V187A+Q249R
V2P+N94K+D96L+Q249R
V2W+S3R+N94K+D96L+Q249R
V2R+S3R+N94K+D96L+Q249R
V2R+S3R+N94K+D96L+Q249R
V2R+S3W+N94K+D96L+Q249R
V2W+S3R+N94K+D96L+Q249R
N94K+D96L+Q249R
V2G+S3T+D57G+N94K+D96L+L97M+Q249R
V2G+S3T+Q4P+D5E+D57G+N94K+D96L+L97M+Q249R
V2G+D5Q+L6M +D57G+N94K+D96L+L97M+Q249R

The following variants are of particular interest:

DS7G+GS9V+N94K+D96L+L97M+S116P+S170P+Q249R
A49P+D167G+E210V
E56K+D57G+D62R+S83T+S85F+D96L+D102Y+E210K

D57G +N94K +D96L+L97M+Q249R
D137G+D167G+E210V+W221L
N94K+F95L+D96H+N101S+F181L+D234Y+I252L+P256T+G263A+L264Q
I90F+D96L+E99K+V187A
N94K+D96A+Q249R
A19P+D167G+E210V+W221L
N94K+D96L+L97M+Q249R
D57G+N94K+D96L+Q249R
I90F+D96L+E99K+D137G+V187A
N94K+D96L+E99K+Q249R
N94K+D96L+E99K+T231R+N233R+D234R+Q249R
N94K+D96L+E99K+D111N+F211A+G225P+Q249R+T267R
N94K+D96L+E99K+D111N+F211A+G225P+T231R+N233R+D234R+Q249R+ T267R
E1K+N94K+D96L+E99K+Q249R
N94K+D96L+K223R+Q249R
N94K+D96L+E99K+N233R
N94K+D96L+E99K+T231R+N233R+Q249R
N94K+D96L+E99K+N233R+Q249R
N94K+D96L+E99K+D234R+Q249R

The variant of the invention may advantageously comprise an additional mutation in position E1, the mutation being a deletion of E1 or a replacement of E by any other amino acid residue, in particular P or S.

In addition the above specific variants may comprise any of the N-terminal or C-terminal peptide extensions discussed herein, specific examples of which are SPIRR, TAIRPRK, SPIRPRP, SPPRRP, RP, GPIRPRP, SRSRHNA, SALRPRK, STRRPRP, SPRRPRT, APPPRPRPLLPIS, SPIRK, SPPRPRP, WP, SPPPRPRP, SPIRRP, APPPRPRPRPR or SPIRPR. An N-terminal extension is e.g. applied to the amino acid residue E1 of the mature parent lipase or is applied to amino acid residue 2-20, such as 2, 3, 4 or 5 of the mature parent enzyme, the residue E1 (and optionally more amino acid residues of the non-structural part of the parent enzyme, e.g. amino acid residues within the 2-20 N-terminal part of the mature parent enzyme) being deleted. In addition, the peptide addition may be applied so that the one or more of the last amino acid residues of the peptide extensions mentioned herein replaces the amino acid residue(s) of the mature parent enzyme occupying position 1, and optionally 2 and further positions. For instance, the peptide extension "SPPRRP" may be applied by substituting E1 of the mature parent *H. lanuginosa* lipase with the last "P" of the peptide addition and substituting the wildtype propeptide "SPIRR" with "SPPRR".

When no replacements are to be performed in the N-terminal part of the mature parent enzyme, the N-terminal addition may be applied either as a result of the variants having been expressed in *S. cerevisiae* (if the N-terminal extension is identical to (a part of) the propeptide of the parent enzyme, or more preferably by the relevant modification of the part of the DNA sequence encoding the parent enzyme, which encodes the (pre)pro sequence or another sequence downstream of the codon encoding amino acid residue 1 of the mature parent enzyme.

The presently most preferred variants of the inventions include:

SPIRPRP+D57G+N94K+D96L+Q249R
SPPRRP+I90F+D96L+E99K+D137G+V187A
SPIRPRP+N94K+D96L+L97M+Q249R
SPPPRPRP+N94K+D96L+L97M+Q249R
SPIRPRP+D57G+N94K+D96L+L97M+Q249R
SPPRRP+I90F+D96L+E99K+V187A
SPIRPRP+D137G+D167G+E21V+W221L
E1SPIRPRP+I90F+D96L+E99K+V187A
E1SRKRKRK+I90F+D96L+E99K+V187A
E1SPRIKPRIK+I90F+D96L+E99K+V187A
E1SPPRRP+D62R+I90F+D96L+E99K+V187A
E1SPPRRP+I90F+D96L+E99K+V187A+N200R+R209A
E1SPPRRP+I90F+D96L+E99K+V187A+T199R+N200R+R209A
E1SPIRPRP+D57G+D62R+N94K+D96L+Q249R
E1SPIRPRP+D57G+N94K+D96L+N200R+R209A+Q249R
E1SPIRPRP+D57G+N94K+D96L+T199R+N200R+Q249R
E1SPPRRP+I90F+D96L+E99K+V187A+T199R
E1SPTRPRP+D57G+N94K+D96L+T199R+R209A+Q249R

E1SPIRPRP+I90F+D96L+E99K+V187A+Q249R
E1SPPRRP+I90F+D96L+E99K+V187A+P253R
E1SPPRRP+I90F+D96L+E99K+D137G+D167G+V187A+Q249R
E1SPPRRP+I90F+D96L+E99K+D137G+V187A+Q249R
E1SPPRRP+D96L+E99K+V187A+Q249R
E1SPPRPR+V2P+N94K+D96L+Q249R
E1SPPWWP+V2W+S3R+N94K+D96L+Q249R
E1SPPWRP+V2R+S3R+N94K+D96L+Q249R
E1SPPRWP+V2R+S3R+N94K+D96L+Q249R
E1SPPWWP+V2R+S3W+N94K+D96L+Q249R
E1SPPRWP+V2W+S3R+N94K+D96L+Q249R
E1SPPRWP+V2R+S3W+N94K+D96L+Q249R
E1SPPRWP+N94K+D96L+Q249R
E1SPPRRP+N94K+D96L+Q249R
E1APPPRPRPRPRP+V2G+S3T+D57G+N94K+D96L+L97M+Q249R
E1APPPRTRPRPRS+V2G+S3T+Q4P+D5E+D57G+N94K+D96L+L97M+Q249R
E1APPPKASPRQRP+V2G+D5Q+L6M+D57G+N94K+D96L+L97M+Q249R
SCIRR+N94K+D96L+E239C+Q249R
E1SPPRRP+D57G+N94K+D96L+Y53C+K127C+Q249R
E1SPPRRPR+V2R+S3P+N94K+D96L+Q249R
E1SPPWPRP+V2R+S3P+N94K+D96L+Q249R
E1SPPRRP+N94K+D96L+E99K
E1SPPRRP+N94K+D96L+E99K+Q249R
E1SPPCGRRP+N94K+D96L+E239C+Q249R
E1SPCRPRP+N94K+D96L+E239C+Q249R
SPPCRRRP+N94K+D96L+E239C+Q249R

and the variants disclosed in the Examples hereinafter. When an N-terminal extension is present in the above specified variants the nomenclature "E1...." is intended to indicate that the E in position 1 has been replaced by the last amino acid residue of the peptide addition listed after "E1", the remaining residues having been fused to the amino acid residue occupying position 1. For instance, "EISPPEQP" is intended to indicate that amino acid residue E1 has been replaced by "P" and that the remaining residues "SPPEQ" have been fused to E1P. In practice, such variants are conveniently constructed by replacing amino acid residues (-5)-(-1) of the unprocessed parent enzyme with the relevant amino acid residues of the peptide extension and replacing E1 with the relevant amino acid residue, the replacements being performed by introducing the relevant mutations in the corresponding DNA sequence, and subsequently produce the resulting variant in an expression system allowing at least a portion of the expressed variants to maintain their N-terminal extension (as is further disclosed herein). Where no replacements of E1 are indicated the N-terminal peptide addition is simply fused to the amino acid residue occupying position 1 of the mature parent enzyme.

The above variants have initially been constructed using the random mutagenesis and/or gene shuffling methods of the invention and subsequently characterized with respect to the thereby introduced mutations. It will be apparent that an alternative method of constructing these variants would be based on site-directed mutagenesis using suitable oligonucleotide probes in accordance with methods known in the art.

It is contemplated that the good washing performance/first wash activity will be maintained when one of the above specific individual mutations is replaced by a mutation to an amino acid residue belonging to the same charge group as the above suggested mutation. For instance, the mutation N94K may be replaced by N94R, H, the "G" in mutation D137G or D167G may be replaced by one of the other amino acid residues belonging to the neutral group, etc. Furthermore, it may be advantageous to replace an amino acid residue of the neutral group with one belonging to the positive charge group, e.g. the "G" in D 137G and D167G, respectively, may be replaced with a K, R or H resulting in the mutations D137K,R,H and D167K, R,H, respectively.

As already mentioned the *H. lanuginosa* lipolytic enzyme is structurally closely related to other lipolytic enzymes such as those derivable from *Rhizomucor miehei, Penicillium camembertii*, *Absidia* sp. and the various *Rhizopus* sp. disclosed herein. Accordingly, it is believed that modifications corresponding to those mentioned above in *H. lanuginosa* lipase and introduced into homologous positions in other structurally related lipolytic enzyme will also be functional with respect to the first wash performance. Accordingly, in a further aspect the invention relates to a first wash variant of a parent lipolytic enzyme which has an amino acid sequence or a three-dimensional structure which can be aligned with the *H. lanuginosa* lipase amino acid sequence or structure (with e.g. at least 20 % sequence identity allowing gaps or an overall protein similarity of at least 50%, such as at leas 60% or 70% using the UWGGG GAP programme or a "structural" similarity), the amino acid sequence of which has been modified so as to introduce mutations corresponding

to those of the *H. lanuginosa* lipase mentioned above and/or to introduce amino acid residues found in the wildtype *H. lanuginsa* lipase into the parent lipolytic enzyme in question. The amino acid residues or positions to be modified in the structurally or sequence homologous lipases may be identified from an alignment of the relevant structure/sequence with that of the *H. lanuginosa* lipase. Such variants may be constructed by a method of constructing a first wash lipolytic enzyme variant prepared from a parent lipolytic enzyme exhibiting structural and/or sequence homology to the *H. lanuginosa* lipase (such lipolytic enzymes being identified above), which method comprises aligning the sequence of the parent enzyme in question with that of the *H. lanuginosa* lipase or a first wash variant thereof or superimposing the structure of the parent enzyme in question with that of the *H. lanuginosa* lipase or variant, identifying the position (s) in the parent enzyme which are homologous to position(s) of the *H. lanuginosa* lipase or variant believed to be essential for achieving first wash activity (cf the mutations disclosed above), and replacing the amino acid residue occupying the relevant position(s) according to t, and producing the resulting variant enzyme.

**Cloning a DNA sequence encoding a parent lipolytic enzyme**

The DNA sequence encoding a parent lipolytic enzyme from which a first wash lipolytic enzyme is created in accordance with the present invention may be isolated from any cell or microorganism producing the parent enzyme in question by use of methods known in the art.

For instance, the DNA sequence may be isolated by establishing a cDNA or genomic library from an organism expected to harbour the sequence, and screening for positive clones by conventional procedures. Examples of such procedures are hybridization to oligonucleotide probes prepared on the basis of the amino acid or DNA sequence of the parent enzyme (if sequence information is available) or of a related lipolytic enzyme (if sequence information as to the parent enzyme is not available) in accordance with standard techniques (cf. Sambrook et al., 1989), and/or selection for clones expressing lipolytic activity, and/or selection for clones producing a protein which is reactive with an antibody raised against a parent lipolytic enzyme.

A preferred method of isolating a DNA sequence encoding a parent lipolytic enzyme to be modified in accordance with the invention from a cDNA or genomic library is by use of polymerase chain reaction (PCR) using degenerate oligonucleotide probes prepared on the basis of DNA or amino acid sequence of the parent enzyme. For instance, the PCR may be carried out using the techniques described in US Patent No. 4,683,202 or by R.K. Saiki et al. (1988).

Alternatively, the DNA sequence encoding the parent enzyme may be prepared synthetically by established standard methods, e.g. the phosphoamidite method described by Beaucage and Caruthers (1981), or the method described by Matthes et al. (1984). According to the phosphoamidite method, oligonucleotides are synthesized, e.g. in an automatic DNA synthesizer, purified, annealed, ligated and cloned in appropriate vectors.

Finally, the DNA sequence encoding the parent enzyme may be prepared from DNA of mixed genomic and synthetic, mixed synthetic and cDNA or mixed genomic and cDNA origin prepared by ligating fragments of synthetic, genomic or cDNA origin (as appropriate), the fragments corresponding to various parts of the entire DNA sequence encoding the parent enzyme, in accordance with standard techniques.

**Methods of constructing of first wash lipolytic enzyme variants**

As will be apparent from the brief description of the invention the present inventors have developed a very efficient method for creating lipolytic enzymes capable of removing a substantial amount of fatty matter during a one wash cycle assay as described herein.

Thus, in one highly preferred embodiment the lipolytic enzyme of the invention is a variant of a naturally-occurring parent lipolytic enzyme which is the result of a process comprising at least the following steps:

(a) expressing a variety of mutated DNA sequences originating from a parent lipolytic enzyme in suitable host cells;
(b) screening for host cells expressing a mutated lipolytic enzyme which has a decreased dependence on calcium and/or an improved tolerance towards a detergent or a detergent component as compared to the parent lipolytic enzyme; and
(c) selecting a mutated lipolytic enzyme among those resulting from step (b) which, when present in detergent composition A or B in a concentration of 12500 LU/l, is capable of removing at least 15% more lard from a lard stained swatch, than the same detergent composition without the enzyme, in a one cycle wash assay as described herein.

The variety of mutated DNA sequences referred to in step (a) may conveniently be obtained by subjecting a DNA sequence encoding the parent lipolytic enzyme to mutagenesis to form mutated DNA sequences Although the mutagenesis may be performed by any suitable method, such as by site-directed mutagenesis, it is presently preferred that the mutagenesis is carried out as a random mutagenesis. Thus, by use of random mutagenesis it is possible to

create a much higher number of mutated DNA sequences than would be possible by use of site-directed mutagenesis. The random mutagenesis is explained in further detail below in the section entitled "Random mutagenesis". In that section it is also described how one or more of the steps (a)-(c) of the method may be repeated one or more times in order to make successive improvements. For instance, the mutated lipolytic enzyme selected from the first round of steps (a)-(c) is subjected to a second round of the method in which the screening step (b) involves selection at more stringent conditions than those used in the screening step (b) of the first round thereby selecting for mutated lipolytic enzymes which has a decreased calcium dependence and/or an improved tolerance towards a detergent or a detergent component as compared to the mutated lipolytic enzyme resulting from the first round.

*Random mutagenesis*

The random mutagenesis of the DNA sequence encoding the parent lipolytic enzyme (or the peptide addition) to be performed in accordance with step a) of the above method may conveniently be performed by use of any method known in the art.

For instance, the random mutagenesis may be performed by use of a suitable physical or chemical mutagenizing agent, by use of a suitable oligonucleotide, or by subjecting the DNA sequence to PCR generated mutagenesis. Furthermore, the random mutagenesis may be performed by use of any combination of these mutagenizing agents.

The mutagenizing agent may, e.g., be one which induces transitions, transversions, inversions, scrambling, deletions, and/or insertions.

Examples of a physical or chemical mutagenizing agent suitable for the present purpose includes ultraviolet (UV) irradiation, hydroxylamine, N-methyl-N'-nitro-N-nitrosoguanidine (MNNG), O-methyl hydroxylamine, nitrous acid, ethyl methane sulphonate (EMS), sodium bisulphite, formic acid, gamma irradiation, 1-methyl-3-nitro-1-nitrosoguanidine (NTG), and nucleotide analogues.

When such agents are used the mutagenesis is typically performed by incubating the DNA sequence encoding the parent enzyme to be mutagenized in the presence of the mutagenizing agent of choice under suitable conditions for the mutagenesis to take place, and selecting for mutated DNA having the desired properties.

When the mutagenesis is performed by the use of an oligonucleotide, the oligonucleotide may be doped or spiked with the three non-parent nucleotides during the synthesis of the oligonucleotide at the positions wanted to be changed. The doping or spiking may be done so that codons for unwanted amino acids are avoided by lowering the amount of or completely avoiding the nucleotides resulting in these codons. State of the art knowledge and computer programs can be used for calculating the most optimal nucleotide mixture for a given amino acid preference. The doped or spiked oligonucleotide can be incorporated into the DNA encoding the lipolytic enzyme by any published technique using e.g. PCR, LCR or any DNA polymerase and ligase.

When PCR generated mutagenesis is used either a chemically treated or non-treated gene encoding a parent lipolytic enzyme is subjected to PCR under conditions that increases the misincorporation of nucleotides (Deshler 1992, Leung et al. 1989).

A mutator strain of *E. coli* (Fowler et al. 1974), *S. cerevisiae* or any other microbial organism may be used for the random mutagenesis of the DNA encoding the lipolytic enzyme by e.g. transforming a plasmid containing the parent enzyme into the mutator strain, growing the mutator strain with the plasmid and isolating the mutated plasmid from the mutator strain. The mutated plasmid may subsequently be transformed into the expression organism.

The DNA sequence to be mutagenized may conveniently be present in a genomic or cDNA library prepared from an organism expressing the parent lipolytic enzyme. Alternatively, the DNA sequence may be present on a suitable vector such as a plasmid or a bacteriophage, which as such may be incubated with or otherwise exposed to the mutagenizing agent. The DNA to be mutagenized may also be present in a host cell either by being integrated in the genome of said cell or by being present on a vector harboured in the cell. Finally, the DNA to be mutagenized may be in isolated form. The DNA sequence to be subjected to random mutagenesis is preferably a cDNA or a genomic DNA sequence.

In some cases it may be convenient to amplify the mutated DNA sequence prior to the expression or screening being performed. Such amplification may be performed in accordance with methods known in the art, the presently preferred method being PCR generated amplification using oligonucleotide primers prepared on the basis of the DNA or amino acid sequence of the parent enzyme.

Subsequent to the incubation with or exposure to the mutagenizing agent, the mutated DNA is expressed by culturing a suitable host cell carrying the DNA sequence under conditions allowing expression to take place. The host cell used for this purpose may be one which has been transformed with the mutated DNA sequence, optionally present on a vector, or one which carried the DNA sequence encoding the parent enzyme during the mutagenesis treatment. Examples of suitable host cells are given below. It is particularly preferred to use a yeast cell as a host cell, in particular when the parent lipolytic enzyme is derived from a fungus such as a filamentous fungus or yeast. The mutated DNA

sequence may further comprise a DNA sequence encoding functions permitting expression of the mutated DNA sequence.

It will be understood that the screening criteria mentioned in step (b) of the method of the invention have been carefully selected. Thus, without being limited to any theory the screening for a decreased dependency on calcium at alkaline pH (pH above 7) is believed to result in variants having an over-all improved performance in that the requirement for calcium may be considered a limiting factor for optimal activity, in particular under most wash conditions which are caracterized by the fact that the concentration of free calcium ions is deliberately lowered by chelating agents in the detergent matrix (builders).

The detergent or detergent component towards which the variant has improved tolerance may be of any type, e.g. as further described below. Preferably, the detergent component is a non-ionic, anionic, cationic, zwitterionic or amphoteric surfactant. Examples of non-ionic surfactants include an alcohol ethoxylate, examples of anionic surfactants include LAS, alkyl sulphate, alcohol ethoxy sulphate and the like. The choice of detergent will, e.g., depend on the inherent weakness (in relation to detergent tolerances) of the parent lipolytic enzyme.

In relation *Humicola lanuginosa* lipolytic enzymes and homologous enzymes (such as the *Penicillium*, *Rhizomucor, Rhizopus and Absidia* sp. lipolytic enzymes), it is contemplated that an improved tolerance towards a non-ionic surfactant alcohol ethoxylate, a commercially available example of which is Dobanol® 25-7, may be indicative of improved wash performance. In relation to *Pseudomonas* type lipolytic enzymes such as *P*. *pseudoalcaligenes, P. cepacia,* it is contemplated that an improved tolerance towards an anionic surfactant such as an alkyl sulphate (a commercially available example of which is NEODOL 45) or LAS (a commercially available example of which is Nansa 1169/P) may be indicative of improved wash performance.

The screening of step (b) is conveniently performed by use of a filter assay based on the following principle:

A microorganism capable of expressing the mutated lipolytic enzyme of interest is incubated on a suitable medium and under suitable conditions for the enzyme to be secreted, the medium being provided with a double filter comprising a first protein-binding filter and on top of that a second filter exhibiting a low protein binding capability. The microorganism is located on the second filter. Subsequent to the incubation, the first filter comprising enzymes secreted from the microorganisms is separated from the second filter comprising the microorganisms. The first filter is subjected to screening for the desired enzymatic activity and the corresponding microbial colonies present on the second filter are identified.

Alternatively, the second filter carrying the colonies may be used directly on the screening plate. This makes it easier to pick the right colonies and in some cases gives a stronger signal. And using only one filter, either protein binding or none-protein binding is sufficient in many cases.

The filter used for binding the enzymatic activity may be any protein binding filter e.g. nylon or nitrocellulose. The topfilter carrying the colonies of the expression organism may be any filter that has no or low affinity for binding proteins e.g. cellulose acetate or Durapore™. The filter may be pretreated with any of the conditions to be used for screening or may be treated during the detection of enzymatic activity.

The enzymatic activity may be detected by a dye, fluorescence, precipitation, pH indicator, IR-absorbance or any other known technique for detection of enzymatic activity.

The detecting compound may be immobilized by any immobilizing agent e.g. agarose, agar, gelatine, polyacrylamide, starch, filter paper, cloth; or any combination of immobilizing agents.

Lipolytic activity may be detected by Brilliant green, Rhodamine B or Sudan Black in combination with a lipid e.g. olive oil or lard. The screening criteria for identifying variants of parent lipolytic enzymes having improved washing performance may be e.g. EGTA, EDTA, non-ionic and/or anionic tensides, alkaline pH, or any detergent composition in combination with one of the above detectors of enzymatic activity.

Subsequent to the screening in step (b) lipolytic enzymes having desired properties (i.e. as defined by the screening criteria) are isolated and their first wash capability tested in the one cycle wash assay described in the Materials and Methods section herein.

If the first wash activity of the enzyme is not sufficiently good after one round of the above treatment, the enzyme may be modified, e.g. by site-directed or random mutagenesis in order to improve the first wash activity of the enzyme, e.g. in accordance with any of the principles given further above for modifacation of lipases to achieve a first wash performance.

Most convenient, the host cells produced in step (b) may be subjected to further rounds of mutagenesis as defined in steps (a)-(b) and optionally (c) above, conveniently by using more stringent selection criteria than employed in a previous mutagenesis treatment. The further round(s) of mutagenesis may be random, localized random or site-directed so as to introduce previously identified advantageous mutations, in particular D96L, Q249R, E87K, D254K, E210K or to introduce random mutations in selected regions, e.g. the lipid contact zone, in particular random mutations with doped or spiked oligonucleotides towards introduction of positive and/or hydrophobic amino acid residues, or to introduce any of the other specific mutations mentioned herein. Alternatively, genes encoding different homologous parent lipolytic enzymes may be combined in a random manner in order to obtain a novel variant carrying one or more

mutations from each variant. This is discussed in further detail below in the section entitled "Combination of DNA sequences encoding lipolytic enzymes".

The host cells selected for in step (b) or (c) may be used directly for the production of the variant of the lipolytic enzyme. Alternatively, DNA encoding the variant may be isolated from the host cell and inserted into another suitable host cell, conveniently by use of the procedure described below in the section entitled "Expression of a variant of the invention", in which suitable host cells are also listed.

*Localized random mutagenesis*

In accordance with the invention the random mutagenesis may advantageously be located to a part of the parent lipolytic enzyme in question. This may, e.g., be advantageous when a certain region of the enzyme has been identified to be of particular importance for a given property of the enzyme, and which, when modified, is expected to result in a variant having improved properties. Such region may normally be identified when the tertiary structure of the parent enzyme has been elucidated and related to the function of the enzyme.

One area of particular interest for modification amino acid residues located at the surface of the parent enzyme within or outside the lipid contact zone, i.e. the part of the lipolytic enzyme which is in contact with the lipid substrate and e.g. comprising the lid region, the hydrophobic cleft or any part of these structures. Another area of interest for lipolytic enzymes of the invention which contains a peptide addition or another modification within a non-structural part of the N-terminal or C-terminal end of the mature parent enzyme.

The localized random mutagenesis is conveniently performed by use of PCR generated mutagenesis techniques as described above or any other suitable technique known in the art. Especially for mutagenizing large peptide additions, it may be relevant to use PCR generated mutagenesis (e.g. as described by Deshler 1992 or Leung et al., 1989), in which one or more suitable oligonucleotide probes are used which flanks the area to be mutagenized. For mutagenesis of shorter peptide additions, it is more preferably perform the localized random mutagenesis by use of doped or spiked oligonucleotides. The doping or spiking is used, e.g., to avoid codons for unwanted amino acid residues or to increase the likelihood that a particular type of amino acid residue, such as a positively charged or hydrophobic amino acid residue, is introduced at a desired position.

Alternatively, the DNA sequence encoding the part of the DNA sequence to be modified may be isolated, e.g. by being inserted into a suitable vector, and said part may subsequently be subjected to mutagenesis by use of any of the mutagenesis methods discussed above.

Of particular interest is that the DNA sequence subjected to random mutagenesis comprises a part of or constitutes a part of a DNA sequence encoding the lipid contact zone or the lid region of the parent lipolytic enzyme. The localized random mutagenesis may be performed in one or more of these regions and/or one or more of the regions constituting the lipid contact zone, and is preferably performed in at least two of the regions. Parent lipolytic enzymes of particular interest for modification according to this aspect of the invention includes the *H. lanuginosa* lipolytic enzyme obtainable from strain DSM 4109 or a variant or analogue thereof, a parent lipolytic enzyme derived from *Penicillium camembertii*, a parent lipolytic enzyme derived from *Rhizopus oryzae*, a parent lipolytic enzyme derived from *Rhizomucor miehei*, a parent lipolytic enzyme derived from a *Absidia* sp. lipolytic enzyme, a parent lipolytic enzyme derived from a *Pseudomonas* sp. , prefereably belonging to the *Ps. aeroginosa* family such as the *Pseudomonas cepacia* lipase, the *Pseudomonas pseudoalcaligenes* lipase, the *Pseudomonas glumae* lipase, the *Pseudomonas mendocina* lipase, the *Pseudomonas wisconsinensis,* or the *Pseudomonas sp.* lipase (SD705) (Liposam®) shown in SEQ ID NO 99. The lipid contact zones and lid regions are identified in the Definitions section above.

The localized random mutagenesis may be done by use of doped oligonucleotides which are doped in the direction of L, I, V, F, W, A (hydrophobic amino acid residues) or K,R (positive amino acid residues), for instance under conditions ensuring about 90-93 % wildtype and about 7-10% mutant. Specific examples of suitable doping regimes are given in the Examples section below.

*In vivo recombination*

According to a preferred embodiment of the invention a DNA sequence encoding a first wash lipolytic enzyme may be constructed by a method, which as an important step involves combination of selected DNA sequences encoding different parent lipolytic enzymes or parts of such DNA sequences.

Preferably, the DNA sequences to be combined are derived from genes encoding lipolytic enzymes which has a satisfactory washing and/or dishwashing performance (e.g. as identified in Example 6) The aim of combining the DNA sequences is that the best elements from each "parent enzyme" are combined into one and the same variant enzyme.

In the present context the term "satisfactory washing performance" is intended to indicate that the parent enzymes are capable of removing fatty stains during one or several wash cycles when present in a suitable detergent.

Preferably, the parent enzyme in question has a better washing performance than Lipolase(TM).

The combination of DNA sequences may be performed by any suitable method known in the art. For instance, when the DNA sequences to be combined comprises homologous fragments, the combination is preferably achieved by homologous cross-over, e.g. by use of conventional methods such as US 5, 093, 257, or by gene shuffling (Stemmer (1994), Proc. Natl. Acad. Sci. USA, vol. 91, 10747-10751; Stemmer (1994), Nature, vol. 370, 389-391; Smith (1994), Nature vol 370, page 324-25), WO 95/17413. Gene shuffling means recombination of nucleotide sequence(s) between two or more homologous DNA sequences resulting in output DNA sequences having a number of nucleotides exchanged.

Of particular interest is an *in vivo* Gene Shuffling Method which is based on the following procedure:

a) forming at least one circular expression vector comprising a DNA sequence encoding a parent lipolytic enzyme or a substantial part thereof,
b) opening said circular expression vector within the DNA sequence encoding the lipolytic enzyme or part thereof,
c) preparing at least one DNA fragment comprising a DNA sequence homologous to at least a part of the enzyme coding region on at least one of the circular expression vector(s),
d) introducing at least one of said opened vector(s), together with at least one of said homologous DNA fragment (s) covering full-length DNA sequences encoding said lipolytic enzyme(s) or a part thereof, into a recombination host cell,
e) cultivating said yeast recombination host cell under conditions conducive for recombination betwen the homologous DNA fragments to take place, and
f) screening for positive lipolytic enzyme variants with an improved wash performance.

The vector used in step a) above may be a yeast expression vector which can be transformed into and expressed in a yeast recombination host cell. Examples of such expression vectors include yeast expression vectors constructed from pYES 2.0 (Invitrogene), such as pJSO37 comprising the wild type *Humicola lanuginosa* lipase gene

Opening of the vector in step b) may be accomplished by any conventional techniques known in the art, and may for instance be performed by opening the vector within the lipase gene by cutting at a single site or by gapping the vector *(i.e.* cutting e.g. at two sites resulting in cutting out a little part of the gene).

The preparation of the homologous DNA fragment(s) in step c) may be performed by amplifying homologous DNA sequence(s) (*e.g.* comprising one or more mutation in the lipolytic gene and comprising in a plasmid or vector) by any suitable methods, such as by a standard PCR amplification method described in US 4,683,202 or Saiki et al., (1988), Science 239, 487 - 491).

The vector(s) may be introduced into the recombination host cell (in step d) by transformation. In the case of the recombination host cell is a strain of *Saccharomyces cerevisiae*, such as *Saccharomyces cerevisiae* YNG318 (described below) the transformation may be performed as described by Sambrooks et al., (1989), Molecular Cloning: A Laboratory Manual, 2nd Ed., Cold Spring Harbor, NY, USA).

The screening for positive lipolytic enzyme variants may, *e.g.*, be performed by the screening method described in connection with the random mutagenesis above.

One of more cycle of step a) to f) may be performed before a selection of a first wash lipolytic enzyme variant is made using the selection conditions defined further above.

According to the shuffling method significantly more than two DNA sequences can be shuffled. Any number of different DNA fragments and homologous lipolytic enzymes comprised in suitable plasmids may be shuffles at the same time.

The DNA sequences to be combined may be entire genes of which at least one part exhibits sufficient homology to the other genes to allow for recombination of the genes to take place. Alternatively, the DNA sequences may be partial genes, which when combined can give rise to a functional gene capable of expressing a lipolytic enzyme.

When the DNA sequences to be combined are highly homologous or partially identical controlled combination may be performed, e.g. in the case of combination of two DNA sequences, to combine the N-terminal part of one of the sequences with the C-terminal part of the other (corresponding to the remaining part of the first sequence) or by combining other relevant parts of the respective genes in question.

Naturally occurring enzymes may be genetically modified by random, localized random or site directed mutagenesis as described above prior to being subjected to gene shuffling. Alternatively, part of one enzyme may be replaced by a part of another to obtain a chimeric enzyme. This replacement can be achieved either by conventional *in vitro* gene splicing techniques or by *in vivo* recombination or by combinations of both techniques. When using conventional *in vitro* gene splicing techniques, a desired portion of the lipolytic enzyme gene may be deleted using appropriate site-specific restriction enzymes; the deleted portion of the coding sequence may then be replaced by insertion of a desired portion of a different lipolytic enzyme coding sequence so that a chimeric nucleotide sequence encoding a new lipolytic enzyme is produced. Alternatively, lipolytic enzyme genes may be fused, e. g. by use of the PCR overlay

addition method described by Higuchi et al. 1988. The *in vivo* recombination techniques depend on the fact that different DNA segments with highly homologous regions (identity of DNA sequence) may recombine, i.e. break and exchange DNA, and establish new bonds in the homologous regions. Accordingly, when the coding sequences for two or more different but homologous lipolytic enzymes are used to transform a host cell, recombination of homologous sequences *in vivo* will result in the production of chimeric gene sequences. Translation of these coding sequences by the host cell will result in production of a chimeric lipolytic enzyme gene product. Specific *in vivo* recombination techniques are described in US 5,093,257 and EP 252 666.

In order to allow homologous recombination to take place it is desirable that the lipolytic enzymes comprises parts which are at least 60% homologous. It is particularly preferred that the entire enzymes are at least 60% homologous. The enzymes to be combined may be different variants of the same parent enzyme, e.g. variants derived from the *H*. *lanuginosa* lipolytic enzyme disclosed herein, or variants derived from the *Ps*. *alcaligenes* or *Ps. pseudoalcaligenes* lipolytic enzymes referred to further above, or variants derived from the *F*. *solani pisi* lipolytic enzyme (cf above), or variants derived from the *P*. *mendocina* lipolytic enzyme or the *Pseudomonas* sp. lipase (Liposam) (cf. above). It will be understood that the random recombination may be performed between a naturally-occurring lipolytic enzymes and one or more variants of said enzyme, between differerent naturally ocurring enzymes, between variants of naturally ocurring enzymes (the variants being variants of the same parent enzyme or of different enzymes), or between any combination of naturally occurring enzymes and variants of naturally occurring enzyme as long as the corresponding DNA sequences are capable of recombinng. When the DNA sequences to be combined are variants of a parent enzyme these variants may conveniently be prepared by the mutagenesis, in particular random mutagenesis method disclosed above.

In an alternative embodiment, the hybrid enzyme may be synthesized by standard chemical methods known in the art. For example, see Hunkapiller et al. (1984). Accordingly, peptides having the amino acid sequences described above may be synthesized in whole or in part and joined to form the hybrid enzymes of the invention.

In a highly preferred embodiment first wash lipolytic enzymes of the invention are constructed by a method which comprises subjecting a parent lipolytic enzyme to mutagenesis, in particular random mutagenesis, to form a variety of mutated DNA sequences, expressing the mutated DNA sequences in a suitable host and screening for host cells which produces a mutated lipolytic enzyme which has a decreased dependency on calcium and/or an improved tolerance towards a detergent or a detergent component, subjecting the DNA sequence encoding the mutated lipolytic enzyme selected in said screening to in vivo recombination, in particular gene shuffling or sexual PCR, with one or more other mutated DNA sequences prepared in a similar manner from the same parent lipolytic enzyme, expressing the mutated recombined DNA sequences in a suitable host, optionally selecting for host cells producing a mutated lipolytic enzyme which has a decreased dependency on calcium and/or an improved tolerance towards a detergent or a detergent component, optionally repeating either or both of the above mutagenesis and in vivo recombination procedures one or more times using more stringent screening criteria, and finally selecting a recombined DNA sequence endoding a lipolytic enzyme exhibiting first wash activity as defined herein.

Furthermore, it will be understood that a first wash lipolytic enzyme of the invention which comprises a peptide addition as well as mutation(s) in a structural part of the parent enzyme may be constructed by a method which involves localized mutagenesis, in particular localized random mutagenesis, in the part of the DNA sequence encoding the peptide addition and selected parts of the DNA sequence encoding the mature part of the parent lipolytic enzyme, i. e. a combination of the random mutagenesis method according to the second aspect of the invention performed in a structural part of the parent enzyme and random mutagenesis in a non-structural part of the N-terminal and/or C-terminal end and/or in a peptide addition applied to the N-terminal and/or C-terminal part.

It will be understood that the in vivo recombination and mutagenesis methods disclosed herein may be applied to any of the parent lipolytic enzymes mentioned in the "Parent Lipolytic Enzymes" section herein. Particularly preferred parent lipolytic enzymes are derived from *Humicola lanuginosa* and from *Pseudomonas* sp. such as *Ps. alcaligenes* and *Ps*. *pseudoalcaligenes*.

**Expression of a lipolytic enzyme of the invention**

An isolated nucleic acid sequence encoding a first wash lipolytic enzyme of the invention may be manipulated in a variety of ways to provide for expression of the enzyme. Manipulation of the nucleic acid sequence encoding a first wash lipolytic enzyme prior to its insertion into a vector may be desirable or necessary depending on the expression vector. The techniques for modifying nucleic acid sequences utilizing cloning methods are well known in the art.

The term "control sequences" is defined herein to include all components which are necessary or advantageous for expression of the coding sequence of the nucleic acid sequence. Each control sequence may be native or foreign to the nucleic acid sequence encoding the lipolytic enzyme. Such control sequences include, but are not limited to, a leader, a polyadenylation sequence, a propeptide sequence, a promoter, a signal sequence, and a transcription terminator. At a minimum, the control sequences include a promoter, and transcriptional and translational stop signals.

The control sequences may be provided with linkers for the purpose of introducing specific restriction sites facilitating ligation of the control sequences with the coding region of the nucleic acid sequence encoding the lipolytic enzyme.

The control sequence may be an appropriate promoter sequence, a nucleic acid sequence which is recognized by a host cell for expression of the nucleic acid sequence. The promoter sequence contains transcription and translation control sequences which mediate the expression of the first wash lipolytic enzyme. The promoter may be any nucleic acid sequence which shows transcriptional activity in the host cell of choice and may be obtained from genes encoding extracellular or intracellular polypeptides either homologous or heterologous to the host cell. Examples of suitable promoters for directing the transcription of the nucleic acid constructs of the present invention, especially in a bacterial host cell, are the promoters obtained from the *E. coli lac* operon, the *Streptomyces coelicolor* agarase gene (*dagA*), the *B. subtilis* levansucrase gene (*sacB*) or the alkaline protease gene, the *B. licheniformis* alpha-amylase gene (*amyL*), the *B. stearothermophilus* maltogenic amylase gene (*amyM*), the *B. amyloliquefaciens* alpha-amylase gene (*amyQ*), the *B.licheniformis* penicillinase gene (*penP*), the *B. subtilis xylA* and *xylB* genes, the *B. pumilus* xylosidase gene, and the prokaryotic beta-lactamase or tryptophan gene (Villa-Kamaroff *et al*., 1978, *Proceedings of the National Academy of Sciences USA* 75:3727-3731), as well as the *tac* gene (DeBoer *et al*., 1983, *Proceedings of the National Academy of Sciences USA* 80:21-25). Further promoters are described in "Useful proteins from recombinant bacteria" in *Scientific American*, 1980, 242:74-94; and in Sambrook *et al*., 1989, *supra.* Examples of suitable promoters for directing the transcription of the nucleic acid constructs of the present invention in a filamentous fungal host cell are promoters obtained from the genes encoding *A. oryzae* TAKA amylase, *A. oryzae* triose phosphate isomerase, *Rhizomucor miehei* aspartic proteinase, *A. niger* neutral alpha-amylase, *A. niger* acid stable alpha-amylase, *A. niger* or *A.. awamori* glucoamylase (*glaA*), *Rhizomucor miehei* lipase, *A. oryzae* alkaline protease, *A. oryzae* triose phosphate isomerase, *A. nidulans* acetamidase, *Fusarium oxysporum* trypsin-like protease (as described in U.S. Patent No. 4,288,627, which is incorporated herein by reference), or the ADH-3 promoter (McKnight et al., (1985), The EMBO J. 4, 2093-3099) and hybrids thereof. Particularly preferred promoters for use in filamentous fungal host cells is the TAKA amylase and the *glaA* promoters. In a yeast host, promoters from yeast glycolytic genes (Hitzeman et al.,(1980), J. Biol. Chem. 255, 12073-12080; Alber and Kawasaki, (1982), J. Mol. Appl. Gen. 1, 419-434) or alcohol dehydrogenase genes (Young et al., in Genetic Engineering of Microorganisms for Chemicals (Hollaender et al, eds.), Plenum Press, New York, 1982), or the TPI1 (US 4,599,311) or ADH2-4c (Russell et al., (1983), Nature 304, 652-654) promoters. useful promoters are obtained from the *S. cerevisiae* enolase (ENO-1) gene, the *S. cerevisiae* galactokinase gene (GAL1), the *S. cerevisiae* alcohol dehydrogenase/glyceraldehyde-3-phosphate dehydrogenase genes (ADH2/GAP), and the *S. cerevisiae* 3-phosphoglycerate kinase gene. Other useful promoters for yeast host cells are described by Romanos *et al*., 1992, *Yeast* 8:423-488.

The control sequence may also be a suitable transcription terminator sequence, a sequence recognized by a host cell to terminate transcription. The terminator sequence is operably linked to the 3' terminus of the nucleic acid sequence encoding the modified lipolytic enzyme. The terminator sequence may be native to the nucleic acid sequence encoding the lipolytic enzyme or may be obtained from foreign sources. Any terminator which is functional in the host cell of choice may be used in the present invention. Preferred terminators for filamentous fungal host cells are obtained from the genes encoding *A. oryzae* TAKA amylase, *A. niger* glucoamylase, *A. nidulans* anthranilate synthase, *A. niger* alpha-glucosidase, and *Fusarium oxysporum* trypsin-like protease. Preferred terminators for yeast host cells are obtained from the genes encoding *S. cerevisiae* enolase, *S. cerevisiae* cytochrome C (CYC1), or *S. cerevisiae* glyceraldehyde-3-phosphate dehydrogenase. Other useful terminators for yeast host cells are described by Romanos *et al*., 1992, *supra.*

The control sequence may also be a suitable leader sequence, a nontranslated region of a mRNA which is important for translation by the host cell. The leader sequence is operably linked to the 5' terminus of the nucleic acid sequence encoding the lipolytic enzyme. The leader sequence may be native to the nucleic acid sequence encoding the lipolytic enzyme or may be obtained from foreign sources. Any leader sequence which is functional in the host cell of choice may be used in the present invention. Preferred leaders for filamentous fungal host cells are obtained from the genes encoding *A. oryzae* TAKA amylase and *A. oryzae* triose phosphate isomerase. Suitable leaders for yeast host cells are obtained from the *S. cerevisiae* enolase (ENO-1) gene, the *S. cerevisiae* 3-phosphoglycerate kinase gene, the *S. cerevisiae* alpha-factor, and the *S. cerevisiae* alcohol dehydrogenase/glyceraldehyde-3-phosphate dehydrogenase genes (ADH2/GAP).

The control sequence may also be a polyadenylation sequence, a sequence which is operably linked to the 3' terminus of the nucleic acid sequence and which, when transcribed, is recognized by the host cell as a signal to add polyadenosine residues to transcribed mRNA. The polyadenylation sequence may be native to the nucleic acid sequence encoding the lipolytic enzyme or may be obtained from foreign sources. Any polyadenylation sequence which is functional in the host cell of choice may be used in the present invention. Preferred polyadenylation sequences for filamentous fungal host cells are obtained from the genes encoding *A. oryzae* TAKA amylase, *A. niger* glucoamylase, *A. nidulans* anthranilate synthase, and *A. niger* alpha-glucosidase. Useful polyadenylation sequences for yeast host cells are described by Guo and Sherman, 1995, *Molecular Cellular Biology* 15:5983-5990. Polyadenylation sequences

are well known in the art for mammalian host cells.

The control sequence may also be a signal peptide coding region, which codes for an amino acid sequence linked to the amino terminus of the first wash lipolytic enzyme which can direct the expressed first wash lipolytic enzyme into the cell's secretory pathway. The signal peptide coding region may be native to the first wash lipolytic enzyme of the invention or may be obtained from foreign sources. The 5' end of the coding sequence of the nucleic acid sequence may inherently contain a signal peptide coding region naturally linked in translation reading frame with the segment of the coding region which encodes the secreted first wash lipolytic enzyme. Alternatively, the 5' end of the coding sequence may contain a signal peptide coding region which is foreign to that portion of the coding sequence which encodes the secreted first wash lipolytic enzyme. The foreign signal peptide coding region may be required where the coding sequence does not normally contain a signal peptide coding region. Alternatively, the foreign signal peptide coding region may simply replace the natural signal peptide coding region in order to obtain enhanced secretion of the lipolytic enzyme relative to the natural signal peptide coding region normally associated with the coding sequence. The signal peptide coding region may be obtained from a glucoamylase or an amylase gene from an *Aspergillus* species, a lipase or proteinase gene from a *Rhizomucor* species, the gene for the a-factor from *Saccharomyces cerevisiae*, an amylase or a protease gene from a *Bacillus* species, or the calf preprochymosin gene. An effective signal peptide coding region for bacterial host cells is the signal peptide coding region obtained from the maltogenic amylase gene from *Bacillus* NCIB 11837, the *B. stearothermophilus* alpha-amylase gene, the *B. licheniformis* subtilisin gene, the *B. licheniformis* beta-lactamase gene, the *B. stearothermophilus* neutral proteases genes (*nprT*, *nprS*, *nprM*), and the *B. subtilis* PrsA gene. Further signal peptides are described by Simonen and Palva, 1993, *Microbiological Reviews* 57: 109-137. An effective signal peptide coding region for filamentous fungal host cells is the signal peptide coding region obtained from *A. oryzae* TAKA amylase gene, *A. niger* neutral amylase gene, the *Rhizomucor miehei* aspartic proteinase gene, the *H. lanuginosa* cellulase gene, or the *Rhizomucor miehei* lipase gene. Useful signal peptides for yeast host cells are obtained from the genes for *S. cerevisiae* a-factor and *S. cerevisiae* invertase. Other useful signal peptide coding regions are described by Romanos *et al*., 1992, *supra.* However, any signal peptide coding region capable of directing the expressed enzyme into the secretory pathway of a host cell of choice may be used in the present invention.

The nucleic acid constructs of the present invention may also comprise one or more nucleic acid sequences which encode one or more factors that are advantageous in the expression of the first wash lipolytic enzyme, *e.g*., an activator (*e.g*., a trans-acting factor), a chaperone, and a processing protease. The nucleic acids encoding one or more of these factors are not necessarily in tandem with the nucleic acid sequence encoding the first wash lipolytic enzyme. An activator is a protein which activates transcription of a nucleic acid sequence encoding a first wash lipolytic enzyme (Kudla *et al*., 1990, *EMBO Journal* 9:1355-1364; Jarai and Buxton, 1994, *Current Genetics* 26:2238-244; Verdier, 1990, *Yeast* 6:271-297). The nucleic acid sequence encoding an activator may be obtained from the genes encoding *B. stearothermophilus* NprA (*nprA*), *S. cerevisiae* heme activator protein 1 (*hap1*), *S. cerevisiae* galactose metabolizing protein 4 (*gal4*), and *A. nidulans* ammonia regulation protein (*areA*). For further examples, see Verdier, 1990, *supra* and MacKenzie *et al*., 1993, *Journal of General Microbiology* 139:2295-2307. A chaperone is a protein which assists another polypeptide in folding properly (Hartl *et al*., 1994, *TIBS* 19:20-25; Bergeron *et al*., 1994, *TIBS* 19:124-128; Demolder *et al*., 1994, *Journal of Biotechnology* 32:179-189; Craig, 1993, *Science* 260:1902-1903; Gething and Sambrook, 1992, *Nature* 355:33-45; Puig and Gilbert, 1994, *Journal of Biological Chemistry* 269:7764-7771; Wang and Tsou, 1993, *The FASEB Journal* 7:1515-11157; Robinson *et al*., 1994, *Bio/Technology* 1:381-384). The nucleic acid sequence encoding a chaperone may be obtained from the genes encoding *B. subtilis* GroE proteins, *A. oryzae* protein disulphide isomerase, *S. cerevisiae* calnexin, *S. cerevisiae* BiP/GRP78, and *S. cerevisiae* Hsp70. For further examples, see Gething and Sambrook, 1992, *supra,* and Hartl *et al*., 1994, *supra*. Any factor that is functional in the host cell of choice may be used in the present invention.

It may also be desirable to add regulatory sequences which allow the regulation of the expression of the first wash lipolytic enzyme relative to the growth of the host cell. Examples of regulatory systems are those which cause the expression of the gene to be turned on or off in response to a chemical or physical stimulus, including the presence of a regulatory compound. Regulatory systems in prokaryotic systems would include the *lac, tac,* and *trp* operator systems. In yeast, the ADH2 system or GAL1 system may be used. In filamentous fungi, the TAKA alpha-amylase promoter, *A. niger* glucaamylase promoter, and the *A. oryzae* glucoamylase promoter may be used as regulatory sequences. Other examples of regulatory sequences are those which allow for gene amplification. In eukaryotic systems, these include the dihydrofolate reductase gene which is amplified in the presence of methotrexate, and the metallothionein genes which are amplified with heavy metals. In these cases, the nucleic acid sequence encoding the first wash lipolytic enzyme would be placed in tandem with the regulatory sequence.

## Expression Vectors

The present invention also relates to recombinant expression vectors comprising a nucleic acid sequence of the present invention, a promoter, and transcriptional and translational stop signals. The various nucleic acid and

control sequences described above may be joined together to produce a recombinant expression vector which may include one or more convenient restriction sites to allow for insertion or substitution of the nucleic acid sequence encoding the first wash lipolytic enzyme at such sites. Alternatively, the nucleic acid sequence of the present invention may be expressed by inserting the nucleic acid sequence or a nucleic acid construct comprising the sequence into an appropriate vector for expression. In creating the expression vector, the coding sequence is located in the vector so that the coding sequence is operably linked with the appropriate control sequences for expression, and possibly secretion.

The recombinant expression vector may be any vector which can be conveniently subjected to recombinant DNA procedures and can bring about the expression of the nucleic acid sequence. The choice of the vector will typically depend on the compatibility of the vector with the host cell into which the vector is to be introduced. The vectors may be linear or closed circular plasmids. The vector may be an autonomously replicating vector, *i.e.*, a vector which exists as an extrachromosomal entity, the replication of which is independent of chromosomal replication, *e.g.*, a plasmid, an extrachromosomal element, a minichromosome, or an artificial chromosome. The vector may contain any means for assuring self-replication. Alternatively, the vector may be one which, when introduced into the host cell, is integrated into the genome and replicated together with the chromosome(s) into which it has been integrated. The vector system may be a single vector or plasmid or two or more vectors or plasmids which together contain the total DNA to be introduced into the genome of the host cell, or a transposon.

The vectors of the present invention preferably contain one or more selectable markers which permit easy selection of transformed cells. A selectable marker is a gene the product of which provides for biocide or viral resistance, resistance to heavy metals, prototrophy to auxotrophs, and the like. Examples of bacterial selectable markers are the dal genes from *B. subtilis* or *B. licheniformis*, or markers which confer antibiotic resistance such as ampicillin, kanamycin, chloramphenicol or tetracycline resistance. A frequently used mammalian marker is the dihydrofolate reductase gene. Suitable markers for yeast host cells are ADE2, HIS3, LEU2, LYS2, MET3, TRP1, and URA3. A selectable marker for use in a filamentous fungal host cell may be selected from the group including, but not limited to, *amdS* (acetamidase), *argB* (ornithine carbamoyltransferase), bar (phosphinothricin acetyltransferase), *hygB* (hygromycin phosphotransferase), *niaD* (nitrate reductase), *pyrG* (orotidine-5'-phosphate decarboxylase), *sC* (sulfate adenyltransferase), *trpC* (anthranilate synthase), and glufosinate resistance markers, as well as equivalents from other species. Preferred for use in an *Aspergillus* cell are the *amdS* and *pyrG* markers *of A. nidulans* or *A*. *oryzae* and the *bar* marker of *Streptomyces hygroscopicus*. Furthermore, selection may be accomplished by co-transformation, *e.g.*, as described in WO 91/17243, where the selectable marker is on a separate vector.

The vectors of the present invention preferably contain an element(s) that permits stable integration of the vector into the host cell genome or autonomous replication of the vector in the cell independent of the genome of the cell.

The vectors of the present invention may be integrated into the host cell genome when introduced into a host cell. For integration, the vector may rely on the nucleic acid sequence encoding the first wash lipolytic enzyme or any other element of the vector for stable integration of the vector into the genome by homologous or nonhomologous recombination. Alternatively, the vector may contain additional nucleic acid sequences for directing integration by homologous recombination into the genome of the host cell. The additional nucleic acid sequences enable the vector to be integrated into the host cell genome at a precise location(s) in the chromosome(s). To increase the likelihood of integration at a precise location, the integrational elements should preferably contain a sufficient number of nucleic acids, such as 100 to 1,500 base pairs, preferably 400 to 1,500 base pairs, and most preferably 800 to 1,500 base pairs, which are highly homologous with the corresponding target sequence to enhance the probability of homologous recombination. The integrational elements may be any sequence that is homologous with the target sequence in the genome of the host cell. Furthermore, the integrational elements may be non-encoding or encoding nucleic acid sequences. On the other hand, the vector may be integrated into the genome of the host cell by non-homologous recombination. These nucleic acid sequences may be any sequence that is homologous with a target sequence in the genome of the host cell, and, furthermore, may be non-encoding or encoding sequences.

For autonomous replication, the vector may further comprise an origin of replication enabling the vector to replicate autonomously in the host cell in question. Examples of bacterial origins of replication are the origins of replication of plasmids pBR322, pUC19, pACYC177, pACYC184, pUB110, pE194, pTA1060, and pAMβ1. Examples of origin of replications for use in a yeast host cell are the 2 micron origin of replication, the combination of CEN6 and ARS4, and the combination of CEN3 and ARS1. The origin of replication may be one having a mutation which makes its functioning temperature-sensitive in the host cell (see, *e.g.,* Ehrlich, 1978, *Proceedings of the National Academy of Sciences USA* 75:1433).

More than one copy of a nucleic acid sequence encoding a first wash lipolytic enzyme of the present invention may be inserted into the host cell to amplify expression of the nucleic acid sequence. Stable amplification of the nucleic acid sequence can be obtained by integrating at least one additional copy of the sequence into the host cell genome using methods well known in the art and selecting for transformants.

The procedures used to ligate the elements described above to construct the recombinant expression vectors

of the present invention are well known to one skilled in the art (see, *e.g.*, Sambrook *et al.*, 1989, *supra*).

**Host Cells**

The present invention also relates to recombinant host cells, comprising a nucleic acid sequence of the invention, which are advantageously used in the recombinant production of the first wash lipolytic enzymes. The cell is preferably transformed with a vector comprising a nucleic acid sequence of the invention followed by integration of the vector into the host chromosome. "Transformation" means introducing a vector comprising a nucleic acid sequence of the present invention into a host cell so that the vector is maintained as a chromosomal integrant or as a self-replicating extra-chromosomal vector. Integration is generally considered to be an advantage as the nucleic acid sequence is more likely to be stably maintained in the cell. Integration of the vector into the host chromosome may occur by homologous or non-homologous recombination as described above.

The choice of a host cell will to a large extent depend upon the gene encoding the first wash lipolytic enzyme and its source. In addition, the choice of host cell will often depend on the proteolytic enzyme system of the host cell and its impact on the production of a first wash lipolytic enzyme of the invention. Accordingly, it may be desirable to use a host cell which is deficient in one or more proteolytic enzymes or other enzyme processing means. Protease deficient host cells of bacteria as well as fungal (filamentous fungal and yeast) cells are well-known in the art. When the first wash lipolytic enzyme of the invention comprises a peptide addition it may be advantageous that the host is a strain reduced or deficient in one or more exo-proteases capable of cleaving the modified lipolytic enzyme at a site close to the peptide addition or a protease capable of cleaving within the peptide addition. For instance, the host cell may be reduced or deficient in a tripeptidyl-aminopeptidase (TPAP) (see e.g. WO 96/14404 from Novo Nordisk A/S), a dipeptidyl-aminopeptidase (DPAP), and/or a Kex2 protease or Kex2-like protease and therefore not capable of cleaving at di-basic sites such as Arg-Arg (RR).

Other examples of host cells include alkaline protease deficient or reduced host cells, aspartic proteinase deficient host cells (EP 429 490), and host cells deficient of proteolytic enzymes such as the host cells described in WO 93/00925, WO 92/17595, EP 341 215, EP 574 347, and PCT/DK96/00111.

The host cell may be a unicellular microorganism or a non-unicellular microorganism. Useful unicellular cells are bacterial cells such as gram positive bacteria including, but not limited to, a *Bacillus* cell, *e.g.*, *B. subtilis*, *B. licheniformis*, *B. lentus*, *B. brevis*, *B. stearothermophilus*, *B. alkalophilus, B. amyloliquefaciens*, *B. coagulans, B.circulans*, *B. lautus*, *B. megaterium*, and *B. thuringiensis*; or a *Streptomyces* cell, *e.g.*, *S. lividans* or *S. murinus*, or gram negative bacteria such as *E. coli* and *Pseudomonas* sp. (especially when a bacterial lipolytic enzyme, such as a *Pseudomonas sp*. enzyme is to be produced). The transformation of a bacterial host cell may, for instance, be effected by protoplast transformation (see, *e.g.*, Chang and Cohen, 1979, *Molecular General Genetics* 168:111-115), by using competent cells (see, *e.g.*, Young and Spizizin, 1961, *Journal of Bacteriology* 81:823-829, or Dubnar and Davidoff-Abelson, 1971, *Journal of Molecular Biology* 56:209-221), by electroporation (see, *e.g.*, Shigekawa and Dower, 1988, *Biotechniques* 6:742-751), or by conjugation (see, *e.g.*, Koehler and Thorne, 1987, *Journal of Bacteriology* 169:5771-5278).

The host cell may be a eukaryote, and is preferably a fungal, i.e. a yeast cell or a filamentous fungal cell, especially for the production of a modified lipolytic enzyme of eukaryotic origin.

"Yeast" as used herein includes ascosporogenous yeast (Endomycetales), basidiosporogenous yeast, and yeast belonging to the Fungi Imperfecti (Blastomycetes). The ascosporogenous yeasts are divided into the families Spermophthoraceae and Saccharomycetaceae. The latter is comprised of four subfamilies, Schizosaccharomycoideae (*e.g.*, genus *Schizosaccharomyces*), Nadsonioideae, Lipomycoideae, and Saccharomycoideae (*e.g.*, genera *Pichia*, *Kluyveromyces* and *Saccharomyces*). The basidiosporogenous yeasts include the genera *Leucosporidim, Rhodosporidium, Sporidiobolus, Filobasidium*, and *Filobasidiella*. Yeast belonging to the Fungi Imperfecti are divided into two families, Sporobolomycetaceae (*e.g.*, genera *Sorobolomyces* and *Bullera)* and Cryptococcaceae (*e.g.*, genus *Candida*). Since the classification of yeast may change in the future, for the purposes of this invention, yeast shall be defined as described in *Biology and Activities of Yeast* (Skinner, F.A., Passmore, S.M., and Davenport, R.R., eds, Soc. App. Bacteriol. Symposium Series No. 9, 1980. The biology of yeast and manipulation of yeast genetics are well known in the art (see, *e.g., Biochemistry and Genetics of Yeast,* Bacil, M., Horecker, B.J., and Stopani, A.O.M., editors, 2nd edition, 1987; *The Yeasts*, Rose, A.H., and Harrison, J.S., editors, 2nd edition, 1987; and *The Molecular Biology of the Yeast Saccharomyces,* Strathern *et al*., editors, 198i). In connection with the present invention the use of yeast cells which typically have another proteolytic enzyme processing system that, e.g., bacteria and filamentous fungi, may be of particular use for preparing modified lipolytic enzymes which, as the peptide addition, comprise a part or all of the natural prosequences of the parent lipolytic enzyme in question. When the fungal host cell is a yeast cell (e.g. to be used in applying a peptide addition (in the form of part of or the entire prosequence of the parent enzyme, the yeast host cell may be a cell of a species of *Candida, Kluyveromyces, Saccharomyces, Schizosaccharomyces, Pichia*, or *Yarrowia, such as* a *S. cerevisiae* cell, a *S.s carlsbergensis*, a *S. diastaticus* cell, a *S. douglasii* cell, a *S. kluyveri* cell, a *S. norbensis* cell, or a *S. oviformis* cell.

"Fungi" as used herein includes the phyla Ascomycota, Basidiomycota, Chytridiomycota, and Zygomycota (as defined by Hawksworth *et al., In, Ainsworth and Bisby's Dictionary of The* Fungi, 8th edition, 1995, CAB International, University Press, Cambridge, UK) as well as the Oomycota (as cited in *Hawksworth et al*., 1995, *supra*, page 171) and all mitosporic fungi (Hawksworth *et al*., 1995, *supra*). Representative groups of Ascomycota include, *e.g., Neurospora, Eupenicillium* (*=Penicillium*), *Emericella* (*=Aspergillus*), *Eurotium* (*=Aspergillus*), and the true yeasts listed above. Examples of Basidiomycota include mushrooms, rusts, and smuts. Representative groups of Chytridiomycota include, *e. g., Allomyces, Blastocladiella, Coelomomyces*, and aquatic fungi. Representative groups of Oomycota include, *e.g*., Saprolegniomycetous aquatic fungi (water molds) such as *Achlya*. Examples of mitosporic fungi include *Aspergillus, Penicillium, Candida,* and *Alternaria.* Representative groups of Zygomycota include, *e.g., Rhizopus* and *Mucor.*

"Filamentous fungi" include all filamentous forms of the subdivision Eumycota and Oomycota (as defined by Hawksworth *et al*., 1995, *supra*). The filamentous fungi are characterized by a vegetative mycelium composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

In a preferred embodiment, the fungal host cell is a filamentous fungal cell. In a more preferred embodiment, the filamentous fungal host cell is a cell of a species of, but not limited to, *Acremonium, Aspergillus, Fusarium, Humicola, Myceliophthora, Mucor, Neurospora, Penicillium, Thielavia, Tolypocladium*, and *Trichoderma*. In an even more preferred embodiment, the filamentous fungal host cell is an *Aspergillus* cell. In another even more preferred embodiment, the filamentous fungal host cell is a *Fusarium* cell. In a most preferred embodiment, the filamentous fungal host cell is an *A. oryzae* cell, an *A. niger* cell, an *A. foetidus* cell, or an *A. japonicus* cell. In another most preferred embodiment, the filamentous fungal host cell is a *Fusarium oxysporum* cell or a *F. graminearum* cell.

Fungal cells may be transformed by a process involving protoplast formation, transformation of the protoplasts, and regeneration of the cell wall in a manner known *per se.* Suitable procedures for transformation of *Aspergillus* host cells are described in EP 238 023 and Yelton *et al*., 1984, *Proceedings of the National Academy of Sciences USA* 81: 1470-1474. A suitable method of transforming *Fusarium* species is described by Malardier *et al*., 1989, *Gene* 78: 147-156 or in WO 96/00787. Yeast may be transformed using the procedures described by Becker and Guarente, In Abelson, J.N. and Simon, M.I., editors, *Guide to Yeast Genetics and Molecular Biology,* Methods in Enzymology, Volume 194, pp 182-187, Academic Press, Inc., New York; Ito *et al*., 1983, *Journal of Bacteriology* 153:163; and Hinnen *et al*., 1978, *Proceedings of the National Academy of Sciences USA* 75:1920. Mammalian cells may be transformed by direct uptake using the calcium phosphate precipitation method of Graham and Van der Eb (1978, *Virology* 52:546).

**Methods of Production**

The present invention also relates to methods for producing a first wash lipolytic enzyme of the invention comprising (a) cultivating a host cell transformed with a DNA sequence encoding the enzyme under conditions conducive to expression of the first wash lipolytic enzyme; and (b) recovering the first wash lipolytic enzyme.

The host cells may be cultivated in a nutrient medium suitable for production of the first wash lipolytic enzyme using methods known in the art. For example, the cell may be cultivated by shake flask cultivation, small-scale or large-scale fermentation (including continuous, batch, fed-batch, or solid state fermentations) in laboratory or industrial fermentors performed in a suitable medium and under conditions allowing the modified lipolytic enzyme to be expressed and/or isolated. The cultivation takes place in a suitable nutrient medium comprising carbon and nitrogen sources and inorganic salts, using procedures known in the art (see, *e.g*., references for bacteria and yeast; Bennett, J.W. and LaSure, L., editors, *More Gene Manipulations in Fungi,* Academic Press, CA, 1991). Suitable media are available from commercial suppliers or may be prepared according to published compositions (*e.g*., in catalogues of the American Type Culture Collection). If the first wash lipolytic enzyme is secreted into the nutrient medium, the lipolytic enzyme can be recovered directly from the medium. If the first wash lipolytic enzyme is not secreted, it is recovered from cell lysates.

The resulting first wash lipolytic enzyme may be recovered by methods known in the art. For example, the first wash lipolytic enzyme may be recovered from the nutrient medium by conventional procedures including, but not limited to, centrifugation, filtration, extraction, spray-drying, evaporation, or precipitation. The recovered first wash lipolytic enzyme may then be further purified by a variety of chromatographic procedures, *e.g*., ion exchange chromatography, gel filtration chromatography, affinity chromatography, or the like.

The first wash lipolytic enzymes of the present invention may be purified by a variety of procedures known in the art including, but not limited to, chromatography (*e.g*., ion exchange, affinity, hydrophobic, chromatofocusing, and size exclusion), electrophoretic procedures (*e.g*., preparative isoelectric focusing (IEF), differential solubility (*e.g*., ammonium sulfate precipitation), or extraction (see, *e.g., Protein Purification*, J.-C. Janson and Lars Ryden, editors, VCH Publishers, New York, 1989).

In accordance with the invention, it is also contemplated to apply, to the first wash lipolytic lipolytic enzyme,

one or more charged amino acids which permit effective purification of the modified enzyme. Techniques for doing this is well known by a person skilled in the art of molecular biology.

The host cell, cultivation condition and/or recovery conditions are preferably selected so that at the most a partial processing of the pre, pro or preproform of the parent enzyme has occured resulting in that at least 5%, such as at least 10%, such as at least 15%, such as at least 20%, such as at least 25%, such as at least 50 % or at least 75% of the produced modified enzyme molecules comprises the desired , e.g. the entire pre-sequence or a substantial part thereof.

**Enzyme composition of the invention**

In a further aspect the invention relates to an enzyme composition comprising a first wash lipolytic enzyme of the invention.

As defined herein, a "substantially pure" enzyme is an enzyme which is essentially free of other homologous contaminants (originating from the same source as the first wash lipolytic enzyme), *e.g.*, at least about 20% pure, preferably at least about 40% pure, more preferably about 60% pure, even more preferably about 80% pure, most preferably about 90% pure, and even most preferably about 95% pure, as determined by SDS-PAGE.

When the first wash lipolytic enzyme of the invention comprises a peptide addition it has been found that not all of the first wash lipolytic enzyme molecules expressed by a given host cell are processed at the same cleavage site. This has the consequence that the first wash lipolytic enzyme product recovered from the fermentation by such host cells comprise a portion having the full length peptide addition and one or more other portions with only a part of the peptide addition. The inventors found that this does not influence the wash performance significantly. Consequently, even though not all of the lipolytic enzyme of the enzyme composition of the invention may have retained the full length peptide addition the enzyme composition is still capable of exerting a first wash effect. Actually, it has been found that as long as at least about 5 % of the total amount of first wash lipolytic enzyme of the invention containing a peptide addition has the intact peptide addition as disclosed above, this may be found to be sufficient for providing the desired first wash effect. The remaining part of the first wash lipolytic enzyme molecules may then have a peptide addition which is shorter than the one intended (e.g. as a consequence of one or more amino acid residues have been cut off during processing of the enzyme by the host organism).Therefore, the enzyme composition of the invention need only to comprise at least about 5%, preferably at least about 10%, such as at least about 25 %, better at least about 50%, especially at least about 75 % of the first wash lipolytic enzyme with its full length addition.

Said enzyme composition may further comprise an enzyme selected from the group of proteases, cellulases, peroxidases, cutinases, amylases and/or lipases, and when intended for washing also ingredients normally used in detergent compositions.

DETERGENT DISCLOSURE

<u>Surfactant system</u>

The detergent compositions according to the present invention comprise a surfactant system wherein the surfactants can be selected from nonionic and/or anionic and/or cationic and/or ampholytic and/or zwitterionic and/or semi-polar surfactants.

The surfactants are typically present at a level of from 0.1 % to 60 % by weight.

The surfactant is preferably formulated to be compatible with enzyme components present in the composition. In liquid or gel compositions the surfactant is most preferably formulated such that it promotes, or at least does not degrade, the stability of any enzyme in these compositions.

Preferred surfactant systems to be used according to the present invention comprise as a surfactant one or more of the nonionic and/or anionic surfactants described herein.

Nonionic detergent surfactants normally consist of a water-solubilizing polyalkoxylene or a mono- or di-alkanolamide group in chemical combination with an organic hydrophobic group derived, for example, from alkylphenols in which the alkyl group contains from about 6 to about 12 carbon atoms, dialkylphenols in which each alkyl group contains from 6 to 12 carbon atoms, primary, secondary or tertiary aliphatic alcohols (or alkyl-capped derivatives thereof), preferably having from 8 to 20 carbon atoms, monocarboxylic acids having from 10 to about 24 carbon atoms in the alkyl group and polyoxypropylenes. Also common are fatty acid mono- and dialkanolamides in which the alkyl group of the fatty acid radical contains from 10 to about 20 carbon atoms and the alkyloyl group having from 1 to 3 carbon atoms. Optionally, in any of the mono- and di-alkanolamide derivatives there may be a polyoxyalkylene moiety joining the latter groups and the hydrophobic part of the molecule. In all polyalkoxylene containing surfactants, the polyalkoxylene moiety preferably consists of from 2 to 20 groups of ethylene oxide or of ethylene oxide and propylene oxide groups.

Polyethylene, polypropylene, and polybutylene oxide condensates of alkyl phenols are suitable for use as the nonionic surfactant of the surfactant systems of the present invention, with the polyethylene oxide condensates being preferred. These compounds include the condensation products of alkyl phenols having an alkyl group containing from about 6 to about 14 carbon atoms, preferably form about 8 to about 14 carbon atoms, in either a straightchain or branched-chain configuration with the alkylene oxide. In a preferred embodiment, the ethylene oxide is present in an amount equal to from about 2 to about 25 moles, more preferably from about 3 to about 15 moles, of ethylene oxide per mole of alkyl phenol. Commercially available nonionic surfactants of this type include Igepal™ CO-630, marketed by the GAF Corporation; and Triton™ X-45, X-114, X-100 and X-102, all marketed by the Rohm & Haas Company. These surfactants are commonly referred to as alkylphenol alkoxylates (e.g., alkyl phenol ethoxylates).

The condensation products of primary and secondary aliphatic alcohols with from about 1 to about 25 moles of ethylene oxide are suitable for use as the nonionic surfactant of the nonionic surfactant systems of the present invention. The alkyl chain of the aliphatic alcohol can either be straight or branched, primary or secondary, and generally contains from about 8 to about 22 carbon atoms. Preferred are the condensation products of alcohols having an alkyl group containing from about 8 to about 20 carbon atoms, more preferably from about 10 to about 18 carbon atoms, with from about 2 to about 10 moles of ethylene oxide per mole of alcohol. About 2 to about 7 moles of ethylene oxide and most preferably from 2 to 5 moles of ethylene oxide per mole of alcohol are present in said condensation products. Examples of commercially available nonionic surfactants of this type include Tergitol™ 15-S-9 (The condensation product of $C_{11}$-$C_{15}$ linear alcohol with 9 moles ethylene oxide), Tergitol™ 24-L-6 NMW (the condensation product of $C_{12}$-$C_{14}$ primary alcohol with 6 moles ethylene oxide with a narrow molecular weight distribution), both marketed by Union Carbide Corporation; Neodol™ 45-9 (the condensation product of $C_{14}$-$C_{15}$ linear alcohol with 9 moles of ethylene oxide), Neodol™ 23-3 (the condensation product of $C_{12}$-$C_{13}$ linear alcohol with 3.0 moles of ethylene oxide), Neo dol™ 45-7 (the condensation product of $C_{14}$-$C_{15}$ linear alcohol with 7 moles of ethylene oxide), Neodol™ 45-5 (the condensation product of $C_{14}$-$C_{15}$ linear alcohol with 5 moles of ethylene oxide) marketed by Shell Chemical Company, Kyro™ EOB (the condensation product of $C_{13}$-$C_{15}$ alcohol with 9 moles ethylene oxide), marketed by The Procter & Gamble Company, and Genapol LA 050 (the condensation product of $C_{12}$-$C_{14}$ alcohol with 5 moles of ethylene oxide) marketed by Hoechst. Preferred range of HLB in these products is from 8-11 and most preferred from 8-10.

The detergent composition of the invention may comprise a nonionic material which is alkoxylated aliphatic alcohols containing at least 25 alkylene oxide groups, preferably at least 50 alkylene oxide groups, and more preferably at least 80 alkylene oxide groups.

Also useful as the nonionic surfactant of the surfactant systems of the present invention are alkylpolysaccharides disclosed in US 4,565,647, having a hydrophobic group containing from about 6 to about 30 carbon atoms, preferably from about 10 to about 16 carbon atoms and a polysaccharide, e.g. a polyglycoside, hydrophillic group containing from about 1.3 to about 10, preferably from about 1.3 to about 3, most preferably from about 1.3 to about 2.7 saccharide units. Any reducing saccharide containing 5 or 6 carbon atoms can be used, e.g., glucose, galactose and galactosyl moieties can be substituted for the glucosyl moieties (optionally the hydrophobic group is attached at the 2-, 3-, 4-, etc. positions thus giving a glucose or galactose as opposed to a glucoside or galactoside). The intersaccharide bonds can be, e.g., between the one position of the additional saccharide units and the 2-, 3-, 4-, and/or 6- positions on the preceding saccharide units.

Another useful nonionic surfactant is a glycoside of a uronic acid, a uronic acid salt or a uronic acid lactone or polyuronic acid with a straight-chain or branced saturated or unsaturated aliphatic chain of from 6 to 24 carbon atoms, optionally containing an aromatic, cycloaliphatic, mixed aromatic-aliphatic or polyalkyloxyalkyl radical, as described in WO 95/10524.

The preferred alkylpolyglycosides have the formula

$$R^2O(C_nH_{2n}O)_t(glycosyl)_x$$

wherein $R^2$ is selected from the group consisting of alkyl, alkylphenyl, hydroxyalkyl, hydroxyalkylphenyl, and mixtures thereof in which the alkyl groups contain from about 10 to about 18, preferably from about 12 to about 14, carbon atoms; n is 2 or 3, preferably 2; t is from 0 to about 10, preferably 0; and x is from about 1.3 to about 10, preferably from about 1.3 to about 3, most preferably from about 1.3 to about 2.7. The glycosyl is preferably derived from glucose. To prepare these compounds, the alcohol or alkylpoly-ethoxy alcohol is formed first and then reacted with glucose, or a source of glucose, to form the glucoside (attachment at the 1-position). The additional glycosyl units can then be attached between their 1-position and the preceding glycosyl units 2-, 3-, 4-, and/or 6-position, preferably predominately the 2-position.

The condensation products of ethylene oxide with a hydrophobic base formed by the condensation of propylene oxide with propylene glycol are also suitable for use as the additional nonionic surfactant systems of the present invention. The hydrophobic portion of these compounds will preferably have a molecular weight of from about 1500 to

about 1800 and will exhibit water insolubility. The addition of polyoxyethylene moieties to this hydrophobic portion tends to increase the water solubility of the molecule as a whole, and the liquid character of the product is retained up to the point where the polyoxyethylene content is about 50 % of the total weight of the condensation product, which corresponds to condensation with up to about 40 moles of ethylene oxide. Examples of compounds of this type include certain of the commercially available Pluronic™ surfactants, marketed by BASF.

Also suitable for use as the nonionic surfactant of the nonionic surfactant system of the present invention, are the condensation products of ethylene oxide with the product resulting from the reaction of propylene oxide and ethylenediamine. The hydrophobic moiety of these products consists of the reaction product of ethylenediamine and excess propylene oxide, and generally has a molecular weight of from about 2500 to about 3000. This hydrophobic moiety is condensed with ethylene oxide to the extent that the condensation product contains from about 40% to about 80% by weight of polyoxyethylene and has a molecular weight of from about 5,000 to about 11.000. Examples of this type of nonionic surfactant include certain of the commercially available Tetronic™ compounds, marketed by BASF.

Preferred for use as the nonionic surfactant of the surfactant systems of the present invention are polyethylene oxide condensates of alkyl phenols, condensation products of primary and secondary aliphatic alcohols with from about 1 to about 25 moles of ethyleneoxide, alkylpolysaccharides, and mixtures hereof. Most preferred are $C_8$-$C_{14}$ alkyl phenol ethoxylates having from 3 to 15 ethoxy groups and $C_8$-$C_{18}$ alcohol ethoxylates (preferably $C_{10}$ avg.) having from 2 to 10 ethoxy groups, and mixtures thereof. Highly preferred nonionic surfactants are polyhydroxy fatty acid amide surfactants of the formula

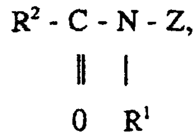

wherein $R^1$ is H, or $R^1$ is $C_{1-4}$ hydrocarbyl, 2-hydroxy ethyl, 2-hydroxy propyl or a mixture thereof, $R^2$ is $C_{5-31}$ hydrocarbyl, and Z is a polyhydroxyhydrocarbyl having a linear hydrocarbyl chain with at least 3 hydroxyls directly connected to the chain, or an alkoxylated derivative thereof. Preferably, $R^1$ is methyl, $R^2$ is straight $C_{11-15}$ alkyl or $C_{16-18}$ alkyl or alkenyl chain such as coconut alkyl or mixtures thereof, and Z is derived from a reducing sugar such as glucose, fructose, maltose, lactose, in a reductive amination reaction.

A nonionic surfactant system comprising an alkoxylated nonionic surfactant having an average degree of alkoxylation of at least 6 and an aldobionamide of the structure $ANR_1R_2$ wherein A is a sugar moiety which is an aldobionic acid except that it does not contain the OH group normally extending from the carbonyl group on the aldonic acid. $NR_1R_2$ is attached where the hydroxyl group on the aldobionic acid would normally be found. $R_1R_2$, may be the same or different, is a hydrogen atom, an aliphatic hydrocarbon radical, an aromatic radical a cycloaliphatic radical an amino acid ester, or an ether amine. $R_1$ and $R_2$ cannot both be hydrogen atoms as described in WO 95/2770.

Other so-called nonionic detergent compounds include long chain tertiary amine oxides, long chain tertiary phosphine oxides and dialkyl sulphoxides.

Highly preferred anionic surfactants include alkyl alkoxylated sulfate surfactants hereof are water soluble salts or acids of the formula $RO(A)_mSO_3M$ wherein R is an unsubstituted $C_{10}$-$C$-$_{24}$ alkyl or hydroxyalkyl group having a $C_{10}$-$C_{24}$ alkyl component, preferably a $C_{12}$-$C_{20}$ alkyl or hydroxyalkyl, more preferably $C_{12}$-$C_{18}$ alkyl or hydroxyalkyl, A is an ethoxy or propoxy unit, m is greater than zero, typically between about 0.5 and about 6, more preferably between about 0.5 and about 3, and M is H or a cation which can be, for example, a metal cation (e.g., sodium, potassium, lithium, calcium, magnesium, etc.), ammonium or substituted-ammonium cation. Alkyl ethoxylated sulfates as well as alkyl propoxylated sulfates are contemplated herein. Specific examples of substituted ammonium cations include methyl-, dimethyl, trimethyl-ammonium cations and quaternary ammonium cations such as tetramethyl-ammonium and dimethyl piperdinium cations and those derived from alkylamines such as ethylamine, diethylamine, triethylamine, mixtures thereof, and the like. Exemplary surfactants are $C_{12}$-$C_{18}$ alkyl polyethoxylate (1.0) sulfate ($C_{12}$-$C_{18}E(1.0)M$), $C_{12}$-$C_{18}$ alkyl polyethoxylate (2.25) sulfate ($C_{12}$-$C_{18}(2.25)M$, and $C_{12}$-$C_{18}$ alkyl polyethoxylate (3.0) sulfate ($C_{12}$-$C_{18}E(3.0)M$), and $C_{12}$-$C_{18}$ alkyl polyethoxylate (4.0) sulfate ($C_{12}$-$C_{18}E(4.0)M$), wherein M is conveniently selected from sodium and potassium. Suitable anionic surfactants to be used are alkyl ester sulfonate surfactants including linear esters of $C_8$-$C_{20}$ carboxylic acids (i.e., fatty acids) which are sulfonated with gaseous $SO_3$ according to "The Journal of the American Oil Chemists Society", 52 (1975), pp. 323-329. Suitable starting materials would include natural fatty substances as derived from tallow, palm oil, etc.

The preferred alkyl ester sulfonate surfactant, especially for laundry applications, comprise alkyl ester sulfonate surfactants of the structural formula:

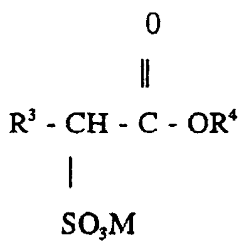

wherein $R^3$ is a $C_8$-$C_{20}$ hydrocarbyl, preferably an alkyl, or combination thereof, $R^4$ is a $C_1$-$C_6$ hydrocarbyl, preferably an alkyl, or combination thereof, and M is a cation which forms a water soluble salt with the alkyl ester sulfonate. Suitable salt-forming cations include metals such as sodium, potassium, and lithium, and substituted or unsubstituted ammonium cations, such as monoethanolamine, diethonolamine, and triethanolamine. Preferably, $R^3$ is $C_{10}$-$C_{16}$ alkyl, and $R^4$ is methyl, ethyl or isopropyl. Especially preferred are the methyl ester sulfonates wherein $R^3$ is $C_{10}$-$C_{16}$ alkyl.

Other suitable anionic surfactants include the alkyl sulfate surfactants which are water soluble salts or acids of the formula $ROSO_3M$ wherein R preferably is a $C_{10}$-$C_{24}$ hydrocarbyl, preferably an alkyl or hydroxyalkyl having a $C_{10}$-$C_{20}$ alkyl component, more preferably a $C_{12}$-$C_{18}$ alkyl or hydroxyalkyl, and M is H or a cation, e.g., an alkali metal cation (e.g. sodium, potassium, lithium), or ammonium or substituted ammonium (e.g. methyl-, dimethyl-, and trimethyl ammonium cations and quaternary ammonium cations such as tetramethyl-ammonium and dimethyl piperdinium cations and quaternary ammonium cations derived from alkylamines such as ethylamine, diethylamine, triethylamine, and mixtures thereof, and the like). Typically, alkyl chains of $C_{12}$-$C_{16}$ are preferred for lower wash temperatures (e.g. below about 50°C) and $C_{16}$-$C_{18}$ alkyl chains are preferred for higher wash temperatures (e.g. above about 50°C).

Other anionic surfactants useful for detersive purposes can also be included in the laundry detergent compositions of the present invention. Theses can include salts (including, for example, sodium, potassium, ammonium, and substituted ammonium salts such as mono- di- and triethanolamine salts) of soap, $C_8$-$C_{22}$ primary or secondary alkanesulfonates, $C_8$-$C_{24}$ olefinsulfonates, sulfonated polycarboxylic acids prepared by sulfonation of the pyrolyzed product of alkaline earth metal citrates, e.g., as described in GB 1,082,179, $C_8$-$C_{24}$ alkylpolyglycolethersulfates (containing up to 10 moles of ethylene oxide); alkyl glycerol sulfonates, fatty acyl glycerol sulfonates, fatty oleyl glycerol sulfates, alkyl phenol ethylene oxide ether sulfates, paraffin sulfonates, alkyl phosphates, isethionates such as the acyl isethionates, isethionates esters of alkoxy carboxylic acids (as described in WO95/14661), N-acyl taurates, alkyl succinamates and sulfosuccinates, monoesters of sulfosuccinates (especially saturated and unsaturated $C_{12}$-$C_{18}$ monoesters) and diesters of sulfosuccinates (especially saturated and unsaturated $C_6$-$C_{12}$ diesters), acyl sarcosinates, oleoyl sarcosinate, sulfates of alkylpolysaccharides such as the sulfates of alkylpolyglucoside (the nonionic nonsulfated compounds being described below), branched primary alkyl sulfates, and alkyl polyethoxy carboxylates such as those of the formula $RO(CH_2CH_2O)_k$-$CH_2COO$-M+ wherein R is a $C_8$-$C_{22}$ alkyl, k is an integer from 1 to 10, and M is a soluble salt forming cation. Resin acids and hydrogenated resin acids are also suitable, such as rosin, hydrogenated rosin, and resin acids and hydrogenated resin acids present in or derived from tall oil. Alkylbenzene sulfonate is highly preferred, in particular linear alkyl benzene sulfonate (LAS) where the alkyl group contains preferably from 10 to 18 carbon atoms.

Further examples are described in "Surface Active Agents and Detergents" (Vol. I and II by Schwartz, Perrry and Berch). A variety of such surfactants are also generally disclosed in US 3,929,678, (Column 23, line 58 through Column 29, line 23).

When included therein, the laundry detergent compositions of the present invention typically comprise from about 1 % to about 40%, preferably from about 3% to about 20 % by weight of such anionic surfactants.

The laundry detergent compositions of the present invention may also contain cationic, ampholytic, zwitterionic, and semi-polar surfactants, as well as the nonionic and/or anionic surfactants other than those already described herein.

Cationic detersive surfactants suitable for use in the laundry detergent compositions of the present invention are those having one long-chain hydrocarbyl group. Examples of such cationic surfactants include the ammonium surfactants such as alkyltrimethylammonium halogenides, and those surfactants having the formula:

$$[R^2(OR^3)_y][R^4(OR^3)_y]_2R^5N+X-$$

wherein $R^2$ is an alkyl or alkyl benzyl group having from about 8 to about 18 carbon atoms in the alkyl chain, each $R^3$ is selected form the group consisting of $-CH_2CH_2-$, $-CH_2CH(CH_3)-$, $-CH_2CH(CH_2OH)-$, $-CH_2CH_2CH_2-$, and mixtures thereof; each $R^4$ is selected from the group consisting of $C_1$-$C_4$ alkyl, $C_1$-$C_4$ hydroxyalkyl, benzyl ring structures formed

by joining the two $R^4$ groups, $-CH_2CHOHCHOHCOR^6CHOHCH_2OH$ wherein $R^6$ is any hexose or hexose polymer having a molecular weight less than about 1000, and hydrogen when y is not 0; $R^5$ is the same as $R^4$ or is an alkyl chain wherein the total number of carbon atoms or $R^2$ plus $R^5$ is not more than about 18; each y is from 0 to about 10 and the sum of the y values is form 0 to about 15; and X is any compatible anion.

Highly preferred cationic surfactants are the water soluble quaternary ammonium compounds useful in the present composition having the formula:

$$R_1R_2R_3R_4N^+X^- \qquad (i)$$

wherein $R_1$ is $C_8$-$C_{16}$ alkyl, each of $R_2$, $R_3$ and $R_4$ is independently $C_1$-$C_4$ alkyl, $C_1$-$C_4$ hydroxy alkyl, benzyl, and $-(C_2H_{40})_xH$ where x has a value from 2 to 5, and X is an anion. Not more than one of $R_2$, $R_3$ or $R_4$ should be benzyl.

The preferred alkyl chain length for $R_1$ is $C_{12}$-$C_{15}$ particularly where the alkyl group is a mixture of chain lengths derived from coconut or palm kernel fat or is derived synthetically by olefin build up or OXO alcohols synthesis.

Preferred groups for $R_2R_3$ and $R_4$ are methyl and hydroxyethyl groups and the anion X may be selected from halide, methosulphate, acetate and phosphate ions. Examples of suitable quaternary ammonium compounds of formulae (i) for use herein are:

coconut trimethyl ammonium chloride or bromide;
coconut methyl dihydroxyethyl ammonium chloride or bromide;
decyl triethyl ammonium chloride;
decyl dimethyl hydroxyethyl ammonium chloride or bromide;
$C_{12-15}$ dimethyl hydroxyethyl ammonium chloride or bromide;
coconut dimethyl hydroxyethyl ammonium chloride or bromide;
myristyl trimethyl ammonium methyl sulphate;
lauryl dimethyl benzyl ammonium chloride or bromide;
lauryl dimethyl (ethenoxy)$_4$ ammonium chloride or bromide;
choline esters (compounds of formula (i) wherein $R_1$ is

$$CH_2\text{-}CH_2\text{-}O\text{-}\underset{\underset{O}{\|}}{C}\text{-}C_{12\text{-}14}\ \text{alkyl and}\ R_2R_3R_4\ \text{are methyl).}$$

di-alkyl imidazolines [compounds of formula (i)].

Other cationic surfactants useful herein are also described in US 4,228,044 and in EP 000 224.

When included therein, the laundry detergent compositions of the present invention typically comprise from 0.2 % to about 25%, preferably from about 1% to about 8% by weight of such cationic surfactants.

Ampholytic surfactants are also suitable for use in the laundry detergent compositions of the present invention. These surfactants can be broadly described as aliphatic derivatives of secondary or tertiary amines, or aliphatic derivatives of heterocyclic secondary and tertiary amines in which the aliphatic radical can be straight- or branched-chain. One of the aliphatic substituents contains at least about 8 carbon atoms, typically from about to about 18 carbon atoms, and at least one contains an anionic water-solubilizing group, e.g. carboxy, sulfonate, sulfate. See US 3,929,678 (column 19, lines 18-35) for examples of ampholytic surfactants.

When included therein, the laundry detergent compositions of the present invention typically comprise from 0.2% to about 15%, preferably from about 1% to about 10% by weight of such ampholytic surfactants.

Zwitterionic surfactants are also suitable for use in laundry detergent compositions. These surfactants can be broadly described as derivatives of secondary and tertiary amines, derivatives of heterocyclic secondary and tertiary amines, or derivatives of quaternary ammonium, quaternary phosphonium or tertiary sulfonium compounds. See US 3,929,678 (column 19, line 38 through column 22, line 48) for examples of zwitterionic surfactants.

When included therein, the laundry detergent compositions of the present invention typically comprise from 0.2% to about 15%, preferably from about 1% to about 10% by weight of such zwitterionic surfactants.

Semi-polar nonionic surfactants are a special category of nonionic surfactants which include water-soluble amine oxides containing one alkyl moiety of from about 10 to about 18 carbon atoms and 2 moieties selected from the

group consisting of alkyl groups and hydroxyalkyl groups containing from about 1 to about 3 carbon atoms; watersoluble phosphine oxides containing one alkyl moiety of form about 10 to about 18 carbon atoms and 2 moieties selected from the group consisting of alkyl groups and hydroxyalkyl groups containing from about 1 to about 3 carbon atoms; and water-soluble sulfoxides containing one alkyl moiety of from about 10 to about 18 carbon atoms and a moiety selected from the group consisting of alkyl and hydroxyalkyl moieties of from about 1 to about 3 carbon atoms.

Semi-polar nonionic detergent surfactants include the amine oxide surfactants having the formula:

$$\overset{\displaystyle O}{\overset{|}{R^3(OR^4)xN(R^5)2}}$$

wherein $R^3$ is an alkyl, hydroxyalkyl, or alkyl phenyl group or mixtures thereof containing from about 8 to about 22 carbon atoms; $R^4$ is an alkylene or hydroxyalkylene group containing from about 2 to about 3 carbon atoms or mixtures thereof; x is from 0 to about 3: and each $R^5$ is an alkyl or hydroxyalkyl group containing from about 1 to about 3 carbon atoms or a polyethylene oxide group containing from about 1 to about 3 ethylene oxide groups. The $R^5$ groups can be attached to each other, e.g., through an oxygen or nitrogen atom, to form a ring structure.

These amine oxide surfactants in particular include $C_{10}$-$C_{18}$ alkyl dimethyl amine oxides and $C_8$-$C_{12}$ alkoxy ethyl dihydroxy ethyl amine oxides.

When included therein, the laundry detergent compositions of the present invention typically comprise from 0.2% to about 15%, preferably from about 1% to about 10% by weight of such semi-polar nonionic surfactants.

Builder system

The compositions according to the present invention may further comprise a builder system. Any conventional builder system is suitable for use herein including aluminosilicate materials, silicates, polycarboxylates and fatty acids, materials such as ethylenediamine tetraacetate, metal ion sequestrants such as aminopolyphosphonates, particularly ethylenediamine tetramethylene phosphonic acid and diethylene triamine pentamethyle-nephosphonic acid. Phosphate builders can also be used herein, e.g pyrophosphates, orthophosphates or polyphosphates.

Suitable builders can be an inorganic ion exchange material, commonly an inorganic hydrated aluminosilicate material, more particularly a hydrated synthetic zeolite such as hydrated zeolite A, X, B, HS or MAP. Another suitable inorganic builder material is layered silicate, e.g. SKS-6 (Hoechst). SKS-6 is a crystalline layered silicate consisting of sodium silicate ($Na_2Si_2O_5$).

Suitable polycarboxylates containing one carboxy group include lactic acid, glycolic acid and ether derivatives thereof as disclosed in BE 831,368, BE 821,369 and BE 821,370. Polycarboxylates containing two carboxy groups include the water-soluble salts of succinic acid, malonic acid, (ethylenedioxy) diacetic acid, maleic acid, diglycollic acid, tartaric acid, tartronic acid and fumaric acid, as well as the ether carboxylates described in DE 2,446,686, DE 2,446,487, US 3,935,257 and the sulfinyl carboxylates described in BE 840,623. Polycarboxylates containing three carboxy groups include, in particular, water-soluble citrates, aconitrates and citraconates as well as succinate derivatives such as the carboxymethyloxysuccinates described in GB 1,379,241, lactoxysuccinates described in NL application 7205873, and the oxypolycarboxylate materials such as 2-oxa-1,1,3-propane tricarboxylates described in GB 1,387,447.

Polycarboxylates containing four carboxy groups include oxydisuccinates disclosed in GB 1,261,829, 1,1,2,2,-ethane tetracarboxylates, 1,1,3,3-propane tetracarboxylates containing sulfo substituents include the sulfosuccinate derivatives disclosed in GB 1,398,421 and GB 1,398,422 and in US 3,936,448, and the sulfonated pyrolysed citrates described in GB 1,082,179, while polycarboxylates containing phosphone substituents are disclosed in GB 1,439,000.

Alicyclic and heterocyclic polycarboxylates include cis,cis-cis-cyclopentane-tetracarboxylates, pentacarboxylatocyclopentadiene, cis, cis, cis-tetrahydrofuran-tetra-carboxylates, cis-tetrahydrofuran-2,5-dicarboxylates, tetrahydrofuran-2,2,5,5,-tetracarboxylates, hexane-1,2,3,4,5,6-hexacarboxylates and carboxymethyl derivatives of polyhydric alcohols such as sorbitol, mannitol and xylitol. Aromatic polycarboxylates include mellitic acid, pyromellitic acid and the phthalic acid derivatives disclosed in GB 1,425,343. Of the above, the preferred polycarboxylates are hydroxycarboxylates containing up to three carboxy groups per molecule, more particularly citrates.

Preferred builder systems for use in the present compositions include a mixture of a water-insoluble aluminosilicate builder such as zeolite A or of a layered silicate (SKS-6), and a water-soluble carboxylate chelating agent such as citric acid.

A suitable chelant for inclusion in the detergent compositions in accordance with the invention is ethylenediamine-N,N'-disuccinic acid (EDDS) or the alkali metal, alkaline earth metal, ammonium, or substituted ammonium salts thereof, or mixtures thereof. Preferred EDDS compounds are the free acid form and the sodium or magnesium salt

thereof. Examples of such preferred sodium salts of EDDS include $Na_2EDDS$ and $Na_4EDDS$. Examples of such preferred magnesium salts of EDDS include MgEDDS and $Mg_2EDDS$. The magnesium salts are the most preferred for inclusion in compositions in accordance with the invention.

Preferred builder systems include a mixture of a water-insoluble aluminosilicate builder such as zeolite A, and a water soluble carboxylate chelating agent such as citric acid.

Other builder materials that can form part of the builder system for use in granular compositions include inorganic materials such as alkali metal carbonates, bicarbonates, silicates, and organic materials such as the organic phosphonates, amino polyalkylene phosphonates and amino polycarboxylates.

Other suitable water-soluble organic salts are the homo- or co-polymeric acids or their salts, in which the polycarboxylic acid comprises at least two carboxyl radicals separated form each other by not more than two carbon atoms.

Polymers of this type are disclosed in GB-A-1,596,756. Examples of such salts are polyacrylates of MW 2000-5000 and their copolymers with maleic anhydride, such copolymers having a molecular weight of from 20,000 to 70,000, especially about 40,000.

Detergency builder salts are normally included in amounts of from 5% to 80% by weight of the composition. Preferred levels of builder for liquid detergents are from 5% to 30%.

Enzymes

Preferred detergent compositions, in addition to the enzyme of the invention, comprise other enzyme(s) which provides cleaning performance and/or fabric care benefits. Such enzymes include proteases, lipases, cutinases, amylases, cellulases, peroxidases, oxidases (e.g. laccases).

Proteases: Any protease suitable for use in alkaline solutions can be used. Suitable proteases include those of animal, vegetable or microbial origin. Microbial origin is preferred. Chemically or genetically modified mutants are included. The protease may be a serine protease, preferably an alkaline microbial protease or a trypsin-like protease. Examples of alkaline proteases are subtilisins, especially those derived from Bacillus, e.g., subtilisin Novo, subtilisin Carlsberg, subtilisin 309, subtilisin 147 and subtilisin 168 (described in WO 89/06279). Examples of trypsin-like proteases are trypsin (e.g. of porcine or bovine origin) and the Fusarium protease described in WO 89/06270. Preferred commercially available protease enzymes include those sold under the tradenames Alcalase, Savinase, Primase, Durazym, and Esperase by Novo Nordisk A/S (Denmark), those sold under the tradename Maxatase, Maxacal, Maxapem and Properase by Gist-Brocades/Genencor, those sold under the tradename Purafect and Purafect OXP by Genencor International, and those sold under the tradename Opticlean and Optimase by Solvay Enzymes. Protease enzymes may be incorporated into the compositions in accordance with the invention at a level of from 0.0001 % to 2 % of enzyme protein by weight of the composition, in particular at a level of from 0.001 % to 0.1% of enzyme protein by weight of the composition.

Lipases: Any lipase suitable for use in alkaline solutions can be used. Suitable lipases include those of bacterial or fungal origin and chemically or genetically modified lipase mutants.

Examples of useful lipases include a Humicola lanuginosa lipase, e.g., as described in EP 258 068 and EP 305 216, and mutants thereof as described in WO 92/05249, WO 94/25577 and WO 95/22615 a Rhizomucor miehei lipase, e.g., as described in EP 238 023, a Candida lipase, such as a C. antarctica lipase, e.g., the C. antarctica lipase A or B described in EP 214 761, a Pseudomonas lipase such as a P. alcaligenes and P. pseudoalcaligenes lipase, e. g., as described in EP 218 272, or any mutant of said *Pseudomonas* lipases, a P. cepacia lipase, e.g., as described in EP 331 376, a P. stutzeri lipase, e.g., as disclosed in BP 1,372,034, a P. fluorescens lipase, a Bacillus lipase, e.g., a B. subtilis lipase (Dartois et al., (1993), Biochemica et Biophysica acta 1131, 253-260), a B. stearothermophilus lipase (JP 64/744992) and a B. pumilus lipase (WO 91/16422). Furthermore, a number of cloned lipases may be useful, including the Penicillium camembertii lipase described by Yamaguchi et al., (1991), Gene 103, 61-67), the Geotricum candidum lipase (Schimada, Y. et al., (1989), J. Biochem., 106, 383-388), and various Rhizopus lipases such as a R. delemar lipase (Hass, M.J et al., (1991), Gene 109, 117-113), a R. niveus lipase (Kugimiya et al., (1992), Biosci. Biotech. Biochem. 56, 716-719) and a R. oryzae lipase.

Other types of lipolytic enzymes such as cutinases may also be useful, e.g., a cutinase derived from Pseudomonas mendocina as described in WO 88/09367, or a cutinase derived from Fusarium solani pisi (e.g. described in WO 90/09446). Especially suitable lipases are lipases such as M1 Lipase™, Luma fast™ and Lipomax™ (Gist-Brocades/Genencor), Lipolase™ and Lipolase Ultra™ (Novo Nordisk A/S), and Lipase P "Amano" (Amano Pharmaceutical Co. Ltd.).

The lipases are normally incorporated in the detergent composition at a level of from 0.0001 % to 2 % of enzyme protein by weight of the detergent composition, in particular at a level of from 0.001 % to 0.1% of enzyme protein by weight of the composition. Amylases: Any amylase (a and/or b) suitable for use in alkaline solutions can be used. Suitable amylases include those of bacterial or fungal origin. Chemically or genetically modified mutants are

included. Amylases include, for example, a-amylases obtained from a special strain of B. licheniformis, described in more detail in GB 1,296,839. Commercially available amylases are Duramyl™, Termamyl™, Fungamyl™ and BAN™ (available from Novo Nordisk A/S) and Rapidase™ and Maxamyl P™ (available from (Gist-Brocades/Genencor).

The amylases are normally incorporated in the detergent composition at a level of from 0.0001 % to 2% of enzyme protein by weight of the detergent composition, in particular at a level of from 0.001 % to 0.1 % of enzyme protein by weight of the composition. Cellulases: Any cellulase suitable for use in alkaline solutions can be used. Suitable cellulases include those of bacterial or fungal origin. Chemically or genetically modified mutants are included. Suitable cellulases are disclosed in US 4,435,307, which discloses fungal cellulases produced from Humicola insolens. Especially suitable cellulases are the cellulases having color care benefits. Examples of such cellulases are cellulases described in published European patent application No. 0495257. Commercially available cellulases is Celluzyme® produced by a strain of Humicola insolens, (Novo Nordisk A/S), and KAC-500(B)® (Kao Corporation).

Said cellulases are normally incorporated in the detergent composition at a level of from 0.0001 % to 2 % of enzyme protein by weight of the detergent composition, in particular at a level of from 0.001 % to 0.1% of enzyme protein by weight of the composition.

Peroxidases/Oxidases: Peroxidase and/or oxidase enzymes, such as laccase, may be used, the former in combination with hydrogen peroxide sources, e.g., percarbonate, perborate, or persulfate, for solution bleaching, i.e. to prevent transfer of textile dyes from dyed fabrics to other fabrics when said fabrics are washed together in a wash liquor, preferably together with an enhancing agent as described in e.g. WO 94/12621 and WO 95/01426. Suitable enzymes of these types include those of plant, bacterial or fungal origin. Chemically or genetically modified mutants are included.

Said peroxidase and/or oxidase enzymes may be incorporated in the detergent composition at a level of from 0.0001% to 2 % of enzyme protein by weight of the detergent composition, in particular at a level of from 0.001 % to 0.1% of enzyme protein by weight of the composition.

Mixtures of the above mentioned enzymes are encompassed herein, in particular a mixture of a protease, an amylase, a lipase and/or a cellulase.

Optional detergent ingredients

Bleaching agents: Additional optional detergent ingredients that can be included in the detergent compositions of the present invention include peroxide bleaching agents such as sodium perborate-water (1/1), PB1, sodium perborate-water (1/4), PB4 and sodium carbonate-hydrogen peroxide 2/3, percarbonate, with a particle size of 400-800 microns. If included, peroxide bleaching agents will typically be present at levels of from about 1% to about 25%.

Other peroxide-based bleaching agents that can be used are percarboxylic acids and salts thereof. Suitable examples of this class of agents include magnesium monoperoxyphthalate hexahydrate, the magnesium salt of meta-chloro-perbenzoic acid, 4-nonylamino-4-oxoperoxybutyric acid and diperoxydodecanedioic acid. Such bleaching agents are disclosed in US 4,483,781, US 740,446, EP 0 133 354 and US 4,412,934. Highly preferred bleaching agents also include 6-nonylamino-6-oxoperoxycaproic acid as described in US 4,634,551.

Bleaching agents for use herein may also be other bleaching agents known in the art for use in detergent compositions.

A non-aqeous liquid detergent composition may comprise a peroxyacid material e.g peroxyacids consisting of N,N'-Di(4-Percarboxybenzoyl)ethylenediamine (PCBED), N,N'-Terephthaloyl-di(6-aminopercarboxycaproic acid) (TPCAP), N,N'-Di(4-percarboxybenzoyl)piperazine (PCBPIP), N,N'-Di(4-Percarboxybenzoyl)-1,4-diaminocyclohexane (PCBHEX), N,N'-Di(4-Percarboxybenzoyl)-1,4.butanediamine (PCBBD), N,N'-Di(4-Percarboxyaniline)-terephthalate (DPCAT), N,N,N'N'-1,2,4,5-tetracarboxybenzoyl-di(6-aminopercarboxycaproic acid) (DiPAP), N,N'-Di(percarboxyadipoyl)phenylenediamine (DPAPD), N,N'-Succinoyl-di(4-percarboxy)aniline (SDPCA), $C_3$ analogue of N,N'-Terephthaloyl-di-(8-amino peroxyoctanoic acid) (TPOCT) as described in WO 95/06104.

Another such category of bleaching agents that can be used encompasses the halogen bleaching agents. Examples of hypohalite-releasing bleaching agents, for example, include trichloro-isocyanuric acid and the sodium and potassium dichloroisocyanurates and N-chloro and N-bromo alkanesulfonamides. Such materials are normally added at 0.5-10% by weight of the finished product, preferably 1-5% by weight.

A further type of non-peroxidase based bleaching agent of particular interest includes photoactivated bleaching agents such as the sulfonated zinc and/or aluminum phthalocyanines. These materials can be deposited upon the substrate during the washing process. Upon irradiation with light, in the presence of oxygen, such as by hanging clothes out to dry in the daylight, the sulfonated phthalocyanine complex is activated and, consequently, the substrate is bleached. Preferred zinc phthalocyanine complexes and a photoactivated bleaching process are described in US 4,033,718. Typically, detergent compositions will contain about 0.025% to about 1.25%, by weight, of sulfonated zinc phthalocyanine.

The peroxide-based bleaching agents can be used in combination with bleach activators such as

tetraacetylethylenediamine (TAED), nonanoyloxybenzenesulfonate (NOBS, described in US 4,412,934), 3,5,5-trimethylhexanoyloxybenzenesulfonate (ISONOBS, described in EP 120 591) or pentaacetylglucose (PAG), which are perhydrolyzed to form a peracid as the active bleaching species, leading to improved bleaching effect. In addition, very suitable are the bleach activators 6-octanamidocaproyloxybenzenesulfonate, 6-nonanamidocaproyl)oxybenzenesulfonate and 6-decanamidocaproyloxybenzenesulfonate or mixtures thereof. Also suitable activators are acylated citrate esters such as disclosed in European Patent Application No. 91870207.7.

Further useful bleaching agents, including peroxyacids and bleaching systems comprising bleach activators and peroxygen bleaching compounds for use in cleaning compositions according to the invention, are described in application USSN 08/136,626.

Hydrogen peroxide may also be generated in the detergent solution by adding an enzymatic system (i.e. an enzyme and a substrate therefore) which is capable of generation of hydrogen peroxide at the beginning of or during the washing and/or rinsing process. Such enzymatic systems are disclosed in published EP Patent Application No 0537381.

The release of peracid from a peroxygen bleach source may be activated by use of a lipase of the invention. The necessary components for the enzymatic hydrolysis system is the peracid precursor substrate: a diacyl peroxide $R_1$-CO-O-O-CO-R, where R and $R_1$ may be saturated or unsaturated alkyl, an aryl group or an alkaryl. The lipase reacts with the substrate and releases peracids in the wash.

Imine quaternary salts may used as bleach catalysts together with a peroxygen compound as described in WO 95/13352.

Peroxide-based bleaching systems may also comprise a manganese catalyst. The manganese catalyst may, e.g., be one of the compounds described in "Efficient manganese catalysts for low-temperature bleaching", Nature 369, 1994, pp. 637-639.

Suds suppressors: Another optional ingredient is a suds suppressor, exemplified by silicones, and silica-silicone mixtures. Silicones can be generally represented by alkylated polysiloxane materials while silica is normally used in finely divided forms exemplified by silica aerogels and xerogels and hydrophobic silicas of various types. Theses materials can be incorporated as particulates in which the suds suppressor is advantageously releasably incorporated in a water-soluble or waterdispersible, substantially non surface-active detergent impermeable carrier. Alternatively the suds suppressor can be dissolved or dispersed in a liquid carrier and applied by spraying on to one or more of the other components.

A preferred silicone suds controlling agent is disclosed in US 3,933,672. Other particularly useful suds suppressors are the self-emulsifying silicone suds suppressors, described in DE 2,646,126. An example of such a compound is DC-544, commercially available form Dow Corning, which is a siloxane-glycol copolymer. Especially preferred suds controlling agent are the suds suppressor system comprising a mixture of silicone oils and 2-alkyl-alkanols. Suitable 2-alkyl-alkanols are 2-butyl-octanol which are commercially available under the trade name Isofol 12 R.

Such suds suppressor system are described in published European Patent Application No. 0593841.

Especially preferred silicone suds controlling agents are described in published European Patent Application No. 0573699. Said compositions can comprise a silicone/silica mixture in combination with fumed nonporous silica such as Aerosil$^R$.

The suds suppressors described above are normally employed at levels of from 0.001 % to 2% by weight of the composition, preferably from 0.01% to 1% by weight.

Other components: Other components used in detergent compositions may be employed such as soil-suspending agents, soil-releasing agents, optical brighteners, abrasives, bactericides, tarnish inhibitors, coloring agents, and/or encapsulated or nonencapsulated perfumes.

Especially suitable encapsulating materials are water soluble capsules which consist of a matrix of polysaccharide and polyhydroxy compounds such as described in GB 1,464,616.

Other suitable water soluble encapsulating materials comprise dextrins derived from ungelatinized starch acid esters of substituted dicarboxylic acids such as described in US 3,455,838. These acid-ester dextrins are, preferably, prepared from such starches as waxy maize, waxy sorghum, sago, tapioca and potato. Suitable examples of said encapsulation materials include N-Lok manufactured by National Starch. The N-Lok encapsulating material consists of a modified maize starch and glucose. The starch is modified by adding monofunctional substituted groups such as octenyl succinic acid anhydride.

Antiredeposition and soil suspension agents suitable herein include cellulose derivatives such as methylcellulose, carboxymethylcellulose and hydroxyethylcellulose, and homo- or co-polymeric polycarboxylic acids or their salts. Polymers of this type include the polyacrylates and maleic anhydride-acrylic acid copolymers, e.g Sokalan CP5, previously mentioned as builders, as well as copolymers of maleic anhydride with ethylene, methylvinyl ether or methacrylic acid, the maleic anhydride constituting at least 20 mole percent of the copolymer. These materials are normally used at levels of from 0.5% to 10% by weight, more preferably form 0.75% to 8%, most preferably from 1% to 6% by weight of the composition.

Preferred optical brighteners are anionic in character, examples of which are disodium 4,4'-bis-(2-diethanolamino-4-anilino -s- triazin-6-ylamino)stilbene-2:2' disulphonate, disodium 4, - 4'-bis-(2-morpholino-4-anilino-s-triazin-6-ylamino-stilbene-2:2' - disulphonate, disodium 4,4' - bis-(2,4-dianilino-s-triazin-6-ylamino)stilbene-2:2' - disulphonate, monosodium 4',4" - bis-(2,4-dianilino-s-tri-azin-6 ylamino)stilbene-2-sulphonate, disodium 4,4' -bis-(2-anilino-4-(N-methyl-N-2-hydroxyethylamino)-s-triazin-6-ylamino)stilbene-2,2' - disulphonate, di-sodium 4,4' -bis-(4-phenyl-2,1,3-triazol-2-yl)-stilbene-2,2' disulphonate, di-so-dium 4,4'bis(2-anilino-4-(1-methyl-2-hydroxyethylamino)-s-triazin-6-ylami-no)stilbene-2,2'disulphonate, sodium 2(stilbyl-4"-(naphtho-1',2':4,5)-1,2,3, - triazole-2"-sulphonate and 4,4'-bis (2-sulphostyryl)biphenyl.

Other useful polymeric materials are the polyethylene glycols, particularly those of molecular weight 1000-10000, more particularly 2000 to 8000 and most preferably about 4000. These are used at levels of from 0.20% to 5% more preferably from 0.25% to 2.5% by weight. These polymers and the previously mentioned homo- or co-polymeric polycarboxylate salts are valuable for improving whiteness maintenance, fabric ash deposition, and cleaning performance on clay, proteinaceous and oxidizable soils in the presence of transition metal impurities.

A graft polymer as described in WO 95/22593 may also be used.

Soil release agents useful in compositions of the present invention are conventionally copolymers or terpolymers of terephthalic acid with ethylene glycol and/or propylene glycol units in various arrangements. Examples of such polymers are disclosed in US 4,116,885, US 4,711,730 and EP 0 272 033. A particular preferred polymer in accordance with EP 0 272 033 has the formula:

$$(CH_3(PEG)_{43})_{0.75}(POH)_{0.25}[T\text{-}PO)_{2.8}(T\text{-}PEG)_{0.4}]T(POH)_{0.25}((PEG)_{43}CH_3)_{0.75}$$

where PEG is $-(OC_2H_4)O-$, PO is $(OC_3H_6O)$ and T is $(pcOC_6H_4CO)$.

Also very useful are modified polyesters as random copolymers of dimethyl terephthalate, dimethyl sulfoisophthalate, ethylene glycol and 1-2 propane diol, the end groups consisting primarily of sulphobenzoate and secondarily of mono esters of ethylene glycol and/or propane-diol. The target is to obtain a polymer capped at both end by sulphobenzoate groups, "primarily", in the present context most of said copolymers herein will be endcapped by sulphobenzoate groups. However, some copolymers will be less than fully capped, and therefore their end groups may consist of monoester of ethylene glycol and/or propane 1-2 diol, thereof consist "secondarily" of such species.

The selected polyesters herein contain about 46% by weight of dimethyl terephthalic acid, about 16% by weight of propane -1.2 diol, about 10% by weight ethylene gluycol about 13% by weight of dimethyl sulfobenzoic acid and about 15% by weight of sulfoisophthalic acid, and have a molecular weight of about 3.000. The polyesters and their method of preparation are described in detail in EP 311 342.

<u>Softening agents:</u> Fabric softening agents can also be incorporated into laundry detergent compositions in accordance with the present invention. These agents may be inorganic or organic in type. Inorganic softening agents are exemplified by the smectite clays disclosed in GB-A-1 400898 and in US 5,019,292. Organic fabric softening agents include the water insoluble tertiary amines as disclosed in GB-A1 514 276 and EP 0 011 340 and their combination with mono $C_{12}$-$C_{14}$ quaternary ammonium salts are disclosed in EP 026 528 and di-long-chain amides as disclosed in EP 0 242 919. Other useful organic ingredients of fabric softening systems include high molecular weight polyethylene oxide materials as disclosed in EP 0 299 575 and 0 313 146.

Levels of smectite clay are normally in the range form 5% to 15%, more preferably form 8% to 12% by weight, with the material being added as a dry mixed component to the remainder of the formulation. Organic fabric softening agents such as the water-insoluble tertiary amines or dilong chain amide materials are incorporated at levels of from 0.5% to 5% by weight, normally from 1% to 3% by weight whilst the high molecular weight polyethylene oxide materials and the water soluble cationic materials are added at levels of from 0.1% to 2%, normally from 0.15% to 1.5% by weight. These materials are normally added to the spray dried portion of the composition, although in some instances it may be more convenient to add them as a dry mixed particulate, or spray them as molten liquid on to other solid components of the composition.

<u>Polymeric dye transfer inhibiting agents:</u> The detergent compositions according to the present invention may also comprise from 0.001% to 10%, preferably from 0.01% to 2%, more preferably form 0.05% to 1% by weight of polymeric dye transfer inhibiting agents. Said polymeric dye transfer inhibiting agents are normally incorporated into detergent compositions in order to inhibit the transfer of dyes from colored fabrics onto fabrics washed therewith. These polymers have the ability to complex or adsorb the fugitive dyes washed out of dyed fabrics before the dyes have the opportunity to become attached to other articles in the wash. Especially suitable polymeric dye transfer inhibiting agents are polyamine N-oxide polymers, copolymers of N-vinylpyrrolidone and N-vinylimidazole, polyvinylpyrrolidone polymers, polyvinyloxazolidones and polyvinylimidazoles or mixtures thereof. Addition of such polymers also enhances the performance of the enzymes according the invention.

The detergent composition according to the invention can be in liquid, paste, gels, bars or granular forms.

Non-dusting granulates may be produced, e.g., as disclosed in US 4,106,991 and 4,661,452 (both to Novo Industri A/S) and may optionally be coated by methods known in the art. Examples of waxy coating materials are poly(ethylene oxide) products (polyethyleneglycol, PEG) with mean molecular weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591.

Granular compositions according to the present invention can also be in "compact form", i.e. they may have a relatively higher density than conventional granular detergents, i.e. form 550 to 950 g/l; in such case, the granular detergent compositions according to the present invention will contain a lower amount of "Inorganic filler salt", compared to conventional granular detergents; typical filler salts are alkaline earth metal salts of sulphates and chlorides, typically sodium sulphate; "Compact" detergent typically comprise not more than 10% filler salt. The liquid compositions according to the present invention can also be in "concentrated form", in such case, the liquid detergent compositions according to the present invention will contain a lower amount of water, compared to conventional liquid detergents. Typically, the water content of the concentrated liquid detergent is less than 30%, more preferably less than 20%, most preferably less than 10% by weight of the detergent compositions.

The compositions of the invention may for example, be formulated as hand and machine laundry detergent compositions including laundry additive compositions and compositions suitable for use in the pretreatment of stained fabrics, rinse added fabric softener compositions, and compositions for use in general household hard surface cleaning operations and dishwashing operations.

Particular forms of laundry detergent compositions within the invention include:

1) A detergent composition formulated as a granulate having a bulk density of at least 600 g/l comprising

| | |
|---|---|
| Linear alkylbenzenesulfonate (calculated as acid) | 7 - 12% |
| Alcohol ethoxysulfate (e.g. $C_{12-18}$ alcohol, 1-2 EO) or alkyl sulfate (e.g. $C_{16-18}$) | 1 - 4% |
| Alcohol ethoxylate (e.g. $C_{14-15}$ alcohol, 7 EO) | 5 - 9% |
| Sodium carbonate (as $Na_2CO_3$) | 14 - 20% |
| Soluble silicate (as $Na_2O,2SiO_2$) | 2 - 6% |
| Zeolite (as $NaAlSiO_4$) | 15 - 22% |
| Sodium sulfate (as $Na_2SO_4$) | 0 - 6% |
| Sodium citrate/citric acid (as $C_6H_5Na_3O_7/C_6H_8O_7$) | 0 - 15% |
| Sodium perborate (as $NaBO_3.H_2O$) | 11 - 18% |
| TAED | 2 - 6% |
| Carboxymethylcellulose | 0 - 2% |
| Polymers (e.g. maleic/acrylic acid copolymer, PVP, PEG) | 0 - 3% |
| Enzymes (calculated as pure enzyme protein) | 0.0001 - 0.1% |
| Minor ingredients (e.g. suds suppressors, perfume, optical brightener, photobleach) | 0 - 5% |

2) A detergent composition formulated as a granulate having a bulk density of at least 600 g/l comprising

| | |
|---|---|
| Linear alkylbenzenesulfonate (calculated as acid) | 6 - 11% |
| Alcohol ethoxysulfate (e.g. $C_{12-18}$ alcohol, 1-2 EO or alkyl sulfate (e.g. $C_{16-18}$) | 1 - 3% |
| Alcohol ethoxylate (e.g. $C_{14-15}$ alcohol, 7 EO) | 5 - 9% |
| Sodium carbonate (as $Na_2CO_3$) | 15 - 21% |
| Soluble silicate (as $Na_2O,2SiO_2$) | 1 - 4% |
| Zeolite (as $NaAlSiO_4$) | 24 - 34% |
| Sodium sulfate (as $Na_2SO_4$) | 4 - 10% |

(continued)

| | |
|---|---|
| Sodium citrate/citric acid (as $C_6H_5Na_3O_7/C_6H_8O_7$) | 0 - 15% |
| Carboxymethylcellulose | 0 - 2% |
| Polymers (e.g. maleic/acrylic acid copolymer, PVP, PEG) | 1 - 6% |
| Enzymes (calculated as pure enzyme protein) | 0.0001 - 0.1% |
| Minor ingredients (e.g. suds suppressors, perfume) | 0 - 5% |

3) A detergent composition formulated as a granulate having a bulk density of at least 600 g/l comprising

| | |
|---|---|
| Linear alkylbenzenesulfonate (calculated as acid) | 5 - 9% |
| Alcohol ethoxylate (e.g. $C_{12-15}$ alcohol, 7 EO) | 7 - 14% |
| Soap as fatty acid (e.g. $C_{16-22}$ fatty acid) | 1 - 3% |
| Sodium carbonate (as $Na_2CO_3$) | 10 - 17% |
| Soluble silicate (as $Na_2O,2SiO_2$) | 3 - 9% |
| Zeolite (as $NaAlSiO_4$) | 23 - 33% |
| Sodium sulfate (as $Na_2SO4$) | 0 - 4% |
| Sodium perborate (as $NaBO_3.H_2O$) | 8 - 16% |
| TAED | 2 - 8% |
| Phosphonate (e.g. EDTMPA) | 0 - 1% |
| Carboxymethylcellulose | 0 - 2 % |
| Polymers (e.g. maleic/acrylic acid copolymer, PVP, PEG) | 0 - 3% |
| Enzymes (calculated as pure enzyme protein) | 0.0001 - 0.1% |
| Minor ingredients (e.g. suds suppressors, perfume, optical brightener) | 0 - 5% |

4) A detergent composition formulated as a granulate having a bulk density of at least 600 g/l comprising

| | |
|---|---|
| Linear alkylbenzenesulfonate (calculated as acid) | 8 - 12% |
| Alcohol ethoxylate (e.g. $C_{12-15}$ alcohol, 7 EO) | 10 - 25% |
| Sodium carbonate (as $Na_2CO_3$) | 14 - 22% |
| Soluble silicate (as $Na_2O,2SiO_2$) | 1 - 5% |
| Zeolite (as $NaAlSiO_4$) | 25 - 35% |
| Sodium sulfate (as $Na_2SO_4$) | 0 - 10% |
| Carboxymethylcellulose | 0 - 2% |
| Polymers (e.g. maleic/acrylic acid copolymer, PVP, PEG) | 1 - 3% |
| Enzymes (calculated as pure enzyme protein) | 0.0001 - 0.1% |
| Minor ingredients (e.g. suds suppressors, perfume) | 0 - 5% |

5) An aqueous liquid detergent composition comprising

| | |
|---|---|
| Linear alkylbenzenesulfonate (calculated as acid) | 15 - 21% |
| Alcohol ethoxylate (e.g. $C_{12-15}$ alcohol, 7 EO or $C_{12-15}$ alcohol, 5 EO) | 12 - 18% |
| Soap as fatty acid (e.g. oleic acid) | 3 - 13% |

(continued)

| | |
|---|---|
| Alkenylsuccinic acid ($C_{12-14}$) | 0 - 13% |
| Aminoethanol | 8 - 18% |
| Citric acid | 2 - 8% |
| Phosphonate | 0 - 3% |
| Polymers (e.g. PVP, PEG) | 0 - 3% |
| Borate (as $B_4O_7$) | 0 - 2% |
| Ethanol | 0 - 3% |
| Propylene glycol | 8 - 14% |
| Enzymes (calculated as pure enzyme protein) | 0.0001 - 0.1% |
| Minor ingredients (e.g. dispersants, suds suppressors, perfume, optical brightener) | 0 - 5 % |

6) An aqueous structured liquid detergent composition comprising

| | |
|---|---|
| Linear alkylbenzenesulfonate (calculated as acid) | 15 - 21% |
| Alcohol ethoxylate (e.g. $C_{12-15}$ alcohol, 7 EO, or $C_{12-15}$ alcohol, 5 EO) | 3 - 9% |
| Soap as fatty acid (e.g. oleic acid) | 3 - 10% |
| Zeolite (as $NaAlSiO_4$) | 14 - 22% |
| Potassium citrate | 9 - 18% |
| Borate (as $B_4O_7$) | 0 - 2% |
| Carboxymethylcellulose | 0 - 2% |
| Polymers (e.g. PEG, PVP) | 0 - 3% |
| Anchoring polymers such as, e.g., lauryl methacrylate/acrylic acid copolymer; molar ratio 25:1; MW 3800 | 0 - 3% |
| Glycerol | 0 - 5% |
| Enzymes (calculated as pure enzyme protein) | 0.0001 - 0.1% |
| Minor ingredients (e.g. dispersants, suds suppressors, perfume, optical brighteners) | 0 - 5% |

7) A detergent composition formulated as a granulate having a bulk density of at least 600 g/l comprising

| | |
|---|---|
| Fatty alcohol sulfate | 5 - 10% |
| Ethoxylated fatty acid monoethanolamide | 3 - 9% |
| Soap as fatty acid | 0 - 3% |
| Sodium carbonate (as $Na_2CO_3$) | 5 - 10% |
| Soluble silicate (as $Na_2O,2SiO_2$) | 1 - 4% |
| Zeolite (as $NaAlSiO_4$) | 20 - 40% |
| Sodium sulfate (as $Na_2SO_4$) | 2 - 8% |
| Sodium perborate (as $NaBO_3.H_2O$) | 12 - 18% |
| TAED | 2 - 7% |
| Polymers (e.g. maleic/acrylic acid copolymer, PEG) | 1 - 5% |
| Enzymes (calculated as pure enzyme protein) | 0.0001 - 0.1% |
| Minor ingredients (e.g. optical brightener, suds suppressors, perfume) | 0 - 5% |

8) A detergent composition formulated as a granulate comprising

| | |
|---|---|
| Linear alkylbenzenesulfonate (calculated as acid) | 8 - 14% |
| Ethoxylated fatty acid monoethanolamide | 5 - 11% |
| Soap as fatty acid | 0 - 3% |
| Sodium carbonate (as $Na_2CO_3$) | 4 - 10% |
| Soluble silicate (as $Na_2O,2SiO_2$) | 1 - 4% |
| Zeolite (as $NaAlSiO_4$) | 30 - 50% |
| Sodium sulfate (as $Na_2SO_4$) | 3 - 11% |
| Sodium citrate (as $C_6H_5Na_3O_7$) | 5 - 12% |
| Polymers (e.g. PVP, maleic/acrylic acid copolymer, PEG) | 1 - 5% |
| Enzymes (calculated as pure enzyme protein) | 0.0001 - 0.1% |
| Minor ingredients (e.g. suds suppressors, perfume) | 0 - 5% |

9) A detergent composition formulated as granulate

| | |
|---|---|
| Alkyl sulfate (e.g $C_{12-18}$) | 6 -12 % |
| Soap, Na-salt | 0 - 3 % |
| Nonionic, (eg. alcohol ethoxylate $C_{10-18}$, 2-7 EO) | 2 - 8 % |
| Alkylglucamid (e.g $C_{16-18}$) | 2 - 6 % |
| Zeolite ($NaAlSiO_4$) | 14 - 24 % |
| Sodium carbonate ($Na_2CO_3$) | 7 - 13 % |
| Sodium disillicate; ($Na_2O:2SiO_2$) (e.g SKS6) | 10 - 14 % |
| Sodium sulfate ($Na_2SO_4$) | 5 - 9 % |
| Sodium percarbonate | 10 - 16 % |
| TAED | 1-5% |
| CMC | 0 - 3 % |
| Polycarboxylate | 1 - 7 % |
| Polymers (e.g PVP, PEG, maleic/acrylic acid copolymer) | 0 - 2 % |
| Enzymes including lipolytic enzymes (calculated as pure enzyme protein) | 0.0001 - 0.1 % |
| Minor ingrediens (e.g suds supressors, perfume, optical brightener, photobleach) | 0 - 5 % |
| Bulk density (g/l) at least | 700 |

10) A detergent composition formulated as granulate

| | |
|---|---|
| Alkyl sulfate (e.g $C_{12-18}$) | 7 - 11 % |
| Soap, Na-salt | 0 - 3 % |
| Nonionic, (eg. alcohol ethoxylate $C_{10-18}$, 2-7 EO) | 7 - 11 % |
| Alkylglucamid (e.g $C_{16-18}$) | 2 - 6 % |
| Zeolite ($NaAlSiO_4$) | 19 - 29 % |
| Sodium carbonate ($Na_2CO_3$) | 12 - 18 % |
| Sodium disillicate; ($Na_2O:2SiO_2$) (e.g SKS6) | 7 - 11 % |

(continued)

| | |
|---|---|
| Sodium sulfate ($Na_2SO_4$) | 5 - 9 % |
| CMC | 0-3 % |
| Polycarboxylate | 4 - 8 % |
| Polymers (e.g PVP, PEG, maleic/acrylic acid copolymer) | 1 - 3 % |
| Enzymes including lipolytic enzymes (calculated as pure enzyme protein) | 0.0001 - 0.1 % |
| Minor ingrediens (e.g suds supressors, perfume, optical brightener, photobleach) | 0 - 5 % |
| Bulk density (g/l) at least | 700 |

11) A detergent composition formulated as granulate

| | |
|---|---|
| Alkyl sulfate (e.g $C_{12-18}$) | 4 - 10 % |
| Nonionic, (eg. alcohol ethoxylate $C_{10-18}$, 2-7 EO) | 2 - 7 % |
| Phosphate (as STPP) | 17 - 27 % |
| Sodium carbonate ($Na_2CO_3$) | 4 - 8 % |
| Sodium disillicate; ($Na_2O:2SiO_2$) (e.g SKS6) | 4 - 8 % |
| Sodium sulfate ($Na_2SO_4$) | 18 - 26 % |
| Sodium perborate tetrahydrate | 13 - 19 % |
| TAED | 1 - 4 % |
| CMC | 0 - 2 % |
| Enzymes including lipolytic enzymes (calculated as pure enzyme protein) | 0.0001 - 0.1 % |
| Minor ingrediens (e.g suds supressors, perfume, optical brightener, photobleach) | 0 - 5 % |
| Bulk density (g/l) at least | 600 |

12) A detergent composition formulated as granulate

| | |
|---|---|
| Alkyl sulfuric acid (e.g $C_{12-18}$) | 2 - 9 % |
| Soap, Na-salt | 1 - 4 % |
| Nonionic, (eg. alcohol ethoxylate $C_{10-18}$, 2-7 EO) | 9 - 15 % |
| Zeolite ($NaAlSiO_4$) | 35 - 45 % |
| Sodium carbonate ($Na_2CO_3$) | 3 - 10 % |
| Sodium disillicate; ($Na_2O:2SiO_2$) (e.g SKS6) | 0 - 4 % |
| Sodium sulfate ($Na_2SO_4$) | 2 - 6 % |
| Sodium percarbonate | 14 - 20 % |
| TAED | 2 - 7 % |
| CMC | 0 - 3 % |
| Polycarboxylate | 0 - 2 % |
| Enzymes including lipolytic enzymes (calculated as pure enzyme protein) | 0.0001 - 0.1 |
| Minor ingrediens (e.g suds supressors, perfume, optical brightener, photobleach) | 0 - 5 % |
| Bulk density (g/l) at least | 700 |

13) A detergent composition formulated as granulate

| | |
|---|---|
| Alkyl benzene sulfonic acid | 8 - 14 % |
| Alkyl sulfuric acid (e.g $C_{12-18}$) | 2 - 6 % |
| Nonionic, (eg. alcohol ethoxylate $C_{10-18}$, 2-7 EO) | 9 - 13 % |
| Zeolite ($NaAlSiO_4$) (e.g Zeolite 4A) | 20 - 30 % |
| Sodium carbonate ($Na_2CO_3$) | 0 - 6 % |
| Sodium disillicate; ($Na_2O$:$2SiO_2$) | 0 - 3 % |
| Sodium sulfate ($Na_2SO_4$) | 0 - 6 % |
| Sodium perborate tetrahydrate | 22 - 28 % |
| TAED | 5 - 9 % |
| CMC | 0 - 2 % |
| Polymers (e.g maleic/acrylic acid copolymer) | 0 - 4 % |
| Enzymes including lipolytic enzymes (calculated as pure enzyme protein) | 0.0001 - 0.1 |
| Minor ingrediens (e.g suds supressors, perfume, optical brightener, photobleach) | 0 - 5 % |
| Bulk density (g/l) at least | 600 |

14) A detergent composition formulated as granulate

| | |
|---|---|
| Alkyl benzene sulfonic acid | 6 - 12 % |
| Alkyl ether sulfuric acid (e.g. $C_{12-18}$ alcohol, 4-10 EO) or alkyl sulfate (e.g $C_{12-18}$) | 2 - 6 % |
| Soap, Na-salt | 0 - 2 % |
| Nonionic, (eg. alcohol ethoxylate $C_{12-18}$, 3-10 EO) | 9 - 13 % |
| Zeolite ($NaAlSiO_4$) (e.g Zeolite 4A) | 39 - 49 % |
| Sodium carbonate ($Na_2CO_3$) | 2 - 8 % |
| Sodium disillicate; ($Na_2O$:$2SiO_2$) | 0 - 3 % |
| Sodium sulfate ($Na_2SO_4$) | 2 - 8 % |
| CMC | 0 - 3 % |
| Polymers (e.g maleic/acrylic acid copolymer) | 0 - 4 % |
| Enzymes including lipolytic enzymes (calculated as pure enzyme protein) | 0.0001 - 0.1 |
| Minor ingrediens (e.g suds supressors, perfume, optical brightener, photobleach) | 0 - 5 % |
| Bulk density (g/l) at least | 600 |

15) A detergent composition formulated as granulate

| | |
|---|---|
| Alkyl sulfate (e.g $C_{12-18}$) | 2- 8 % |
| Soap, Na-salt | 0 - 3 % |
| Nonionic, (eg. alcohol ethoxylate $C_{12-16}$, 3-10 EO) | 10 - 16 % |
| Zeolite ($NaAlSiO_4$) (e.g Zeolite 4A) | 47 - 57 % |
| Sodium carbonate ($Na_2CO_3$) | 15 - 23 % |
| Sodium citrate /citric acid ($C_6H_5Na_3O_7$/$C_6H_8O_7$) | 0 - 16 % |
| Sodium sulfate ($Na_2SO_4$) | 1 - 5 % |
| CMC | 0 - 3% |

(continued)

| | |
|---|---|
| Polycarboxylate | 0 - 2 % |
| Polymers (e.g PVP) | 0 - 2 % |
| Enzymes including lipolytic enzymes (calculated as pure enzyme protein) | 0.0001 - 0.1 |
| Minor ingrediens (e.g suds supressors, perfume, optical brightener, photobleach) | 0 - 5 % |
| Bulk density (g/l) at least | 800 |

16) A detergent composition formulated as granulate

| | |
|---|---|
| Alkyl benzene sulfonic acid | 0 - 3 % |
| Nonionic, (eg. alcohol ethoxylate $C_{12-18}$, 3-10 EO) | 1 - 5 % |
| Phosphate (as STPP) | 12 - 18 % |
| Sodium carbonate ($Na_2CO_3$) | 16 - 24 % |
| Sodium disillicate; ($Na_2O$:$2SiO_2$) | 1 - 3 % |
| Sodium sulfate ($Na_2SO_4$) | 38 - 48 % |
| Sodium perborate tetrahydrate | 8 - 14 % |
| TAED | 0 - 3 % |
| CMC | 0-3 % |
| Enzymes including lipolytic enzymes (calculated as pure enzyme protein) | 0.0001 - 0.1 |
| Minor ingrediens (e.g suds supressors, perfume, optical brightener, photobleach) | 0 - 5 % |
| Bulk density (g/l) at least | 500 |

17) A detergent composition formulated as granulate

| | |
|---|---|
| Soap, Na-salt | 1 - 3 % |
| Nonionic, (eg. alcohol ethoxylate $C_{12-18}$, 3-10 EO) | 2 - 6 % |
| Betaine (e.g Alkylamidopropylbetaine) | 0 - 3 % |
| Phosphate (as STPP) | 27 - 37 % |
| Sodium carbonate ($Na_2CO_3$) | 17 - 23 % |
| Sodium disillicate; ($Na_2O$:$2SiO_2$) | 3 - 7 % |
| Sodium sulfate ($Na_2SO_4$) | 4 - 11 % |
| Sodium perborate tetrahydrate | 15 - 21 % |
| TAED | 1 - 4 % |
| CMC | 1 - 3 % |
| Enzymes including lipolytic enzymes (calculated as pure enzyme protein) | 0.0001 - 0.1 |
| Minor ingrediens (e.g suds supressors, perfume, optical brightener, photobleach) | 0 - 5 % |
| Bulk density (g/l) at least | 600 |

18) A detergent composition formulated as granulate

| | |
|---|---|
| Alkyl benzene sulfonic acid | 5 - 11 % |
| Soap, Na-salt | 0 - 3 % |

(continued)

| | |
|---|---|
| Nonionic, (eg. alcohol ethoxylate $C_{12-18}$, 3-10 EO) | 3 - 7 % |
| Zeolite ($NaAlSiO_4$) (e.g Zeolite 4A) | 20 - 30 % |
| Sodium carbonate ($Na_2CO_3$) | 15 - 23 % |
| Sodium citrate /citric acid ($C_6H_5Na_3O_7/C_6H_8O_7$) | 0 - 3 % |
| Sodium sulfate ($Na_2SO_4$) | 4 - 10 % |
| Sodium percarbonate | 7 -13 % |
| TAED | 2 - 6 % |
| CMC | 1 - 3 % |
| Enzymes including lipolytic enzymes (calculated as pure enzyme protein) | 0.0001 - 0.1 |
| Minor ingrediens (e.g suds supressors, perfume, optical brightener, photobleach) | 0 - 5 % |
| Bulk density (g/l) at least | 600 |

19) A detergent composition formulated as granulate

| | |
|---|---|
| Alkyl benzene sulfonic acid | 0 - 4 % |
| Soap, Na-salt | 0 - 3 % |
| Nonionic, (eg. alcohol ethoxylate $C_{12-18}$, 3-10 EO) | 2 - 6 % |
| Zeolite ($NaAlSiO_4$) (e.g Zeolite 4A) | 11 - 17 % |
| Phosphate (as STPP) | 25 - 35 % |
| Sodium carbonate ($Na_2CO_3$) | 3 - 7 % |
| Sodium sillicate; ($Na_2O:SiO_2$) | 0 - 19 % |
| Sodium sulfate ($Na_2SO_4$) | 20 - 28 % |
| Sodium perborate tetrahydrate | 9 - 13 % |
| TAED | 1 - 5 % |
| CMC | 0 - 2 % |
| Enzymes including lipolytic enzymes (calculated as pure enzyme protein) | 0.0001 - 0.1 |
| Minor ingrediens (e.g suds supressors, perfume, optical brightener, photobleach) | 0 - 5 % |
| Bulk density (g/l) at least | 600 |

20) A detergent composition formulated as granulate

| | |
|---|---|
| Alkyl benzene sulfonic acid | 17 - 23 % |
| Soap, Na-salt | 0 - 3 % |
| Nonionic, (eg. alcohol ethoxylate $C_{12-18}$, 3-10 EO) | 11 - 15 % |
| | |
| Zeolite ($NaAlSiO_4$) (e.g Zeolite 4A) | 60 - 70 % |
| Sodium carbonate ($Na_2CO_3$) | 0 - 3 % |
| Sodium sulfate ($Na_2SO_4$) | 5 - 11 % |
| CMC | 0-3 % |
| Polymers (e.g PVP, PEG, maleic/acrylic acid copolymer)) | 2 - 6 % |

(continued)

| | |
|---|---|
| Enzymes including lipolytic enzymes (calculated as pure enzyme protein) | 0.0001 - 0.1 |
| Minor ingrediens (e.g suds supressors, perfume, optical brightener, photobleach) | 0 - 5 % |
| Bulk density (g/l) at least | 350 |

21) A detergent composition formulated as granulate

| | |
|---|---|
| Alkyl benzene sulfonic acid | 16 - 22 % |
| Soap, Na-salt | 0 - 2 % |
| Nonionic, (eg. alcohol ethoxylate $C_{12-18}$, 3-10 EO) | 3 - 9 % |
| Zeolite ($NaAlSiO_4$) | 25 - 33 % |
| Sodium carbonate ($Na_2CO_3$) | 3 - 7 % |
| Sodium sillicate; ($Na_2O:SiO_2$) | 0 - 4 % |
| Sodium sulfate ($Na_2SO_4$) | 5 - 11 % |
| Phosphonate | 0 - 3 % |
| Sodium perborate monohydrate | 15 - 19 % |
| TAED | 3 - 7 % |
| CMC | 0-3 % |
| Polymers (e.g PVP, PEG, maleic/acrylic acid copolymer)) | 0 - 3 % |
| Enzymes including lipolytic enzymes (calculated as pure enzyme protein) | 0.0001 - 0.1 |
| Minor ingrediens (e.g suds supressors, perfume, optical brightener, photobleach) | 0 - 5 % |
| Bulk density (g/l) at least | 700 |

22) A detergent composition formulated as granulate

| | |
|---|---|
| Alkyl benzene sulfonic acid | 4 - 8 % |
| Alkyl sulfate (e.g $C_{12-18}$) | 0 - 3 % |
| Nonionic, (eg. alcohol ethoxylate $C_{12-18}$, 3-10 EO) | 5 - 9 % |
| Zeolite ($NaAlSiO_4$) | 20 - 28 % |
| Sodium carbonate ($Na_2CO_3$) | 9 - 15 % |
| Sodium disillicate; ($Na_2O:2SiO_2$) | 0 - 4 % |
| Sodium perborate tetrahydrate | 21 - 31 % |
| TAED | 1 - 5 % |
| CMC | 0 - 3 % |
| Polymers (e.g PVP, PEG, maleic/acrylic acid copolymer)) | 0 - 3 % |
| Enzymes including lipolytic enzymes (calculated as pure enzyme protein) | 0.0001 - 0.1 |
| Minor ingrediens (e.g suds supressors, perfume, optical brightener, photobleach) | 0 - 5 % |
| Bulk density (g/l) at least | 600 |

23) A detergent composition formulated as a granulate comprising

| | |
|---|---|
| Linear alkylbenzenesulfonate (calculated as acid) | 6 - 12% |

(continued)

| | |
|---|---|
| Nonionic surfactant | 1 - 4% |
| Soap as fatty acid | 2 - 6 % |
| Sodium carbonate (as $Na_2CO_3$) | 14 - 22 % |
| Zeolite (as $NaAlSiO_4$) | 18 - 32 % |
| Sodium sulfate (as $Na_2SO_4$) | 5 - 20% |
| Sodium citrate (as $C_6H_5Na_3O_7$) | 3 - 8% |
| Sodium perborate (as $NaBO_3.H_2O$) | 4 - 9% |
| Bleach activator (e.g. NOBS or TAED) | 1 - 5% |
| Carboxymethylcellulose | 0 - 2 % |
| Polymers (e.g. polycarboxylate or PEG) | 1 - 5 % |
| Enzymes (calculated as pure enzyme protein) | 0.0001 - 0.1% |
| Minor ingredients (e.g. optical brightener, perfume) | 0 - 5% |

24) An aqueous liquid detergent composition comprising

| | |
|---|---|
| Linear alkylbenzenesulfonate (calculated as acid) | 15 - 23 % |
| Alcohol ethoxysulfate (e.g. $C_{12-15}$ alcohol, 2-3 EO) | 8 - 15% |
| Alcohol ethoxylate (e.g. $C_{12-15}$ alcohol, 7 EO, or $C_{12-15}$ alcohol, 5 EO) | 3 - 9% |
| Soap as fatty acid (e.g. lauric acid) | 0 - 3% |
| Aminoethanol | 1 - 5% |
| Sodium citrate | 5 - 10% |
| Hydrotrope (e.g. sodium toluensulfonate) | 2 - 6% |
| Borate (as $B_4O_7$) | 0 - 2 % |
| Carboxymethylcellulose | 0 - 1% |
| Ethanol | 1 - 3% |
| Propylene glycol | 2 - 5% |
| Enzymes (calculated as pure enzyme protein) | 0.0001 - 0.1 % |
| Minor ingredients (e.g. polymers, dispersants, perfume, optical brighteners) | 0 - 5% |

25) An aqueous liquid detergent composition comprising

| | |
|---|---|
| Linear alkylbenzenesulfonate (calculated as acid) | 20 - 32 % |
| Alcohol ethoxylate (e.g. $C_{12-15}$ alcohol, 7 EO, or $C_{12-15}$ alcohol, 5 EO) | 6 - 12% |
| Aminoethanol | 2 - 6% |
| Citric acid | 8 - 14% |
| Borate (as $B_4O_7$) | 1 - 3% |
| Polymer (e.g. maleic/acrylic acid copolymer, anchoring polymer such as, e.g., lauryl methacrylate/ acrylic acid copolymer) | 0 - 3% |
| Glycerol | 3 - 8% |
| Enzymes (calculated as pure enzyme protein) | 0.0001 - 0.1% |
| Minor ingredients (e.g. hydrotropes, dispersants, perfume, optical brighteners) | 0 - 5 % |

26) A detergent composition formulated as concentrated liquid.

| | |
|---|---|
| Alkyl benzene sulfonic acid | 6 - 12 % |
| Alkyl sulfuric acid | 0 - 4 % |
| Monoethanolamin | 2 - 6 % |
| Nonionic, (eg. alcohol ethoxylate $C_{12-18}$, 3-10 EO) | 9 - 15 % |
| Sodium citrate /citric acid ($C_6H_5Na_3O_7/C_6H_8O_7$) | 2 - 8 % |
| Glycerol | 2 - 6 % |
| Borate (as $Na_2B_4O_7$) | 0 - 4 % |
| Polymers (e.g PVP, PEG, maleic/acrylic acid copolymer)) | 0 - 3 % |
| Enzymes including lipolytic enzymes (calculated as pure enzyme protein) | 0.0001 - 0.1 |
| Minor ingrediens (e.g suds supressors, perfume, optical brightener, photobleach) | 0 - 5 % |
| Total water | 40 % |

27) A detergent composition formulated as concentrated liquid.

| | |
|---|---|
| Alkyl benzene sulfonic acid | 11 - 17 % |
| Alkyl ether sulfuric acid (e.g. $C_{12-18}$ alcohol, 4-10 EO) or alkyl sulfate (e.g $C_{12-18}$) | 0 - 4 % |
| Triethanolamin | 0 - 3 % |
| Nonionic, (eg. alcohol ethoxylate $C_{12-18}$, 310 EO) | 6 - 10 % |
| Sodium citrate /citric acid ($C_6H_5Na_3O_7/C_6H_8O_7$) | 2 - 6 % |
| Hydrotropes (Sodium toluene sulfonate) | 1 - 5 % |
| Glycerol | 6 - 12 % |
| MPG | 0 - 5 % |
| Ethanol | 0 - 3 % |
| Enzymes including lipolytic enzymes (calculated as pure enzyme protein) | 0.0001 - 0.1 |
| Minor ingrediens (e.g suds supressors, perfume, optical brightener, photobleach) | 0 - 5 % |
| Total water | 55 % |

28) A detergent composition formulated as a granulate having a bulk density of at least 600 g/l comprising

| | |
|---|---|
| Anionic surfactant (linear alkylbenzenesulfonate, alkyl sulfate, alpha-olefinsulfonate, alpha-sulfo fatty acid methyl esters, alkanesulfonates, soap) | 25 - 40% |
| Nonionic surfactant (e.g. alcohol ethoxylate) | 1 - 10% |
| Sodium carbonate (as $Na_2CO_3$) | 8 - 25 % |
| Soluble silicates (as $Na_2O$, $2SiO_2$) | 5 - 15% |
| Sodium sulfate (as $Na_2SO_4$) | 0 - 5 % |
| Zeolite (as $NaAlSiO_4$) | 15 - 28% |
| Sodium perborate (as $NaBO_3.4H_2O$) | 0 - 20% |
| Bleach activator (TAED or NOBS) | 0 - 5% |
| Enzymes (calculated as pure enzyme protein) | 0.0001 - 0.1% |
| Minor ingredients (e.g. perfume, optical brighteners) | 0 - 3% |

29) A detergent composition formulated as granulate

| | |
|---|---|
| Alkyl benzene sulfonic acid | 25 - 35 % |
| Nonionic, (eg. alcohol ethoxylate $C_{12-18}$, 3-10 EO) | 0 - 3% |
| Zeolite ($NaAlSiO_4$) | 3 - 9 % |
| Phosphate (as STPP) | 25 - 35 % |
| Sodium carbonate ($Na_2CO_3$) | 0 - 3 % |
| Sodium disillicate; ($Na_2O$:$2SiO_2$) | 2 - 8 % |
| Sodium sulfate ($Na_2SO_4$) | 17 - 23 % |
| Sodium perborate monohydrate | 1 - 5 % |
| TAED | 0 - 3 % |
| CMC | 0 - 3 % |
| Enzymes including lipolytic enzymes (calculated as pure enzyme protein) | 0.0001 - 0.1 |
| Minor ingrediens (e.g suds supressors, perfume, optical brightener, photobleach) | 0 - 5 % |
| Bulk density (g/l) at least | 600 |

30) A detergent composition formulated as granulate

| | |
|---|---|
| Alkyl benzene sulfonic acid | 25 - 35 % |
| Soap, fatty acid Na-salt | 0 - 3 % |
| Nonionic, (eg. alcohol ethoxylate $C_{13-15}$ , 7 EO) | 4 - 9 % |
| Zeolite ($NaAlSiO_4$) | 7 - 11 % |
| Phosphate (as STPP) | 26 - 36 % |
| Sodium carbonate ($Na_2CO_3$) | 6 - 12 % |
| Sodium disillicate; ($Na_2O$:$2SiO_2$) | 4 - 10 % |
| Sodium sulfate ($Na_2SO_4$) | 4 - 8 % |
| CMC | 0-3 % |
| Enzymes including lipolytic enzymes (calculated as pure enzyme protein) | 0.0001 - 0.1 |
| Minor ingrediens (e.g suds supressors, perfume, optical brightener, photobleach) | 0 - 5 % |
| Bulk density (g/l) at least | 700 |

The following specific compositions are meant to exemplify compositions for the present invention, but are not necessarily meant to limit or otherwise define the scope of the invention.
In the detergent compositions, the abbreviated component identifications have the following meanings:

| | |
|---|---|
| LAS: | Sodium linear $C_{12}$ alkyl benzene sulphonate |
| TAS: | Sodium tallow alkyl sulphate |
| XYAS: | Sodium $C_{1X}$ - $C_{1Y}$ alkyl sulfate |
| SS: | Secondary soap surfactant of formula 2-butyl octanoic acid |
| 25EY: | A $C_{12}$ - $C_{15}$ predominantly linear primary alcohol condensed with an average of Y moles of ethylene oxide |
| 45EY: | A $C_{14}$ - $C_{15}$ predominantly linear primary alcohol condensed with an average of Y moles of ethylene oxide |
| XYEZS: | $C_{1X}$ - $C_{1Y}$ sodium alkyl sulfate condensed with an average of Z moles of ethylene oxide per mole |
| Nonionic: | $C_{13}$ - $C_{15}$ mixed ethoxylated/propoxylated fatty alcohol with an average degree of ethoxylation of 3.8 and an average degree of propoxylation of 4.5 sold under the tradename Plurafax LF404 |

by BASF Gmbh

| | |
|---|---|
| CFAA: | $C_{12}$ - $C_{14}$ alkyl N-methyl glucamide |
| TFAA: | $C_{16}$ - $C_{18}$ alkyl N-methyl glucamide |
| Silicate: | Amorphous Sodium Silicate ($SiO_2$:$Na_2O$ ratio = 2.0) |
| NaSKS-6: | Crystalline layered silicate of formula d-$Na_2Si_2O_5$ |
| Carbonate: | Anhydrous sodium carbonate |
| Phosphate: | Sodium tripolyphosphate |
| MA/AA: | Copolymer of 1:4 maleic/acrylic acid, average molecular weight about 80,000 |
| Polyacrylate: | Polyacrylate homopolymer with an average molecular weight of 8,000 sold under the tradename PA30 by BASF Gmbh |
| Zeolite A: | Hydrated Sodium Aluminosilicate of formula $Na_{12}(AlO_2SiO_2)_{12}$. $27H_2O$ having a primary particle size in the range from 1 to 10 micrometers |
| Citrate: | Tri-sodium citrate dihydrate |
| Citric: | Citric Acid |
| Perborate: | Anhydrous sodium perborate monohydrate bleach, empirical formula $NaBO_2.H_2O_2$ |
| PB4: | Anhydrous sodium perborate tetrahydrate |
| Percarbonate: | Anhydrous sodium percarbonate bleach of empirical formula $2Na_2CO_3.3H_2O_2$ |
| TAED: | Tetraacetyl ethylene diamine |
| CMC: | Sodium carboxymethyl cellulose |
| DETPMP: | Diethylene triamine penta (methylene phosphonic acid), marketed by Monsanto under the Tradename Dequest 2060 |
| PVP: | Polyvinyl pyrrolidone polymer |
| EDDS: | Ethylenediamine -N, N'- disuccinic acid, [S,S] isomer in the form of the sodium salt |
| Suds Suppressor: | 25% paraffin wax Mpt 50°C, 17% hydrophobic silica, 58% paraffin oil |
| Granular Suds: | 12% Silicone/silica, 18% stearyl alcohol, 70 % starch in granular form |
| Sulphate: | Anhydrous sodium sulphate |
| HMWPEO: | High molecular weight polyethylene oxide |
| TAE 25: | Tallow alcohol ethoxylate (25) |

Composition 1

A granular fabric cleaning composition in accordance with the invention may be prepared as follows:

| | |
|---|---|
| Sodium linear $C_{12}$ alkyl benzene sulfonate | 6.5 |
| Sodium sulfate | 15.0 |
| Zeolite A | 26.0 |
| Sodium nitrilotriacetate | 5.0 |
| Enzyme of the invention | 0.1 |
| PVP | 0.5 |
| TAED | 3.0 |
| Boric acid | 4.0 |
| Perborate | 18.0 |
| Phenol sulphonate | 0.1 |
| Minors | Up to 100 |

Composition 2

A compact granular fabric cleaning composition (density 800/l) in accord with the invention may be prepared as follows:

| | |
|---|---|
| 45AS | 8.0 |
| 25E3S | 2.0 |
| 25E5 | 3.0 |
| 25E3 | 3.0 |
| TFAA | 2.5 |

(continued)

| | |
|---|---|
| Zeolite A | 17.0 |
| NaSKS-6 | 12.0 |
| Citric acid | 3.0 |
| Carbonate | 7.0 |
| MA/AA | 5.0 |
| CMC | 0.4 |
| Enzyme of the invention | 0.1 |
| TAED | 6.0 |
| Percarbonate | 22.0 |
| EDDS | 0.3 |
| Granular suds suppressor | 3.5 |
| water/minors | Up to 100% |

Composition 3

Granular fabric cleaning compositions in accordance with the invention which are especially useful in the laundering of coloured fabrics were prepared as follows:

| | | |
|---|---|---|
| LAS | 10.7 | - |
| TAS | 2.4 | - |
| TFAA | - | 4.0 |
| 45AS | 3.1 | 10.0 |
| 45E7 | 4.0 | - |
| 25E3S | - | 3.0 |
| 68E11 | 1.8 | - |
| 25E5 | - | 8.0 |
| Citrate | 15.0 | 7.0 |
| Carbonate | - | 10 |
| Citric acid | 2.5 | 3.0 |
| Zeolite A | 32.1 | 25.0 |
| Na-SKS-6 | - | 9.0 |
| MA/AA | 5.0 | 5.0 |
| DETPMP | 0.2 | 0.8 |
| Enzyme of the invention | 0.10 | 0.05 |
| Silicate | 2.5 | - |
| Sulphate | 5.2 | 3.0 |
| PVP | 0.5 | - |
| Poly (4-vinylpyridine)-N-Oxide/copolymer of vinyl-imidazole and vinyl-pyrrolidone | - | 0.2 |
| Perborate | 1.0 | - |
| Phenol sulfonate | 0.2 | - |
| Water/Minors | Up to 100% | |

Composition 4

Granular fabric cleaning compositions in accordance with the invention which provide "Softening through the wash" capability may be prepared as follows:

| | | |
|---|---|---|
| 45AS | - | 10.0 |

(continued)

| | | |
|---|---|---|
| LAS | 7.6 | - |
| 68AS | 1.3 | - |
| 45E7 | 4.0 | - |
| 25E3 | - | 5.0 |
| Coco-alkyl-dimethyl hydroxy-ethyl ammonium chloride | 1.4 | 1.0 |
| Citrate | 5.0 | 3.0 |
| Na-SKS-6 | - | 11.0 |
| Zeolite A | 15.0 | 15.0 |
| MA/AA | 4.0 | 4.0 |
| DETPMP | 0.4 | 0.4 |
| Perborate | 15.0 | - |
| Percarbonate | - | 15.0 |
| TAED | 5.0 | 5.0 |
| Smectite clay | 10.0 | 10.0 |
| HMWPEO | - | 0.1 |
| Enzyme of the invention | 0.10 | 0.05 |
| Silicate | 3.0 | 5.0 |
| Carbonate | 10.0 | 10.0 |
| Granular suds suppressor | 1.0 | 4.0 |
| CMC | 0.2 | 0.1 |
| Water/Minors | Up to 100% | |

Composition 5

Heavy duty liquid fabric cleaning compositions in accordance with the invention may be prepared as follows:

| | I | II |
|---|---|---|
| LAS acid form | - | 25.0 |
| Citric acid | 5.0 | 2.0 |
| 25AS acid form | 8.0 | - |
| 25AE2S acid form | 3.0 | - |
| 25AE7 | 8.0 | - |
| CFAA | 5 | - |
| DETPMP | 1.0 | 1.0 |
| Fatty acid | 8 | - |
| Oleic acid | - | 1.0 |
| Ethanol | 4.0 | 6.0 |
| Propanediol | 2.0 | 6.0 |
| Enzyme of the invention | 0.10 | 0.05 |
| Coco-alkyl dimethyl hydroxy ethyl ammonium chloride | - | 3.0 |
| Smectite clay | - | 5.0 |
| PVP | 2.0 | - |
| Water / Minors | Up to 100% | |

Composition 6

A detergent composition formulated as a granulate

| | | |
|---|---|---|
| Alcohol ethoxylate $C_{12-18}$, 5-7 EO | 6 | 4 |
| Alkyl benzene sulfonate; $C_{11-13}$ | 5 | 20 |
| Linear alkyl sulfate; $C_{16-18}$ (e.g Sulfopon) | 5 | 0 |
| Soap | 1 | 4 |
| Sodium carbonate | 10 | 0 |
| Zeolith Na-A | 25 | 35 |
| Sodium sillicate; $Na_2O$:$SiO_2$=3 | 2 | 2 |
| Sodium sulfate ($Na_2SO_4$) | 1 | 20 |
| Polycarboxylate; (Sokalan- CP5) | 5 | 5 |
| Carboxylic acids (Sokalan DCS) | 0 | 4 |
| Sodium perborate tetrahydrate | 20 | 0 |
| TAED | 6 | 0 |
| Suds supressors | 5 | 0 |
| Enzymes including lipolytic enzymes (calculated as pure enzyme protein) | 0.0001 - 0.1 | 0.0001 - 0.1 |

Composition 7

A bleach containing detergent composition formulated as a granulate

| | | |
|---|---|---|
| Primary alcohol sulfate (CocoPAS) | 5 | 6 |
| Nonion 3EO (e.g Alkohol ethoylate C12-15; 3EO) | 5 | 0 |
| Nonion 7EO (Alkohol ethoylate C12-15; 7EO) | 8 | 14 |
| Sokalan HP22* | 0,7 | 0,8 |
| Soap | 1,3 | 0 |
| Zeolite MAP | 39 | 39 |
| Sodium citrate | 4 | 5 |
| Sodium carbonate | 3,3 | 1 |
| Water/ salts | 0,4 | 0,5 |
| Antifoam/flourescer/perfume | 4 | 5 |
| TAED | 5 | 5 |
| Mn-catalyst | 1,7 | 1,7 |
| Percarbonate | 21 | 21 |
| EDTMP | 0,4 | 0,4 |
| Enzymes including lipolytic enzymes (calculated as pure enzyme protein) | 0.0001 - 0.1 | 0.0001 - 0.1 |

* a graft polymer as decribed in WO 95/22393

Composition 8

A non-bleach detergent composition formulated as a granulate

| | | | | | | |
|---|---|---|---|---|---|---|
| Primary alcohol sulfate (CocoPAS) | 6 | 6 | 11 | 9 | 6 | 6 |
| Nonion 3EO (e.g Alkohol ethoylate C12-15; 3EO) | 6 | 0 | 4 | 4 | 6 | 0 |

(continued)

| | | | | | | |
|---|---|---|---|---|---|---|
| Nonion 7EO (e.g Alkohol ethoylate C12-15; 7EO) | 6 | 14 | 6 | 6 | 9 | 15 |
| Sokalan HP22* | 0,7 | 0,7 | 0,6 | 0,7 | 0,5 | 0,5 |
| Soap | 2 | 2 | 2 | 2 | 2 | 2 |
| Zeolite MAP | 39 | 39 | 30 | 32 | 40 | 40 |
| Sodium citrate | 25 | 25 | 30 | 32 | 22 | 22 |
| Sodium carbonate | 1 | 1 | 2 | 2 | 1 | 1 |
| Sodium CMC | 0 | 0 | 0 | 0 | 0,7 | 0,7 |
| Water/ salts | 5 | 6 | 5 | 6 | 6 | 6 |
| Antifoam/PVP/perfume | 3 | 3 | 4 | 4 | 3 | 3 |
| EDTMP | 1,4 | 1,4 | 1,4 | 1,4 | 1,4 | 1,4 |
| Enzymes including lipolytic enzymes (calculated as pure enzyme protein) | 0.0001 - 0.1 | | | | | |

*A graft polymer as decribed in the invention WO95622593

Composition 9

A detergent composition formulated as a granulate

| | | | |
|---|---|---|---|
| Alcohol ethoylat $C_{12-18}$, 5EO | 0,5 | 0 | 0 |
| Alkyl glycoside, $C_{12-14}$. Degree of polymerization: 1,4 | 5 | 5 | 10 |
| Linear alkyl sulfate $C_{16-18}$ (e.g Sulfopon, Henkel) | 10 | 18 | 15 |
| Soap | 1 | 6 | 6 |
| Sodium carbonate | 12 | 0 | 0 |
| Zeolith Na-A | 23 | 50 | 33 |
| Sodium silicate ($SiO_2$:$Na_2O$ = 3.0) | 5 | 0 | 3 |
| Polycarboxylate (Sokalan CP5) | 6 | 0 | 0 |
| Carboxylic acids (Sokalan DCS) | 0 | 4 | 0 |
| Sodium percarbonate | 0 | 0 | 15 |
| TAED | 6 | 0 | 5 |
| Sud supressors | 6 | 6 | 6 |
| Enzymes including lipolytic enzymes (calculated as pure enzyme protein) | 0.0001 - 0.1 | | |
| Water | to 100 | | |

Composition 10

A detergent composition formulated as a granulate

| | | | | | |
|---|---|---|---|---|---|
| Zeolite A | 38 | 38 | 0 | 0 | 0 |
| Sodium disilicate (e.g SKS-6, Hoechst) | 0 | 0 | 25 | 30 | 30 |
| Amorphes sodium silicate $Na_2O$:$SiO_2$ = 1:2.0 | 5.5 | 5.5 | 0 | 0 | 0 |
| Sodium carbonate | 3 | 3 | 3 | 5 | 0 |
| Polycarboxylate (Sokalan CP5) | 0 | 2 | 6 | 5 | 5 |

(continued)

| | | | | | |
|---|---|---|---|---|---|
| Mixt. of 80% Alcohol ethoxylate $C_{12-18}$, 5 EO and 20 % Alcohol ethoxylate $C_{12-14}$, 3 EO (Dehydol LST 80/20) | 2.5 | 2.5 | 2.5 | 2.5 | 2.5 |
| Alkohol ethoxylate $C_{16-18}$; 14 EO (e.g Dehydol TA 14) | 2 | 2 | 2 | 2 | 2 |
| Sodium alkyl benzene sulfonate; $C_{12}$ | 0 | 0 | 2 | 0 | 0 |
| Alkyl sulfate; $C_{16-18}$ | 7.5 | 7.5 | 5.5 | 7.5 | 7.5 |
| Perborate tetrahydrate | 25 | 25 | 25 | 25 | 25 |
| TAED | 2 | 2 | 2 | 2 | 2 |
| Enzymes including lipolytic enzymes (calculated as pure enzyme protein) | 0.0001 - 0.1 | | | | |
| Water, perfume, sud supressor ect. | to 100 | | | | |

Composition 11

A detergent composition formulated as a granulate

| | |
|---|---|
| Linear alkylbenzene sulfonate | 9 |
| Nonionic ethoxylated alcohol 7EO - (e.g Synperonic A7) | 2 |
| Nonionic ethoxylated alcohol, 1:1 mixt. of Superionic A3 & A7 (3 / 7 EO groups) | 5 |
| Zeolite 4A | 29 |
| Poly carboxylate (e.g Sokalan CP 5) | 4 |
| Sodium carbonate | 7 |
| Granular sodium silicate | 4 |
| TAED | 8 |
| Sodium perborate monohydrate | 15 |
| EDTMP (Ethylene diamine tetramethylene phosphonic acid) | 0.4 |
| Nonionic material containing at least 25 EO groups (e.g Lutensol AT - BASF and BRIJ- ICI) | 0-1 |
| Minor ingrediens (Fluorescer, CMC, salts antifoam granules, perfume | 4 |
| Enzymes including lipolytic enzymes (calculated as pure enzyme protein) | 0.0001 - 0.1 |
| Moisture | to 100 |

Composition 12

A detergent composition formulated as a granulate

| | |
|---|---|
| Sodium primary alkyl sulphate (PAS) | 6 |
| Nonionic ethoxylated alcohol 3EO - (e.g Synperonic A3) | 7 |
| Nonionic ethoxylated alcohol 7EO - (e.g Synperonic A7) | 6 |
| Zeolite MAP | 36 |
| Stearic acid | 2 |
| Tallow 80EO | 0.2 |
| Sodium silicate | 3 |
| TAED | 5 |
| Manganese catalyst | 2 |

(continued)

| | |
|---|---|
| Sodium percarbonate | 21 |
| Dequest 2047 | 0,4 |
| Minor ingrediens (Fluorescer, CMC, salts antifoam granules, perfume) | 4 |
| Enzymes including lipolytic enzymes (calculated as pure enzyme protein) | 0.0001 - 0.1 |
| Moisture | to 100 |

Composition 13

A detergent composition formulated as a granulate

| | | |
|---|---|---|
| Decylidene or dodecylidene diglycerol | 17 | 0 |
| Decylidene or dodecylidene triglycerol | 0 | 9 |
| C12-15 EO 7 ethoxylate | | 9 |
| Zeolite | 32 | 32 |
| Sodium carbonate | 12 | 12 |
| Alkaline sodium silicate | 1 | 1 |
| Fatty acid soap | 2 | 2 |
| Sodium carboxy methyl cellulose | 1 | 1 |
| Sodium perborate monohydrate | 15 | 15 |
| TAED | 7 | 7 |
| Bleach stabiliser (EDTMP) | 0.4 | 0.4 |
| Silicone suds suppressor | 0.4 | 0.4 |
| flourescer/perfume | 1 | 1 |
| Enzymes including lipolytic enzymes (calculated as pure enzyme protein) | 0.0001 - 0.1 | |
| Moisture | to 100 | |

Composition 14

A detergent composition formulated as a granulate

| | | | | | | |
|---|---|---|---|---|---|---|
| Alkyl benzene sulfonate $C_{10-13}$ | 18 | 13 | 15 | 11 | 15 | 3 |
| Linear alkyl sulfate; $C_{16-18}$ (e.g Sulfopon) | | 3 | | 3 | 4 | 14 |
| Alcohol ethoxylate; $C_{16-18}$ ; 5 EO | 1.5 | 0.5 | | 0.5 | | |
| Cetyl/oleyl alcohol 5 EO | 1.5 | 1 | | 0.7 | 1.4 | |
| Cetyl/oleyl alcohol 10 EO | 1.5 | 1 | | 0.7 | 1.4 | |
| Alkohol ethoxylate $C_{14-15}$ ; 7 EO (Dobanol 45-7) | | | 2 | | | 0.5 |

(continued)

| | | | | | | |
|---|---|---|---|---|---|---|
| Alcohol ethoxylate $C_{14-15}$ ; 4 EO (Dobanol 45-4) | | | 6 | | | 0.5 |
| Zeolith Na-A | 50 | 15 | 42 | 15 | 51 | 42 |
| Na-silicate | | | 5 | | | 5 |
| Na-carbonate | | | 12 | | | 12 |
| Na-sulfate | 1 | 45 | 1 | 42 | | |
| Polycarboxylate (Sokalan CP5) | 5 | 3 | 5 | 2 | 5 | 4 |
| Na-hydrogensulfate | | 3 | | | | |
| Org. acid (Sokalan DCS) | 3 | | | | | 1,5 |
| Water | 14.5 | 11.8 | 10.1 | 14.5 | 16.0 | 13.1 |
| Soap; $C_{12-18}$ | 3 | 3 | 1 | 3 | 4 | 3 |
| Enzymes including lipolytic enzymes (calculated as pure enzyme protein) | 0.0001 - 0.1 | | | | | |

Composition 15

A powdered detergent composition

| | | | | | | |
|---|---|---|---|---|---|---|
| Sodium Alkylbenzene sulfonate $C_{11-13}$ | 11 | 11.5 | 7 | 11 | 15 | |
| Alcohol ethoxy sulfate (Sulfated Alfonic 1412-70) | | 5.5 | | | | |
| Primary alcohol sulfate ( | 10 | | | 9 | 5 | |
| Alcohol Ethoxylate (e.g Neodol 25-9) | | 3 | | 2 | 3 | 10 |
| Soap | I | | | | 1 | |
| | | | | | | |
| Sodium tripolyphosphate | | | | | 25 | |
| Aluminosilicates, e.g Zeolite 4A | 10-35 | 0-15 | 5-20 | 0-12 | | |
| Polycarboxylate (e.g. CP5) | 0-3 | | | | | |
| MA/AA/Hydrophobe terpolymers * | 2-25 | 2-25 | 2-25 | 2-25 | 5 | 2-20 |
| | | | | | | |
| Alkaline Silicate | 2-5 | 20 | 5 | 3-20 | 15 | 15 |
| Sodium carbonate | 18 | 18 | 15 | 30 | 20 | 40 |
| | | | | | | |
| Quaternary amines | | | 2.4 | | | |
| Ethoxylated Amine (e.g Varonic U202) | | | 2 | | | |
| Swelling clay | | | 10 | | | |
| Flourescers (Tinopal AMS) | 0.15 | 0.2 | 0.25 | 0.15 | 1.5 | 1.5 |
| Perfume | 0.1 | 0.2 | 0.1 | 0.1 | 0.1 | 0.1 |
| Enzymes including lipolytic enzymes (calculated as pure enzyme protein) | 0.0001 - 0.1 | | | | | |
| Sodium sulfate | to balance | | | | | |

* As described in US 5,308,530

Composition 16

An aqueous liquid washing agent

| | | | | | | | | | |
|---|---|---|---|---|---|---|---|---|---|
| Alkyl benzene sulfonate; $C_{10-13}$; Monoethanolaminsalt | 0 | 0 | 0 | 0 | 9 | 17 | 0 | 0 | 0 |
| Alkyl ether sulphate, $C_{12-14}$; 2 EO | 16 | 10 | 10 | 21 | 11 | 0 | 14 | 38 | 21 |
| Sodium lauryl sulfate | 0 | 5 | 5 | 0 | 0 | 0 | 4 | 0 | 0 |
| Soap ($C_{12-18}$- fatty acid) | 5 | 5 | 5 | 5 | 5 | 5 | 5 | 5 | 5 |
| Alkohol ethoxylate $C_{12-15}$; 7EO | 22 | 30 | 30 | 30 | 30 | 30 | 20 | 25 | 30 |
| Alkylglycoside, $C_{8-10}$- degree of oligomerization: 1,6 | 15 | 0 | 7 | 0 | 0 | 0 | 0 | 0 | 0 |
| Alkylglycoside,$C_{12-16}$- degree of oligomerization: 1,4 | 0 | 5 | 0 | 5 | 5 | 5 | 10 | 0 | 5 |
| 1,2-propanediol | 15 | 15 | 15 | 15 | 15 | 15 | 15 | 15 | 15 |
| Ethanol | 5 | 5 | 5 | 5 | 5 | 5 | 5 | 5 | 5 |
| Enzymes including lipolytic enzymes (calculated as pure enzyme protein) | 0.0001 - 0.1 | | | | | | | | |
| Water | to 100 | | | | | | | | |

Composition 17

| A liquid detergent composition | |
|---|---|
| Fattyacid monoglyceride (e.g Cutina AGS, Henkel) | 0.5 |
| $C_{12-18}$ fatty acid (e.g Edenor K 12-18, Henkel) | 5 |
| Alkohol ethoxylate $C_{12-18}$; 7EO | 20 |
| Alkylglycoside, $C_{12-14}$ ,degree of polymerization: 1,4 | 20 |
| Alkyl sulfate $C_{16-18}$ (e.g Sulfopon K35, Henkel) | 5 |
| Ethanol | 5 |
| 1,2 propylene glycol | 8 |
| Enzymes including lipolytic enzymes (calculated as pure enzyme protein) | 0.0001 - 0.1 |
| Water | to 100 |

Composition 18

A detergent composition formulated as non-aqueous liquid detergent

| | |
|---|---|
| Alkohol ethoxylate C10-12; 7EO | 28 |
| Alkohol ethoxylate C10-12; 3EO | 23 |
| Glycerol triacetate | 6 |
| Silicone antifoam | 1.5 |
| Alkyl benzene sulphonic acid | 7 |
| Sodium carbonate | 20 |

(continued)

| | |
|---|---|
| Calcite | 7 |
| Antiseeding polymer | 2 |
| Silica | 4 |
| Carboxy methyl cellulose | 2 |
| Peracids* | 0 - 1 |
| Brightener | 0,2 |
| Perfume | 0.6 |
| Enzymes including lipolytic enzymes (calculated as pure enzyme protein) | 0.0001 - 0.1 |

* As described in WO 95/06104

Composition 19

A detergent composition formulated as a non aqueous liquid detergent:

| | |
|---|---|
| Decylidene or dodecylidene diglycerol | 25 |
| C10-15 EO 7 ethoxylate | 25 |
| Sodium carbonate | 17 |
| Sodium perborate monohydrate | 11 |
| Alkylbenzene sulphonic acid | 6 |
| Calcium carbonate | 6 |
| Silica (dispersant) | 4 |
| Silicone suds suppressor | 3 |
| flourescer/antiashing polymer/antiredeposition polymer | 3 |
| Enzymes including lipolytic enzymes (calculated as pure enzyme protein) | 0.0001 - 0.1 |

Composition 20

A detergent composition formulated as aqueous liq. detergents

| | | |
|---|---|---|
| Decyl- or dodecylidene triglycerol | 25 | 0 |
| Decyl- or dodecylidene diglycerol | 0 | 12.5 |
| $C_{10-15}$ EO 7, ethoxylate | 0 | 12.5 |
| Fatty acid | 4.5 | 4.5 |
| Potassium hydroxide | 10 | 10 |
| Zeolite | 15 | 15 |
| Citric acid | 8 | 8 |
| Glycerol | 2 | 2 |
| Borax | 1.5 | 1.5 |
| Polymer | 1 | 1 |
| Silicone oil | 0.3 | 0.3 |
| Perfume | 0.5 | 0.5 |
| Enzymes including lipolytic enzymes (calculated as pure enzyme protein) | 0.0001 - 0.1 | |
| Water | to 100 | |

Composition 21

A liquid detergent composition comprising

| | | | | | | |
|---|---|---|---|---|---|---|
| Sodium C11-C15 Alkylbenzene sulfonate | 8 | 17 | 10 | | | 7 |
| Alcohol ethoxy sulfate (C12-14, 60% ethylene oxide by weight) | 12 | | 6 | | | 1 |
| Alcohol Ethoxylate (12-14C alcohol)ethoxylate | 8 | 7 | 8 | 16 | 8 | 4 |
| Alkylpolyglycoside | | | | | 16 | 15 |
| Trisodium citrate | 0-15 | 0-15 | 0-10 | 0-20 | 10 | 10 |
| Soap | 0-10 | 0-15 | | | 5 | 4 |
| Carboxymethylenoxysuccinate, trisodium | | | | | 10 | 0-20 |
| Oxysuccinate - tetrasodium | | | | | | 6 |
| MA/AA/Hydrophobe terpolymers * | 5-15 | 2-20 | 2-15 | 1-10 | 5 | 2-15 |
| Monoethanolamine | 1 | 2 | 2 | 0-4 | | 2 |
| Triethanolamine | | | 2 | | 4 | 4 |
| Sodium carbonate | | | | | | 1 |
| Borax pentahydrate | | | 3,5 | | 4 | 4 |
| Glycerol | | | 4 | | 6 | 5 |
| Propylene glycol | 10 | | | 10 | 2 | 5 |

* As described in the invention US 5,308,530

(continued)

| | | | | | | |
|---|---|---|---|---|---|---|
| Formic acid | 1 | | | 1 | | 1 |
| Calcium chloride | 1 | | 1 | 1 | 1 | 1 |
| Quaternary amines | | | | 2 | | |
| Ethoxylated Amine | 1 | | | 2 | 1 | |
| Alkyldimethyl Amine Oxide | | | | 1,5 | | |
| Na Xylene Sulfonates | 3 | 6 | 3 | 2 | | 3 |
| Ethanol | 10 | | 2 | 8 | 3 | 3 |
| Flourescers | 0.25 | 0.2 | 0.25 | 0.25 | 0.2 | 0.15 |
| Perfume | 0.2 | 0.15 | 0.1-0.3 | 0.2 | 0.25 | 0.1-0.25 |
| Enzymes including lipolytic enzymes (calculated as pure enzyme protein) | 0.0001 - 0.1 | | | | | |
| Sodium sulfate | to balance | | | | | |

Composition 22

An aqueous liquid detergent composition

| | |
|---|---|
| Silicon antifoam | 0.3 |
| Citric acid | 8 |
| Glycerol | 2 |
| Borax | 2 |
| KOH | 10 |
| Zeolite 4A | 8 |
| Polymer: Deflocculating polymer w. chemical structure as polymer A11 in EP 346,995 | 1.0 |
| QCC 200 : Bentonite clay | 8 |
| Oleic acid | 5 |
| LAS-acid | 17 |
| Synperonic A3 | 5 |
| Synperonic A7 | 5 |
| PVP | 0.3 |
| Perfume | 0.5 |
| Enzymes including lipolytic enzymes (calculated as pure enzyme protein) | 0.0001 - 0.1 |
| Water | To 100 |

**Dishwashing Composition**

The dishwashing detergent composition comprises a surfactant which may be anionic, non-ionic, cationic, amphoteric or a mixture of these types. The detergent will contain 0-90% of non-ionic surfactant such as low- to non-foaming ethoxylated propoxylated straight-chain alcohols.

The detergent composition may contain detergent builder salts of inorganic and/or organic types. The detergent builders may be subdivided into phosphorus-containing and non-phosphorus-containing types. The detergent composition usually contains 1-90% of detergent builders.

Examples of phosphorus-containing inorganic alkaline detergent builders, when present, include the water-soluble salts especially alkali metal pyrophosphates, orthophosphates, polyphosphates, and phosphonates. Examples

of non-phosphorus-containing inorganic builders, when present, include water-soluble alkali metal carbonates, borates and silicates as well as the various types of water-insoluble crystalline or amorphous alumino silicates of which zeolites are the best-known representatives.

Examples of suitable organic builders include the alkali metal, ammonium and substituted ammonium, citrates, succinates, malonates, fatty acid sulphonates, carboxymetoxy succinates, ammonium polyacetates, carboxylates, polycarboxylates, aminopolycarboxylates, polyacetyl carboxylates and polyhydroxsulphonates.

Other suitable organic builders include the higher molecular weight polymers and co-polymers known to have builder properties, for example appropriate polyacrylic acid, polymaleic and polyacrylic/polymaleic acid copolymers and their salts.

The dishwashing detergent composition may contain bleaching agents of the chlorine/bromine-type or the oxygen-type. Examples of inorganic chlorine/bromine-type bleaches are lithium, sodium or calcium hypochlorite and hypobromite as well as chlorinated trisodium phosphate. Examples of organic chlorine/bromine-type bleaches are heterocyclic N-bromo and N-chloro imides such as trichloroisocyanuric, tribromoisocyanuric, dibromoisocyanuric and dichloroisocyanuric acids, and salts thereof with water-solubilizing cations such as potassium and sodium. Hydantoin compounds are also suitable.

The oxygen bleaches are preferred, for example in the form of an inorganic persalt, preferably with a bleach precursor or as a peroxy acid compound. Typical examples of suitable peroxy bleach compounds are alkali metal perborates, both tetrahydrates and monohydrates, alkali metal percarbonates, persilicates and perphosphates. Preferred activator materials are TAED and glycerol triacetate.

The dishwashing detergent composition of the invention may be stabilized using conventional stabilizing agents for the enzyme(s), e.g. a polyol such as e.g.propylene glycol, a sugar or a sugar alcohol, lactic acid, boric acid, or a boric acid derivative, e.g. an aromatic borate ester.

The dishwashing detergent composition may also comprise other enzymes, in particular an amylase, a protease and/or a cellulase.

The dishwashing detergent composition of the invention may also contain other conventional detergent ingredients, e.g. deflocculant material, filler material, foam depressors, anti-corrosion agents, soil-suspending agents, sequestering agents, anti-soil redeposition agents, dehydrating agents, dyes, bactericides, fluorescers, thickeners and perfumes.

The first wash lipolytic enzyme of the invention may be incorporated in concentrations conventionally employed in detergents. It is at present contemplated that, in the detergent composition of the invention, the lipolytic enzyme may be added in an amount corresponding to 0.00001-1 mg (calculated as pure enzyme protein) of lipolytic enzyme per liter of wash liquor.

Below, specifically preferred diswashing compositions are exemplified:

1) POWDER AUTOMATIC DISHWASHING COMPOSITION

| | |
|---|---|
| Nonionic surfactant | 0.4 - 2.5% |
| Sodium metasilicate | 0 - 20% |
| Sodium disilicate | 3 - 20% |
| Sodium triphosphate | 20 - 40% |
| Sodium carbonate | 0 - 20% |
| Sodium perborate | 2 - 9% |
| Tetraacetyl ethylene diamine (TAED) | 1 - 4% |
| Sodium sulphate | 5 - 33% |
| Enzymes | 0.0001 - 0.1 % |

2) POWDER AUTOMATIC DISHWASHING COMPOSITION

| | |
|---|---|
| Nonionic surfactant (e.g. alcohol ethoxylate) | 1 - 2% |
| Sodium disilicate | 2 - 30% |
| Sodium carbonate | 10 - 50% |
| Sodium phosphonate | 0 - 5% |

(continued)

| | |
|---|---|
| Trisodium citrate dihydrate | 9 - 30% |
| Nitrilotrisodium acetate (NTA) | 0 - 20% |
| Sodium perborate monohydrate | 5 - 10% |
| Tetraacetyl ethylene diamine (TAED) | 1 - 2 % |
| Polyacrylate polymer (e.g. maleic acid/acrylic acid copolymer) | 6 - 25% |
| Enzymes | 0.0001 - 0.1 % |
| Perfume | 0.1 - 0.5% |
| Water | 5 - 10 |

3) POWDER AUTOMATIC DISHWASHING COMPOSITION

| | |
|---|---|
| Nonionic surfactant | 0.5 - 2.0% |
| Sodium disilicate | 25 - 40% |
| Sodium citrate | 30 - 55% |
| Sodium carbonate | 0 - 29% |
| Sodium bicarbonate | 0 - 20% |
| Sodium perborate monohydrate | 0 - 15 % |
| Tetraacetyl ethylene diamine (TAED) | 0 - 6 % |
| Maleic acid/acrylic acid copolymer | 0 - 5% |
| Clay | 1 - 3% |
| Polyamino acids | 0 - 20% |
| Sodium polyacrylate | 0 - 8% |
| Enzymes | 0.0001 - 0.1% |

4) POWDER AUTOMATIC DISHWASHING COMPOSITION

| | |
|---|---|
| Nonionic surfactant | 1 - 2% |
| Zeolite MAP | 15 - 42 % |
| Sodium disilicate | 30 - 34 % |
| Sodium citrate | 0 -12% |
| Sodium carbonate | 0 -20% |
| Sodium perborate monohydrate | 7 - 15 % |
| Tetraacetyl ethylene diamine (TAED) | 0 - 3% |
| Polymer | 0 - 4% |
| Maleic acid/acrylic acid copolymer | 0 - 5 % |
| Organic phosphonate | 0 - 4 % |
| Clay | 1 - 2% |
| Enzymes | 0.0001 - 0.1% |
| Sodium sulphate | Balance |

5) POWDER AUTOMATIC DISHWASHING COMPOSITION

| | |
|---|---|
| Nonionic surfactant | 1 - 7% |
| Sodium disilicate | 18 - 30% |
| Trisodium citrate | 10 - 24% |
| Sodium carbonate | 12 - 20% |
| Monopersulphate (2 $KHSO_5.KHSO_4.K_2SO_4$) | 15 -21% |
| Bleach stabilizer | 0.1 - 2% |
| Maleic acid/acrylic acid copolymer | 0 - 6% |
| Diethylene triamine pentaacetate, pentasodium salt | 0 - 2.5 % |
| Enzymes | 0.0001 - 0.1 % |
| Sodium sulphate, water | Balance |

6) POWDER AND LIQUID DISHWASHING COMPOSITION WITH CLEANING SURFACTANT SYSTEM

| | |
|---|---|
| Nonionic surfactant | 0 - 1.5% |
| Octadecyl dimethylamine N-oxide dihydrate | 0 - 5% |
| 80:20 wt.C18/C16 blend of octadecyl dimethylamine N-oxide dihydrate and hexadecyldimethyl amine N-oxide dihydrate | 0 - 4% |
| 70:30 wt.C18/C16 blend of octadecyl bis (hydroxyethyl)amine N-oxide anhydrous and hexadecyl bis (hydroxyethyl)amine N-oxide anhydrous | 0 - 5% |
| $C_{13}$-$C_{15}$ alkyl ethoxysulfate with an average degree of ethoxylation of 3 | 0 - 10% |
| $C_{12}$-$C_{15}$ alkyl ethoxysulfate with an average degree of ethoxylation of 3 | 0 - 5% |
| $C_{13}$-$C_{15}$ ethoxylated alcohol with an average degree of ethoxylation of 12 | 0 - 5% |
| A blend of $C_{12}$-$C_{15}$ ethoxylated alcohols with an average degree of ethoxylation of 9 | 0 - 6.5% |
| A blend of $C_{13}$-$C_{15}$ ethoxylated alcohols with an average degree of ethoxylation of 30 | 0 - 4% |
| Sodium disilicate | 0 - 33% |
| Sodium tripolyphosphate | 0 - 46% |
| Sodium citrate | 0 - 28% |
| Citric acid | 0 - 29% |
| Sodium carbonate | 0 - 20% |
| Sodium perborate monohydrate | 0 -11.5% |
| Tetraacetyl ethylene diamine (TAED) | 0 - 4% |
| Maleic acid/acrylic acid copolymer | 0 - 7.5% |
| Sodium sulphate | 0 - 12.5% |
| Enzymes | 0.0001 - 0.1% |

7) NON-AQUEOUS LIQUID AUTOMATIC DISHWASHING COMPOSITION

| | |
|---|---|
| Liquid nonionic surfactant (e.g. alcohol ethoxylates) | 2.0 - 10.0% |
| Alkali metal silicate | 3.0 - 15.0% |
| Alkali metal phosphate | 20.0 - 40.0% |
| Liquid carrier selected from higher glycols, polyglycols, polyoxides, glycolethers | 25.0 - 45.0% |
| Stabilizer (e.g. a partial ester of phosphoric acid and a $C_{16}$-$C_{18}$ alkanol) | 0.5 - 7.0% |

(continued)

| | |
|---|---|
| Foam suppressor (e.g. silicone) | 0 - 1.5% |
| Enzymes | 0.0001 - 0.1% |

8) NON-AQUEOUS LIQUID DISHWASHING COMPOSITION

| | |
|---|---|
| Liquid nonionic surfactant (e.g. alcohol ethoxylates) | 2.0 - 10.0% |
| Sodium silicate | 3.0 - 15.0% |
| Alkali metal carbonate | 7.0 - 20.0% |
| Sodium citrate | 0.0 - 1.5% |
| Stabilizing system (e.g. mixtures of finely divided silicone and low molecular weight dialkyl polyglycol ethers) | 0.5 - 7.0% |
| Low molecule weight polyacrylate polymer | 5.0 - 15.0% |
| Clay gel thickener (e.g. bentonite) | 0.0 - 10.0% |
| Hydroxypropyl cellulose polymer | 0.0 - 0.6% |
| Enzymes | 0.0001 - 0.1% |
| Liquid carrier selected from higher lycols, polyglycols, polyoxides and glycol ethers | Balance |

9) THIXOTROPIC LIQUID AUTOMATIC DISHWASHING COMPOSITION

| | |
|---|---|
| $C_{12}$-$C_{14}$ fatty acid | 0 - 0.5% |
| Block co-polymer surfactant | 1.5 - 15.0% |
| Sodium citrate | 0 - 12% |
| Sodium tripolyphosphate | 0 - 15% |
| Sodium carbonate | 0 - 8% |
| Aluminium tristearate | 0 - 0.1% |
| Sodium cumene sulphonate | 0 - 1.7% |
| Polyacrylate thickener | 1.32 - 2.5% |
| Sodium polyacrylate | 2.4 - 6.0% |
| Boric acid | 0 - 4.0% |
| Sodium formate | 0 - 0.45% |
| Calcium formate | 0 - 0.2% |
| Sodium n-decydiphenyl oxide disulphonate | 0 - 4.0% |
| Monoethanol amine (MEA) | 0 - 1.86% |
| Sodium hydroxide (50%) | 1.9 - 9.3% |
| 1,2-Propanediol | 0 - 9.4% |
| Enzymes | 0.0001 - 0.1 % |
| Suds suppressor, dye, perfumes, water | Balance |

10) LIQUID AUTOMATIC DISHWASHING COMPOSITION

| | |
|---|---|
| Alcohol ethoxylate | 0 - 20 % |
| Fatty acid ester sulphonate | 0 - 30% |

(continued)

| | |
|---|---|
| Sodium dodecyl sulphate | 0 - 20% |
| Alkyl polyglycoside | 0 - 21 % |
| Oleic acid | 0 - 10% |
| Sodium disilicate monohydrate | 18 - 33% |
| Sodium citrate dihydrate | 18 - 33 % |
| Sodium stearate | 0 - 2.5% |
| Sodium perborate monohydrate | 0 - 13% |
| Tetraacetyl ethylene diamine (TAED) | 0 - 8% |
| Maleic acid/acrylic acid copolymer | 4 - 8% |
| Enzymes | 0.0001 - 0.1% |

11) LIQUID AUTOMATIC DISHWASHING COMPOSITION CONTAINING PROTECTED BLEACH PARTICLES

| | |
|---|---|
| Sodium silicate | 5 - 10% |
| Tetrapotassium pyrophosphate | 15 - 25% |
| Sodium triphosphate | 0 - 2% |
| Potassium carbonate | 4 - 8% |
| Protected bleach particles, e.g. chlorine | 5 - 10% |
| Polymeric thickener | 0.7 - 1.5% |
| Potassium hydroxide | 0 - 2% |
| Enzymes | 0.0001 - 0.1% |
| Water | Balance |

11) Automatic dishwashing compositions as described in 1), 2), 3), 4), 6) and 10), wherein perborate is replaced by percarbonate.

12) Automatic dishwashing compositions as described in 1) - 6) which additionally contain a manganese catalyst. The manganese catalyst may, e.g., be one of the compounds described in "Efficient manganese catalysts for low-temperature bleaching", Nature 369, 1994, pp. 637-639.

Furthermore, the first wash lipolytic enzyme of the invention may be used in softening compositions:

The lipolytic enzyme of the invention may be used in fabric softeners, e.g. as described in Surfactant and Consumer Products, Ed. by J. Falbe, 1987, pp 295-296; Tenside Surfactants Detergents, 30 (1993), 6, pp 394-399; JAOCS, Vol. 61 (1984), 2, pp 367-376; EP 517 762; EP 123 400; WO 92/19714; WO 93/19147; US 5,082,578; EP 494 769; EP 544 493; EP 543 562; US 5,235,082; EP 568 297; EP 570 237.

MATERIALS AND METHODS

**Lipase activity (LU)**

A substrate for lipase was prepared by emulsifying glycerine tributyrate (MERCK) using gum-arabic as emulsifier. Lipase activity is assayed at pH 7 using pH stat method. One unit of lipase activity (LU) is defined as the amount needed to liberate one micromole fatty acid per minute.

**Strains and plasmids:**

*Humicola lanuginosa* DSM 4109 available from the Deutsche Sammlung von Mikroorganismen und Zellkulturen GmbH, Mascheroderweg 1b, D-3300 Braunschweig, Federal Republic of Germany (EP 305,216)
*Saccharomyces cerevisiae* YNG318: MATa Dpep4[cir$^+$] ura3-52, leu2-D2, his 4-539 *Aspergillus oryzae* IFO 4177

*A. oryzae* A 1560-T40, a protease deficient derivative of A. oryzae IFO 4177 (WO 91/17243).
*A. oryzae* JaL 125: *Aspergillus oryzae* IFO 4177 available from Institute for Fermention, Osaka; 17-25 Juso Hammachi 2-Chome Yodogawa-ku, Osaka, Japan, having the alkaline protease gene named "alp" (described by Murakami K et al., (1991), Agric. Biol. Chem. 55, p. 2807-2811) deleted by a one step gene replacement method (described by G. May in "Applied Molecular Genetics of Filamentous Fungi" (1992), p. 1-25. Eds. J. R. Kinghorn and G. Turner; Blackie Academic and Professional), using the *A. oryzae* pyrG gene as marker.
*Absidia reflexa* ATTC 44896 is available from ATCC (American Type Culture Collection, 12301, Parklawn Drive, Rockville, Maryland 20852. USA) as *Absidia reflexa* ATTC 44896 and from IFO (Institute for Fermentation, 17-85 Juso-horrmachi 2-chomee, Yodogawa-ku, Osaka 532, Japan) as *Absidia reflexa* IFO 5874 as described in WO 96/113578 (Novo Nordisk A/S).
Yeast cell YPH499 (Stratagene)
*E.coli* DH10B (Gibco)
pTiK04: constructed from pJSO37 including the mature *Ab reflexa* NL 127 lipase gene with a SPIRR encoding extension upstream of the start of the lipase gene.
pTiK05: As pTiK04 without the SPIRR extension
pTiK06: pTik04 with the MF$\alpha$1 signal sequence
pTiK07: pTik05 with the MF$\alpha$1 signal sequence
pYESHL is a yeast/*E. coli* shuttle vector that expresses and secretes a low level of the *H. lanuginosa* lipolytic enzyme in yeast. More specifically pYESHL is a derivative of pYES2 in which the GAL1 promoter was excised and the *H. lanuginosa* lipolytic enzyme gene and the TPI (triose phosphate isomerase) promoter from *S. cerevisiae* (Alber, T. and Kawasaki, G., J.Mol.Appl. Genet 1, 419-434 (1982) were cloned between the SphI and XbaI sites. A restriction map of pYESHL is shown in Fig. 1.
PJSO37 (*S. cerevisiae* expression plasmid)(J.S.Okkels, (1996)"A URA3-promoter deletion in a pYES vector increases the expression level of a fungal lipase in Saccharomyces cerevisiae. Recombinant DNA Biotechnology III: The Integration of Biological and Engineering Sciences, vol. 782 of the Annals of the New York Academy of Sciences) More specifically, the expression plasmid pJSO37, is derived from pYES 2.0 by replacing the inducible GAL1-promoter of pYES 2.0 with the constitutively expressed TPI (triose phosphate isomerase)-promoter from *Saccharomyces cerevisiae* (Albert and Karwasaki, (1982), J. Mol. Appl Genet., 1, 419-434), and deleting the URA3 promoter. A restriction map of pJSO37 is shown in Fig. 6.
pYES 2.0 (Invitrogen Corp., UK)
p960 *A. oryzae* expression plasmid (described in EP 305 216)
pUC19 (Yanish-Perron et al. (1985) Gene 33, 103-119)
pHD414 (*Aspergillus* expression vector being a derivative of the plasmid p775 described in EP 238 023). The construction of pHD414 is further described in WO 93/11249).
PJVi245 (See Figure 8)
pCaHj383 (see Figure 8)
pCaHj385 (see Figure 8)

**Low calcium filter assay**

Procedure

1) Provide SC Ura replica plates (useful for selecting strains carrying the expression vector) with a first protein binding filter (Nylon membrane) and a second low protein binding filter (Cellulose acetate) on the top.
2) Spread yeast cells containing a parent lipolytic enzyme gene or a mutated lipolytic enzyme in question on the double filter and incubate for 2 or 3 days at 30°C.
3) Keep the colonies on the top filter by transferring the topfilter to a new plate.
4) Remove the protein binding filter to an empty petri dish.
5) Pour an agarose solution comprising an olive oil emulsion (2% P.V.A.:Olive oil=3:1), Brilliant green (indicator, 0.004%), 100 mM tris buffer pH9 and EGTA (final concentration 5mM) on the bottom filter so as to identify colonies expressing lipolytic activity in the form of blue-green spots.
6) Identify colonies found in step 5) having a reduced dependency on calcium as compared to the parent lipolytic enzyme.

Dobanol®25-7 filter assay:

The screening for an improved tolerance towards a detergent component is performed by use of a filter assay corresponding to that described above except for the fact that the solution defined in 5) further comprises 0.02% Dobanol®25-7.

An alternative screening assay is the following:

1) Provide SC Ura-plates (useful for selecting strains carrying an expression vector) with first a protein binding filter (e.g. nylon) followed by a non-protein binding filter (e.g. Cellulose acetate) on the top.
2) Spread yeast cells containing a parent lipase gene or a mutated lipase gene on the filter and incubate for 3 or 4 days at 30°C.
3) Keep the colonies on the top filter by transferring the topfilter to a new plate.
4) Remove the protein binding filter to a petri dish containing:
An agarose solution comprising an olive oil emulsion (2% P.V.A.:Olive oil=2:1), Brilliant green (indicator,0.004%), 100 mM tris buffer pH10 and the detergent or detergent component, e.g. PCS-plates. The protein binindg filter should have teh colony side facing the screening plate.
5) Identify colonies expressing lipase activity in the form of blue-green spots found in step 4)

Alternatively, the non-protein binding filter (or a protein binding filter) carrying the yeast colonies may be used directly on the screening plate.

**Construction of random mutagenized libraries**

*a) Rationale and mathematics behind the desing of random mutagenized libraries*

The overall rationale for the random mutagenesis is to mimic the evolution in nature where a low continuous mutagenesis is coupled to a continuous selection for a better mutant which is then further mutagenized. Similarly, the recent in vitro evolution studies described in the litterature have been performed with consecutive rounds of mutagenesis with increasing selection pressure (for a review see Joyce 1992). We have adapted this by using the wt gene in the first rounds of mutagenesis. Improved variants are then used in the next rounds of mutagenesis (to improve by small steps). We have screened under wash correlated conditions that are only just enough to knock out the wt enzyme activity or improved variants activity. This means that we increase the stringency of screening when better and better variants are isolated.

To increase the number of exchanges and to increase the likelyhood of finding improved variants, localized random mutagenesis have also been performed. Important regions deduced from the structure of Lipolase and from results from site-directed mutagenesis were selected. E.g. the whole lipid contact zone was considered as important for improvement, especially the lid region and the lid-contact regions. The lipid contact zone corresponds to 7 regions on the gene which have been mutated. Combinations of the regions have also been done.

*b) Random mutagenesis of an entire lipolytic enzyme coding gene*

The plasmid pYESHL is treated with 12 M formic acid for 20 min. at room temperature. The resulting lipolytic enzyme encoding gene is amplified from the formic acid treated plasmid using PCR under mutagenic conditions (0.5 mM $MnCl_2$ and 1/5 the normal amount of ATP, see e.g. Leung et al., 1989. This treatment is expected to give a broad range of mutations since formic acid gives mainly transversions and PCR generated mutations mainly transitions.

The resulting PCR fragments are cloned either by double recombination (Muhlrad et al., 1992) in vivo into the shuttle vector or digestion and ligation into the shuttle vector and transformation of *E. coli*.

Eight randomly picked clones have been sequenced and were found to contain 2-3 mutations in average - both transversion and transitions.

By use of this method seven libraries were made containing from 10,000 to 140,000 clones.

*c) Localized random mutagenesis*

A mutagenic primer (oligonucleotide) is synthesized which corresponds to the part of the DNA sequence to be mutagenized except for the nucleotide(s) corresponding to amino acid codon(s) to be mutagenized. Subsequently,

the resulting mutagenic primer is used in a PCR reaction with a suitable opposite primer. The resulting PCR fragment is purified and digested and cloned into the shuttle vector. Alternatively and if necessary, the resulting PCR fragment is used in a second PCR reaction as a primer with a second suitable opposite primer so as to allow digestion and cloning of the mutagenized region into the shuttle vector. The PCR reactions are performed under normal conditions. When synthesizing the oligonucleotides used for the localized random mutagenesis, calculation of the doping level is important to estimate the mutagenesis frequency. The frequency of nucleotide exchanges can be calculated using the Binomial distribution formula:

$$P(i) = \frac{N!}{i!(N-i)!} \times p^{i} \times (1-p)^{N-i}$$

Where N = the number of doped oligo nucleotides; p = the fraction of none wt nucleotides; i = number of nucleotide exchanges; P(i) = the probability for the i number of exchanges. It is difficult to calculate the exact number of aa exchanges from the number of nucleotide exchanges, because the third position in a codon for most of the aa can be two or all four nucleotides with out changing the aa. The same is the case for the first or second position for the three aa with 6 codons. For estimating the number of aa exchanges a Monte-Carlo simulation is more appropriate. For example the program called RAMHA performs such a simulation (described in Siderovski and Mak 1993). This program simulates the synthesis of e.g. 10,000 oligonucleotides with the desired doping and calculates the frequency of 0 to n aa exchanges.

*A doping example*

The relationship between doping and aa exchanges in a region of 13 codons is (calculated using a Monte Carlo simulation):

| Percent | 0 | 1 | 2 | 3 | 4 | 5 | 6 | 7 |
|---|---|---|---|---|---|---|---|---|
| doping | mutations | | | | | | | |
| level | | | | | | | | |
| 5% | 0.2 | 0.35 | 0.27 | 0.13 | 0.05 | 0 | 0 | 0 |
| 10% | 0.04 | 0.13 | 0.24 | 0.25 | 0.19 | 0.11 | 0.05 0 | |
| 15% | 0.005 | 0.03 | 0.10 | 0.20 | 0.24 | 0.23 | 0.13 | 0.07 |

The possible number of combinations of aa exchanges for 13 aa can be calculated using the formula:

$$N = \frac{y!}{x!(y-x)!} \, 20^{x}$$

y = number of aa mutagenized
x = number of aa exchanges

1 aa exchange in 13 aa = 260 possible exchanges
2 - - - - = 31200 - combinations
3 - - - - = $2.3 \times 10^{6}$ - -

From this follows that when screening e.g 100,000 colonies of a library doped with 10% in 13 codons giving the distribution shown in the above table will mean screening of about 13,000 with one aa exchange (13%). There are, however, only 260 possible one aa exchanges, so a large number of the same one aa exchanges are being screened. A higher doping of e.g. 15% (in the above table) will give fewer one aa exchanges (about 3 %), however, the two aa exchanges will also be lowered to a degree (10%) that will not enable screening of the 31200 possible combinations with a screenings capacity around 100.000.

Finally, the aa exchanges are biased by the origin of the wt amino acid. E.g. it takes only one nucleotide exchange to change Glu to Ala, but three from Glu to Phe. This means that the probability is lower for the aa exchanges that requires 2 or 3 nucleotide exchanges than for those that requires one nucleotide exchange. Therefore we have in some cases allowed more than one aa at positions where we know it is possible. We have always chosen G/C at the

third position of the codons with four or six codons. This lowers the bias of the wt codon and also lowers the likelyhood of stop codon (from 4.7% to 3.1 % if completely scrambled). For a calculation of the probability of whether a given pool size contain the most probable and least probable replacement mutants, see Palzkill et al. 1994.

*Calculation of population distribution in screening of amplified libraries*

Another consideration may be taken into account. Most of the libraries presented herein are amplified in E. coli before they are transformed into yeast. This means that there is a probability for screening the same amplified clone more than once - see box I.

---

**Box I**

---

Screening of an amplified random mutagenized library of e.g. 100,000 different clones:

64 % of the library is screened when 100,000 colonies have been screened.
90 % - - - - 230,000 - .
95 % - - - - 300,000 - .

This is assuming that all 100,000 clones are amplified evenly.
The following formula can be used to calculate this:

$$N = \frac{\ln(1-P)}{\ln(1-1/D)}$$

N is the number of screened clones, P is the fraction of different clones screened and D is the total number of different clones.

---

*Anti-termination strategies*

In order to avoid premature truncated proteins nonsense mutations should be avoided in the codons with a potential to form stop codons. For codons that can be substituted with alternative codons with out the potential to form stop codons, the following strategies can be used:

| | |
|---|---|
| Gly: GGA | GG(G,C,T) |
| Leu: TT(A,G) | CTN |
| Arg: (A,C)GA | (A,C)GG or CG(C,T) |
| Ser: TC(A,G) | TC(C,T) or AG(C,T) |

The following aa can, however, only be specified with codons exhibiting stop-codon potential: Cys, Glu, Lys, Gln, Trp, and Tyr. Therefore only the doping can be designed to circumvent the random placement of nucleotides producing stop codons. For example:

Glu (similar for Lys and Gln): (90%G/5%C,A) (90%A/3.3%C,G,T) (90%A/3.3%C,G,T). No TAA or TAG = STOP.
Tyr (similar for Cys): (90%T/3.3%A,C,G) (90%A/3.3%C,G,T) (90%C/10%T). No TAG or TAA = STOP.
Trp: (90%T/3.3%A,C,G) (90%G/5%C,T) (90%G/5%C,T). No TGA or TAG = STOP.

Such a strategy will of course abolish certain a.a. exchanges. Using these strategies the number of premature truncated proteins will be lowered dramatically.

### *In vivo* recombination of *Humicola lanuginosa* lipase variants (Gene shuffling)

The DNA sequences of a number of *Humicola lanuginosa* lipase variants can be *in vivo* recombined in the same mixture.
Vectors are prepared from the lipase variants by ligation into the yeast expression vector pJSO37. All vectors are cut open with NruI.
DNA fragment of all homologous DNA sequences are prepared by PCR amplification using standard methods.
The DNA fragments and the opened vectors are mixed and transformed into the yeast *Saccharomyces cerevisiae* YNG318 by standard methods. The recombination host cell is cultivated and screened as described above. Apearing transformants are isolated and tested for improved wash performance using one of the filter assay methods described above.
Positive transformants are variants with improved wash performance resulting from gene shuffling of homologous DNA sequences.

### Site-directed in vitro mutagenesis of lipolytic enzyme gene

One approach which may be used for introducing mutations into the lipolytic enzyme gene is described in Nelson & Long, Analytical Biochemistry, 180, 147-151 (1989). It involves the 3-step generation of a PCR (polymerase chain reaction) fragment containing the desired mutation introduced by using a chemically synthesized DNA-strand as one of the primers in the PCR-reactions. The construction of a PCR fragment may be performed in accordance with methods known in the art. From the PCR generated fragment, a DNA fragment carrying the mutation can be isolated by cleavage with restriction enzymes and re-inserted into the expression plasmid. In figures 4 and 5 the method is further outlined.
An alternative method for the construction of variants of a *H. lanuginosa* lipolytic enzyme involves the use of the commercial kit, Chameleon double-stranded, site-directed mutagenesis kit according to the manufacturer's instructions.
The gene encoding the lipolytic enzyme in question is inserted into the plasmid pHD414. In accordance with the manufacturer's instructions the ScaI site of the Ampicillin gene of pHD414 is changed to a MluI site by use of the following primer:

Primer 3: AGAAATCGGGTATCCTTTCAG

The pHD414 vector comprising the lipolytic gene in question is then used as a template for DNA polymerase and oligos 7258 and 7770 the sequences of which are disclosed in the Examples hereinafter. The desired mutation (*e.g.* in the N-terminal of the lipolytic gene) is introduced into the lipolytic gene in question by addition of an appropriate oligos comprising the desired mutation. When an N-terminal peptide addition is applied this may be accomplished by mutating codons of the DNA sequence encoding the pro- or prepro part of the parent lipolytic enzyme.
PCR reactions are performed according to the manufacturer's recomendations.
<u>DNA sequencing</u> was performed by using Applied Biosystems ABI DNA sequence model 373A according to the protocol in the ABI Dye Terminator Cycle Sequencing kit.

### Expression of Humicola lanuginosa lipolytic enzyme in *Aspergillus oryzae*

Cloning of *H. lanuginosa* lipolytic enzyme is described in EP 305 216. It also describes expression and characterization of the enzyme in *A. oryzae*. The expression plasmid used is named p960.
The expression plasmid used in this application is identical to p960, except for minor modifications just 3' to the lipase coding region. The modifications were made the following way: p960 was digested with NruI and BamHI restriction enzymes. Between these two sites the BamHI/NheI fragment from plasmid pBR322, in which the NheI fragment was filled in with Klenow polymerase, was cloned, thereby creating plasmid pAO1 (figure 2), which contains unique BamHI and NheI sites. Between these unique sites BamHI/XbaI fragments from p960 was cloned to give pAHL (figure 3).

**Transformation of Aspergillus oryzae (general procedure)**

Transformation of *Aspergillus oryzae* (general procedure)

100 ml of YPD (Sherman et al., (1981), Methods in Yeast Genetics, Cold Spring Harbor Laboratory) are inoculated with spores of *A. oryzae* and incubated with shaking for about 24 hours. The mycelium is harvested by filtration through miracloth and washed with 200 ml of 0.6 M $MgSO_4$. The mycelium is suspended in 15 ml of 1.2 M $MgSO_4$, 10 mM $NaH_2PO_4$, pH 5.8. The suspension is cooled on ice and 1 ml of buffer containing 120 mg of Novozym• 234, batch 1687 is added. After 5 min., 1 ml of 12 mg/ml BSA (Sigma type H25) is added and incubation with gentle agitation continued for 1.5-2.5 hours at 37°C until a large number of protoplasts is visible in a sample inspected under the microscope.

The suspension is filtered through miracloth, the filtrate transferred to a sterile tube and overlayed with 5 ml of 0.6 M sorbitol, 100 mM Tris-HCl, pH 7.0. Centrifugation is performed for 15 min. at 1000 g and the protoplasts are collected from the top of the $MgSO_4$ cushion. 2 volumes of STC (1.2 M sorbitol, 10 mM Tris-HCl, pH 7.5, 10 mM $CaCl_2$) are added to the protoplast suspension and the mixture is centrifugated for 5 min. at 1000 g. The protoplast pellet is resuspended in 3 ml of STC and repelleted. This is repeated. Finally, the protoplasts are resuspended in 0.2-1 ml of STC.

100 µl of protoplast suspension are mixed with 5-25 µg of p3SR2 (an *A. nidulans* amdS gene carrying plasmid described in Hynes et al., Mol. and Cel. Biol., Vol. 3, No. 8, 1430-1439, Aug. 1983) in 10 µl of STC. The mixture is left at room temperature for 25 min. 0.2 ml of 60% PEG 4000 (BDH 29576), 10 mM $CaCl_2$ and 10 mM Tris-HCl, pH 7.5 is added and carefully mixed (twice) and finally 0.85 ml of the same solution are added and carefully mixed. The mixture is left at room temperature for 25 min., spun at 2.500 g for 15 min. and the pellet is resuspended in 2 ml of 1.2M sorbitol. After one more sedimentation the protoplasts are spread on minimal plates (Cove, (1966), Biochem. Biophys. Acta 113, 51-56) containing 1.0 M sucrose, pH 7.0, 10 mM acetamide as nitrogen source and 20 mM CsCl to inhibit background growth. After incubation for 4-7 days at 37°C spores are picked, suspended in sterile water and spread for single colonies. This procedure is repeated and spores of a single colony after the second re-isolation are stored as a defined transformant.

Transformation of *A. oryzae* A1560-T40

The plasmid carrying a DNA sequence encoding a variant of the invention is transformed into *Aspergillus oryzae* A1560-T40, a protease deficient derivative of *A. oryzae* IFO 4177, using selection on acetamide by cotransformation with pToC 90 harboring the amdS gene from A. nidulans as a 2.7 kb Xba I fragment (Corrick et al. (1987), GENE 53, 63-71) on a pUC 19 vector (Yannisch-Perron et al. (1985), GENE **33**, 103-119). Transformation is performed as described in EP 238 023.

Fed batch fermentation

Fed batch fermentation is performed in a medium comprising maltodextrin as a carbon source, urea as a nitrogen source and yeast extract. The fed batch fermentation is performed by inoculating a shake flask culture of *A. oryzae* host cells in question into a medium comprising 3.5% of the carbon source and 0.5% of the nitrogen source. After 24 hours of cultivation at pH 5.0 and 34°C the continuous supply of additional carbon and nitrogen sources are initiated. The carbon source is kept as the limiting factor and it is secured that oxygen is present in excess. The fed batch cultivation is continued for 4 days, after which the enzymes can be recovered by centrifugation, ultrafiltration, clear filtration and germ filtration. Further purification may be done by anionexchange chromatographic methods known in the art.

Purification of *H. lanuginosa* lipolytic enzyme variants expressed in *S. cerevisiae*

The fermentation broth is sterile filtered and ammonium acetate (92 g) is added to the filtrate (1400 ml) to give a 0.8 M solution of ammonium acetate. The solution is added onto a Toyopearl Butyl column (XK 16/10). The column is washed with 0.8 M ammonium acetate and the lipolytic enzyme eluted in $H_2O$ at a flow rate of 5 ml/min. 10 ml fractions are collected and lipolytic enzyme containing factions are pooled according to activity in the standard lipase titration assay. The lipase containing pool are filtered and the pH is adjusted to pH 8.5 and added onto a Q-Sepharose column (HPQ XK 26/10). The column is washed with 200 ml 0.1 M Tris-HCl, pH 8.5 and the lipolytic enzyme eluted in a linear gradient of 0 to 0.3 M NaCl in 400 ml of 0.1 M Tris-HCl, pH 8.5 at a flow rate of 5 ml/min. 10 ml fractions are collected and the lipase containing fractions pooled according to activity in the standard lipase titration assay. Fractions containing lipase activity and absorption A280/A260 nm is greater than 1.7 are pooled.

Purification of *H. lanuginosa* lipolytic enzyme variants without peptide addition and expressed *A. oryzae*

Fermentation supernatant from the *A. oryzae* culture is centrifuged and cell debris discarded. The supernatant is filtered though a 0.45 μ millipore filter. Then the is precipitated with 60 % saturated ammonium sulphate. The precipitate is dissolved in water and solid ammonium acetate added to a final concentration of 0.8 M. The solution is applied onto a Butyl Toyopearl column pre-equilibrated with 0.8 M ammonium acetate. The bound enzyme is eluted with gradient using water and 50% ethanol as eluent.

Fractions containing enzyme activity are then pooled and conductance is adjusted to lower than 5 mSi and pH is adjusted to 8.5.

The pools containing activity are then applied onto an anion exchange column (*e.g.* High-performance Q Separose®) pre-equilibrated with 25 mM Tris-acetate buffer, pH 8.5. The bound activity is eluted with linear salt gradient using same buffer and 0.5 M sodium chloride. Fractions containing high lipolytic enzyme activity are pooled. Fractions containing lipase activity and absorption A280/A260 nm is greater than 1.7 are pooled.

**Assay for test of First Wash effect**

The wash performance of lipolytic enzymes was tested in a one cycle wash trial carried out in a thermostated Terg-O-Tometer (TOM) followed by linedrying.

The experimental conditions were as follows:

Wash liquor: 1000 ml per beaker
Swatches: 7 cotton swatches (9 x 9 cm) per beaker.
Stain: Lard coloured with sudan red (Sigma) (0.75 mg sudan red/g of lard). 50 μl of lard/sudan red heated to 70°C were applied to the centre of each swatch. After application of the stain the swatches were heated in an oven for 25 minutes at 75°C.
Stored overnight at room temperature.
Water: 3.2 mM $Ca^{2+}/Mg^{2+}$ (in a ratio of 5:1)
Detergent: 5 g/l of Detergent Composition A or Detergent B. pH adjusted artificially to 10 by NaOH.

Detergent Composition A:

0.300 g/l of alkyl sulphate (AS; $C_{14-16}$)
0.650 g/l of alcohol ethoxylate (AEO; $C_{12-14}$, 6EO)
1.750 g/l of Zeolite P
0.145 g/l of $Na_2CO_3$
0.020 g/l of Sokalan CP5
0.050 g/l of CMC (carboxy- methyl-cellulose)
Mixed in 3.2 mM $Ca^{2+}/Mg^{2+}$ (5:1) in Milli-Q water, pH 10.2

Detergent Composition B

as Detergent Composition A but additional containing the following bleaching agents:

0.900 g/l Sodium carbonate peroxyhydrate
0.300 g/l TAED (tetra-acetyl-ethylene-diamine)

Concentration of lipolytic enzyme (in Detergent composition A as well as B): 0 and 1250 or 12500 LU/l

Wash Time: 20 minutes
Wash temperature: 30°C
Rinse: 15 minutes in running tap water Drying: Overnight at room conditions (approx. 20°C, 30-40% RH).
Evaluation: The reflectance was measured at 460 nm. Afterwards, the fatty matter was extracted from the swatches with chloroform in a Soxhlet extraction apparatus, distilling off the solvent and determining the amount of fatty matter left on the swatches gravimetrically.

The amout of fatty material may alternatively be determined using thin layer chromatography(TLC)/Flame Ionization

Detector (FID)].

The percentage of lard removed is determined as:

1) % removal defined as:
[(remaining fat on swatches washed with detergent without lipolytic enzyme) minus (remaining fat on swatches washed with detergent with lipolytic enzyme)] divided by (remaining fat on swatches washed with detergent without lipolytic enzyme) and multiplied by 100%, or
2) delta refectance(dR) defined as:
(R(swatches washed in detergent with lipase)-R(swatches washed in detergent without lipase). The reflectance (which may also be termed remission) is measured on an Elrepho 2000 apparatus from Datacolor which illuminates the sample with 2 xenon blitzlambs and measures the amount of reflected light so that entirely white correspond to a 100% reflection and entirely black a 0% reflection.

Media and substrates:

YPD: 10 g yeast extract, 20 g peptone, $H_2O$ to 810 ml. Autoclaved, 90 ml 20% glucose (sterile filtered) added.
LB-medium: 10 g Bacto-tryptone, 5 g Bacto yeast extract, 10 g NaCl in 1 litre water.
FG4 medium: 3% soy meal, 3% maltodextrin, 1% peptone, pH adjusted to 7.0 with 4 M NaOH
SC Ura- plates: 10% 10xBasal salts with out amino acids, 0.5% Casamino acids, 0.02% Threonine, 0.01 % Tryptophane, 2 % Glucose, 1.5% Agar. 10xBasal salts with out amino acids: 60g NaOH, 66.8g Yeast nitrogen base with out amino acids (Difco), and 100g Succinic acid in 1 liter water.
Litex Agarose HSB 2000 (CAT NO: F90472)
BG-reagent: 4 mg/ml Brilliant Green (BG) dissolved in water
Substrate 1:

10 ml Olive oil (Sigma CAT NO. 0-1500)
20 ml 2% polyvinyl alcohol (PVA)

The Substrate is homogenised for 15-20 minutes.

PCS detergent

10 g/l:

| | |
|---|---|
| SDS | 0.52 g |
| Dobanol 25-3 | 0.60 g |
| Dobanol 25-1 | 0.58 g |
| $NaBO_3H_2O$ | 1.50 g |

Ad 1 litre 0.1 M Tris buffer (pH 9), and dilute further with the Tris buffer to the double concentration of the desired concentration on the PCS plates.

PCS-plates

| Solution for making PCS plates | |
|---|---|
| Brilliant Green (BG-reagent) | 10 ml |
| Substrate 1 | 24 ml |
| PCS detergent | 500 ml |
| 2% agarose (in TRIS buffer (pH 9) | 500 ml |

Lipase Substrate (Sigma catalogue no. 800-1)
Brilliant Green (Merck, art. No. 1.01310)

EXAMPLES

EXAMPLE 1

**Construction of random lipolytic enzyme variants**

Random mutagenized libraries of the entire *H. lanuginosa* lipolytic enzyme gene and of amino acids (aa) 91-97 and 206-211 thereof were prepared as described in Materials and Methods above.

The amino acid regions 91-97 and 206-211 were chosen for the first round of localized mutagenesis since these regions have been found to be important for wash performance.
Region 91-97 is a part of the lid region of the enzyme and region 206-211 constitutes part of the hydrophobic cleft of the enzyme.

One oligonucleotide was synthesized for each of these regions comprising 93% of the wild type nucleotides and 2.33% of each of the other three nucleotides at amino acid codons wanted to be mutagenized. Where possible without changing the amino acid, the third nucleotide (the wobble base) in codons were synthesized with 50%G/50%C to give a larger likelihood for changes to amino acids with one or two codons. The composition of the mutagenic oligonucleotide of region 91-97 is shown in Table 1.

By use of this oligonucleotide a calculated mutation frequency of approximately 65-70% is obtained in the library for one amino acid change having been introduced in the parent lipolytic enzyme. The mutation frequency for two or more amino acid changes having been introduced are less than 35 %. This low mutation frequency is chosen to ensure that the observed amino acid changes in positive clones are involved in improving the enzyme and not just "neutral" changes due to a high mutation frequency.

The mutagenic primers were used in a PCR reaction with a suitable opposite primer. The resulting PCR fragments were purified and in the case of region 206-211 digested and cloned into the shuttle vector. In the case of region 91-97 the resulting PCR fragment was used in a second PCR reaction as a primer with a second suitable opposite primer. This step was necessary to be able to digest and clone the mutagenized region into the shuttle vector.

Libraries of region 91-97 and of region 206-211 have been prepared containing from 10,000 to 80,000 clones/library. Most colonies were positive (more than 90%) when checked under conditions where the parent lipase is positive, *i.e*. exhibits lipase activity. The positive reaction was determined in a filter assay with 2.5 mM Ca (instead of 5 mM EGTA).

450,000 colonies were screened from the different libraries using the Dobanol®25-7 and low calcium assays described in Materials and Methods above. 25 low calcium positives from the aa 91-97 library (lid-region) and twelve Dobanol®25-7 positives from the whole gene libraries were isolated. Fourteen of the low calcium positives from mutagenesis of aa 91-97 were sequenced.

The three other mutations (in codon 83, 103, 145), outside the mutagenized region, can be explained by PCR misincorporation, although the mutation of S83T is a transversion which is quite unusual for PCR misincorporations.

**Sequence:**

| | | | |
|---|---|---|---|
| 5' | 5 | C | G |
| T | 5 | C | 3' |
| T | 7 | A | |
| A | 8 | G | <u>Bottle 5:</u> 93% A; 2.33% C; 2.33% G and 2.33% T |
| T | 8 | T | |
| T | A/C | T | |
| T | 5 | C | |
| C | 7 | T | |
| T | 5 | C | <u>Bottle 6:</u> 93% C; 2.33% A; 2.33% G and 2.33% T |
| T | 8 | T | |
| T | 8 | A | |
| 6 | C/G | T | |
| 5 | 6 | G | <u>Bottle 7:</u> 93% G; 2.33% A; 2.33% C and 2.33% T |
| 5 | 6 | G | |
| 7 | G | A | |
| 8 | AA | A | |
| 6 | T | C | <u>Bottle 8:</u> 93% T; 2.33% A; 2.33% C and 2.33% G |
| 7 | | | |

**Table 1:** Illustration of the construction of oligonucleotides (SEQ ID No. 4) used for localized random mutagenesis of amino acids 91-97 of the *H. lanuginosa* lipolytic enzyme. The numbers presented in the sequence refer to the bottles the composition of which is apppearing to the right of the sequence.

Table 2

Strain number refers to the originally picked clones cloned into Aspergillus expression vector pAHL. Variant type refers to identical clones, which probably have arisen during amplification of the random mutagenized library. Variant types I and II are active in 0.01 % Dobanol®25-7 while the rest are inactive like wild type.

| Strain number | Variant type | | | | | | |
|---|---|---|---|---|---|---|---|
| 59 | I | | | G91A | N94K | | D96A |
| 60 | II | S83T | | | N94K | | D96N |
| 61 | II | S83T | | | N94K | | D96N |
| 62 | III | | E87K | | | | D96V |
| 63 | IV | | E87K | G91A | | | D96V |
| 64 | II | S83T | | | N94K | | D96N |
| 65 | III | | E87K | | | | D96V |
| 67 | V | | | | N94K | F95L | D96H |
| 69 | V | | | | N94K | F95L | D96H |
| 71 | III | | E87K | | | | D96V |
| 72 | II | S83T | | | N94K | | D96N |

**Table 3**

| Strain number | Variant type | 82 | 83 | 84 | 85 | 86 | 87 | 88 | 89 | 90 | 91 | 92 |
|---|---|---|---|---|---|---|---|---|---|---|---|---|
| | | DNA sequence (Amino acid number above the sequence) | | | | | | | | | | |
| wt | | GGC | TCT | CGT | TCC | ATA | GAG | AAC | TGG | ATC | GGG | AAT |
| 59 | I | | | | | | | | | | C | |
| 60 | II | | A | | | | | | | | C | |
| 61 | II | | A | | | | | | | | C | |
| 62 | III | | | | | | A | | | | C | |
| 63 | IV | | | | | | A | | | | C | |
| 64 | II | | A | | | | | | | | C | |
| 65 | III | | | | | | A | | | | C | |
| 67 | V | | | | | | | | | | C | |
| 52/68 | wt | | | | | | | | | | | |
| 53 | wt | | | | | | | | | | | |
| 69 | V | | | | | | | | | | C | |
| 71 | III | | | | | | A | | | | C | |
| 72 | II | | A | | | | | | | | C | |
| 73 | VI | | | | | | | | | | | |

| Strain number | Variant type | 93 | 94 | 95 | 96 | 97 | 98 | 99 | 100 | -103 | -145 |
|---|---|---|---|---|---|---|---|---|---|---|---|
| wt | | CTT | AAC | TTC | GAC | TTG | AAA | GAA | ATA | -ATT | -CAT |
| 59 | I | G | G | | C | | | | | | |
| 60 | II | G | G | | A | | | | | | |
| 61 | II | G | G | | A | | | | | | |
| 62 | III | | | | T | | | | | | |
| 63 | IV | | | | C | | | | | C | C |
| 64 | II | G | G | | A | | | | | | |
| 65 | III | G | | | T | | | | | | |
| 67 | V | | A | C A | C | | | | | | |
| 52/68 | wt | | | | | | | | | | |
| 53 | wt | | | | | | | | | | |
| 69 | V | | A | C A | C | | | | | | |
| 71 | III | G | | | T | | | | | | |
| 72 | II | G | A | | A | | | | | | |
| 73 | VI | | | | A | ? | | | | | |

**Table 3:** The wild type seqence is shown at the topline. Only nucleotides differing from wt are written at the variant sequences. The base of codon 91 and 93 were doped with 1:1 of C/T and T/G, respectively. Otherwise the nucleotides at codon 91-97 were doped using 93% wt and 2.33 % of the three other nucleotides.

**Results from screening a random mutagenized library of aa 85-99 (the lid region) with a doping based on the results obtained from positives from random mutagenesis of aa 91-97.**

**Construction of the random mutagenized library**

*Background*

Five different types of strong positive mutants were found in screening of the first library of the lid region (aa 91-97, see previous example). D96 was changed to A, V, N or H and amino acid change E87K, G91A and N94K were

found in two to three independent mutants in combination with a change of D96 indicating their importance for independence of calcium of Lipolase. Since these mutations were improved with respect to low calcium/Dobanol activity compared to wt in several assays, they were used as a starting point in a second random mutagenesis of the whole lid region.

**Localized random mutagenesis.**

The amino acid region aa 85-99 + 83S/T were random mutagenized as follows. Doping scheme: S83 - 50%S/50%T; E87 - 93%K/7%X; G91 - 93%A/7%X; N94 - 50%K/50%N; D96 - 100%X; the rest were 93%wt/7%X (the percentages refers to the doping of the codons at the nucleotide level (see the sequence of the oligo). The theoretical percentage of the various amino acid codons resulting from these dopings may be calculated using a state of the art computer program). Where possible without changing the amino acid, the third nucleotide (the wobble base) in codons were synthesized with 50%G/50%C to give a larger likelyhood for changes to amino acids with only one or two codons. The composition of the mutagenic oligonucleotide is shown in SEQ ID NO. oligo 2. (SEQ ID NO 6). The none mutagenized nucleotide region were chosen using the Oligo program optimizing for stability and no secondary structure.

This mutagenesis gives a calculated frequency of approximately 93% changes of the starting point (not including S83, N94 and D96) in the library. This is a very high mutation frequency which should give the chance af major changes of the lid region.

The mutagenic primer were used in a PCR reaction with a suitable opposite primer. The resulting PCR fragment was used in a second PCR reaction as a primer with a second suitable opposite primer. This step was necessary to be able to digest and clone the mutagenized region into the yeast expression vector pYESHL. It is important to take the A added to the 3' end of the PCR fragment by Taq polymerase into account when designing a mutagenic primer for such a two step PCR method.

In this way random mutagenized libraries of the region aa 85-99 + 83S/T were prepared.

**Screening**

The low calcium filter assay was used with Dobanol and LAS. Screening of the lid2 library was made with 5 mM EGTA, 0.01 % Dobanol and 0.006% LAS. Several positives were detected and isolated, sequenced, transformed into Aspergillus, purified and tested in wash tests.

**Sequence and wash results of selected positives**

Underlined shows conditions used in the filter assay. IF = improvement factor in 3-cycle wash.
<u>5 mM EGTA,0.01% Dobanol,0.006% LAS</u>
E87K,G91A,L93I,N94K,D96A. IF=1.3
<u>5 mM EGTA,0.02 % Dobanol</u>
N73D,S85T,E87K,G91A,N94K,D96A. IF=1.1
S83T,E87K,W89G,G91A,N94K,D96V. IF=0.8
E87K,G91A,D96R,I100V. IF=5.2
S83T,E87K,Q249R
<u>2g/l PCS</u>
E87K,G91A. IF=5.0

**Sequence of Oligo-lid2 (SEQ ID NO. 6):**

**5'-C ATT TAT 886 888 655 (C/G)(A/C/G/T)(A/C/G/T) 755 (C/G)88 (A/C)57 588 (C/G)76 (7/8)58 665 788 688 (8/7)58 775 ACG AG(A/T) GCC ACG-3'**

<u>Flask 5</u>: **93% A**; 2,33% C; 2,33% G og 2,33 % T.
<u>Flask 6</u>: **93% C**; 2,33% A; 2,33% G og 2,33 % T.
<u>Flask 7</u>: **93% G**; 2,33% A; 2,33% C og 2,33 % T.
<u>Flask 8</u>: **93% T**; 2,33% A; 2,33% C og 2,33 % G.

**Local random mutagenesis performed on two regions simultaneously**

Random mutagenized libraries of aa region 56 to 64 and 81 to 99 + 102 were prepared as described in the Materials and Methods using the two oligo nucleotides 004 and 005 as shown in Table 4 in a PCR reaction. Oligo 004 was synthesized for the aa region 81 to 99 + 102 with 93% wt nucleotides and 2.33% of each of the other 3 nucleotides in each position except for the S83 codon which was doped to give 50%S/50%T (see table 4). For aa with 4 or 6 codons a 50%/50% mixture of G/C or A/C was used for the third base (see table 4). For the third base of the Ile codon a 50%/50% mixture of bottle 7 and 8 was used. D96L was used as starting point in the random mutagenesis since it was found in previous good performing variants. Oligo 005 was synthesized for the aa region 56 to 64 with 93 % wt nucleotides and 2.33% of each of the other 3 nucleotides in each position. For the positions 56, 57 and 62 a bias of positively charged aa among others were introduced (see table 4). For aa with 4 or 6 codons a 50%/50% mixture of G/C or G/T was used for the third base. In general the PCR reaction may introduce mutations outside the doped region which is an advantage since such mutations may benefit to the property of a variant.

The oligo 004 was also used in combination with oligo 006 (see table 4) cloned by a double PCR reaction resulting in library covering region 81 to 99 + 102 and region 248-257,259, 263-269. The oligo 006 was synthesized for the aa region 248-257,259, 263-269 with 93% wt nucleotides and 2.33% of each of the other 3 nucleotides in each position. For aa with 4 or 6 codons a 50%/50% mixture of G/C or A/C was used for the third base (see table 4). For the third base of the Ile codon a 50%/50% mixture of bottle 7 and 8 was used.

The oligo 005 and 006 were also used for construction of random mutagenized libraries using positives in the lid region as a template.

**Table 4:**

| | | | | |
|---|---|---|---|---|
| Oligo.004 | 102: | T | C | A |
| Lid3 | | C | T | G |
| (antisense) | 5'- | C | G | C |
| aa 81-99, | G | C | C | C |

| | | | | |
|---|---|---|---|---|
| G | G | 5 | 6 | A/C |
| G | C | C?G | 5 | 6 |
| A | A | 5 | 7 | 7 |
| | A | 7 | 8 | G |
| | A | 7 | 8 | A |
| | T | 5 | 6 | A |
| | 7 | 5 | 8 | A |
| | 8 | C/G | 6 | G |
| | 6 | 8 | 7/8 | -3' |
| | A | 8 | 5 | Bottle 5: **93% A**; 2,33% C; 2,33% G og 2,33 % T. |
| | T | A/C | 8 | |
| | T | 5 | C/G | |
| | T | 7 | 7 | Bottle 6: **93% C**; 2,33% A; 2,33% G og 2,33 % T. |
| | A | 5 | 5 | |
| | T | 8 | A/C | |
| | 8 | 8 | 6 | Bottle 7: **93% G**; 2,33% A; 2,33% C og 2,33 % T. |
| | 8 | C/G | 7 | |
| | 6 | 6 | A | |
| | 8 | 6 | G | Bottle 8: **93% T**; 2,33% A; 2,33% C og 2,33 % G. |
| | 8 | 7/8 | A/T | |
| | 8 | 5 | C/G | |
| | 6 | 8 | 6 | |
| | 5 | 6 | 6 | |

Oligo.005 lid-contact aa 56-64:

5'-
G
A
A
A
T
G
C
A
T
G
C
C
C
C
G

A
G
G
T
A
G
A
G
A
A
G
G
C
G
G
A
T
G
C
A
A
C
G
T
T
T
C
T
C
T
A
C
T
C
G
T
T
T
7/5
7/5
7/5
6/7

5/7
6/8

8
6
T/G
7
7
G/T
7
8
G/C
7
7
G/C
6/7
5/7
6/8
7
8
G/C
5
6
G/C

G
G
C
T
T
C
3'

**<u>Bottle 5</u>:**
**93%** A;
2,33% C;
2,33% G og
2,33 % T.
<u>Bottle 6</u>:
**93% C;**
2,33% A;
2,33% G og
2,33 % T.
<u>Bottle 7</u>:
**93% G;**
2,33% A;
2,33% C og
2,33 % T.
<u>Bottle 8</u>:
**93% T;**
2,33% A;
2,33% C og
2,33 % G.

| | | | | |
|---|---|---|---|---|
| Oligo 006 | 6 | A | C/G | G |
| C-terminal | 5 | C | 7 | C |
| aa 248- | A/C | C | 7 | C |
| 257,259, | 7 | A | 5/8 | **-3'** |
| 263-269 | 8 | A/C | 5 | Bottle 5: **93% A;** |
| | C/G | 5 | 8 | 2,33% C; 2,33% G |
| **5'-** | 6 | 7 | 7 | og 2,33 % T. |
| G | 6 | G | 8 | Bottle 6: **93% C;** |
| C | 5/8 | T | 8 | 2,33% A; 2,33% G |
| C | 5 | G | A/C | og 2,33 % T. |
| C | 8 | C/G | 7 | Bottle 7: **93% G;** |
| T | A/C | 7 | 7 | 2,33% A; 2,33% C |
| C | 5 | 6 | 6 | og 2,33 % T. |
| T | 7 | A/C | 8 | Bottle 8: **93% T;** |
| A | C/G | 7 | 7 | 2,33% A; 2,33% C |
| G | 6 | 7 | 7 | og 2,33 % G. |
| A | 6 | 7/8 | 8 | |
| C | G | 5 | 8 | |
| T | A | 8 | A | |
| A | A | 5 | T | |
| A/C | G | 8 | T | |
| 5 | T | 6 | | |
| 7 | | | | |
| 5 | | | | |

Some of the positives obtained from screening these libraries on detergent containing plates are shown below:

E56R+D57L+I90F+D96L+E99K
E56R+D57L+V60M+D62N+S83T+D96P+D102E
D57G+N94K+D96L+L97M
E87K+G91A+D96R+I100V+E129K+K237M+I252L+P256T+G263A+L264Q
E56R+D57G+S58F+D62C+T64R+E87G+G91A+F95L+D96P+K98I
A47V+D62G+D96L+A121T
E56G+D57G+V60E+D62G+N94K+D96L

The following variants were obtained from random mutagenesis of the whole gene alone (by PCR or PCR + formic acid as described in the Materials and Methods section) and screened on detergent containing plates:

I34V+S54P+F80L+S85T+D96G+R108W+G109V+D111G+S116P+L124S+V132M+V140Q+V141A+F142S+H145R+N162T+I166V+F181P+F183S+R205G+A243T+D254G+ F262L

A19T+D167G+E210V+W221L (random mutagenesis based on D167G+E210V)

A49P+D167G+E210V (random mutagenesis based on D167G+E210V)

EXAMPLE 2

**Construction of first wash variants of the *H. lanuginosa* lipolytic enzyme**

*1. Domain shuffling by recombination and screening*

20 *H. lanuginosa* lipolytic enzyme variants having a very good washing performance (as evaluated in various wash related tests) some of which were constructed according to Example 1 were allowed to recombine by an *in vivo*

recombination method in *S. cerevisiae* YNG318 as described in the Materials and Methods section herein. The lipolytic enzyme variants used are apparent from table 1. Most of these variants had been constructed by random or localized random mutagenesis as described in the Materials and Methods section above and screening for a decreased dependence on calcium and an improved tolerance towards the detergent component Dobanol 25-7 (cf. the Materials and Methods section above). Some of the variants are the result of two or more consecutive rounds of mutagenesis and screening.

The restriction enzyme opened vector and the PCR fragments apparent from the table below and further discussed in the Materials and Methods section were mixed in a molar ratio of approximately 1:1 and used for transformation of competent *S. cerevisiae* cells (made by the lithium acetate method as described in Current Protocols in Molecular Biology, eds. F.M. Ausubel et al., chapter 13.7, John Wiley & Sons, Inc., USA.). The transformed cells were plated on filters and screened for a reduced calcium dependency and an increased detergent tolerance using the filter assay described in the Materials and Methods section above.

Colonies giving a positive signal were streaked out to single colonies on new plates and filters and re-screened. After 2 to 4 rescreenings positive colonies were fermented as follows:

<u>Fermentation of *H. lanuginosa* lipolytic enzyme and variants in yeast</u>

10 ml of SC-ura$^-$ medium are inoculated with a *S. cerevisiae* colony and grown at 30°C for 2 days. The 10 ml is used for inoculating 300 ml SC-ura$^-$ medium which after inoculation is grown at 30°C for 3 days. The 300 ml is used for inoculation 5 l of the following G-substrate:

| | |
|---|---|
| 400 g | Amicase |
| 6.7 g | yeast extract (Difco) |
| 12.5 g | L-Leucine (Fluka) |
| 6.7 g | $(NH_4)_2SO_4$ |
| 10 g | $MgSO_4 \cdot 7H_2O$ |
| 17 g | $K_2SO_4$ |
| 10 ml | Trace compounds, cf below |
| 5 ml | Vitamin solution, cf below |
| 6.7ml | $H_3PO_4$ |
| 25 ml | 20% Pluronic (antifoam) |

In a total volume of 5000 ml.

Tracecompounds:

| | |
|---|---|
| 6.8 g | $ZnCl_2$ |
| 54.0 g | $FeCl_2 \cdot 6H_2O$ |
| 19.1 g | $MnCl_2 \cdot 4H_2O$ |
| 2.2 g | $CuSO_4 \cdot 5H_2O$ |
| 2.58 g | $CoCl_2$ |
| 0.62 g | $H_3BO_3$ |
| 0.024g | $(NH_4)_6Mo_7O_{24} \cdot 4H_2O$ |
| 0.2 g | KI |
| 100 ml | HCl (concentrated) |

In a total volume of 1 l.

Vitamin solution:

| | |
|---|---|
| 250 mg | Biotin |

(continued)

| | |
|---|---|
| 3 g | Thiamine |
| 10 g | D-Calciumpantothenat |
| 100 g | Myo-Inositol |
| 50 g | Cholinechlorid |
| 1.6 g | Pyridoxin |
| 1.2 g | Niacinamid |
| 0.4 g | Folicacid |
| 0.4 g | Riboflavin |

In a total volume of 1 l.

The yeast cells were fermented for 5 days at 30°C in 5000 ml medium. Glucose was fed to the fermenter with a start dosage of 100 ml 70% glucose and addition of 400 ml 70% glucose/day. The pH of the fermentation broth was kept at pH 5.0 by controlled addition of a 10% $NH_3$ solution.

Agitation was 300 rpm for the first 22 h followed by 900rpm for the rest of the fermentation. Air was given with 1 l air/l/min for the first 22 h followed by 1.5 l air/l/min for the rest of the fermentation.

After purification the capability of the variant in removing lard was tested in the one cycle wash assay described in the Materials and Methods section above. The results were given in Example 2 and 3 hereinbelow.

Table 1:

**Variants used for recombination**

*Humicola lanuginosa* lipase variants used for preparing vector (opened with NruI) for invo recombination (gene shuffling):

E56R,D57L,I90F,D96L,E99K
E56R,D57L,V60M,D62N,S83T,D96P,D102E
D57G,N94K,D96L,L97M
E87K,G91A,D96R,I100V,E129K,K237M,I252L,P256T,G263A,L264Q
E56R,D57G,S58F,D62C,T64R,E87G,G91A,F95L,D96P,K98I,(K237M)
E210K

*Humicola lanuginosa* lipase variants used for preparing DNA fragments (by standard PCR amplification of the whole gene from the plasmids containing the variant) for invo recombination (gene shuffling):

S83T,N94K,D96N
E87K,D96V
N94K,D96A
E87K,G91A,D96A
D167G,E210V
S83T,G91A,Q249R
E87K,G91A
S83T,E87K,G91A,N94K,D96N,D111N.
N73D,E87K,G91A,N94I,D96G.
L67P,I76V,S83T,E87N,I90N,G91A,D96A,K98R.
E210K
S83T,E87K,G91A,N92H,N94K,D96M
S85P,E87K,G91A,D96L,L97V.
E87K,I90N,G91A,N94S,D96N,I100T.
I34V,S54P,F80L,S85T,D96G,R108W,G109V,D111G,S116P,L124S,V132M,V140Q,V141 A,F142S,H145R, N162T,I166V,F181P,F183S,R205G,A243T,D254G,F262L.
E56R,D57L,I90F,D96L,E99K
E56R,D57L,V60M,D62N,S83T,D96P,D102E
D57G,N94K,D96L,L97M
E87K,G91A,D96R,I100V,E129K,K237M,I252L,P256T,G263A,L264Q
E56R,D57G,S58F,D62C,T64R,E87G,G91A,F95L,D96P,K98I,(K237M)

*2. Domaine shuffling by traditional cloning of two positives together*

The *Aspergillus* expression vector pHD414 containing a DNA sequence encoding the *Humicola lanuginosa* lipase variant (D57G+N94K+D96L+L97M) were digested with the restriction enzymes NarI and XbaI resulting in two fragments. The fragments were separated by agarose gel electrophoresis and the largest fragment were isolated from the agarose gel. This fragment were ligated to the smallest fragment from digestion of the variant (S83T+G91A+Q249R) with NarI and XbaI. The ligation was transformed into *E. coli* and the resulting plasmid constructions were isolated from one of the transformants and sequenced to test for the correct assembly. The plasmid was transformed into *Aspergillus oryzae* fermented and purified as described in the Materials and Methods section.This variant contained the following mutations D57G+N94K+D96L+L97M+Q249R.

EXAMPLE 3

Construction and expression of *H. lanuginosa* lipolytic enzyme variant HL9 in *Aspergillus oryzae* JaL125

The variant HL9 contains the following mutations in the mature part: E1P+D57G+N94K+D96L+Q249R and the N-terminal peptide addition SPIRPR fused to E1P (resulting in the overall N-terminal peptide addition SPIRPRP. An N-terminal peptide addition was applied to the parent *H. lanuginosa* (DSM 4109) lipolytic enzyme having the amino acid and DNA sequence, respectively, apparent from EP 305 216 and in addition carrying the following mutations D57G+N94K+D96L+Q249R in its mature part (inserted by conventional site-directed mutagenesis) in the DNA sequence (EP 305 216). The peptide addition SPIRPRP was applied to the N-terminus of the parent enzymes as follows:

Construction of pIVI220:

The plasmid was constructed using the Chamelon double stranded, site-directed mutagenesis kit from Stratagene according to the described protocol.

pHL296 was used as the plasmid template. Said plasmid contains the gene encoding the *H. lanuginosa* lipolytic enzyme with the above mentioned mutations (D57G+N94K+D96L+L97M+Q249R) cloned into pHD414.

Primer no. 7258 was used as the selection primer.
7258: 5'p gaa tga ctt ggt tga cgc gtc acc agt cac 3'
(Thus changing the ScaI site found in the ampicillin resistance gene and used for cutting to a MluI site).

Primer no. 7770 was used as the selection primer.
7770: 5'p tct age cca gaa tac tgg atc aaa tc 3' (Changes the ScaI site found in the *H. lanuginosa* lipase gene without changing the amino acid sequence).

Primer no.8479 was used as the mutagenic primer.
8479: 5'p gcg tgg acg gcc ttg gct agc cct att cgt cct cga ccg gtc tcg cag gat ctg (replacing the propeptide and the N-terminal E1 of the parent *H. lanuginosa* enzyme (SPIRRE by SPIRPRP).

Construction of pIVI245:

The plasmid was constructed using the Chameleon double-stranded, site-directed mutagenesis kit from Stratagene (cat no. 200509) according to the described protocol.

pIVI220 was used as the plasmid templated and primer no.7887 as the selection primer (changing the introduced Mlu1 site found in the ampicillin resistance gene and used for cutting to a ScaI site). 7887: 5'p-gaa tga ctt ggt tga gta ctc acc agt cac 3'

Primer no. 8932 was used as the mutagenic primer (8932: 5'p-g aac tgg ata gga aat ttg aag ttc ctg ttg aaa gaa ata aat gac 3' (thus changing M97 back to L97 as wildtype and still preserving the two mutations N94K and D96L)).

2. Construction of the *A. orxzae* expression plasmid pCaHj483

pCaHj483 is depicted in Figure 8. It is build from the following fragments :

a) The vector pToC65 (WO91/17243) cut with *EcoRI* and *XbaI*.
b) A 2.7 kb *XbaI* fragment from *A. nidulans* carrying the *amdS* gene (C. M. Corrick et al., (1987), Gene 53, p. 63-71). The *amdS* gene is used as a selective marker in fungal transformations. The *amdS* gene has been modified so that the *BamHI* site normally present in the gene is destroyed. This has been done by introducing a silent point mutation using Primer 3: AGAAATCGGGTATCCTTTCAG (SEQ ID No. 1)
c) A 0.6 kb *EcoRI*/*BamHI* fragment carrying the *A. niger* NA2 promoter fused to a 60bp DNA fragment of the

sequence encoding the 5' untranslated end of the mRNA of the *A. nidulans tpi* gene. The NA2 promoter was isolated from the plasmid pNA2 (EP 383 779) and fused to the 60 bp tpi sequence by PCR. The primer (Primer 4 SEQ ID No. 7) encoding the 60 bp *tpi* sequence had the following sequence :

5'-
GCTCCTCATGGTGGATCCCCAGTTGTGTATATAGAGGATTGAGGAAGGAAGAG
AAGTGTGGATAGAGGTAAATTGAGTTGGAAACTCCAAGCATGGCATCCTTGC -
3´

d) A 675 bp *XbaI* fragment carrying the *A. niger* glucoamylase transcription terminator.

The fragment was isolated from the plasmid pICAMG/Term (EP 238 023).

The *BamHI* site of fragment c) was connected to the *XbaI* site in front of the transcription terminator on fragment d) via the pIC19R linker (*BamHI* to *XbaI*)

Construction of the HL9 expression plasmid pCaHj485

The plasmid pJVi 245 was digested with BamH I and Sal I, and the resulting 904 bp fragment encoding the HL9 lipolytic enzyme was isolated. pCaHj 483 was digested with BamH I and Sal I, and the large vector fragment (6757) was ligated to the HL9 fragment. The ligation mixture was used to transform *E. coli* DH5α cells, and a transformant harbouring the expected plasmid was isolated. The plasmid was termed pCaHj485.

3. Transformation of pCaHi 485 into JaL125

*Aspergillus oryzae* JaL 125 was transformed with pCaHj 485 using selection on acetamide as described in patent EP 0 531 372. Transformants were spore reisolated twice. Spores from second reisolation of each transformant were used to inoculate 200 µl YPM (1% yeast extract, 2 % peptone, 2 % maltose) in 96 well microtiter dishes. The YPM cultures were grown for 4 days at 34°C, and the higest producers were selected using a p-nitro phenylbutyrate assay:

Stock solution: 18 µl p nitro phenyl butyrate was dissolved in 1 ml isopropanol.
Working solution: 0.1 ml stock solution was mixed with 10 ml 50 mM Tris/HCl pH 7.5; 10 mM $CaCl_2$.
Assay: 1 µl of YPM supernatant was mixed with 200 µl of working solution in 96 well microtiterdishes+ and the color development was measured at 450 nm using an ELISA reader.

One transformant was selected for tank fermentation.

4. Tank fermentation of JaL 125/pCaHj 485

The fermentation was carried out as a fed-batch fermentation using a constant medium temperature of 34°C and a start volume of 1.2 litre. The initial pH of the medium was set to 6.5. Once the pH had increased to 7.0 this value was maintained through addition of 10% $H_3PO_4$. The level of dissolved oxygen in the medium was controlled by varying the agitation rate and using a fixed aeration rate of 1.0 litre air per litre medium per minute. The feed addition rate was maintained at a constant level during the entire fed-batch phase.

The batch medium contained maltose syrup as carbon source, urea and yeast extract as nitrogen source and a mixture of trace metals and salts. The feed added continuously during the fed-batch phase contained maltose syrup as carbon source whereas yeast extract and urea were added in order to assure a sufficient supply of nitrogen.

5. Purification of the first wash lipolytic enzyme

1) Fermentation supernatant was filtered through milipore filter Cat. No. AP2504700 Filter type AP25.
2) Fermentation supernatant was filtered once more on through the sterile filter from Millipore membrane Type GS 0.22 micron.

3) Fermenation supernatent was then adjusted to 0.8 M ammonium acetate by adding solid ammonium acetate.
4) A Hydrophobic chromatography on TSK gel Butyl-Toyopearl 650. 50 ml column was packed with the Butyl-Toyopearl matrix. The column was washed and equilibrated with 0.8 M ammonium acetate. One liter ferementation supernatent adjusted with amonium acetate was then applied on the Butyl column. The column was washed with 0.8 M ammonium acetate till all unbound material was washed out. Bound material was then eluted with water and 50 % ethanol sequentially. Fractions were collected and analyzed for lipase activity using Standard LU assay. Fractions containing lipase activity were pooled and diluted to adjust conductivity of the pool below 4 mSi and pH to 8.5.
5) Anion exchange chromatography on High Performance Q sepharose (Pharmacia, Code No. 17-1014-01). 50 ml column was packed and washed with 50 mM Borate buffer pH 8.5. Pool containing lipolytic enzyme activity was then appllied on The High performance Q sepharose column. Unbound material was washed with the Borate buffer pH 8.5. Bound activity was then eluted with linear gradient using Borate buffer conatining 1 M Sodium Chloride pH 8.5.

Fractions were collected and assayed for Lipase activity. Fractions containing Lipase activity with a ratio of UV absorbence at A280and A260 more than 1.7 are pooled.

EXAMPLE 4

Construction of N-terminal additions by random mutagenesis

Random mutagenesis of the part of the DNA sequence encoding the N-terminal addition SPIRPRP added to the first amino acid residue of the mature *H. lanuginosa* lipolytic enzyme (obtainable from DSM 4109) and containing the following further mutations in its mature part: D57G+N94K+D96L+L97M+Q249R was performed. The mutations in the mature part of the parent lipolytic enzyme was performed by PCR driven site-directed mutagenesis using the appropriate primer sequences using the procedures described in WO 95/26215. The peptide addition SPIRPRP was applied as described in Example 3, (i.e. the last P replacing E1).

The nucleotide doping scheme of the SPIRPRP codons was as follows:

**Oligo 1: 5'-** GCG TGG ACG GCC TTG GCC 86(T/A) 66(A/T) 58(T/A) 67(T/A) 66(T/A) 575 66(T/A) GAG GTC TCG CAG GAT CTG **-3'** (57-mer) **(SEQ ID NO 11)**

the numbers referring to which of the following flasks to be used.
Flask 5: **80%** A; 6.66% C; 6.66% G og 6.66 % T.
Flask 6: **80%** C; 6.66% A; 6.66% G og 6.66 % T.
Flask 7: **80%** G; 6.66% A; 6.66% C og 6.66 % T.
Flask 8: **80%** T; 6.66% A; 6.66% C og 6.66 % G.

A two step PCR reaction protocol was used: The first step with the above primer as the 5' primer and with the primer 2056 (5'gca cgt aat gtt tgt acc 3') as the 3' primer conducted using pHL296 as the plasmid template. The product of the first PCR round was used in a new PCR with 4699 (5'cgg tac ccg ggg atc cac 3') as the 5' primer (to introduce the BamHI site and the first part of the coding sequence) and with the PCR product as the 3' primer using the same template. The resulting product was purified on Spin 100 (from Clonetech Lab., Inc.) and cut with BamHI and PvuII. The resulting DNA fragment was purified from the agarose gel with SpinX (Costar) and ligated into the yeast expression vector pJSO37 containing the *H. lanuginosa* lipolytic enzyme gene from pHL296 cloed as a BamHI-XbaI fragment cut with BamHI and PvuII. The resulting DNA was electrotransformed into DH10/DH12 *E. coli* cells (Gibco/BRG Lifetechnologies) using the conventional technique.

After transformation into *E. coli* and amplification the plasmid was purified and transformed into *S. cerevisiae* YNG 318. The resulting *S. cerevisiae* cells were screened for good performers in the alternative lipase filter assay containing detergent (3 g/l of PCS). The positives were sequenced and found to contain the following peptide additions: GPIRPRP, SHSRHNA, TAIRPRK, SALRRRP, STRRPRP, SPRRPRT, SPIPPGP, LPFRQRP, SPFRPKL, and SALRRP (termed HL10s1-10, respectively).

The one-cycle wash performance of each of HL10s1-6 was tested as described in the Materials and methods section above (Assay for test of first wash effect) at a temperature 30°C and using 5 g/l of enzyme inactivated Ariel Futur as detergent. The amount of fatty material removed by each of the modifed enzymes are shown below:

| Lipase variant | Low dosage | % lard removed | High dosage | % lard removed |
|---|---|---|---|---|
| HLv10s1 | 1250 LU/l | 26 | 12500 LU/l | 54 |
| HLv10s2 | 1250 LU/l | 22 | 12500 LU/l | 53 |
| HLv10s3 | 1250 LU/l | 34 | 12500 LU/l | 55 |
| HLv10s4 | 1250 LU/l | 33 | 12500 LU/l | 55 |
| HLv10s5 | 1250 LU/l | 23 | 12500 LU/l | 47 |
| HLv10s6 | 1250 LU/l | 30 | 12500 LU/l | 53 |

The tendency was that the best performers had more positive charged amino acids in the N-terminal addition.

Analogously, random mutagenesis of the N-terminal addition RPRPRPRP added to the *H. lanuginosa* lipase variant E1*+ D57G+ N94K+ D96L+ L97M + Q249R plus other variants were performed. The nucleotide doping scheme of the RPRPRPRP codons was as follows:

Oligo 2: 5'-GTC TCT GCG TGG ACG GCC TTG GCG GCG CCA CCT CCA 67(T/A) 66(T/A) 575 66(T/A) 67(T/A) 66(T/A) 575 66(T/A) (6/7)(7/8)(C/G) 57(C/G) C57 (5/7)5(C/G) CTG TTT AAC CAG TTC AAT CTC-3' (93-mer)

Flask 5: **80% A**; 6.66% C; 6.66% G og 6.66 % T.
Flask 6: **80% C**; 6.66 % A; 6.66 % G og 6.66 % T.
Flask 7: **80% G**; 6.66% A; 6.66% C og 6.66 % T.
Flask 8: **80% T**; 6.66% A; 6.66% C og 6.66 % G.

APPP is added in the N-terminal of the randomly mutagenized RPRPRPRP and prior to the signal peptide in order to protect against proteolytic degradation of the N-terminal addition. This may not be required. E1 was deleted in order to remove one negatively charged amino acid. The amino acids in position 2 to 5 of the mature *H. lanuginosa* lipase sequence were also mutagenized in order to find improved mutants in this non-structural part of the lipase. Otherwise the procedure is as stated above for the random mutagenesis of SPIRPRP.

The following N-terminal peptide additions were obtained:

Ala-Pro-Pro-Pro-Arg-Pro-Arg-Leu-Leu-Pro-Ile-Ser( APPPRPRLLPIS) (in addition to the deleted E1 residue this variant carries the additional mutation D5E in its non-structural N-terminal part of the mature enzyme).

Ala-Pro-Pro-Pro-Thr-Arg-Gln-Arg-Gln-Ser-Pro(APPPTRQRQSP) (in addtion to the deleted E1 residue this variant carries the additional mutations V2L, S3T and D5V in its non-structural N-terminal part of the mature enzyme).

Ala-Pro-Pro-Pro-Arg-Thr-Ile-Pro-Arg-Ser-Ser-Pro( APPPRTIPRSSP) (in addition to the deleted E1 residue this variant carries the additional mutations V2L, S3R and D5E in its non-structural N-terminal part of the mature enzyme).

Ala-Pro-Pro-Pro-Arg-Pro-Arg-Pro-Arg-Pro-Arg-Pro (APPPRPRPRPRP) (in addtion to the deleted E1 residue this variant carries the additional mutations V2G and D5E in its non-structural N-terminal part of the mature enzyme).

Ala-Pro-Pro-Pro-Arg-Thr-Arg-Pro-Arg-Pro-Arg-Ser (APPPRTRPRPRS) (in addtion to the deleted E1 residue this variant carries the additional mutations V2GL, S3T, Q4P and D5E in its non-structural N-terminal part of the mature enzyme).

Ala-Pro-Pro-Pro-Lys-Ala-Ser-Pro-Arg-Gln-Arg-Pro (APPPKASPRQRP) (in addtion to the deleted E1 residue this variant carries the additional mutations V2GL, D5Q and L6M in its non-structural N-terminal part of the mature enzyme).

EXAMPLE 5

**First wash activity of lipolytic enzymes of the invention**

The first wash activity of lipolytic enzymes was tested using the "Assay for test of First Wash effect" described in the Materials and Methods section above with Detergent Composition A or B. A few of the new first wash lipase are compared to what is considered as being the present state of art within lipolytic enzymes for detergents.

Note: ¤ are produced in Aspergillus oryzae as described in example 2.
* are produced in yeast as described in example 4.

| Detergent Composition A | | |
|---|---|---|
| Lipolytic Enzyme | % removal at 1250 LU/l | % removal at 12500 LU/l |
| E1SPIRPRP+D57G+N94K+D96L+L97M+ Q249R | 15% | 49% |
| Lumafast™ (Ps. mendocina) | 0% | 2% |
| Lipomax™ (Ps. pseuodoalcaligenes L21M) | 0% | 9% |
| Fusarium solani *pisi* | 0% | 0% |
| Lipolase | 0 % | 0 % |
| Lipolase Ultra | 0% | 0% |

| Detergent Composition B | | |
|---|---|---|
| Lipolytic Enzyme | % removal at 1250 LU/l | % removal at 12500 LU/l |
| E1SPIRPRP+D57G+N94K+D96L+L97M+ Q249R | 15% | 46% |
| Lumafast™ (Ps. mendocina) | 6% | 6% |
| Lipomax™ (Ps. pseuodoalcaligenes L21M) | 0% | 0% |
| Liposam™ | 4% | 7% |
| Fusarium solani *pisi* | 2% | 5% |
| Lipolase | 5% | 6 % |
| Lipolase Ultra | 6 % | 0 % |

Additional examples:

| Detergent Composition A | |
|---|---|
| Lipolytic Enzyme | % removal at 12500 LU/l |
| SPIRR+D57G+G59V+N94K+D96L+L97M+S116P+S1 70P+Q249R * | 42% |
| SPIRR+A49P+D167G+E210V * | 44% |
| SPIRR+E56K+D57G+D62R+S83T+S85F+D96L+D10 2Y+E210K * | 36% |
| SPIRR+N94K+F95L+D96H+N101S+F181L+D234Y+I 252L+P256T+G263A+L264Q * | 41% |

EXAMPLE 6

**Activity-in-Detergent (AiD) assay**

The AiD assay is an analytical assay that is useful for selecting parent lipolytic enzymes to be used in the construction of a first wash lipolytic enzyme as described herein.
Equipment:Water bath with 150 ml beakers. Stirring is obtained by an agitator.
Lipolytic enzyme dosage: 12500 LU/l.
Substrate:6 pieces (3.5*3.5 cm) of cotton with 6 µl olive oil for one test.
Detergent:0.5 g/l model liquid detergent* dissolved in 0.36 mM $Ca^{2\cdot}/Mg^{2\cdot}$ (5:1), adjusted to pH 10, 100 ml per beaker.
After stirring the sample for 60 min. at 30°C the remaining detergent on the swatches is removed by addition of tap water for 15 min. The swatches are put into a flask containing 10 ml Tetrahydrofuran and 6.25 ml 4 M HCl and evaporated over night, after which the sample is redissolved in Tetrahydrofuran. The fat composition is determined by TLC/FID and the amount of % FFA (free fatty acids) is used to distinguish between the lipolytic enzymes.
Note ¤ are produced in Aspergillus oryzae ast as described in example 2

*detergent formulation below

* all variants are produced in yeast as described in example 4.

| AiD assay | |
|---|---|
| Lipolytic Enzyme | % FFA |
| SPIRR+D57G+G59V+N94K+D96L+L97M+S116P+ S170P+Q249R * | 20% |
| SPIRR+A49P+D167G+E210V * | 25% |
| SPIRR+E56K+D57G+D62R+S83T+S85F+D96L+ D102Y+E210K * | 25% |
| SPIRR+N94K+F95L+D96H+N101S+F181L+D234Y+I 252L+P256T+G263A+L264Q * | 20% |
| E1SPIRPRP+D57G+N94K+D96L+L97M+Q249R¤ | 27% |
| Lumafast™ (Ps. mendocina) | 5% |
| Lipomax™Cos (Ps. pseudoalcaligenes) | 31% |
| Fusarium solani *pisi* | 6% |
| Lipolase | 5% |
| Lipolase Ultra | 5% |

| Model liquid detergent: | |
|---|---|
| Component | % w/w |
| LAS | 17.50 |
| AEO | 14.40 |
| DTSA | 10.00 |
| Oleic acid | 3.00 |
| Coconut oil | 5.00 |
| MEA | 14.50 |
| MPG | 10.70 |
| Ethanol | 1.40 |
| Phosphonate | 1.00 |
| Boric acid | 0.80 |
| Citric acid | 3.90 |
| Sodium chloride | 0.13 |
| Potassium chloride | 0.38 |
| Hydrochloric acid 4 M | 6.00 |
| Water | 9.7 |

EXAMPLE 7

The first wash activity of a large number of potential first wash lipolytic enzyme was tested using the "Assay for test of First Wash effect" described in the Materials and Methods section above with a specific commercial detergent - Ariel Futur (commercially available batch No.4279 B 23:35). The enzymes already present in the detergents were inactivated by heat (4 minutes at 85°C in micro oven) prior to wash.
The first table can be used to compare to example 5 & 6.
Afterwards the results are divided as follows:

a) % removal when dosing after LU-units (see methods & materials for definition)
b) % removal when dosing after milligrams of pure enzyme protein

c) delta Reflectance when dosing after LU-units (see methods & materials for definition)
d) delta Reflectance when dosing after milligrams of pure enzyme protein

Note: ¤ are produced in Aspergillus oryzae as described in example 2
* are produced in yeast as described in example 4.

The following results were obtained:

| Enzyme Inactivated Commercial European Detergent | | |
|---|---|---|
| Lipolytic Enzyme | % removal | |
| | at 1250 LU/l | 12500 LU/l |
| SPIRR+D57G+G59V+N94K+D96L+L97M+ S116P+S170P+Q249R * | 12% | 37% |
| SPIRR+A49P+D167G+E210V * | 8% | 38% |
| SPIRR+E56K+D57G+D62R+S83T+S85F+ D96L+D102Y+E210K * | 8% | 34% |
| SPIRR+N94K+F95L+D96H+N101S+P181L+ D234Y+I252L+P256T+G263A+L264Q * | 11% | 37% |
| E1SPIRPRP+D57G+N94K+D96L+L97M+ Q249R¤ | 27% | 53% |
| Lumafast™ (Ps. mendocina) | 3% | 3% |
| Lipomax™ (Ps. pseuodoalcaligenes L21M) | 1% | 0% |
| Fusarium solani *pisi* | 0% | 1 % |
| Lipolase | 0% | 0% |

| a) Enzyme Inactivated Commercial European Detergent | | | |
|---|---|---|---|
| Lipolytic Enzyme | % removal 1250 LU/l | at 2500 LU/l | 12500 LU/l |
| SPIRR+D57G+N94K+D96L+L97M+Q249R* | 12% | n.d. | 38% |
| SPIRR+N94K+D96L+Q249R* | 13% | n.d. | 45% |
| SPIRR+I90F+D96L+E99K+V187A* | n.d. | 27% | 48% |
| SPIRR+D137G+D167G+D210V+W221L* | 13% | n.d. | 47% |
| SHSRHNA+D57G+N94K+D96L+L97M+Q249R* | n.d. | 22% | 53% |
| GPIRPRP+D57G+N94K+D96L+L97M+Q249R* | n.d. | 26% | 54% |
| TAIRPRK+D57G+N94K+D96L+L97M+Q249R* | n.d. | 34% | 55% |

| b) Enzyme Inactivated Commercial European Detergent | | |
|---|---|---|
| Lipolytic Enzyme | % removal | |
| | 0.25 mg/l | at 2.50 mg/l |
| I90F+D96L+E99K+V187A¤ | 1% | 26% |
| E1PSPIRPR+D57G+N94K+D96L+L97M+Q249R¤ | 21% | 51% |

| c) Enzyme Inactivated Commercial European Detergent | | | |
|---|---|---|---|
| Lipolytic Enzyme | delta Reflectance (dR) | | |
| | 1250 LU/l | 5000 LU/l | 12500 LU/l |
| A47V+D92G+D96L+A121T¤ | 1 | n.d. | 2 |

(continued)

| c) Enzyme Inactivated Commercial European Detergent | | | |
|---|---|---|---|
| Lipolytic Enzyme | delta Reflectance (dR) | | |
| | 1250 LU/l | 5000 LU/l | 12500 LU/l |
| D57G+N94K+D96L+P256T¤ | 0 | n.d. | 2 |
| N94K+D96A+Q249R¤ | 0 | n.d. | 2 |
| SPIRR+Lipolase™* | n.d. | n.d. | 3 |
| D57G+G59V+N94K+D96L+L97M+S116P¤ | 1 | n.d. | 3 |
| D57G+N94K+D96L+L97M+D167G+E210V¤ | 1 | n.d. | 3 |
| QPIRR+D57G+N94K+D96L+L97M+Q249R¤ | 1 | n.d. | 3 |
| SPIR+D57G+N94K+D96L+L97M+Q249R¤ | n.d. | n.d. | 4 |
| SHWQQ+D57G+N94K+D96L+L97M+Q249R¤ | 1 | n.d. | 4 |
| I90F+D96L+E99K+V187A+D234Y¤ | 1 | 4 | n.d. |
| E1AWWPSPIRPRP+D57G+N94K+D96L+L97M+ Q249R¤ | 2 | 6 | n.d. |
| SPIRR+A19T+D167G+E210V+W221L* | 1 | n.d. | 6 |
| SPIRR+D57G+N94K+D96L+P256T* | 1 | n.d. | 6 |
| SPIRR+E56K+D57G+D62R+S83T+S85F+ D96L+D102Y+E210K* | 4 | n.d. | 11 |
| SPIRR+N94K+F95L+D96H+N101S+F181L+ D234Y+Y252L+P256T+G263A+L264Q* | 4 | n.d. | 11 |
| SPIRR+D57G+G59V+N94K+D96L+L97M+S116 P+S170P+Q249R* | 5 | n.d. | 11 |
| SPIRR+A49P+D167G+E210V* | 3 | n.d. | 12 |
| SPIRR+N94K+D96L+Q249R* | 4 | n.d. | 13 |
| SPIRR+D137G+D167G+E210V+W221L* | 5 | n.d. | 13 |
| SPIRR+D57G+N94K+D96L+L97M+Q249R* | 6 | n.d. | 13 |
| SPIRR+I90F+D96L+E99K+V187A* | 6 | n.d. | 15 |

| d) Enzyme Inactivated Commercial European Detergent | | | |
|---|---|---|---|
| Lipolytic Enzyme | delta Reflectance (dR) | | |
| | 0.25 mg/l | 1.00 mg/l | 2.50 mg/l |
| D57G+N94K+D96L+L97M+D167G+E210V¤ | 1 | n.d | 3 |
| S3R.D137G+D167G+E210V+W221L¤ | 0 | 2 | 2 |
| D57G+N94K+D96L+L97M+E210K¤ | n.d. | n.d. | 3 |
| E1SPPRRP+I90F+D96L+E99K+D137G+D167G+ V187A+Q249R¤ | n.d. | 4 | n.d. |
| E87K+G91A+D167G+E210V¤ | n.d. | n.d. | 4 |
| E87K+G91A+E210K¤ | 1 | n.d. | 4 |
| I90F+D96L+E99K¤ | 0 | 2 | 5 |
| APPPRTRPRPRPR+E1S+V2G+S3T+Q4P+D5E+ D57G+N94K+D96L+L97M+Q249R¤ | 0 | 2 | n.d. |
| N94K+D96L+L97M+N233R+Q249R¤ | 0 | 3 | n.d. |
| SPIRKSPIRR+I90F+D96L+E99K+V187A¤ | 1 | 3 | 5 |

(continued)

| d) Enzyme Inactivated Commercial European Detergent | | | |
|---|---|---|---|
| Lipolytic Enzyme | delta Reflectance (dR) | | |
| | 0.25 mg/l | 1.00 mg/l | 2.50 mg/l |
| D137G+D167G+E210V+W221L+N233R¤ | 1 | 3 | 5 |
| SPIRRSPIRR+I90F+D96L+E99K+V187A¤ | 1 | 3 | 6 |
| D167G+E210V+N233R+Q249R¤ | 1 | 3 | n.d. |
| E1W+V2P+N94K+D96L+Q249R¤ | 1 | 3 | n.d. |
| D96L+E99K+V187A¤ | 1 | 3 | n.d. |
| E1SPPWWPRW+N94K+D96L+Q249R¤ | 2 | 3 | n.d. |
| N94K¤ | 2 | 3 | n.d. |
| D96L+D137G+D167G+E210V¤ | 2 | 3 | n.d. |
| E1SQRIKQRIK+I90F+D96L+E99K+V187A¤ | 0 | 4 | n.d. |
| E1SPPRRP+I90F+D96L+E99K+D137G+D167G+ V187A+Q249R¤ | 0 | 4 | n.d. |
| I90F+D96L+E99K+V187A+D234Y+Q249R¤ | 0 | 4 | n.d |
| I90F.D96L+E99K+V187A+N233R¤ | 1 | 4 | n.d |
| E1A+S3R+N94K+D96L+Q249R¤ | 1 | 4 | n.d. |
| S3R+I90F+D96L+E99K+V187A+Q249R¤ | 1 | 4 | n.d |
| E1A+I90F+D96L+E99K+V187A¤ | 1 | 4 | 7 |
| I90F+D96L+E99K+V187A¤ | 1 | 4 | 8 |
| E1SPIRPRP+D57G+N94K+D96L¤ | 2 | 4 | n.d. |
| E1SPPWWP.N94K+D96L+Q249R¤ | 2 | 4 | n.d. |
| SPIRK+D57G+N94K+D96L+L97M+Q249R¤ | 3 | 4 | 10 |
| SPIRRP+D57G+N94K+D96L+L97M+Q249R¤ | 3 | n.d. | 11 |
| I90F+D96L+E99K+V187A+Q249R¤ | 1 | 5 | 8 |
| I90F+D96L+E99K+V187A+T231R¤ | 2 | 5 | n.d. |
| E1SPPRWP+N94K+D96L+Q249R¤ | 2 | 5 | n.d. |
| E1SPPRWPWR+N94K+D96L+Q249R¤ | 2 | 5 | n.d. |
| N94K+D96L+E99K¤ | 2 | 5 | n.d. |
| E1A+I90F+D96L+E99K+Q249R* | 1 | 6 | n.d. |
| E1K+D96L+D167G+E210V+N233R+Q249R¤ | 2 | 6 | n.d. |
| E1SPIRKPRIK+I90F+D96L+E99K+V187A¤ | 2 | 6 | n.d. |
| SHWRK++D57G+N94K+D96L+L97M+Q249R¤ | 3 | 6 | n.d. |
| SPIRKAWWP+I90F+D96L+E99K+V187A¤ | 2 | 7 | 10 |
| N94K+D96L+E99K+Q249R¤ | 2 | 7 | n+d. |
| E1SPPWRPRR+N94K+D96L+Q249R¤ | 2 | 7 | n.d. |
| E1SPPRWPRR+N94K+D96L+Q249R¤ | 2 | 7 | n.d. |
| D137G+D167G+E210V+W221L+D234R¤ | 2 | 7 | n.d. |
| P-4C+N94K+D96L+E239C+Q249R¤ | 3 | 7 | n.d. |
| E1SPIRPRPSPIRPRP+D57G+N94K+D96L+L97M 3 +Q249R¤ | | 7 | n.d. |

(continued)

| d) Enzyme Inactivated Commercial European Detergent | | | |
|---|---|---|---|
| Lipolytic Enzyme | delta Reflectance (dR) | | |
| | 0.25 mg/l | 1.00 mg/l | 2.50 mg/l |
| E1APPPRPRPRPRP+V2G+D5E+D57G+N94K+ 4 D96L+L97M+Q249R* | | 7 | n.d. |
| E1SPPWPRPRP+N94K+D96L+Q249R¤ | 2 | 8 | n+d. |
| E1SPKRKPRP+D137G+D167G+E210V+W221L¤ | 3 | 8 | n.d. |
| E1SPPRRP+D96L+E99K+D137G+D167G+ V187A+Q249R¤ | 4 | 8 | n.d. |
| E1SPPRRP+D57G+N94K+D96L+Q249R¤ | 4 | 9 | 11 |
| E1SPIRPRP+N94K+D96A+Q249R¤ | 4 | 9 | n.d. |
| E1SPPRRP+D57G+N94K+D96L+L97M+Q249R¤ | 5 | 9 | n.d. |
| E1SPPRRP+I90F+D96L+E99K+D137G+V187A¤ | 5 | 9 | n.d. |
| E1SPPRRP+Y53C+D57G+N94K+D96L+K127C+ Q249R¤ | 5 | 9 | n.d. |
| E1SPPRRP+I90F+D96L+E99K+D137G+V187A+ Q249R¤ | 4 | 10 | n.d. |
| E1SPPRRP+N94K+D96L+Q249R¤ | 5 | 10 | n.d. |
| E1SPPRRP+N94K+D96L+E99K¤ | 5 | 10 | n.d. |
| E1SPPRRP+N94K+D96L+E99K+Q249R¤ | 5 | 10 | n.d. |
| E1SPIRPRP+D57G+N94K+D96L+Q249R¤ | 6 | 10 | 13 |
| E1SPPRRP+I90F+D96L+E99K+V187A¤ | 6 | 10 | 15 |
| E1SPIRPRP+N94K+D96L+L97M+Q249R¤ | 6 | 10 | n.d. |
| E1SPPRPRP+N94K+D96L+Q249R¤ | 6 | 10 | n.d. |
| APPPRPRLLPIS+D5E+D57G+N94K+D96L+ L97M+Q249R* | 6 | 10 | n.d. |
| E1SPIRPRP+D137G+D167G+E210V+W221L¤ | 7 | 10 | 13 |
| E1SPPPRPRP+N94K+D96L+L97M+Q249R¤ | 7 | 10 | n.d. |
| E1SPIRPRP+N94K+D96L+Q249R¤ | 7 | 11 | n.d. |
| E1SPIRPRP+D57G+N94K+D96L+L97M+Q249R¤ | 7 | 13 | 16 |

EXAMPLE 8

The first wash activity of one first wash lipolytic enzyme was tested using the "Assay for test of First Wash effect" described in the Materials and Methods section above with an array of commercial detergents. The enzymes already present in the detergents were inactivated by heat (4 minutes at 85°C in microoven) prior to wash.

The Lipolase variant E1SPTRPRP+D57G+N94K+D96L+L97M+Q249R produced in Aspergillus oryzae as described in example 2 was used.

The following different geographic condtions were used:

| | | |
|---|---|---|
| European | Time | 20 min. |
| | Temperature | 30°C |
| | Water hardness | 3.2 mM $Ca^{2\cdot}/Mg^{2\cdot}$ (5:1) ~18°dH |
| US | Time | 10 min. |
| | Temperature | 30°C |
| | Water hardness | 1.07 mM $Ca^{2\cdot}/Mg^{2\cdot}$ (5:1) ~ 6°dH |

| E1SPIRPRP+D57G + N94K + D96L + L97M + Q249R⌑ in US detergents | | |
|---|---|---|
| Detergent | **dR at 0.25mg/l** | **dR at 1.00 mg/l** |
| Wisk HDL (2 g/l) | 3 | 5 |
| Wisk w. bleach (1 g/l) | 3 | 7 |
| Surf w. bleach (1 g/l) | 1 | 4 |
| Tide HDL (2 g/l) | 1 | 4 |
| Tide w. bleach (1 g/l) | 2 | 5 |

| E1SPIRPRP+D57G + N94K + D96L + L97M + Q249R⌑ in European detergents | | |
|---|---|---|
| Detergent | **dR at 0.25mg/l** | **dR at 1.00 mg/l** |
| Ariel Futur (5 g/l) UBA 06731122 | 6 | 12 |
| Ariel Futur color (5 g/l) UBA 06730101 | 7 | 11 |
| Tandil Ultra Plus (5 g/l) UBA 02500191 | 4 | 12 |
| Tandil Ultra Plus Color (5g/l) UBA 05761612 | 5 | 13 |
| Sunil Aktiv (5.5 g/l) UBA 05580168 | 4 | 15 |
| Sunil Aktiv Citrus (5.5 g/l) UBA 05580168 | 3 | 13 |
| Sunil Aktiv Color (5.5 g/l) UBA 05580169 | 4 | 13 |
| Persil Megapearls (5 g/l) UBA 04163661 | 2 | 12 |
| Persil Megapearls Color (5 g/l) UBA 04163662 | 3 | 16 |

EXAMPLE 9

PRODUCTION OF A FIRST WASH LIPASE IN *F. GRAMINARUM*

**Strains and Media**

The starting strain is *Fusarium graminearum* A3/5 (ATCC 20334).

COVE plates are comprised of 343.3 g of sucrose, 20 ml of COVE salts solution, 10 ml of 1 M acetamide, 10 ml of 3 M CsCl, and 25 g of Noble agar per liter. The COVE salts (50X) solution is comprised of 26 g of KCl, 26 g of $MgSO_4$-$7H_2O$, 76 g of $KH_2PO_4$, and 50 ml of COVE trace metals solution. COVE trace metals solution is comprised of 0.04 g of $NaB_4O_7$-$10H_2O$, 0.040 g of $CuSO_4$-$5H_2O$, 0.70 g of $FeSO_4$-$H_2O$, 0.80 g of $Na_2MoO_2$-$2H_2O$, and 10 g of $ZnSO_4$ per liter.

M400Da medium is comprised of 50 g of maltodextrin, 2.0 g of $MgSO_4$-$7H_2O$, 2.0 g of $KH_2PO_4$, 4.0 g of citric acid, 8.0 g of yeast extract, 2.0 g of urea, and 0.5 ml of trace metals solution per liter. The medium is adjusted to pH 6.0 with 5 N NaOH. The trace metals solution is comprised of 14.3 g of $ZnSO_4$-$7H_2O$, 2.5 g of $CuSO_4$-$5H_2O$, 0.5 g of $NiCl_2$-$6H_2O$, 13.8 g of $FeSO_4$-$7H_2O$, 8.5 g of $MnSO_4$-$H_2O$, and 3.0 g of citric acid per liter.

**Construction of the *H. lanuginosa* lipolytic enzyme variant HL A (E1SPIRPRP+D57G+N94K+D96L+L97M+Q249R) expression plasmid for *Fusarium graminearum***

The construction an expression plasmid for *Fusarium graminearum* is outlined in Fig. 7.

Specifically, a *Fusarium* expression cassette is made using the technique of overlapping PCR (Higuchi. R., *In* Innis, M.A.. Gelfond, D.H., Snisky. J.J., and White. T.J., editors. *PCR Protocols: A Guide to Methods and Applications,* pages 177-183,. Academic Press, Inc., New York) to fuse the 1.24 kb *Fusarium oxysporum* trypsin promoter to the 1.1 kb *Fusarium oxysporum* trypsin terminator (Royer *et al*., 1995 *Bio/Technology* 13: 1479-1483). A polylinker region containing *Swa*I, *Kpn*I and *Pac*I restriction sites is inserted between the promoter and terminator as part of the overlapping PCR reaction. At the 5'end of the promoter an *Xho*I site is added and the native *Eco*RI site is preserved. At the 3' end of the terminator, *Eco*RI, *Hind*III and *Nsi*I sites are incorporated by the PCR reaction.

A PCR fragment containing -1208 to -1 of the *Fusarium oxysporum* trypsin promoter plus a 25 bp polylinker is generated from plasmid pJRoy20 (Royer *et al.*, 1995, *supra*) using the following primers:

*Xho*I *Eco*RI 5'end of promoter

Forward primer 1: 5'-gagctcgagGAATTCTTACAAACCTTCAAC-3'

*Pac*I *Kpn*I *Swa*I 3'end of promoter

Reverse primer 2: 5'-ttaattaaggtacctgaatttaaatGGTGAAGAGATAGATATCCAAG-3'

Upper case letters are the native sequence of the *Fusarium oxysporum* trypsin promoter.

The PCR conditions used are 95°C for 3 minutes followed by 25 cycles each at 95°C for 30 seconds, 50°C for 1 minute, and 72°C for 1 minute. The final extension cycle is at 72°C for 5 minutes. *Pwo* DNA polymerase (Boehringer Mannheim, Indianapolis, IN.) is used with the manufacturer's supplied buffer.

A PCR fragment containing -5 to -1 of the *Fusarium oxysporum* trypsin promoter, the 25 bp polylinker and 1060 bp of the 3' untranslated region of the *Fusarium oxysporum* trypsin gene (terminator region) is generated from plasmid pJRoy20 using the following primers:

promoter *Swa*I *Kpn*I *Pac*I 5' end of terminator

Forward primer 3: 5'-TCACCatttaaattcaggtaccttaattaaATTCCTTGTTGGAAGCGTCGA-3'

*Nsi*I *Hind*III *Eco*RI 3' end of terminator

Reverse primer 4: 5'-tggtatgcataagcttgaattcAGGTAAACAAGATATAATTT-3'

Upper case letters are the native sequence of the *Fusarium oxysporum* trypsin promoter and terminator. The PCR conditions used are as described above.

The final 2.3 kb overlapping PCR fragment which contains -1208 to -1 of the *Fusarium oxysporum* trypsin promoter, the 25 bp polylinker and 1060 bp of the *Fusarium oxysporum* trypsin terminator is made using 0.2 µl of the first PCR (promoter) reaction and 3 µl of the second (terminator) reaction as template and primers number 1 and 4. The PCR conditions used are 95°C for 3 minutes followed by 30 cycles each at 95°C for 30 seconds, 62°C for 1 minute, and 72°C for 3 minutes. The final extension cycle is 5 minutes at 72°C. Pwo DNA polymerase is also used for this reaction.

The resulting 2.3 kb band is digested with *Xho*I and *Nsi*I and cloned into plasmid pBaNe6 that is digested partially with *Nsi*I and to completion with *Sal*I. In effect, the *Aspergillus* promoter and terminator sequences of pBaNe6 are replaced with the *Fusarium oxysporum* trypsin promoter and terminator sequences. The resulting construct (pDM174.3) is digested with *Swa*I and *Pac*I.

DNA primers HLIP-A and HLIP-B shown below are used in a PCR reaction to amplify the HLA lipase gene from plasmid pJVi220:

HLIP-A (Primer 5): 5'-cccatttaaatATGAGGAGCTCCCTTGTGCTG-3'

HLIP-B (Primer 6): 5'-ccettaattaaCTAAAGACATGTCCCAATTAA-3'

Uppercase letters represent sequences in the lipase gene

The PCR is performed in a 50 µl reaction containing *ca*. 50 ng of pHLA, 0.05 mM each of dATP, dTTP, dGTP, dCTP, 100 pmol each of HLIP-A and HLIP-B. 1X *Pwo*I Buffer (Boehringer Mannheim, Indianapolis, IN), and 2.5 units *Pwo*I (Boehringer Mannheim, Indianapolis, IN). The PCR conditions are 95°C for 3 minutes, 30 cycles each at 95°C for 1 minute, 60°C for 1 minute; and 72°C for 1.5 minutes, and then 72°C for 5 minutes. The PCR reaction mixture is run on a agarose gel and the *ca*. 0.9 kb HLA DNA band is excised. The DNA is purified by solubilization of the agarose with 3 volumes Qia-ex solubilization buffer (Qiagen, Los Angeles, CA) followed by a Qiaquick PCR spin column according to the manufacturer's directions (Qiagen, Los Angeles, CA). The DNA is recovered in 50 µl of 1 mM EDTA-10 mM Tris pH 8. A 20 µl aliquot of the DNA is cut in a final volume of 25 µl containing 1X restriction enzyme buffers and restriction enzymes *Pac*I and *Swa*I as suggested by the manufacturers. The reaction mixture is then heated at 80°C for 10 minutes. One µl of the *Pac*I/*Swa*I cut HLA lipase gene is ligated into *Pac*I/*Swa*I cut plasmid pBANe6. The ligation mixture is used to transform *E. coli* strain DH5α. The plasmid containing pBANe6 and the HLA sequences is designated pJeRS33.

The 0.9 kb *Swa*I/*Pac*I HLA fragment from pJeRS33 is cloned into the *Swa*I/*Pac*I digested pDM174.3 vector to create plasmid pDM177.

**Transformation of *Fusarium graminearum***

*F. graminearum* strain A3/5 (ATCC 20334) is grown on 10 x 15 mm petri plates of Vogels medium (Vogel, 1964, *Am. Nature* 98: 435-446) plus 1.5% glucose and agar for 3 weeks at 25°C. Conidia (approximately $10^8$ per plate) are dislodged in 10 ml of sterile water using a transfer loop and purified by filtration through 4 layers of cheesecloth and finally through one layer of Miracloth. Conidial suspensions are concentrated by centrifugation. Fifty ml of YPG medium comprised of 1% yeast extract, 2% bac-topeptone, and 2% glucose are inoculated with approximately 100 conidia, and incubated for 14 hours at 24°C, 150 rpm. Resulting hyphae are trapped on a sterile 0.4 mm filter and washed successively with sterile distilled water and 1.0 M $MgSO_4$. The hyphae are resuspended in 10 ml of NOVOZYM 234™ (Novo Nordisk A/S, Bagsvaerd, Denmark) solution (2-10 mg/ml in 1.0 M $MgSO_4$) and digested for 15-30 minutes at 34°C with agitation at 80 rpm. Undigested hyphal material is removed from the resulting protoplast suspension by successive filtration through 4 layers of cheesecloth and through Miracloth. Twenty ml of 1 M sorbitol are passed through the cheesecloth and Miracloth and combined with the protoplast solution. After mixing, protoplasts (approximately 5 x $10^8$) are pelleted by centrifugation and washed successively by resuspension and centrifugation in 20 ml of 1 M sorbitol and in 20 ml of STC (0.8 M sorbitol, 0.05 M Tris pH 8.0, 0.05 M $CaCl_2$). The washed protoplasts are resuspended in 4 parts STC and 1 part SPTC (0.8 M sorbitol, 40 % PEG 4000, 0.05 M Tris pH 8.0, 0.05 M $CaCl_2$) at a concentration of 1-2 x $10^8$/ml. One hundred µl of protoplast suspension are added to 3 µg pDM177 DNA and 5 µl heparin (5 mg/ml in STC) in polypropylene tubes (17 x 100 mm) and incubated on ice for 30 minutes. One ml of SPTC is mixed gently into the protoplast suspension and incubation is continued at room temperature for 20 minutes. Fifteen ml of molten solution (cooled to 40°C) consisting of COVE salts, 0.8 M sucrose and 1 % low melting agarose (Sigma Chemical Company, St. Louis, MO) are mixed with the protoplasts and then plated onto a 150 mm petri plate containing COVE agar. Incubation is continued at room temperature for 10 to 14 days.

**Expression of lipase activity**

Five pDM177 transformants of *Fusarium graminearum* A3/5 are cultured on M400Da medium in shake flasks for 7 days at 30°C. As a control culture, an A3/5 transformant of plasmid pDM155 (Royer *et al*., 1995 *Bio*/*Technology* 13: 1479-1483), which contains the wild-type *Humicola lanuginosa* lipase inserted between the *Fusarium oxysporum* trypsin promoter and terminator, is grown at the same time under the same conditions.

**First wash activity of HL A**

The above produced lipase as well as the analogous lipase (carrying the same mutations, but produced in *A. oryzae*) was tested in the "Assay for test of First Wash effect" described herein using enzyme inactivated Ariel Futur. The following results were obtained:

| E1SPIRPRP+D57G+N94K+D96L+L97M+Q249R in | dR at | |
|---|---|---|
| | 1250 LU/l | 12500 LU/l |
| A.oryzae | 8 | 15 |
| F.graminearum | 9 | 14 |

EXAMPLE 10 -

**Construction of Absidia reflexa mutants**

Material and Methods

Strains and plasmids are listed in the Materials and Methods section preceeding the examples.
*Primers*: primer TiK57, primer TiK58 , primer TiK59, Primer TiK60, Primer TiK 61 ,
primer TiK62, primer TiK64, primer Tik66, primer TiK72, primer TiK74, primer Tik75, primers TiK76.
*Kits, solutions, media and the like*:

Taq-DNA polymerase (Promega)
LB medium supplemented with 100 µg/ml ampicillin
DNA Maxi-Prep kit (QIAGEN).
Polyethyleneglycol/LiOAc yeast transformation kit (Yeastmaker, Clontech).
Yeast nitrogen base w/o amino acids (Difco 0919-15-3)
Casamino acids (Difco 0230-01-1)
Bacto-agar (Difco)
YPG-medium (20 g casein-peptone and 10 g yeast extract (Difco))
LB medium supplemented with 100 µg/ml ampicillin

*Equipment*

Applied Biosystems 373 DNA Sequencer
Cellulose acetate filters (Schleicher-Schüll)

*SYC-plates*

13.6 g NaOH and 22.6 g succinic acid are completely dissolved in 500 ml $H_2O$, 15 g yeast nitrogen base w/o amino acids (Difco 0919-15-3), 10 g casamino acids (Difco 0230-01-1). 20 ml of a 2 % threonine solution, and 20 ml of a 1 % tryptophan solution are added. The solution is filled up to 1 litre $H_2O$. sterile-filtered, and stored at 4°C. For liquid media preparation 1 litre of SYC is diluted by adding 200 ml of a 20% glucose solution and 800 ml of $H_2O$. For preparation of agar plates 1 litre of SYC is diluted at 60°C with 800 ml of bacto-agar (37.5 g bacto-agar (Difco) dissolved in 1 litre $H_2O$) and 200 ml of a 20% glucose solution.

*Brilliant Green assay*

For preparation of BG-agar plates 10 g of agarose are dissolved by heating in 500 ml of a 100 mM Tris-Cl buffer pH 9.0. This agarose solution is mixed at 60°C with 24 ml of an olive oil emulsion (8 ml olive oil (Sigma) and 16 ml of a 2 % polyvinylalcohol (Sigma) solution in $H_2O$ are mixed and thoroughly homogenized on ice with an Ultra-Turrax T25), 10 ml of a Brilliant Green stock (4 mg/ml $H_2O$), 465 ml of 100 mM Tris-Cl buffer pH 9.0 containing 6.45 g of the detergent, and 1.4 ml of a 250 mM $CaCl_2$ solution.
Transformed yeast colonies are grown for 3 days at 30°C on sterile cellulose acetate filters (Schleicher-Schüll) on SYC-agar plates. The filters are then transferred to the BG-agar plates and incubated for 2-8 hours at 37°C. Colonies which generate a green spot in the agar are judged positive.

*Restriction enzyme analysis and sequencing of mutant libraries*

The recovered plasmid DNA was subjected to *NcoI*-digestion. Correct clones yielded two fragments of 2.9 and 3.1 kb. The plasmid DNA was subsequently either dye-terminator PCR cycle sequenced or sequenced on an

Applied Biosystems 373 DNA Sequencer. In the case of clones isolated from libraries 3 and 4 (Table 1) the oligonucleotides TiK61 and TiK66 were used for double-strand sequencing whereas for libraries 7 and 8 TiK62 and TiK72 were applied.

**Example 10A**

Construction of *Absidia reflexa* ATTC 44896 lipase expression vectors

Two vectors were constructed for expression of the wild-type *Absidia reflexa* ATTC 44896 lipase (with/without SPIRR peptide extension) in *Saccharomyces cere visiae*.

The cDNA clone encoding the mature *Absidia reflexa* ATTC 44896 lipase (SEQ ID No. 13 and Figure 1) was PCR amplified ($T_a$ = 50°C, 25 cycles, 5 units Taq-DNA polymerase) using either the primer pair TiK57/59 (providing a N-terminal SPIRR extension) or the primer pair TiK58/59 (without SPIRR extension). The PCR fragments were ligated via NheI/XbaI-sites into the yeast expression vector pJSO37 (with a NheI site introduced downstream of the signal peptide) which harbours region encoding an active *H. lanuginosa* lipase in the original BamHI/XbaI cloning site.

The gene encoding the active *H. lanuginosa* lipase was slightly modified by the introduction of a NheI-site downstream of the BamHI site (see Figure 2). Since NheI- and XbaI-sites are compatible, the vector was treated with alkaline phosphatase prior to ligation. Finally, two vector constructs were obtained, namely pTiK04 which includes the SPIRR extension just upstream of the start of the mature lipase gene, and pTiK05 which did not contain a SPIRR extension encoding part. In both cases the original signal sequence from the *Humicola lanuginosa* lipase was kept constant between the *BamHI* and *NheI* sites.

MFα1 signal sequence constructs

The *H. lanuginosa* lipase signal sequence was replaced by the mating factor α1 signal.
Genomic DNA of the yeast strain YPH499 (Stratagene) was prepared cording to standard protocols (Ausubel et al., (1995). Current Protocols in Molecular Biology, John Wiley and Sons). One µg of genomic DNA was subjected to PCR ($T_a$ = 50°C, 25 cycles, 5 units Taq-DNA polymerase) with the primer pair TiK74/75. The amplified MFα1 signal sequence fragment (Fig. 3) was inserted into pTiK04 and pTiK05 via the *BamHI* and *NheI* sites to yield pTiK06 and pTiK07, respectively.

**Example 10B**

Construction of *Absidia reflexa* ATTC 44896 lipase variants by mutagenesis with doped oligonucleotides

Four libraries were constructed with doped oligonucleotides.

In libraries A and B (see Table 1 below)) random mutations were introduced in the putative lid region of *Absidia reflexa* ATTC 44896 lipase. In library A the SPIRR sequence was kept constant, in library B it was omitted.

Libraries C and D were constructed with mutations in two putative lipid contact zones in the N-terminus of *Absidia reflexa* ATTC 44896. In library C the SPIRR sequence was again kept constant.

The mutagenesis of the putative lid region of *Absidia reflexa* ATTC 44896 lipase gene (amino acid positions 82-99) was performed by standard PCR (Sambrook et al., (1989), Molecular cloning - A laboratory manual, Cold Spring Harbor Laboratory Press, Cold Spring Harbor. NY) with 5 units Taq-DNA polymerase with the primer pair TiK60/TiK64 at $T_a$ = 50°C and 25 cycles using pTiK05 as template. In a second standard PCR, using primer TiK62 and the agarose gel-purified DNA fragment generated in the first PCR, the *whole Absidia reflexa* ATTC 44896 lipase gene was restored using the same amplification conditions as for the first PCR. In contrast to the first PCR, pTiK04 or pTiK05 were chosen as templates so that libraries (with/without SPIRR N-terminal extension) were obtained.

For mutagenesis of the amino acid positions 30-45 the primers TiK76 and TiK64 were used for the first PCR. The second PCR was in principle identical to the above described one.

The obtained PCR fragments were ligated into pJSO37 via the *BamHI/XbaI*-sites. The transformation of *E. coli* and subsequently of competent YNG318 yeast cells was performed as described above.

Table 1 Summary of the constructed and screened *Absidia reflexa* ATTC 44896 mutant libraries.

| No | Comments and mutagenized regions | Library size in *E.coli* | Screened *S.cerevisiae* colonies | Positives in the BG-assay at X g/l detergent | Positives in the BG-assay at X g/l. 2.round | Restriction enzyme analysis |
|---|---|---|---|---|---|---|
| A | Lid (pos. 82-99) (.SPIRR) | $8 \times 10^6$ | 180000 | 21 at 3g/l | 9 at 3 g/l | 6 |
| B | Lid (pos. 82-99) | $7 \times 10^6$ | 200000 | 1 at 6 g/l<br>5 at 3 g/l | 0 at 6 g/l<br>2 at 3 g/l | -<br>2 |
| C | Pos. 30-45 (.SPIRR) | $1 \times 10^6$ | 160000 | 38 at 3 g/l | 4 at 3 g/l | 3 |
| D | Pos. 30-45 | $6 \times 10^5$ | 170000 | 27 at 3 g/l | 2 at 3 g/l | 1 |

**Example 10C**

Screening of the *Absidia reflexa* ATTC 44896 lipase mutant libraries and recovery of plasmids from positive colonies

Transformed yeast cells obtained as described in Example 10B were spread on a cellulose acetate filter on a 14 cm SYC-agar plate. After selective growth of transformants and transfer of the filter to BG-agar plates, positive colonies were picked, resuspended in $H_2O$, and re-spread on cellulose acetate filters on SYC-agar plates to allow a second confirmatory round of the BG-assay (see Matarials and Methods section).
Positive clones from the second round were transferred to 20 ml SYC-medium and shaken for 2 days at 30°C. Plasmid DNA was prepared from 1.5 ml of this saturated yeast culture according to the standard phenol/glassbead method (Ausubel et al., (1995), Current Protocols in Molecular Biology, John Wiley and Sons).
An aliquot of the plasmid preparation was electroporated into *E.coli* DH10B cells which were then plated on LB-agar plates supplemented with 100 µg/ml ampicillin. The DNA of the colonies was isolated and applied to restriction enzyme analysis and sequencing as described in the Material and Methods section above.

Sequencing

Sequencing of 15 randomly picked clones in library A and B showed that the chosen doping of 10% finally resulted in:

20 % being wild type,
13% with 1 amino acid exchange,
20% with 2 exchanges,
13% with 3,
20% with 4,
0% with 5, and
13% with 6 amino acid exchanges per molecule.

From library A six clones out of $1.8 \times 10^5$ screened colonies at 3 g/l detergent were isolated in the BG-assay (1 per each $3 \times 10^4$ screened), and from library B two positive clones were isolated out of $2.0 \times 10^5$ screened colonies (1 per each $1.0 \times 10^5$ screened, Table 1). The noticed sequence differences of these 8 clones are depicted in Table 2 below.
As mentioned above libraries C and D were constructed with mutations in two putative lipid contact zones in the N-terminus of *Absidia reflexa* ATTC 44896 lipase. Sequencing of 13 randomly picked clones showed that the chosen doping level of 10% resulted in:

8 % being wild type,
15 % with 1 amino acid exchange,
46 % with 2 exchanges,
15% with 3, and
15% with 4 exchanges per molecule.

Library C yielded 3 clones out of 1.6 x $10^5$ screened colonies at 3 g/l of detergent in the BG assay (1 positive per each 53333 screened), and from library D one positive clone could be isolated out of 1.7 x $10^5$ (Table 1). The sequences of these 4 clones are also depicted in Table 2.

Table 2 Sequences of the improved Absidia reflexa ATTC 44896 variants.

Target sequence:

| | | 82 | | | | | | | | | | | | | | | | | 99 | |
|---|---|---|---|---|---|---|---|---|---|---|---|---|---|---|---|---|---|---|---|---|
| Library | Clone number | T | S | S | I | R | N | A | I | A | D | I | V | F | V | P | V | N | Y | |
| A | 303 | | | | | | | W | | | | | T | | | | | | | |
| | 309 | | | | | H | | T | | | | | | | | | | | | |
| | 312 | S | | | | | | | S | | | | | | | | | | | |
| | 315 | | | | V | | | T | W | L | -N | | | | | | L | | | .. |
| H133R | | | | | | | | | | | | | | | | | | | | |
| | 318 | | C | | | | | W | | | K | | | | L | S | | | I | ..V102F |
| | 321 | | | | | | | S | | | | E | | | | | | | | |
| B | 401 | S | | | | | | S | | | | | | A | | | | | | |
| | 402 | | | | | | | | | | | | | | | | | | | ..Y136H.K137H |

| | | | | | | 30 | | | | | | | | | 45 | | |
|---|---|---|---|---|---|---|---|---|---|---|---|---|---|---|---|---|---|
| | | R | T | V | I | P | G | G | R | W | S | C | P | H | C | G | V |
| C | 701 | | | | | | | | | | W | N | | | | | |
| | 702 | | | | | | | | | | W | N | | | | | |
| | 703 | | | | | | | | | | | | | | | C | |
| | ...Q4R | | | | | | | | | | | | | | | | |
| D | 801 | | | | | | | | | | | | | | | | |
| | | | | | | | | | | | | | | ...V95E | | | |

**Example 10D**

Expression of *Absidia reflexa* ATTC 44896 variants and lipase unit measurement

Four of the improved mutants identified from library A were subjected to measurement of LU secreted to the culture medium as well as LU measurement of the crude cytosol/membrane fraction.

10 ml of YPG-medium were dissolved in 900 ml $H_2O$, autoclaved and 100 ml of a 20% glucose solution added) were inoculated with 1 ml of a saturated yeast culture in SYC-medium. The culture was shaken at 30°C for 2 days. The cells were harvested by centrifugation (5 minutes x 4000 g) and the supernatant stored on ice for LU measurement. The cell pellet was treated with Novozym™234 for spheroplast preparation and lysed using the glass bead procedure (Ausubel et al., (1995). Current Protocols in Molecular Biology, John Wiley and Sons). The obtained crude cytosol fraction which also included the membrane fraction was immediately applied to LU measurement (see Materials and Methods section) in order to minimize proteolytic degradation.

The result is shown in Table 3. The four clones showed weak but significant LU in the cytosol/membrane fraction. Moreover, from two out of these four clones weak LU signals could be recorded. All data differing from 0.0 LU/ml are significant enzyme activities as confirmed by repeated measurements.

Table 3

Summary of the obtained lipase unit secreted to the medium or measured from the cytosol fraction of ATTC 44896 variants from library A.
The standard deviation is 10%.

| Sample | Lipase units in the supernatant (LU/ml) | Lipase units in the cytosol (LU/ml) |
|---|---|---|
| YNG318 (negative control) | 0.0 | 0.0 |
| 303 | 0.0 | 1.0 |
| 309 | 0.0 | 0.5 |
| 312 | 1.7 | 0.6 |
| 321 | 0.6 | 1.0 |

EXAMPLE 12

**Substrate affinity of Lipolytic enzymes**

A procedure has been developed for a simple comparison of the ability of lipolytic enzymes to accumulate on/in a substrate phase (olive oil, incl. FFA) at alkaline pH (pH 9.0) and presence of the non-ionic surfactant Dobanol 25-7 (100 ppm) (i.e. a measure for substrate affinity).

**Procedure:**

1. Two identical buffer solutions (5 ml) are prepared in 20 ml sealable vials, ("Sample" (s) and "Reference" (r)).
2. Enzyme is added into "Sample" and "Reference" and the lipase concentration is determined (X LU/ml).
3. Olive oil is added onto the "Sample" and both lipase solutions are shaken vigorously. Incubation at 4°C over night.
4. Remaining lipase concentration in the aqueous phases is determined after incubation, (Yi LU/ml; i=r,s).

**Summary of incubation conditions:**

| | |
|---|---|
| Buffer: | 100 mM Glycine (5 ml). |
| pH: | 9.0. |
| Substrate: | Olive oil (5 ml). |
| Dobanol 25-7: | 100 ppm. |
| T: | 4°C. |
| Lipase: | 5-10 LU/ml. |
| Incubation: | Over night (24-26 hours). |

**Evaluation of data:**

The result after an experiment is calculated by comparing the activity-loss upon incubation in the aqueous phase in contact with olive oil to the activity-loss in the aqueous phase in absence of olive oil:

$$\alpha = Ys/Yr \text{ (see above)}$$

**Results:**

Table 11:

| Lipase | α (%) |
|---|---|
| Lipolase™ | 95 % |
| D57G+N94K+D96L+L97M+Q249R | 65 % |
| SPIRPR+E1P+D57G+N94K+D96L+L97M+Q249R | 45 % |
| SALRPRK+D57G+N94K+D96L+L97M+Q249R | 25 % |
| SPIRPR+E1P+D137G+D167G+E210V+W221L | 50% |

Comparing results presented above to wash data disclosed in examples 5-8, clearly indicate that Lipolase variants with increased first wash performance generally have increased substrate affinity as compared to Lipolase.

**Example 13**

Localized random mutagenesis of the Pseudomonas sp. lipase (Liposam)

A suitable doping scheme to use for introducing mutations contemplated to lead to a first wash activity of the above lipase may comprise localized random mutagenesis in the whole or parts of one or more of the following regions. 93 % wt/7% random means that the respective codons are synthesized with 93% wt nucleotides and 7% of the other 3 nucleotides in the oligonucleotide used for constructing the random mutagenized library. Similarly for 90% wt/10% random.

In the amino acid region 17-37:
Amino acid position 17-18+20-24+26-29+32-37: 93% wt/7% random M19: doped to give preferentially L,I,F

In the amino acid region 109-161:
Amino acid position 109-118: 93% wt/7% random
Amino acid position 120 + 123-137 + 139-161: 90% wt/10% random

In the amino acid region 208-239:
Amino acid position 208-212+214-215+217-231+233-239: 90% wt/10% random

In the amino acid region 253-271:
Amino acid position 253+255+259-268+270-271: 90% wt/10% random
V258: 90% wt/10% random but doped not to be a positive charged amino acid.

The localized random mutagenesis may be performed as described in the Materials and Methods section and in Example 1 herein, and the resulting mutants screened for a reduced dependence to calcium and/or an increased tolerance towards a detergent or detergent component and afterwards first wash activity. Subsequently, and if necessary, localized random mutagenesis of the resulting mutants may be repeated and/or the genes may be subjected to gene shuffling as disclosed herein.

REFERENCES CITED IN THE SPECIFICATION

Muhlrad et al., 1992, Yeast, Vol. 8, 79-82
Shimada. Y, et al. (1989), cDNA Molecular Cloning of Geotrichum candidum Lipase, J. Biochem., 106, 383-388,
Hunkapiller et al., 1984, Nature 310:105-111
R. Higuchi, B. Krummel, and R.K. Saiki (1988), A general method of *in vitro* preparation and specific mutagenesis of DNA fragments: study of protein and DNA interactions, *Nucl. Acids Res.* **16**:7351-7367,
Yamaguchi. S, et al. (1991), Cloning and structure of the mono- and diglycerol lipase-encoding gene from Penicillium camembertii U-150, Gene 103, 61-67,
Hass. M.J. et al. (1991), Cloning, expression and characterization of a cDNA encoding a lipase from Rhizopus delemar, Gene 109. 107-113,
Kugimiya. W. et al. (1992), Cloning and Sequences Analysis of DNA encoding Rhizopus niveus Lipase, Biosci. Biotech. Biochem. 56, 716-719.
Dartois. V. et al. (1993), Cloning, nucleotide sequence and expression in Escherichia coli of a lipase gene from

*Bacillus subtilis* 168, Biochemica et Biophysica acta 1131, 253-260,
Sambrook et al. Molecular Cloning: A Laboratory Manual, 2nd Ed., Cold Spring Harbor, 1989,
R.K. Saiki et al., Science 239, pp. 487-491, 1988,
Beaucage and Caruthers, Tetrahedron Letters 22, pp. 1859-1869, 1981,
Matthes et al., The EMBO J, 3, pp. 801-805, 1984,
J.O. Deshler, (1992) A simple method for randomly mutating cloned DNA fragments by using chemical mutagens and the
polymerase chain reaction, GATA 9(4): 103-106
Leung et al., Technique, Vol. 1, No. 1, pp. 11-15, 1989
Fowler et al., Molec. gen. Genet., 133, pp. 179-191, 1974,
Brady et al., "A Serine Protease Triad Forms the Catalytic Centre of a Triacylglycerol Lipase", Nature 343, 1990, pp. 767-770, 1990,
Tilbeyrgh. H, van Egloff, M.-P., Martinez.C., Rugani.N., Verger.R. and Cambillau (1993) Nature 362, p. 814-820, Interfacial activation of the lipase-prolipase complex by mixed micelles revealed by X-ray crystallography.
Hudson et al., Practical Immunology, Third edition, Blackwell Scientific Publications, 1989 Alber. T. and Kawasaki, G., J.Mol.Appl. Genet 1, 419-434 (1982)
Joyce, G. F.: Directed Molecular Evolution. *Scientific American* December 1992, pp. 48-55.
Siderovski, D. P., and Mak T. W.: RAMHA: A pc-based monte-carlo simulation of random saturation mutagenesis. *Comput. Biol. Med.* 23(6), pp. 463-474, 1993.
Palzkill T., Le Q.-Q., Venkatachalam K. V., LaRocco M., and Ocera H.:Evolution of antibiotic resistance: several different amino acid substitutions in an active site loop alter substrate profile of b-lactamase. *Molecular Microbiology* 12(2), pp. 217-229, 1994. Ollis, D.L. et al (1992) Prot. Eng. 5, p. 197.
Svendsen, A. et al. (1995) Biochim. Biophys. Acta. 1259, p. 9.
Grochulski, P. et al. (1993) J. Biol. Chem. 268, p. 12843.

## Claims

1. A method of preparing a mutated lipolytic enzyme, which method comprises at least the following steps:

a) subjecting a DNA sequence encoding a parent lipolytic enzyme to mutagenesis to form a variety of mutated DNA sequences;
b) expressing the mutated DNA sequences in host cells;
c) screening for host cells expressing a mutated lipolytic enzyme which has a decreased dependence on calcium and/or an improved tolerance towards a detergent or a detergent component as compared to the parent lipolytic enzyme; and
d) selecting a mutated lipolytic enzyme among those resulting from step (c) which, when present in a detergent composition, is capable of removing at least 15% more lard from a lard stained swatch, than the same detergent composition without the lipolytic enzyme, in a one cycle wash assay comprising subjecting 7 lard-stained cotton swatches (9 x 9 cm) per beaker to a one cycle wash in a thermostated TOM, each beaker containing 1000 ml of water comprising 3.2 mM $Ca^{2+}/Mg^{2+}$ (in a ratio of 5:1) and 5 g/l of said detergent composition, adjusted to pH 10, and comprising 12500 LU/l of the lipolytic enzyme, for 20 minutes at a temperature of 30°C, followed by rinsing for 15 minutes in running tap water and overnight linedrying at room temperature, subsequent extraction and quantification of fatty matter from the resulting swatches by Soxhlet extraction.

**2.** The method of claim 1, wherein the mutagenesis of step (a) is a random mutagenesis.

**3.** The method of the preceding claim wherein the mutagenesis is a localized random mutagenesis performed in the lipid contact zone or in any peptide addition present at the N-terminal and/or the C-terminal end of the parent lipolytic enzyme.

**4.** A method of any of claims 1-3 wherein steps (a), (b), (c) and/or (d) are repeated one or more times.

**5.** The method of any of claims 1-4 wherein the parent lipolytic enzyme is derived from *Humicola lanuginosa* strain DSM 4109 and has the amino acid sequence disclosed in EP 0 305 216.

**6.** The method of any preceding claim wherein the mutated lipolytic enzyme comprises at least 3 mutations as compared to the parent lipolytic enzyme.

**7.** The method of any preceding claim wherein the mutated lipolytic enzyme (compared to the parent lipolytic enzyme) comprises substitution of at least one neutral amino acid residue with a positively charged amino acid residue, deletion of at least one negatively charged amino acid residue, or substitution of at least one negatively charged amino acid residue with a neutral or positively charged amino acid residue.

**8.** The method of any preceding claim wherein the mutated lipolytic enzyme, compared to the parent lipolytic enzyme, has been modified by deletion or substitution of at least one amino acid residue in a lipid contact zone of the parent lipolytic enzyme or addition of at least one amino acid residue to said lipid contact zone.

**9.** The method of any preceding claim wherein the mutated lipolytic enzyme, compared to the parent lipolytic enzyme, comprises at least one mutation within and at least one mutation outside the lipid contact zone of the parent lipolytic enzyme.

**10.** The method of any preceding claim wherein the mutated lipolytic enzyme, compared to the parent lipolytic enzyme comprises a peptide addition applied at or within the C- and/or N-terminal end of the mature form of the parent lipolytic enzyme.

**11.** The method of the preceding claim wherein the peptide addition forms a covalent binding to the mature part of the parent lipolytic enzyme.

**12.** The method of any preceding claim wherein the mutated lipolytic enzyme comprises a cysteine residue in the peptide addition, a cysteine residue in the mature part of the parent lipolytic enzyme and a cystine bridge between the two cysteine residues.

**13.** The method of any of claims 10-12 wherein the peptide addition comprises at least one proline residue.

**14.** The method of the preceding claim wherein the peptide addition comprises two or three proline residues.

**15.** The method of any of claims 10-14 wherein the peptide addition comprises at least one positive or hydrophobic

amino acid residue.

**16.** The method of the preceding claim wherein the peptide addition comprises two or three hydrophobic amino acid residues.

**17.** The method of any of claims 10-16 wherein the peptide addition has a length between 1 and 15 amino acids.

**18.** The method of claim 17 wherein the peptide addition has a length from 4 to 10 amino acids.

**19.** The method of any of claims 10-18 wherein the mutated lipolytic enzyme further comprises a mutation in a non-structural part of the N-terminus and/or C-terminus of the parent lipolytic enzyme.

**20.** The method of the preceding claim wherein the mutation results in the removal of at least one negatively charged amino acid residue

**21.** The method of claim 19 or 20 wherein a negatively charged amino acid residue of said non-structural part has been deleted or replaced by a neutral or positively charged amino acid residue or by a hydrophobic amino acid residue, or wherein a neutral amino acid residue has been replaced with a positively charged amino acid residue.

**22.** A method of preparing a mutated lipolytic enzyme, which method comprises at least the following steps:

a) constructing mutated DNA sequences by combining i) a DNA sequence encoding a first parent lipolytic enzyme or a part of said DNA sequence and ii) a DNA sequence encoding a second parent lipolytic enzyme or a part of said DNA sequence and optionally iii) further DNA sequences encoding a third (and optionally further) parent lipolytic enzymes or a part of said DNA sequence(s), the DNA sequences to be combined being sufficiently homologous to allow for homologous recombination between the sequences to take place,
b) expressing the resulting mutated DNA sequences in host cells, and
c) selecting a mutated lipolytic enzyme among those resulting from step (b) which, when present in detergent composition A and/or B is capable of removing at least 15% more lard from a lard stained swatch, than the same detergent composition without the lipolytic enzyme, in a one cycle wash assay comprising subjecting 7 lard-stained cotton swatches (9 x 9 cm) per beaker to a one cycle wash in a thermostated TOM, each beaker containing 1000 ml of water comprising 3.2 mM $Ca^{2+}/Mg^{2+}$ (in a ratio of 5:1) and 5 g/l of said detergent composition, adjusted to pH 10, and comprising 12500 LU/l of the lipolytic enzyme, for 20 minutes at a temperature of 30°C, followed by rinsing for 15 minutes in running tap water and overnight linedrying at room temperature, subsequent extraction and quantification of fatty matter from the resulting swatches by Soxhlet extraction.

**23.** The method of claim 22, wherein prior to step (c) a screening is made for host cells expressing a mutated lipolytic enzyme which has a decreased dependence on calcium and/or an improved tolerance towards a detergent or a detergent component as compared to any of the parent lipolytic enzymes.

**24.** The method of claim 22 or 23, wherein one or more of the homologous DNA sequences i)-iii) used for constructing the mutated DNA sequence of step (a) have been prepared by

a) subjecting a DNA sequence encoding a parent lipolytic enzyme to mutagenesis to form a variety of mutated DNA sequences;
b) expressing the mutated DNA sequences in host cells;
c) screening for host cells expressing a mutated lipolytic enzyme which has a decreased dependence on calcium and/or an improved tolerance towards a detergent or a detergent component as compared to the parent lipolytic enzyme; and optionally
d) selecting a mutated lipolytic enzyme among those resulting from step (c) which, when present in detergent composition A or B, is capable of removing at least 15% more lard from a lard stained swatch, than the same detergent composition without the lipolytic enzyme, in a one cycle wash assay comprising subjecting 7 lard-stained cotton swatches (9 x 9 cm) per beaker to a one cycle wash in a thermostated TOM, each beaker containing 1000 ml of water comprising 3.2 mM $Ca^{2+}/Mg^{2+}$ (in a ratio of 5:1) and 5 g/l of said detergent composition, adjusted to pH 10, and comprising 12500 LU/l of the lipolytic enzyme, for 20 minutes at a temperature of 30°C, followed by rinsing for 15 minutes in running tap water and overnight linedrying at room temperature, subsequent extraction and quantification of fatty matter from the resulting swatches by Soxhlet extraction.

**25.** The method of any of claims 22-24 wherein the parent lipolytic enzymes encoded by the homologous DNA sequences i), ii) and iii) constitute different variants of the same naturally occurring lipolytic enzyme.

**26.** The method of any of claims 22-25 wherein the parent lipolytic enzyme is derived from a fungus or a bacterium.

**27.** The method of the preceding claim, wherein a parent lipolytic enzyme is derived from *Humicola lanuginosa* strain DSM 4109 and has the amino acid sequence disclosed in EP 0 305 216.

**28.** The method of any of claims 22-27 wherein the combination of DNA sequences is performed by sexual PCR or gene shuffling.

**29.** A lipolytic enzyme which is a variant of a parent lipolytic enzyme, wherein the parent lipolytic enzyme is derived from *Humicola lanuginosa* strain DSM 4109 and has the amino acid sequence disclosed in EP 0 305 216, and the variant has one of the following mutations:

a) SPIRPRP+D57G+N94K+D96L+Q249R;
b) SPPRRP+I90F+D96L+E99K+D137G+V187A;
c) SPIRPRP+N94K+D96L+L97M+Q249R;
d) SPPPRPRP+N94K+D96L+L97M+Q249R;
e) SPIRPRP+D57G+N94K+D96L+L97M+Q249R;
f) SPPRRP+I90F+D96L+E99K+V187A;
g) SPIRPRP+D137G+D167G+E21V+W221L;
h) E1SPIRPRP+I90F+D96L+E99K+V187A;
i) E1SRKRKRK+190F+D96L+E99K+V187A;
j) E1SPRIKPRIK+I90F+D96L+E99K+V187A;
k) E1SPPRRP+D62R+190F+D96L+E99K+V187A;
l) E1SPPRRP+I90F+D96L+E99K+V187A+N200R+R209A;
m) E1SPPRRP+190F+D96L+E99K+V187A+T199R+N200R+R209A;
n) E1SPIRPRP+D57G+D62R+N94K+D96L+Q249R;
o) E1SPIRPRP+D57G+N94K+D96L+N200R+R209A+Q249R;
p) E1SPIRPRP+D57G+N94K+D96L+T199R+N200R+Q249R;
q) E1SPPRRP+I90F+D96L+E99K+V187A+T199R;
r) E1SPIRPRP+D57G+N94K+D96L+T199R+R209A+Q249R;
s) E1SPIRPRP+I90F+D96L+E99K+V187A+Q249R;
t) E1SPPRRP+I90F+D96L+E99K+V187A+P253R;
u) E1SPPRRP+I90F+D96L+E99K+D137G+D167G+V187A+Q249R;
v) E1SPPRRP+I90F+D96L+E99K+D137G+V187A+Q249R;
w) E1SPPRRP+D96L+E99K+V187A+Q249R;
x) E1SPPRPR+V2P+N94K+D96L+Q249R;
y) E1SPPWWP+V2W+S3R+N94K+D96L+Q249R;
z) E1SPPWRP+V2R+S3R+N94K+D96L+Q249R;
aa) E1SPPRWP+V2R+S3R+N94K+D96L+Q249R;
bb) E1SPPWWP+V2R+S3W+N94K+D96L+Q249R;
cc) E1SPPRWP+V2W+S3R+N94K+D96L+Q249R;
dd) E1SPPRWP+V2R+S3W+N94K+D96L+Q249R;
ee) E1SPPRWP+N94K+D96L+Q249R;
ff) E1SPPRRP+N94K+D96L+Q249R;
gg) . E1APPPRPRPRPRP+V2G+S3T+D57G+N94K+D96L+L97M+Q249R;
hh) E1APPPRTRPRPRS+V2G+S3T+Q4P+D5E+D57G+N94K+D96L +L97M+Q249R;
ii) E1APPPKASPRQRP+V2G+D5Q+L6M+D57G+N94K+D96L+L97M +Q249R;
jj) SCIRR+N94K+D96L+E239C+Q249R;
kk) E1SPPRRP+D57G+N94K+D96L+Y53C+K127C+Q249R;
ll) E1SPPRRPR+V2R+S3P+N94K+D96L+Q249R;
mm) E1SPPWPRP+V2R+S3P+N94K+D96L+Q249R;
nn) E1SPPRRP+N94K+D96L+E99K;
oo) E1SPPRRP+N94K+D96L+E99K+Q249R;
pp) E1SPPCGRRP+N94K+D96L+E239C+Q249R;
qq) E1SPCRPRP+N94K+D96L+E239C+Q249R;

rr) SPPCRRRP+N94K+D96L+E239C+Q249R; or
ss) E1SPPRRP+D57G+N94K+D96L+Q249R.

**30.** A DNA construct comprising a DNA sequence encoding the lipolytic enzyme of claim 29.

**31.** A vector harboring the DNA construct of claim 30.

**32.** The vector of claim 31 which is a plasmid or a bacteriophage.

**33.** The vector of claim 31 or 32 which is an expression vector further comprising DNA sequences permitting expression of the lipolytic enzyme.

**34.** A host cell harboring the DNA construct of claim 30 or the vector of any of claims 31-33.

**35.** The cell of claim 34 which is a microbial cell.

**36.** The cell of claim 35 which is a cell of a fungal or a bacterial strain.

**37.** The cell of claim 36 which is a cell of the genus *Aspergillus, Fusarium,* or *Saccharomyces.*

**38.** The cell of claim 37 which is A. *niger, A. oryzae*, *A. nidulans*, *F. graminearum* or *S. cerevisiae*.

**39.** A detergent additive in the form of a non-dusting granulate, stabilized liquid or protected enzyme comprising the lipolytic enzyme of claim 29.

**40.** The detergent additive of claim 39 which contains 0.02-200 mg of enzyme protein/g of the additive.

**41.** The detergent additive of claim 39 or 40, which additionally comprises a protease, amylase, peroxidase, cutinase, lipolytic enzyme and/or cellulase.

**42.** A detergent composition comprising a surfactant and the lipolytic enzyme of claim 29.

**43.** The detergent composition of claim 42 which additionally comprises a protease, amylase, peroxidase, cutinase, lipolytic enzyme and/or cellulase.

**Patentansprüche**

**1.** Verfahren zum Zubereiten eines mutierten lipolytischen Enzyms, welches Verfahren mindestens die folgenden Schritte umfasst:

a) Unterwerfen einer DNA-Sequenz, welche ein lipolytisches Eltemenzymkodiert, einer Mutagenese, um eine Vielfalt mutierter DNA-Sequenzen zu bilden;
b) Exprimieren der mutierten DNA-Sequenzen in Wirtszellen;
c) Durchmustern nach Wirtszellen, welche ein mutiertes lipolytisches Enzym exprimieren, welches eine herabgesetzte Abhängigkeit von Kalzium und/oder eine verbesserte Toleranz gegenüber eines Detergens oder einer Detergenskomponente besitzt, verglichen mit dem lipolytischen Elternenzym; und
d) Auswählen eines mutierten lipolytischen Enzyms unter jenen, welche aus Schritt (c) resultieren, welches, wenn es in einer Detergenszusammensetzung vorhanden ist, in der Lage ist, mindestens 15% mehr Schmalz aus einem Schmalz-beschmutzten Textilmuster ("swatch") zu entfernen, als die gleiche Detergenszusammensetzung ohne das lipolytische Enzym, in einem Ein-Zyklus-Waschtest, umfassend das Unterwerfen von 7 Schmalz-beschmutzten Baumwolltextilmustern (9 x 9 cm) pro Becher einer Ein-Zyklus-Waschung in einem Temperatur-geregelten TOM, jeder Becher enthaltend 1.000 ml Wasser, umfassend 3,2 mM $Ca^{2+}/Mg^{2+}$ (in einem Verhältnis von 5 : 1) und 5 g/l der Detergenszusammensetzung, eingestellt auf pH 10, und umfassend 12.500 LU/l des lipolytischen Enzyms, für 20 Minuten bei einer Temperatur von 30 °C, gefolgt von einem Spülen für 15 Minuten in fließendem Leitungswasser und einem ausgebreiteten Trocknen ("linedrying") über Nacht bei Raumtemperatur, nachfolgende Extraktion und Quantifizierung des Schmalzbestandteils aus den resultierenden Textilmustern durch Soxhlet-Extraktion.

2. Verfahren nach Anspruch 1, in welchem die Mutagenese aus Schritt (a) eine Zufallsmutagenese ist.

3. Verfahren nach dem vorangehenden Anspruch, in welchem die Mutagenese eine lokalisierte Zufallsmutagenese ist, durchgeführt in der Lipid-Kontaktzone oder in einer beliebigen Peptid-Hinzufügung, welche an dem N-terminalen und/oder dem C-terminalen Ende des lipolytischen Eltemenzyms vorhanden ist.

4. Verfahren nach einem beliebigen der Ansprüche 1 - 3, in welchem die Schritte (a), (b), (c) und/oder (d) einmal oder mehrmals wiederholt werden.

5. Verfahren nach einem beliebigen der Ansprüche 1 - 4, in welchem das lipolytische Elternenzym von dem *Humicola lanuginosa* Stamm DSM 4109 abgeleitet wird und die in EP 0 305 216 offenbarte Aminosäuresequenz besitzt.

6. Verfahren nach einem beliebigen vorangehenden Anspruch, in welchem das mutierte lipolytische Enzym mindestens 3 Mutationen umfasst, verglichen mit dem lipolytischen Eltemenzym.

7. Verfahren nach einem beliebigen vorangehenden Anspruch, in welchem das mutierte lipolytische Enzym (verglichen mit dem lipolytischen Elternenzym) eine Substitution von mindestens einem neutralen Aminosäurerest durch einen positiv geladenen Aminosäurerest, eine Deletion von mindestens einem negativ geladenen Aminosäurerest, oder eine Substitution von mindestens einem negativ geladenen Aminosäurerest durch einen neutralen oder positiv geladenen Aminosäurerest, umfasst.

8. Verfahren nach einem beliebigen vorangehenden Anspruch, in welchem das mutierte lipolytische Enzym, verglichen mit dem lipolytischen Elternenzym, durch eine Deletion oder eine Substitution von mindestens einem Aminosäurerest in einer Lipid-Kontaktzone des lipolytischen Elternenzyms oder einer Hinzufügung von mindestens einem Aminosäurerest zu der Lipid-Kontaktzone modifiziert worden ist.

9. Verfahren nach einem beliebigen vorangehenden Anspruch, in welchem das mutierte lipolytische Enzym, verglichen mit dem lipolytischen Elternenzym, mindestens eine Mutation innerhalb und mindestens eine Mutation außerhalb der Lipid-Kontaktzone des lipolytischen Elternenzyms umfasst.

10. Verfahren nach einem beliebigen vorangehenden Anspruch, in welchem das mutierte lipolytische Enzym, verglichen mit dem lipolytischen Elternenzym, eine Peptidhinzufügung umfasst, welche an oder innerhalb des C- und/ oder N-terminalen Endes der reifen Form des lipolytischen Elternenzyms angebracht wird.

11. Verfahren nach einem beliebigen vorangehenden Anspruch, in welchem die Peptidhinzufügung eine kovalente Bindung mit dem reifen Teil des lipolytischen Elternenzyms bildet.

12. Verfahren nach einem beliebigen vorangehenden Anspruch, in welchem das mutierte lipolytische Enzym einen Cysteinrest in der Peptidhinzufügung, einen Cysteinrest in dem reifen Teil des lipolytischen Elternenzyms und eine Cysteinbrücke zwischen den beiden Cysteinresten umfasst.

13. Verfahren nach einem beliebigen Ansprüche 10 - 12, in welchem die Peptidhinzufügung mindestens einen Prolinrest umfasst.

14. Verfahren nach dem vorangehenden Anspruch, in welchem die Peptidhinzufügung zwei oder drei Prolinreste umfasst.

15. Verfahren nach einem beliebigen der Ansprüche 10 - 14, in welchem die Peptidhinzufügung mindestens einen positiven oder hydrophoben Aminosäurerest umfasst.

16. Verfahren nach dem vorangehenden Anspruch, in welchem die Peptidhinzufügung zwei oder drei hydrophobe Aminosäurereste umfasst.

17. Verfahren nach einem beliebigen der Ansprüche 10 - 16, in welchem die Peptidhinzufügung eine Länge zwischen 1 und 15 Aminosäuren besitzt.

18. Verfahren nach Anspruch 17, in welchem die Peptidhinzufügung eine Länge von 4 bis 10 Aminosäuren besitzt.

**19.** Verfahren nach einem beliebigen der Ansprüche 10 - 18, in welchem das mutierte lipolytische Enzym weiterhin eine Mutation in einem nicht-strukturellen Teil des N-Terminus und/oder C-Terminus des lipolytischen Elternenzyms umfasst.

**20.** Verfahren nach dem vorangehenden Anspruch, in welchem die Mutation in der Entfernung von mindestens einem negativ geladenen Aminosäurerest resultiert.

**21.** Verfahren nach einem Anspruch 19 oder 20, in welchem ein negativ geladener Aminosäurerest des nicht-strukturellen Teils deletiert oder ersetzt worden ist durch einen neutralen oder positiv geladenen Aminosäurerest oder durch einen hydrophoben Aminosäurerest, oder in welchem ein neutraler Aminosäurerest ersetzt worden ist durch einen positiv geladenen Aminosäurerest.

**22.** Verfahren zum Zubereiten eines mutierten lipolytischen Enzyms, welches Verfahren mindestens die folgenden Schritte umfasst:

a) Konstruieren von mutierten DNA-Sequenzen durch Kombinieren i) einer DNA-Sequenz, welche ein erstes lipolytisches Elternenzym kodiert, oder einen Teil der DNA-Sequenz und ii) einer DNA-Sequenz, welche ein zweites lipolytisches Elternenzym kodiert, oder einen Teil der DNA-Sequenz und wahlweise iii) weiterere DNA-Sequenzen, welche ein drittes (und wahlweise weitere) lipolytische Elternenzyme kodiert (kodieren), oder einen Teil der DNA-Sequenz(en), wobei die zu kombinierenden DNA-Sequenzen ausreichend homolog sind, um zu erlauben, dass eine homologe Rekombination zwischen den Sequenzen stattfindet,
b) Exprimieren der resultierenden mutieren DNA-Sequenzen in Wirtszellen, und
e) Selektieren eines mutierten lipolytischen Enzyms unter jenen, welche aus Schritt (b) resultieren, welches, wenn es in einer Detergenszusammensetzung A und/oder B vorhanden ist, in der Lage ist, mindestens 15% mehr Schmalz aus einem Schmalz-beschmutzten Textilmuster zu entfernen, als die gleiche Detergenszusammensetzung ohne das lipolytische Enzym, in einem Ein-Zyklus-Waschungstest, umfassend ein Unterwerfen von 7 Schmalz-beschmutzten Baumwolltextilmustern (9 x 9 cm) pro Becher einer Ein-Zyklus-Waschung in einer Temperatur-geregelten TOM, jeder Becher enthaltend 1.000 ml Wasser, umfassend 3,2 mM $Ca^{2+}/Mg^{2+}$ (in einem Verhältnis von 5 : 1) und 5 g/l der Detergenszusammensetzung, eingestellt auf pH 10, und umfassend 12.500 LU/l des lipolytischen Enzyms, für 20 Minuten bei einer Temperatur von 30 °C, gefolgt von einem Spülen für 15 Minuten in fließendem Leitungswasser und einem ausgebreiteten Trocknen über Nacht bei Raumtemperatur, nachfolgende Extraktion und Quantifizierung des Schmalzbestandteils aus den resultierenden Textilmustem durch Soxhlet-Extraktion.

**23.** Verfahren nach Anspruch 22, in welchem vor Schritt (c) ein Durchmustern nach Wirtszellen durchgeführt wird, welche ein mutiertes lipolytisches Enzym exprimieren, welches eine herabgesetzte Abhängigkeit von Kalzium und/oder eine verbesserte Toleranz gegenüber eines Detergens oder einer Detergenszusammensetzung besitzt, verglichen mit einem beliebigen der lipolytischen Elternenzyme.

**24.** Verfahren nach Anspruch 22 oder 23, in welchem eine oder mehrere der homologen DNA-Sequenzen i) - iii), welche zum Konstruieren der mutierten DNA-Sequenz aus Schritt (a) verwendet werden, zubereitet worden sind durch

a) Unterwerfen einer DNA-Sequenz, welche ein lipolytisches Eltemenzym kodiert, einer Mutagenese, um eine Vielfalt von mutierten DNA-Sequenzen zu bilden;
b) Exprimieren der mutierten DNA-Sequenzen in Wirtszellen;
c) Durchmustern nach Wirtszellen, welche ein mutiertes lipolytisches Enzym exprimieren, welches eine herabgesetzte Abhängigkeit von Kalzium und/oder eine verbesserte Toleranz gegenüber eines Detergens oder einer Detergenszusammensetzung besitzt, verglichen mit dem lipolytischen Elternenzym; und wahlweise
d) Auswählen eines mutierten lipolytischen Enzyms unter jenen, welche aus Schritt (c) resultieren, welches, wenn es in einer Detergenszusammensetzung vorhanden ist, in der Lage ist, mindestens 15% mehr Schmalz aus einem Schmalz-beschmutzten Textilmuster ("swatch") zu entfernen, als die gleiche Detergenszusammensetzung ohne das lipolytische Enzym, in einem Ein-Zyklus-Waschungstest, umfassend ein Unterwerfen von 7 Schmalz-beschmutzten Baumwolltextilmustern (9 x 9 cm) pro Becher einer Ein-Zyklus-Waschung in einem Temperatur-geregelten TOM, jeder Becher enthaltend 1.000 ml Wasser, umfassend 3,2 mM $Ca^{2+}$ /$Mg^{2+}$ (in einem Verhältnis von 5 : 1) und 5 g/l der Detergenszusammensetzung, eingestellt auf pH 10, und umfassend 12.500 LU/l des lipolytischen Enzyms, für 20 Minuten bei einer Temperatur von 30 °C, gefolgt von einem Spülen für 15 Minuten in fließendem Leitungswasser und einem ausgebreiteten Trocknen über Nacht bei

Raumtemperatur, nachfolgende Extraktion und Quantifizierung des Schmalzbestandteils der resultierenden Textilmustern durch Soxhlet-Extraktion.

**25.** Verfahren nach einem beliebigen der Ansprüche 22 - 24, in welchem die lipolytischen Elternenzyme, welche durch die homologen DNA-Sequenzen i), ii) und iii) kodiert werden, verschiedene Varianten desselben natürlich vorkommenden lipolytischen Enzyms darstellen.

**26.** Verfahren nach einem beliebigen der Ansprüche 22 - 25, in welchem das lipolytische Elternenzym von einem Pilz oder einem Bakterium abgeleitet wird.

**27.** Verfahren nach' dem vorangehenden Anspruch, in welchem ein lipolytisches Elternenzym von dem *Humicola lanuginosa* Stamm DSM 4109 abgeleitet wird, und die in EP 0 305 216 offenbarte Aminosäuresequenz besitzt.

**28.** Verfahren nach einem beliebigen der Ansprüche 22 - 27, in welchem die Kombination der DNA-Sequenzen durch Sexual-PCR oder Gen-Schieben ("gene shuffling") durchgeführt wird.

**29.** Lipolytisches Enzym, welches eine Variante von einem lipolytischen Elternenzym ist, wobei das lipolytische Elternenzym von dem *Humicola lanuginosa* Stamm DSM 4109 abgeleitet ist und die in EP 0 305 216 offenbarte Aminosäuresequenz besitzt, und die Variante eine der folgenden Mutationen besitzt:

a) SPIRPRP+D57G+N94K+D96L+Q249R;
b) SPPRRP+I90F+D96L+E99K+D137G+V187A;
c) SPIRPRP+N94K+D96L+L97M+Q249R;
d) SPPPRPRP+N94K+D96L+L97M+Q249R;
e) SPIRPRP+D57G+N94K+D96L+L97M+Q249R;
f) SPPRRP+190F+D96L+E99K+V187A;
g) SPIRPRP+D137G+D167G+E21V+W221L;
h) E1SPIRPRP+I90F+D96L+E99K+V187A;
i) E1SRKRKRK+I90F+D96L+E99K+V187A;
j) E1SPRIKPRIK+I90F+D96L+E99K+V187A;
k) E1SPPRRP+D62R+I90F+D96L+E99K+V187A;
l) E1SPPRRP+I90F+D96L+E99K+V187A+N200R+R209A;
m) E1SPPRRP+I90F+D96L+E99K+V187A+T199R+N200R+R209A;
n) E1SPIRPRP+D57G+D62R+N94K+D96L+Q249R;
o) E1SPIRPRP+D57G+N94K+D96L+N200R+R209A+Q249R;
p) E1SPIRPRP+D57G+N94K+D96L+T199R+N200R+Q249R;
q) E1SPPRRP+I90F+D96L+E99K+V187A+T199R;
r) E1SPIRPRP+D57G+N94K+D96L+T199R+R209A+Q249R;
s) E1SPIRPRP+I90F+D96L+E99K+V187A+Q249R;
t) E1SPPRRP+I90F+D96L+E99K+V187A+P253R;
u) E1SPPRRP+I90F+D96L+E99K+D137G+D167G+V187A+Q249R;
v) E1SPPRRP+I90F+D96L+E99K+D137G+V187A+Q249R;
w) E1SPPRRP+D96L+E99K+V187A+Q249R;
x) E1SPPRPR+V2P+N94K+D96L+Q249R;
y) E1SPPWWP+V2W+S3R+N94K+D98L+Q249R;
z) E1SPPWRP+V2R+S3R+N94K+D96L+Q249R;
aa) E1SPPRWP+V2R+S3R+N94K+D96L+Q249R;
bb) E1SPPWWP+V2R+S3W+N94K+D96L+Q249R;
cc) E1SPPRWP+V2W+S3R+N94K+D96L+Q249R;
dd) E1SPPRWP+V2R+S3W+N94K+D96L+Q249R;
ee) E1SPPRWP+N94K+D96L+Q249R;
ff) E1SPPRRP+N94K+D96L+Q249R;
gg) E1APPPRPRPRPRP+V2G+S3T+D57G+N94K+D86L+L97M+Q249R;
hh) E1APPPRTRPRPRS+V2G+S3T+Q4P+D5E+D57G+N94K+D96L +L97M+Q249R;
ii) E1APPPKASPRQRP+V2G+D5Q+L6M+D57G+N94K+D96L+L97M +Q249R;
jj) SCIRR+N94K+D96L+E239C+Q249R;
kk) E1SPPRRP+D57G+N94K+D96L+Y53C+K127C+Q249R;
ll) E1SPPRRPR+V2R+S3P+N94K+D96L+Q249R;

mm) E1SPPWPRP+V2R+S3P+N94K+D96L+Q249R;
nn) E1SPPRRP+N94K+D96L+E99K;
oo) E1SPPRRP+N94K+D96L+E99K+Q249R;
pp) E1SPPCGRRP+N94K+D96L+E239C+Q249R;
qq) E1SPCRPRP+N94K+D96L+E239C+Q249R;
rr) SPPCRRRP+N94K+D96L+E239C+Q249R; or
ss) E1SPPRRP+D57G+N94K+D96L+Q249R.

**30.** DNA-Konstrukt, umfassend eine DNA-Sequenz, welche das lipolytische Enzym nach Anspruch 29 kodiert.

**31.** Vektor, das DNA-Konstrukt nach Anspruch 30 beherbergend.

**32.** Vektor nach Anspruch 31, welcher ein Plasmid oder ein Bakteriophage ist.

**33.** Vektor nach Anspruch 31 oder 32, welcher ein Expressionsvektor ist, welcher weiterhin DNA-Sequenzen umfasst, welche die Expression des lipolytischen Enzyms erlauben.

**34.** Wirtszelle, das DNA-Konstrukt nach Anspruch 30 oder den Vektor nach einem beliebigen der Ansprüche 31 bis 33 beherbergend.

**35.** Zelle nach Anspruch 34, welche eine mikrobielle Zelle ist.

**36.** Zelle nach Anspruch 35, welche eine Zelle von einem Pilz- oder Bakterienstamm ist.

**37.** Zelle nach Anspruch 36, welche eine Zelle der Gattung *Aspergillus, Fusarium* oder *Saccharomyces* ist.

**38.** Zelle nach Anspruch 37, welche *A. niger, A. oryzae, A. nidulans, F. graminearum* oder *S. cerevisiae* ist.

**39.** Detergenszusatz in der Form eines nicht-staubenden Granulats, einer stabilisierten Flüssigkeit oder eines geschützten Enzyms, umfassend das lipolytische Enzym nach Anspruch 29.

**40.** Detergenszusatz nach Anspruch 39, welcher 0,02 - 200 mg Enzymprotein/g des Zusatzes enthält.

**41.** Detergenszusatz nach Anspruch 39 oder 40, welcher zusätzlich eine Protease, Amylase, Peroxidase, Cutinase, ein lipolytisches Enzym und/oder eine Cellulase umfasst.

**42.** Detergenszusammensetzung, umfassend einen oberflächenaktiven Stoff und das lipolytische Enzym nach Anspruch 29.

**43.** Detergenszusammensetzung nach Anspruch 42, welche zusätzlich eine Protease, Amylase, Peroxidase, Cutinase, ein lipolytisches Enzym und/oder eine Cellulase umfasst.

## Revendications

**1.** Procédé de préparation d'une enzyme lipolytique mutée, lequel procédé comprend au moins les étapes suivantes :

a) l'exposition d'une séquence d'ADN codant une enzyme lipolytique parentale à une mutagenèse pour former une variété de séquences d'ADN mutées ;
b) l'expression des séquences d'ADN mutées dans des cellules hôtes ;
c) le criblage pour des cellules hôtes exprimant une enzyme lipolytique mutée qui a une dépendance diminuée à l'égard du calcium et/ou une tolérance améliorée à l'égard d'un détergent ou d'un composant de détergent par rapport à l'enzyme lipolytique parentale ; et
d) la sélection d'une enzyme lipolytique mutée parmi celles résultant de l'étape (c) qui, quand elle est présente dans une composition détergente, est capable de retirer au moins 15 % de saindoux d'un échantillon souillé de saindoux de plus que la même composition détergente sans l'enzyme lipolytique, dans un essai de lavage à un cycle comprenant l'exposition de 7 échantillons de coton (9 x 9 cm) souillés de saindoux par becher à un lavage à un cycle dans un TOM thermostaté, chaque becher contenant 1000 ml d'eau comprenant 3,2 mM

de $Ca^{2+}/Mg^{2+}$ (dans un rapport de 5:1) et 5 g/l de ladite composition détergente, ajustée à pH 10, et comprenant 12500 UL/l d'enzyme lipolytique, pendant 20 minutes à une température de 30°C, puis le rinçage pendant 15 minutes dans de l'eau du robinet courante et le séchage sur corde pendant une nuit à la température ambiante, puis l'extraction et la quantification des matières grasses provenant des échantillons résultants par extraction au Soxhlet.

**2.** Procédé selon la revendication 1 où la mutegenèse de l'étape (a) est une mutagenèse aléatoire.

**3.** Procédé selon la revendication précédente où la mutagenèse est une mutagenèse aléatoire localisée réalisée dans une zone de contact des lipides ou dans une addition de peptide quelconque présente à l'extrémité N-terminale et/ou C-terminale de l'enzyme lipolytique parentale.

**4.** Procédé selon l'une quelconque des revendications 1-3 où les étapes (a), (b), (c) et/ou (d) sont répétées une ou plusieurs fois.

**5.** Procédé selon l'une quelconque des revendications 1-4 où l'enzyme lipolytique parentale est dérivée de *Humicola lanuginosa* souche DSM 4109 et a la séquence d'acides aminés décrite dans EP 0 305 216.

**6.** Procédé selon l'une quelconque des revendications précédentes où l'enzyme lipolytique mutée comprend au moins 3 mutations par rapport à l'enzyme lipolytique parentale.

**7.** Procédé selon l'une quelconque des revendications précédentes où l'enzyme lipolytique mutée (par rapport à l'enzyme lipolytique parentale) comprend une substitution d'au moins un résidu d'acide aminé neutre par un résidu d'acide aminé chargé positivement, une délétion d'au moins un résidu d'acide aminé chargé négativement, ou une substitution d'au moins un résidu d'acide aminé chargé négativement par un résidu d'acide aminé neutre ou chargé positivement.

**8.** Procédé selon l'une quelconque des revendications précédentes où l'enzyme lipolytique mutée, par rapport à l'enzyme lipolytique parentale, a été modifiée par délétion ou substitution d'au moins un résidu d'acide aminé dans la zone de contact des lipides de l'enzyme lipolytique parentale ou addition d'au moins un résidu d'acide aminé à ladite zone de contact des lipides.

**9.** Procédé selon l'une quelconque des revendications précédentes où l'enzyme lipolytique mutée, par rapport à l'enzyme lipolytique parentale, comprend au moins une mutation dans et au moins une mutation en dehors de la zone de contact des lipides de l'enzyme lipolytique parentale.

**10.** Procédé selon l'une quelconque des revendications précédentes où l'enzyme lipolytique mutée, par rapport à l'enzyme lipolytique parentale, comprend une addition de peptide appliquée à ou dans l'extrémité C- et/ou N-terminale de la forme mature de l'enzyme lipolytique parentale.

**11.** Procédé selon la revendication précédente où l'addition de peptide forme une liaison covalente avec la partie mature de l'enzyme lipolytique parentale.

**12.** Procédé selon l'une quelconque des revendications précédentes où l'enzyme lipolytique mutée comprend un résidu cystéine dans l'addition de peptide, un résidu cystéine dans la partie mature de l'enzyme lipolytique parentale et un pont cystine entre les deux résidus cystéine.

**13.** Procédé selon l'une quelconque des revendications 10-12 où l'addition de peptide comprend au moins un résidu de proline.

**14.** Procédé selon la revendication précédente où l'addition de peptide comprend deux ou trois résidus de proline.

**15.** Procédé selon l'une quelconque des revendications 10-14 où l'addition de peptide comprend au moins un résidu d'acide aminé positif ou hydrophobe.

**16.** Procédé selon la revendication précédente où l'addition de peptide comprend deux ou trois résidus d'acides aminés hydrophobes.

**17.** Procédé selon l'une quelconque des revendications 10-16 où l'addition de peptide a une longueur de 1 à 15 acides aminés.

**18.** Procédé selon la revendication 17 où l'addition de peptide a une longueur de 4 à 10 acides aminés.

**19.** Procédé selon l'une quelconque des revendications 10-18 où l'enzyme lipolytique mutée comprend en outre une mutation dans une partie non structurale de l'extrémité N-terminale et/ou C-terminale de l'enzyme lipolytique parentale.

**20.** Procédé selon la revendication précédente où la mutation entraîne le retrait d'au moins un résidu d'acide aminé chargé négativement.

**21.** Procédé selon la revendication 19 ou 20 où un résidu d'acide aminé chargé négativement de ladite partie non structurale a été délété ou remplacé par un résidu d'acide aminé neutre ou chargé positivement ou par un résidu d'acide aminé hydrophobe, ou bien où un résidu d'acide aminé neutre a été remplacé par un résidu d'acide aminé chargé positivement.

**22.** Procédé de préparation d'une enzyme lipolytique mutée, lequel procédé comprend au moins les étapes suivantes :

a) la construction de séquences d'ADN mutées par combinaison i) d'une séquence d'ADN codant une première enzyme lipolytique parentale ou d'une partie de ladite séquence d'ADN et ii) d'une séquence d'ADN codant une seconde enzyme lipolytique parentale ou d'une partie de ladite séquence d'ADN et éventuellement iii) d'autres séquences d'ADN codant une troisième enzyme lipolytique parentale (et éventuellement d'autres) ou d'une partie de ladite ou desdites séquences d'ADN, les séquences d'ADN à combiner étant suffisamment homologues pour permettre à une recombinaison homologue d'avoir lieu entre les séquences ;
b) l'expression des séquences d'ADN mutées résultantes dans des cellules hôtes, et
c) la sélection d'une enzyme lipolytique mutée parmi celles résultant de l'étape (b) qui, quand elle est présente dans une composition détergente A et/ou B, est capable de retirer au moins 15 % de saindoux d'un échantillon souillé de saindoux de plus que la même composition détergente sans l'enzyme lipolytique, dans un essai de lavage à un cycle comprenant l'exposition de 7 échantillons de coton (9 x 9 cm) souillés de saindoux par becher à un lavage à un cycle dans un TOM thermostaté, chaque becher contenant 1000 ml d'eau comprenant 3,2 mM de $Ca^{2+}/Mg^{2+}$ (dans un rapport de 5:1) et 5 g/l de ladite composition détergente, ajustée à pH 10, et comprenant 12500 UL/l d'enzyme lipolytique, pendant 20 minutes à une température de 30°C, puis le rinçage pendant 15 minutes dans de l'eau du robinet courante et le séchage sur corde pendant une nuit à la température ambiante, puis l'extraction et la quantification des matières grasses provenant des échantillons résultants par extraction au Soxhlet.

**23.** Procédé selon la revendication 22 où, avant l'étape (c), un criblage est réalisé pour des cellules hôtes exprimant une enzyme lipolytique mutée qui a une dépendance diminuée à l'égard du calcium et/ou une tolérance améliorée à l'égard d'un détergent ou d'un composant de détergent par rapport à l'une quelconque des enzymes lipolytiques parentales.

**24.** Procédé selon la revendication 22 ou 23 où une ou plusieurs des séquences d'ADN homologues i)-iii) utilisées pour construire la séquence d'ADN mutée de l'étape (a) ont été préparées par

a) exposition d'une séquence d'ADN codant une enzyme lipolytique parentale à une mutagenèse pour former une variété de séquences d'ADN mutées ;
b) expression des séquences d'ADN mutées dans des cellules hôtes ;
c) criblage pour des cellules hôtes exprimant une enzyme lipolytique mutée qui a une dépendance diminuée à l'égard du calcium et/ou une tolérance améliorée à l'égard d'un détergent ou d'un composant de détergent par rapport à l'enzyme lipolytique parentale ; et éventuellement
d) sélection d'une enzyme lipolytique mutée parmi celles résultant de l'étape (c) qui, quand elle est présente dans la composition détergente A ou B, est capable de retirer au moins 15 % de saindoux d'un échantillon souillé de saindoux de plus que la même composition détergente sans l'enzyme lipolytique, dans un essai de lavage à un cycle comprenant l'exposition de 7 échantillons de coton (9 x 9 cm) souillés de saindoux par becher à un lavage à un cycle dans un TOM thermostaté, chaque becher contenant 1000 ml d'eau comprenant 3,2 mM de $Ca^{2+}/Mg^{2+}$ (dans un rapport de 5:1) et 5 g/l de ladite composition détergente, ajustée à pH 10, et comprenant 12500 UL/l d'enzyme lipolytique, pendant 20 minutes à une température de 30°C, puis le rinçage

pendant 15 minutes dans de l'eau du robinet courante et le séchage sur corde pendant une nuit à la température ambiante, puis l'extraction et la quantification des matières grasses provenant des échantillons résultants par extraction au Soxhlet.

**25.** Procédé selon l'une quelconque des revendications 22-24 où les enzymes lipolytiques parentales codées par les séquences d'ADN homologues i), ii) et iii) constituent différentes variantes de la même enzyme lipolytique naturelle.

**26.** Procédé selon l'une quelconque des revendications 22-25 où l'enzyme lipolytique parentale est dérivée d'un champignon ou d'une bactérie.

**27.** Procédé selon la revendication précédente où une enzyme lipolytique parentale est dérivée de *Humicola lanuginosa* souche DSM 4109 et a la séquence d'acides aminés décrite dans EP 0 305 216.

**28.** Procédé selon l'une quelconque des revendications 22-27 où la combinaison de séquences d'ADN est réalisée par PCR sexuelle ou redistribution de gènes.

**29.** Enzyme lipolytique qui est une variante d'une enzyme lipolytique parentale, où l'enzyme lipolytique parentale est dérivée de *Humicola lanuginosa* souche DSM 4109 et a la séquence d'acides aminés décrite dans EP 0 305 216, et la variante a l'une des mutations suivantes :

a) SPIRPRP+D57G+N94K+D96L+Q249R;
b) SPPRRP+I90F+D96L+E99K+D137G+V187A;
c) SPIRPRP+N94K+D96L+L97M+Q249R;
d) SPPPRPRP+N94K+D96L+L97M+Q249R;
e) SPIRPRP+D57G+N94K+D96L+L97M+Q249R;
f) SPPRRP+I90F+D96L+E99K+V187A;
g) SPIRPRP+D137G+D167G+E21V+W221L;
h) E1SPIRPRP+I90F+D96L+E99K+V187A;
i) E1SRKRKRK+I90F+D96L+E99K+V187A;
j) E1SPRIKPRIK+I90F+D96L+E99K+V187A;
k) E1SPPRRP+D62R+I90F+D96L+E99K+V187A;
l) E1SPPRRP+I90F+D96L+E99K+V187A+N200R+R209A;
m) E1SPPRRP+I90F+D96L+E99K+V187A+T199R+N200R+R209A;
n) E1SPIRPRP+D57G+D62R+N94K+D96L+Q249R;
o) E1SPIRPRP+D57G+N94K+D96L+N200R+R209A+Q249R;
p) E1SPIRPRP+D57G+N94K+D96L+T199R+N200R+Q249R;
q) E1SPPRRP+I90F+D96L+E99K+V187A+T199R;
r) E1SPIRPRP+D57G+N94K+D96L+T199R+R209A+Q249R;
s) E1SPIRPRP+I90F+D96L+E99K+V187A+Q249R;
t) E1SPPRRP+T90F+D96L+E99K+V187A+P253R;
u) E1SPPRRP+I90F+D96L+E99K+D137G+D167G+V187A+Q249R;
v) E1SPPRRP+I90F+D96L+E99K+D137G+V187A+Q249R;
w) E1SPPRRP+D96L+E99K+V187A+Q249R;
x) E1SPPRPR+V2P+N94K+D96L+Q249R;
y) E1SPPWWP+V2W+S3R+N94K+D96L+Q249R;
z) E1SPPWRP+V2R+S3R+N94K+D96L+Q249R;
aa) E1SPPRWP+V2R+S3R+N94K+D96L+Q249R;
bb) E1SPPWWP+V2R+S3W+N94K+D96L+Q249R;
cc) E1SPPRWP+V2W+S3R+N94K+D96L+Q249R;
dd) E1SPPRWP+V2R+S3W+N94K+D96L+Q249R;
ee) E1SPPRWP+N94K+D96L+Q249R;
ff) E1SPPRRP+N94K+D96L+Q249R;
gg) E1APPPRPRPRPRP+V2G+S3T+D57G+N94K+D96L+L97M+Q249R;
hh) E1APPPRTRPRPRS+V2G+S3T+Q4P+D5E+D57G+N94K+D96L +L97M+Q249R;
ii) E1APPPKASPRQRP+V2G+D5Q+L6M+D57G+N94K+D96L+L97M +Q249R;
jj) SCIRR+N94K+D96L+E239C+Q249R;
kk) E1SPPRRP+D57G+N94K+D96L+Y53C+K127C+Q249R;
ll) E1SPPRRPR+V2R+S3P+N94K+D96L+Q249R;

mm) E1SPPWPRP+V2R+S3P+N94K+D96L+Q249R;
nn) E1SPPRRP+N94K+D96L+E99K;
oo) E1SPPRRP+N94K+D96L+E99K+Q249R;
pp) E1SPPCGRRP+N94K+D96L+E239C+Q249R;
qq) E1SPCRPRP+N94K+D96L+E239C+Q249R;
rr) SPPCRRRP+N94K+D96L+E239C+Q249R;ou
SS) E1SPPRRP+D57G+N94K+D96L+Q249R.

**30.** Construction d'ADN comprenant une séquence d'ADN codant l'enzyme lipolytique selon la revendication 29.

**31.** Vecteur contenant la construction d'ADN selon la revendication 30.

**32.** Vecteur selon la revendication 31 qui est un plasmide ou un bactériophage.

**33.** Vecteur selon la revendication 31 ou 32 qui est un vecteur d'expression comprenant en outre des séquences d'ADN permettant l'expression de l'enzyme lipolytique.

**34.** Cellule hôte contenant la construction d'ADN selon la revendication 30 ou le vecteur selon l'une quelconque des revendications 31-33.

**35.** Cellule selon la revendication 34 qui est une cellule microbienne.

**36.** Cellule selon la revendication 35 qui est une cellule d'une souche fongique ou d'une souche bactérienne.

**37.** Cellule selon la revendication 36 qui est une cellule du genre *Aspergillus, Fusarium* ou *Saccharomyces*.

**38.** Cellule selon la revendication 37 qui est *A. niger, A. oryzae*, *A. nidulans*, *F. graminearum* ou *S. cerevisiae*.

**39.** Additif de détergent sous forme de granulés sans poussière, d'un liquide stabilisé ou d'une enzyme protégée comprenant l'enzyme lipolytique selon la revendication 29.

**40.** Additif de détergent selon la revendication 39 qui contient 0,02-200 mg de protéine enzyme/g d'additif.

**41.** Additif de détergent selon la revendication 39 ou 40 qui comprend en outre une protéase, une amylase, une peroxydase, une cutinase, une enzyme lipolytique et/ou une cellulase.

**42.** Composition détergente comprenant un tensioactif et l'enzyme lipolytique selon la revendication 29.

**43.** Composition détergente selon la revendication 42 qui comprend en outre une protéase, une amylase, une peroxydase, une cutinase, une enzyme lipolytique et/ou une cellulase.

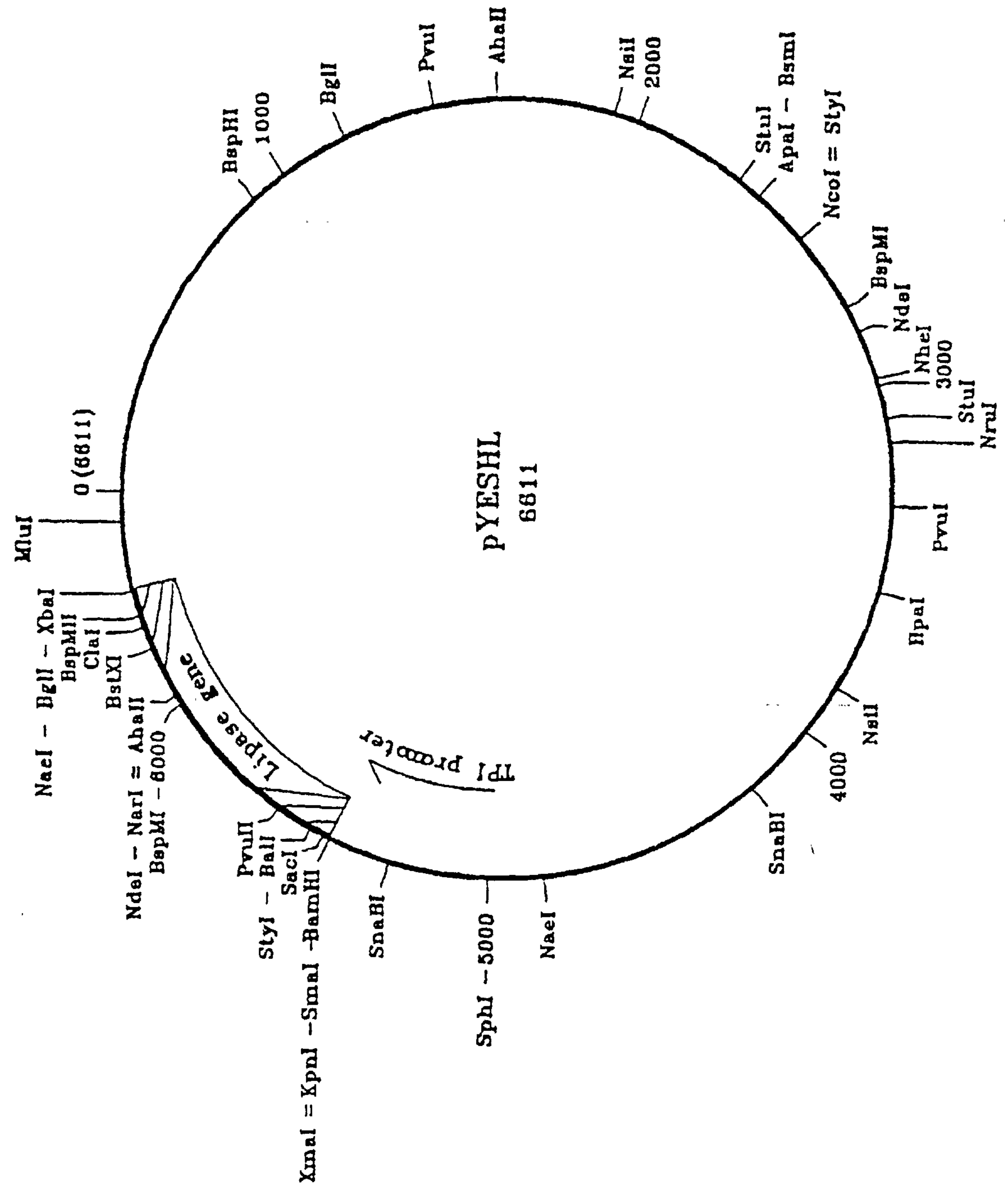
pYESHL
6611
Lipase gene
TPI promoter
0 (6611)
MluI
BspHI
1000
BglI
PvuI
AhaII
NsiI
2000
StuI
ApaI - BsmI
NcoI = StyI
BspMI
NdeI
NheI
3000
StuI
NruI
PvuI
HpaI
NsiI
4000
SnaBI
NaeI
SphI - 5000
SnaBI
XmaI = KpnI - SmaI - BamHI
StyI - BalI
SacI
PvuII
NdeI - NarI = AhaII
BspMI - 6000
NaeI - BglI - XbaI
BspMII
ClaI
BstXI

Fig. 1

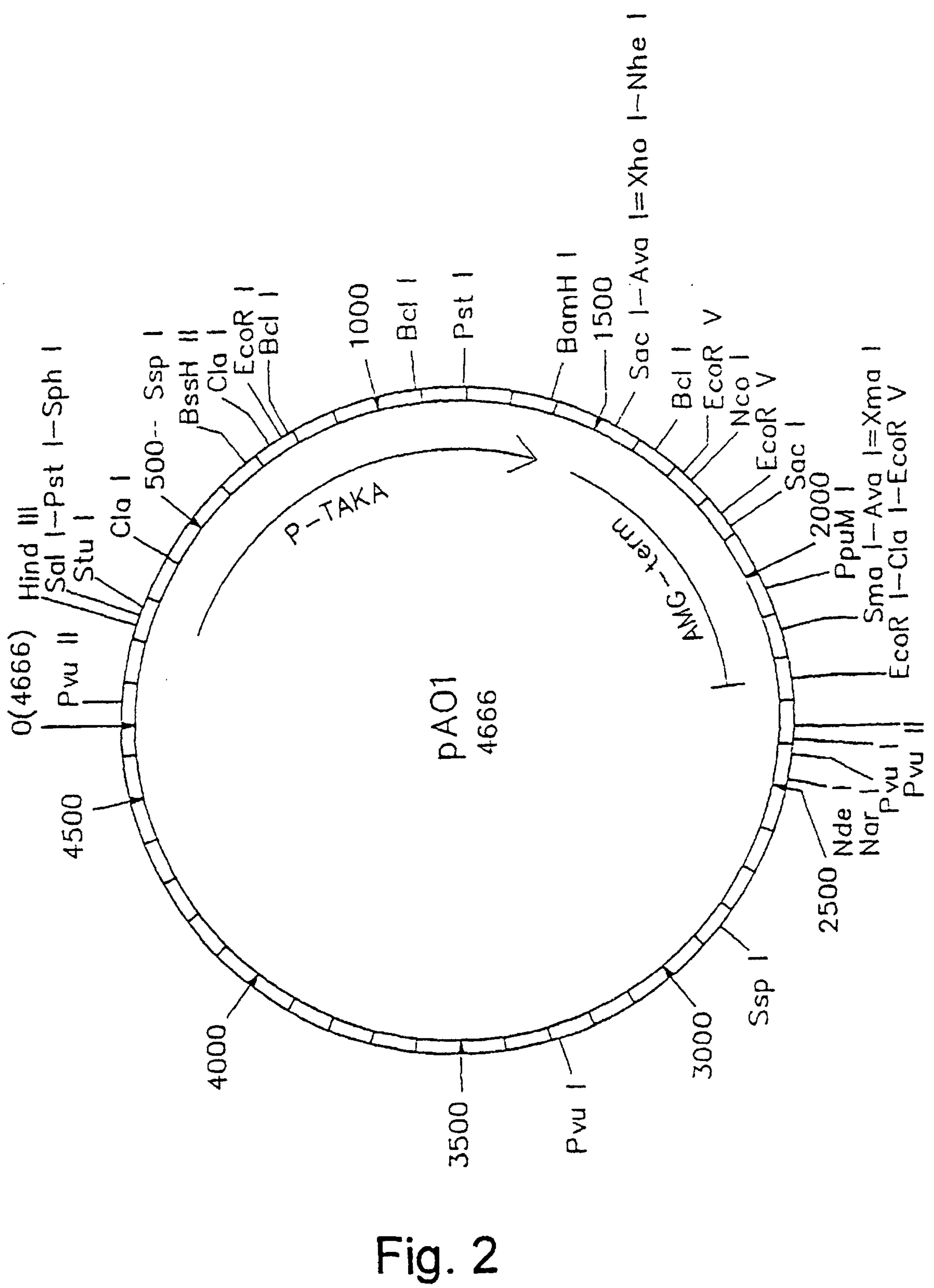
pAO1
4666
P-TAKA
AMG-term
0(4666)
Pvu II
Hind III
Sal I-Pst I-Sph I
Stu I
Cla I
500-- Ssp I
BssH II
Cla I
EcoR I
Bcl I
1000
Bcl I
Pst I
BamH I
1500
Sac I-Ava I=Xho I-Nhe I
Bcl I
EcoR V
Nco I
EcoR V
Sac I
2000
PpuM I
Sma I-Ava I=Xma I
EcoR I-Cla I-EcoR V
Pvu II
Pvu I
Nar I
Nde I
2500
Ssp I
3000
Pvu I
3500
4000
4500

Fig. 2

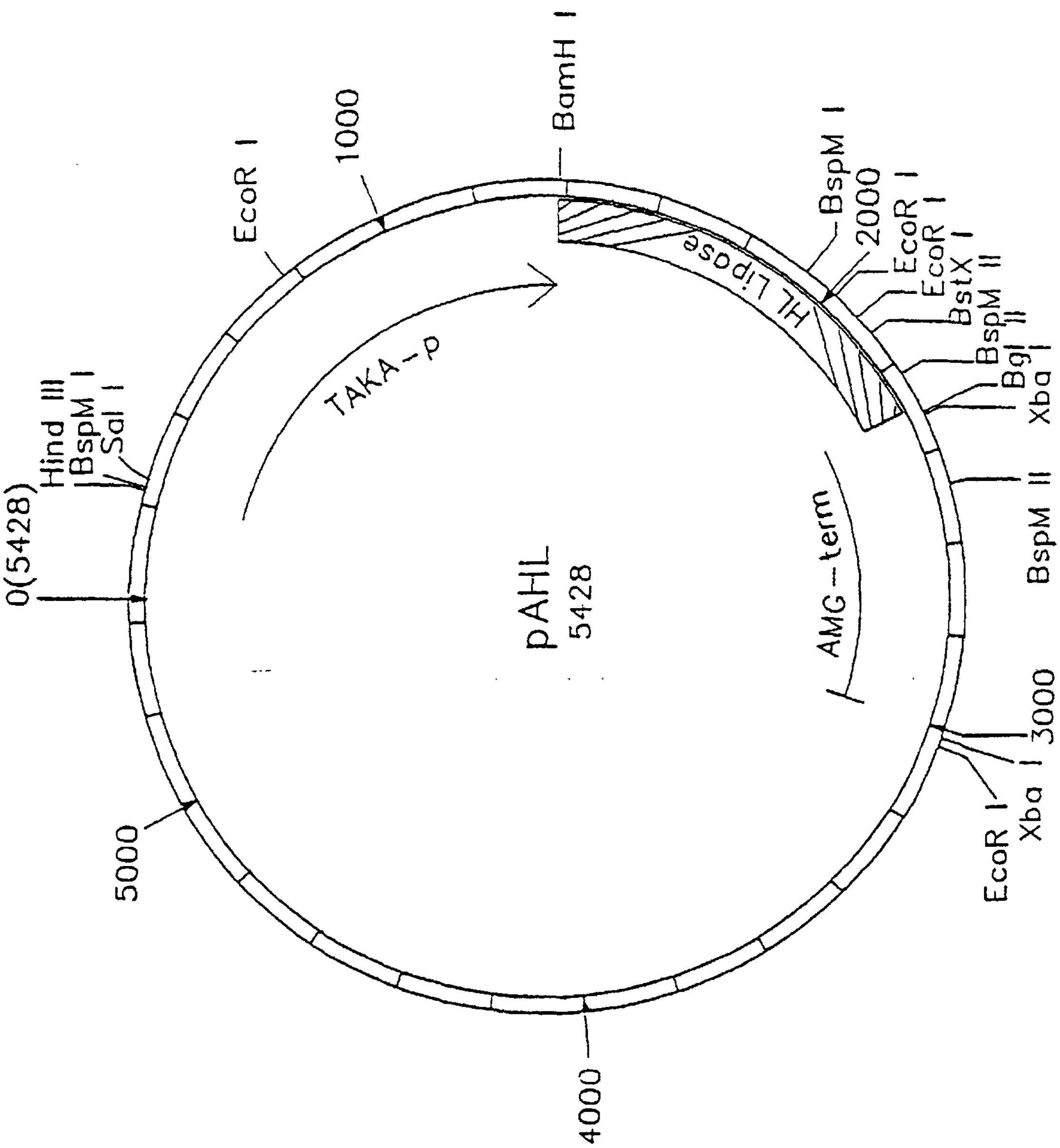
pAHL
5428
TAKA-p
HL Lipase
AMG-term
BamH I
BspM I
2000
EcoR I
EcoR I
BstX I
BspM II
Bgl II
Xba I
BspM II
3000
Xba I
EcoR I
4000
5000
0(5428)
Hind III
BspM I
Sal I
EcoR I
1000

Fig. 3

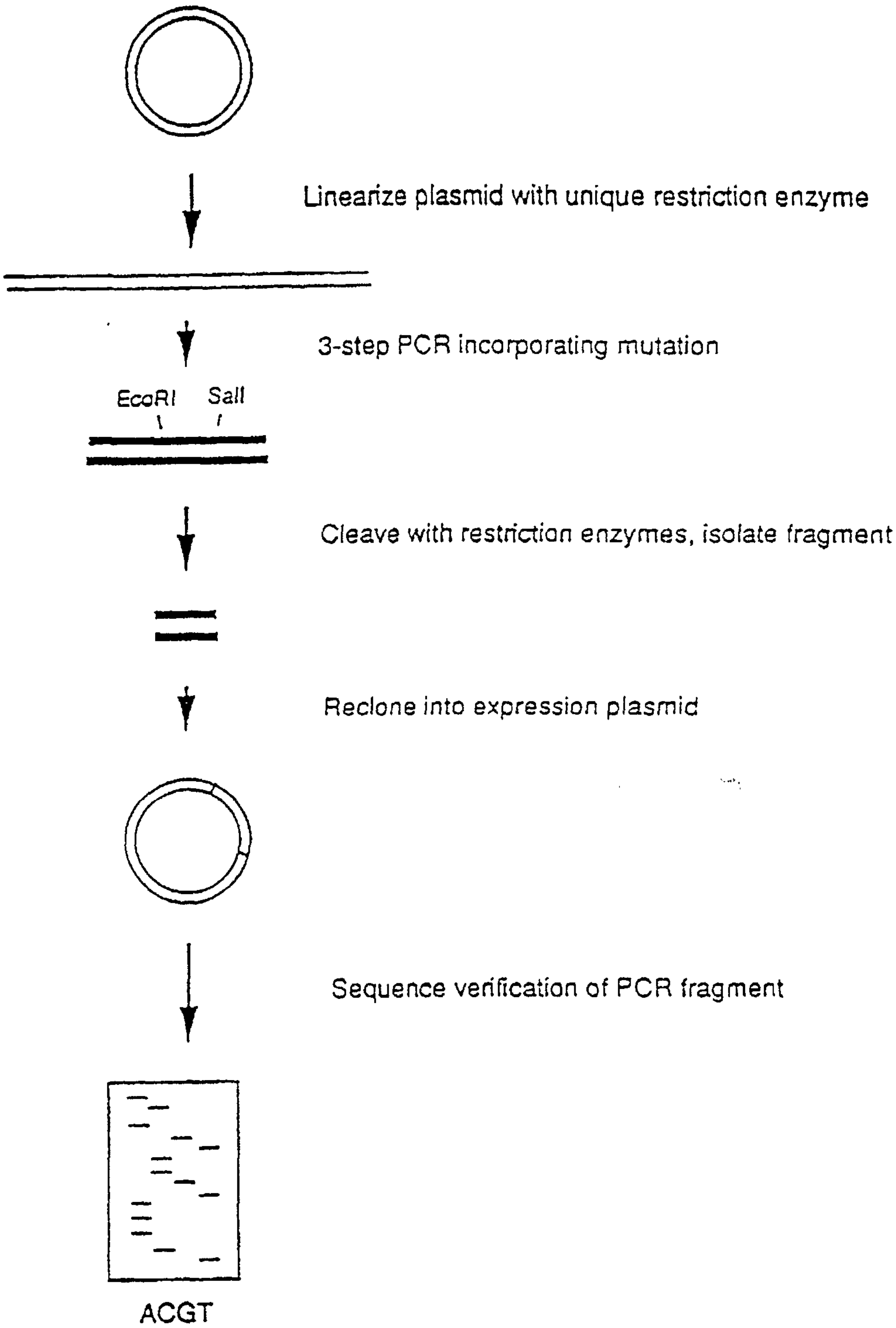
Linearize plasmid with unique restriction enzyme
3-step PCR incorporating mutation
EcoRI
SalI
Cleave with restriction enzymes, isolate fragment
Reclone into expression plasmid
Sequence verification of PCR fragment
ACGT

Fig. 4

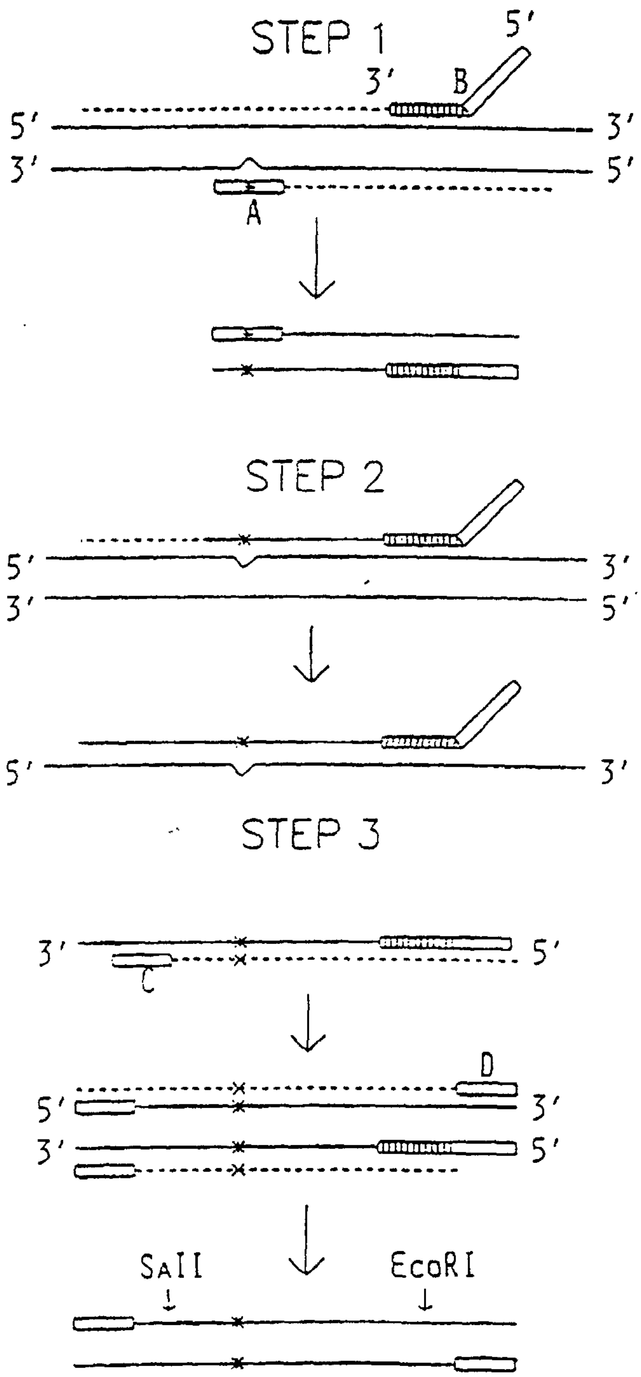


Fig. 5

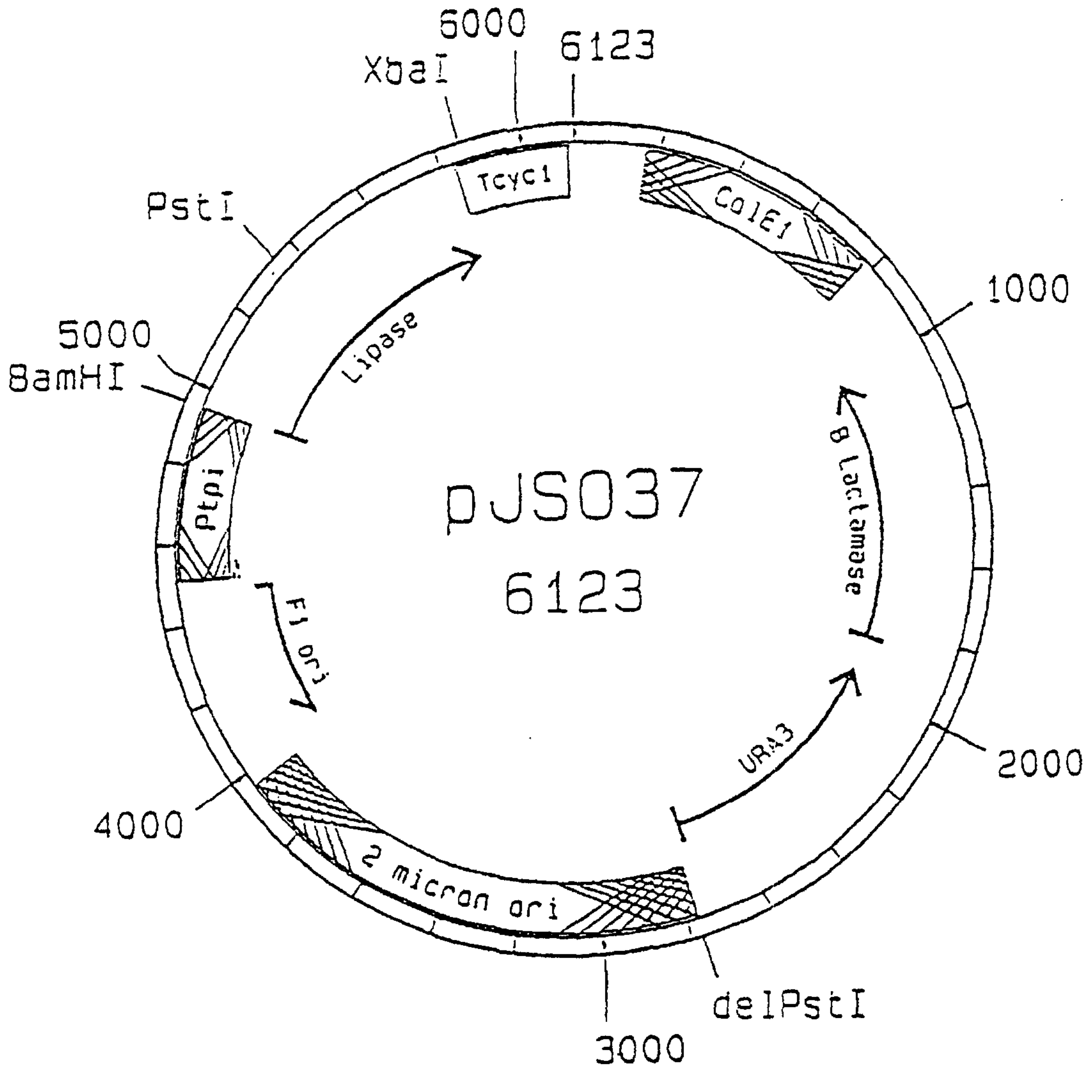
6000
6123
XbaI
Tcyc1
ColE1
PstI
1000
5000
BamHI
Lipase
B Lactamase
Ptpi
pJS037
6123
F1 ori
URA3
2000
4000
2 micron ori
delPstI
3000

pJS037

Fig. 6

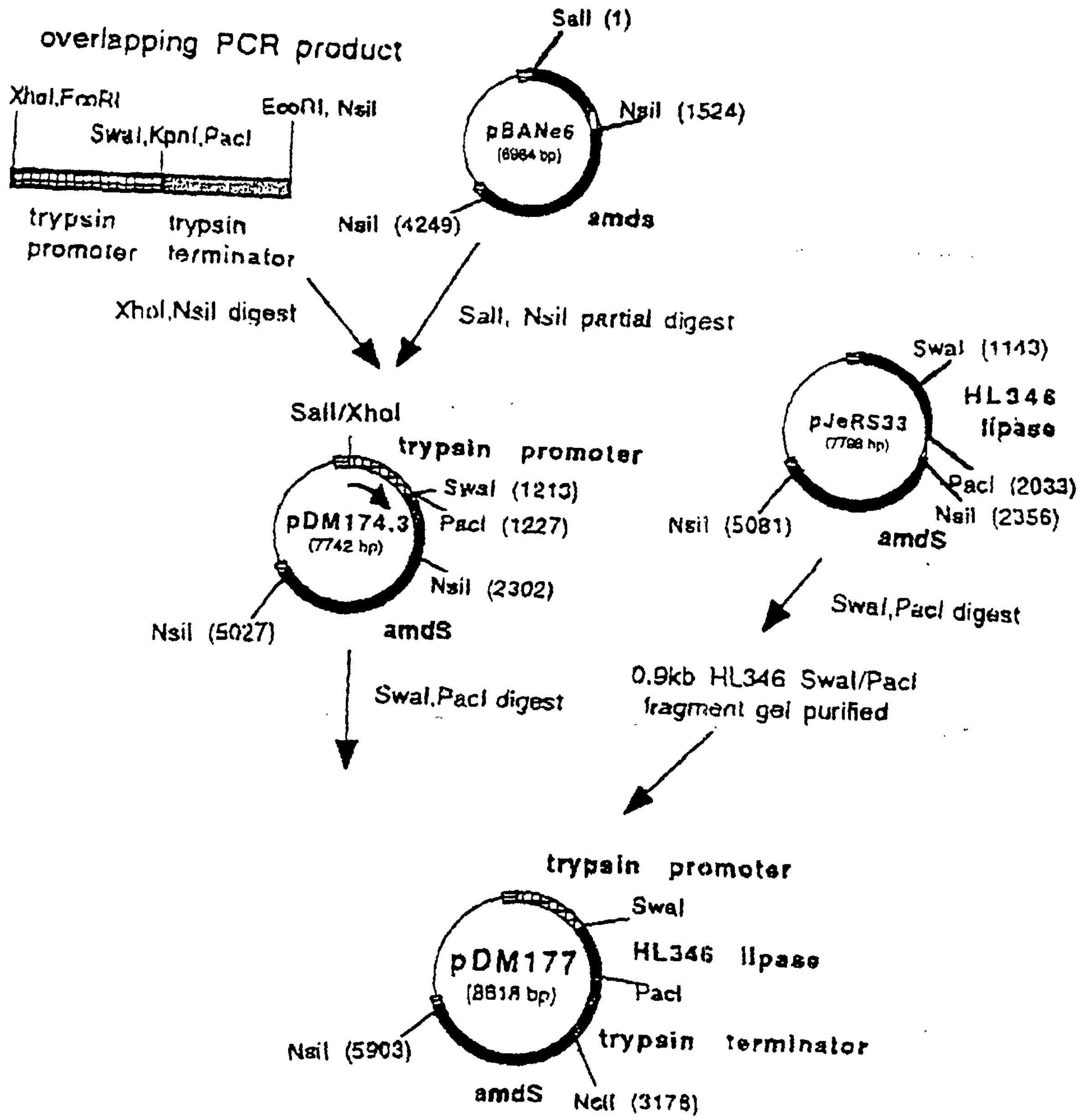
overlapping PCR product
XhoI,EcoRI
EcoRI, NsiI
SwaI,KpnI,PacI
trypsin promoter
trypsin terminator
SalI (1)
pBANe6
(6984 bp)
NsiI (1524)
NsiI (4249)
amds
XhoI,NsiI digest
SalI, NsiI partial digest
SalI/XhoI
trypsin promoter
SwaI (1213)
PacI (1227)
pDM174.3
(7742 bp)
NsiI (2302)
NsiI (5027)
amdS
SwaI (1143)
HL346 lipase
pJeRS33
(7798 bp)
PacI (2033)
NsiI (2356)
NsiI (5081)
amdS
SwaI,PacI digest
SwaI,PacI digest
0.9kb HL346 SwaI/PacI fragment gel purified
trypsin promoter
SwaI
pDM177
(8818 bp)
HL346 lipase
PacI
trypsin terminator
NsiI (5903)
amdS
NsiI (3178)

Fig. 7

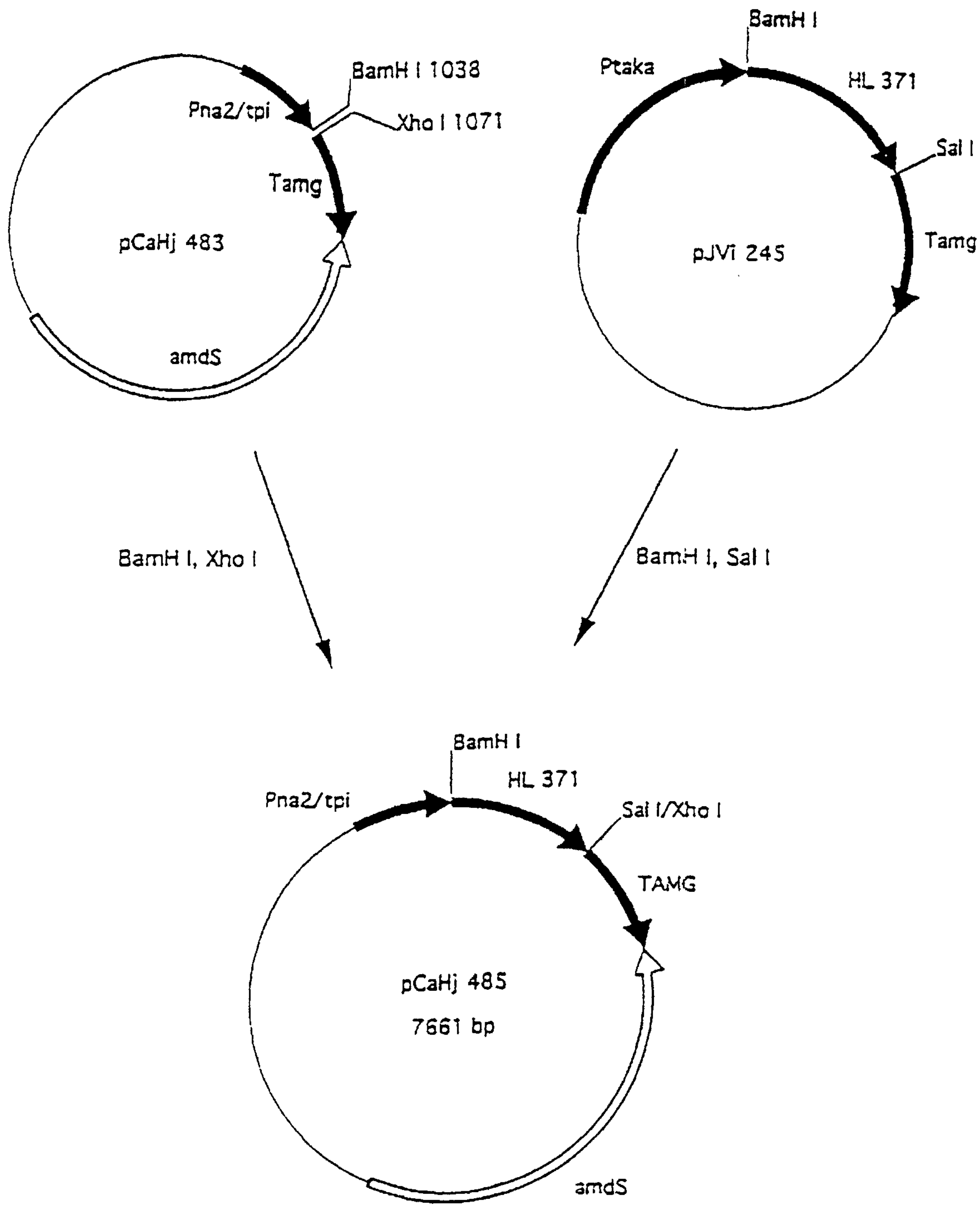
BamH I 1038
Pna2/tpi
Xho I 1071
Tamg
pCaHj 483
amdS
BamH I
Ptaka
HL 371
Sal I
Tamg
pJVi 245
BamH I, Xho I
BamH I, Sal I
BamH I
Pna2/tpi
HL 371
Sal I/Xho I
TAMG
pCaHj 485
7661 bp
amdS

Fig. 8

Length: 1115

1 AAAGGCATTC TCATTTTGTA GTCTTATTGC TAGCAGTATT CATCTGCATG

51 TGCTCTGTAT CGGGTGTGCC ACTGCAAATT GATCCACGCG ATGACAAGAG

101 CTATGTTCCT GAACAATATC CTTTGAAGGT GAATGGTCCT TTGCCAGAAG

151 GTGTAAGCGT GATCCAAGGC TATTGTGAAA ACTGTACCAT GTATCCTGAA

201 AAAAATAGTG TATCGGCATT CTCGTCATCA TCCACACAAG ATTATCGTAT

251 TGCAAGCGAG GCAGAGATTA AGGCACACAC ATTTTACACA GCATTGTCAG

301 CCAATGCATA CTGCAGAACT GTCATTCCTG GTGGTCGATG GAGCTGTCCC

351 CACTGTGGTG TTGCATCCAA TTTGCAAATT ACCAAGACTT TCAGCACCTT

401 AATCACTGAT ACTAATGTCT TGGTGGCTGT TGGCGAAAAG GAGAAGACCA

451 TCTATGTAGT TTTTCGTGGT ACAAGCTCAA TTCGCAACGC CATTGCTGAC

501 ATTGTTTTTG TACCAGTGAA TTATCCACCT GTTAATGGAG CCAAAGTACA

551 CAAAGGATTT CTTGATAGCT ATAACGAAGT CCAGGATAAA CTTGTTGCTG

601 AAGTCAAGGC ACAACTTGAT CGTCATCCAG GATACAAGAT CGTCGTCACT

651 GGACATTCCT TGGGAGGTGC AACAGCTGTT CTCAGTGCAC TTGACCTTTA

701 TCACCATGGC CATGCCAATA TCGAAATCTA TACTCAAGGT CAGCCACGTA

751 TAGGTACTCC AGCATTTGCA AACTATGTGA TAGGCACCAA GATTCCATAC

801 CAACGTCTTG TCCATGAGCG TGACATTGTT CCTCACCTTC CACCTGGTGC

851 ATTTGGTTTC TTGCATGCTG GTGAAGAGTT TTGGATCATG AAAGATAGCT

901 CGTTGCGCGT ATGTCCAAAT GGCATTGAAA CTGACAACTG CAGCAACTCC

951 ATTGTTCCCT TCACTAGTGT CATTGACCAT TTAAGCTATC TTGACATGAA

1001 CACTGGTCTC TGTTTATAAT CTTTAGTATC ATCCACTCCT CCTCTTTAAT

1051 GCAATACTTT TTAAGATAAA TCACAAGTAT ACTTTGTACA AAACCAAAAA

1101 AAAAAAAAAA AAAAA

Fig. 9

```
    CACATACAGGAATTCATTCAAGAATAGTTCAAACAAGAAGATTACAAACTATCAATTTCA
4861 ---------+---------+---------+---------+---------+---------+ 4920
    GTGTATGTCCTTAAGTAAGTTCTTATCAAGTTTGTTCTTCTAATGTTTGATAGTTAAAGT

     H T G I H S R I V Q T R R L Q T I N F I
                           B
                           Bs
                       S N     sp
              N  BB BB    N a ISS  CBi1    B
             BB IRAAssKasDMMNNIAuBaccS AvaH2S    s
             aa aslvaapmtpsnccal3slrm linK8a     e
             en lawaJJnHYnpliilwAllFFa uJlA6c    R
             II VIIIIIIIIIIIVIIIIIII IIIII    I
               / /// / / /// /  /// / ///
    TACACAATATAAACGACGGTACCCGGGGATCCACCATGAGGAGCTCCCTTGTGCTGTTCT
4921 ---------+---------+---------+---------+---------+---------+ 4980
    ATGTGTTATATTTGCTGCCATGGGCCCCTAGGTGGTACTCCTCGAGGGAACACGACAAGA

     H N I N D G T R G S T M R S S L V L F F

                H
      B      CBa  C C C B             C C
      s     M  vseS  vBaNAv c           Mv a   M
      m      w  ialt  ifchli e         ni c   w
      A      o  JJly  Ja8euJ f          lR 8   o
      I      I  IIII  IIIIII            III  I
                 //   //
    TTGTCTCTGCGTGGACGGCCTTGGCTAGCTCCACACAAGATTATCGTATTGCAAGCGAGG
4981 ---------+---------+---------+---------+---------+---------+ 5040
    AACAGAGACGCACCTGCCGGAACCGATCGAGGTGTGTTCTAATAGCATAACGTTCGCTCC

c    V S A W T A L A S S T Q D Y R I A S E A
                     |
                     START of the MATURE ATTC 44896
                        C   C   C
       M                M v    v N  S v P
       s                w i    i s f i s
       e                o J    R i c R t
       I                I I    II III
    CAGAGATTAAGGCACACACATTTTACACAGCATTGTCAGCCAATGCATACTGCAGAACTG
5041 ---------+---------+---------+---------+---------+---------+ 5100
    GTCTCTAATTCCGTGTGTGTAAAATGTGTCGTAACAGTCGGTTACGTATGACGTCTTGAC

c    E I K A H T F Y T A L S A N A Y C R T V

                             T   T
      E A                    s   s
      c Sc B      C      T   C  p  S C p
      o ce s  T  B  Av   F  BM s B v  5  f v 5
      R rl m  a  c  li   o  ss p s i  0  a i 0
      I Fl F  q  c  uJ   k  IIR b R   9  N R 9
      IIII I  I  II   I  IIII I  I  III
              /
```

Fig. 10

```
     TCATTCCTGGTGGTCGATGGAGCTGTCCCCACTGTGGTGTTGCATCCAATTTGCAAATTA
5101 ---------+---------+---------+---------+---------+---------+ 5160
     AGTAAGGACCACCAGCTACCTCGACAGGGGTGACACCACAACGTAGGTTAAACGTTTAAT

c    I P G G R W S C P H C G V A S N L Q I T

     CCAAGACTTTCAGCACCTTAATCACTGATACTAATGTCTTGGTGGCTGTTGGCGAAAAGG
5161 ---------+---------+---------+---------+---------+---------+ 5220
     GGTTCTGAAAGTCGTGGAATTAGTGACTATGATTACAGAACCACCGACAACCGCTTTTCC

c    K T F S T L I T D T N V L V A V G E K E

     AGAAGACCATCTATGTAGTTTTTCGTGGTACAAGCTCAATTCGCAACGCCATTGCTGACA
5221 ---------+---------+---------+---------+---------+---------+ 5280
     TCTTCTGGTAGATACATCAAAAAGCACCATGTTCGAGTTAAGCGTTGCGGTAACGACTGT

c    K T I Y V V F R G T S S I R N A I A D I

     TTGTTTTTGTACCAGTGAATTATCCACCTGTTAATGGAGCCAAAGTACACAAAGGATTTC
5281 ---------+---------+---------+---------+---------+---------+ 5340
     AACAAAAACATGGTCACTTAATAGGTGGACAATTACCTCGGTTTCATGTGTTTCCTAAAG

c    V F V P V N Y P P V N G A K V H K G F L
```

Fig. 10 (cont.)

```
   TTGATAGCTATAACGAAGTCCAGGATAAACTTGTTGCTGAAGTCAAGGCACAACTTGATC
5341 ---------+---------+---------+---------+---------+---------+ 5400
   AACTATCGATATTGCTTCAGGTCCTATTTGAACAACGACTTCAGTTCCGTGTTGAACTAG

c     D  S  Y  N  E  V  Q  D  K  L  V  A  E  V  K  A  Q  L  D  R

   GTCATCCAGGATACAAGATCGTCGTCACTGGACATTCCTTGGGAGGTGCAACAGCTGTTC
5401 ---------+---------+---------+---------+---------+---------+ 5460
   CAGTAGGTCCTATGTTCTAGCAGCAGTGACCTGTAAGGAACCCTCCACGTTGTCGACAAG

c     H  P  G  Y  K  I  V  V  T  G  H  S  L  G  G  A  T  A  V  L

   TCAGTGCACTTGACCTTTATCACCATGGCCATGCCAATATCGAAATCTATACTCAAGGTC
5461 ---------+---------+---------+---------+---------+---------+ 5520
   AGTCACGTGAACTGGAAATAGTGGTACCGGTACGGTTATAGCTTTAGATATGAGTTCCAG

c     S  A  L  D  L  Y  H  H  G  H  A  N  I  E  I  Y  T  Q  G  Q

   AGCCACGTATAGGTACTCCAGCATTTGCAAACTATGTGATTGGCACCAAGATTCCATACC
5521 ---------+---------+---------+---------+---------+---------+ 5580
   TCGGTGCATATCCATGAGGTCGTAAACGTTTGATACACTATCCGTGGTTCTAAGGTATGG

c     P  R  I  G  T  P  A  F  A  N  Y  V  L  G  T  K  I  P  Y  Q
```

Fig. 10 (cont.)

```
     AACGTCTTGTCCATGAGCGTGACATTGTTCCTCACCTTCCACCTGGTGCATTTGGTTTCT
5581 ---------+---------+---------+---------+---------+---------+ 5640
     TTGCAGAACAGGTACTCGCACTGTAACAAGGAGTGGAAGGTGGACCACGTAAACCAAAGA

c     R L V H E R D I V P H L P P G A F G F L

       N        S   N
     C C I      a M  I C    C
     v a aEMNS     Hu DbR  aAj    Av   M  HT
     i c lassp    p3 poc  lle    li   w  hh
     R 8 lrlph    hA nla  lwP    uJ   o  aa
     I I IIIII   II III III   II   I II
       ///        // /    /    /
     TGCATGCTGGTGAAGAGTTTTGGATCATGAAAGATAGCTCGTTGCGCGTATGTCCAAATG
5641 ---------+---------+---------+---------+---------+---------+ 5700
     ACGTACGACCACTTCTCAAAACCTAGTACTTTCTATCGAGCAACGCGCATACAGGTTTAC

c     H A G E E F W I M K D S S L R V C P N G

             CB
           S vsPT      B       SB           M
           f ioss     'b      pf          s
           c RFte      v       ea          e
           I IIII      I       II          I
             /
     GCATTGAAACTGACAACTGCAGCAACTCCATTGTTCCCTTCACTAGTGTCATTGACCATT
5701 ---------+---------+---------+---------+---------+---------+ 5760
     CGTAACTTTGACTGTTGACGTCGTTGAGGTAACAAGGGAAGTGATCACAGTAACTGGTAA

c     I E T D N C S N S I V P F T S V I D H L

                                        T
            N                   S   H  N  s
      C     I      T  B          a C Ba  I  p
      Av    a       Bs  Bs   M  XB   u vAseT  a  5
      li    l      sp  sm   n  bf   9 icola  I  0
      uJ    I       rR  aA   I  aa   6 JiFlu  I  9
      II    I      II  II   I  II   I IIIII  I  I
      /           / /           ///
     TAAGCTATCTTGACATGAACACTGGTCTCTGTTTATAGTCTAGAGGGCCGCATGATGTAA
5761 ---------+---------+---------+---------+---------+---------+ 5820
     ATTCGATAGAACTGTACTTGTGACCAGAGACAAATATCAGATCTCCCGGCGTACTACATT

c     S Y L D M N T G L C L * S R G P H D V I
                  STOP of ATTC 44896
      MT
      as      C             B    R C
      ep      j F          M A s    I j
      I4      e o          n c r    e e
      I5      P k          I i B    A P
      II      I I          I II     I I
      /
     TTAGTTATGTCACGCTTACATTCACGCCCTCCCCCCACATCCGCTCTAACCGAAAAGGAA
```

Fig. 10 (cont.)

```
5821 ---------+---------+---------+---------+---------+---------+ 5880
     AATCAATACAGTGCGAATGTAAGTGCGGGAGGGGGGTGTAGGCGAGATTGGCTTTTCCTT

c      S Y V T L T F T P S P H I R S N R K G R

                  E
                  c
                  o PS    E
         B        AONsa    c
         s      B v1lpu    o               M
         m      f a0a59    5               s
         F      a l9ll6    7               e
         l      l llVll    l               l
                  / //
     GGAGTTAGACAACCTGAAGTCTAGGTCCCTATTTATTTTTTTATAGTTATGTTAGTATTA
5881 ---------+---------+---------+---------+---------+---------+ 5940
     CCTCAATCTGTTGGACTTCAGATCCAGGGATAAATAAAAAAATATCAATACAATCATAAT
c      S * T T * S L G P Y L F F Y S Y V S I K
```

Fig. 10 (cont.)

```
                B           BC
       B        Ts  B B MA     MAsvMT
       b        so  b p wc     nloiws
       v        eF  v m oi     luFJoe
       I        II  I I II     IIIIII
                                ///
ATGAGATTTCCTTCTATTTTTACTGCTGTTTTATTCGCTGCTTCCTCCGCTTTAGCTGCT
1 ---------+---------+---------+---------+---------+---------+ 60
TACTCTAAAGGAAGATAAAAATGACGACAAAATAAGCGACGAAGGAGGCGAAATCGACGA

a    M R F P S I F T A V L F A A S S A L A A -

                     T
   H                sE    M      M
   i       T      CM  pc   BCsP C     a
B  nB      s       vb A5 o  Acvpv Av     e
s  cs      p       io p0 5  leiAu li     l
r  Ib      R       JI o9 7  ufJ1I uJ     I
I  II      I       II II I  IIIII II     I
                   / /     /// /
CCAGTCAACACTACCACTGAAGATGAAACGGCTCAAATTCCAGCTGAAGCTGTCATCGGT
61 ---------+---------+---------+---------+---------+---------+ 120
GGTCAGTTGTGATGGTGACTTCTACTTTGCCGAGTTTAAGGTCGACTTCGACAGTAGCCA

a    P V N T T T E D E T A Q I P A E A V I G -

  E                           H
  c                           g
  o       T  H         C        i
  5       a  p         j      E
  7       q  h         e      I
  I       I  I         I      I
TACCTTGATTTAGAAGGTGATTTCGATGTTGCTGTTTTGCCATTTTCCAACTCCACCAAT
121 ---------+---------+---------+---------+---------+---------+ 180
ATGAGACTAAATCTTCCACTAAAGCTACAACGACAAAACGGTAAAAGGTTGAGGTGGTTA

a    Y L D L E G D F D V A V L P F S N S T N -

  T
  s                 B  B
  p    M     C   B    sM TsM
  4    m     j   b    rw son
  C    e     e   v    Do eFI
  I    I     I   I    II III
AACGGTTTATTGTTTATCAATACTACTATTGCCTCCATTGCTGCTAAAGAAGAAGGTGTT
181 ---------+---------+---------+---------+---------+---------+ 240
TTGCCAAATAACAAATAGTTATGATGATAACGGAGGTAACGACGATTTCTTCTTCCACAA

a    N G L L F I N T T I A S I A A K E E G V -

M     H
b     iT
o     nf
I     fi
I     II
      /
TCTTTGGATAAAAGA
241 ---------+----- 255
AGAAACCTATTTTCT

a    S L D K R  -
```

Fig. 11

SEQUENCE LISTING

(1) GENERAL INFORMATION:

(i) APPLICANT:

(A) NAME: Novo Nordisk A/S
(B) STREET: Novo Alle
(C) CITY: Bagsvaerd
(E) COUNTRY: Denmark
(F) POSTAL CODE (ZIP): DK-2880
(G) TELEPHONE: +45 4444 8888
(H) TELEFAX: +45 4449 3256

(ii) TITLE OF INVENTION: Novel Lipolytic enzyme
(iii) NUMBER OF SEQUENCES: 100
(iv) (iv) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30B (EPO)

(2) INFORMATION FOR SEQ ID NO: 1:

(i) SEQUENCE CHARACTERISTICS:

(A) LENGTH: 21 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer 3"

(xi) SEQUENCE DESCRIPTION: SEQ ID No. 1:

AGAAATCGGG TATCCTTTCA G 21

(2) INFORMATION FOR SEQ ID NO: 2:

(i) SEQUENCE CHARACTERISTICS:

(A) LENGTH: 30 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer 7258"

(xi) SEQUENCE DESCRIPTION: SEQ ID NO: 2:

gaatgacttg gttgacgcgt caccagtcac 30

(2) INFORMATION FOR SEQ ID NO: 3:

(i) SEQUENCE CHARACTERISTICS:

(A) LENGTH: 26 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer 7770"

(xi) SEQUENCE DESCRIPTION: SEQ ID NO: 3:

tctagcccag aatactggat caaatc 26

(2) INFORMATION FOR SEQ ID NO:4:

(i) SEQUENCE CHARACTERISTICS:

(A) LENGTH: 53 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "randon 1"
(B) Bottle 5: 93% A; 2.33% C; 2.33% G and 2.33% T

Bottle 6: 93% C; 2.33% A; 2.33% G and 2.33% T
Bottle 7: 93% G; 2.33% A; 2.33% C and 2.33% T
Bottle 8: 93% T; 2.33% A; 2.33% C and 2.33% G

(xi) SEQUENCE DESCRIPTION: SEQ ID NO: 4:

| | | | |
|---|---|---|---|
| 5' | 5 | C | G |
| T | 5 | C | 3' |
| T | 7 | A | |
| A | 8 | G | |
| T | 8 | T | |
| T | A/C | T | |
| T | 5 | C | |
| C | 7 | T | |
| T | 5 | C | |
| T | 8 | T | |
| T | 8 | A | |
| 6 | C/G | T | |
| 5 | 6 | G | |
| 5 | 6 | G | |
| 7 | G | A | |
| 8 | AA | A | |
| 6 | T | C | |
| 7 | | | |

(2) INFORMATION FOR SEQ ID NO: 5:

(i) SEQUENCE CHARACTERISTICS:

(A) LENGTH: 93 base pairs
(B) TYPE: nucleic acid
(C) STRANDEDNESS: single
(D) TOPOLOGY: linear

(ii) MOLECULE TYPE: other nucleic acid

(A)DESCRIPTION: /desc = "Oligo 2"
Flask 5: 80% A; 6,66% C; 6,66% G og 6,66 % T.
Flask 6: 80% C; 6,66% A; 6,66% G og 6,66 % T.
Flask 7: 80% G; 6,66% A; 6,66% C og 6,66 % T.
Flask 8: 80% T; 6,66% A; 6,66% C og 6,66 % G.

(xi) SEQUENCE DESCRIPTION: SEQ ID NO: 5:

GTCTCTGCGT GGACGGCCTT GGCGGCGCCA CCTCCA67(T/A)
66(T/A) 575 66(T/A) 67(T/A) 66(T/A) 575 66(T/A) (6/7)(7/8)(C/G)
57(C/G) C57 (5/7)5(C/G) CTGT TTAACCAGTT CAATCTC 93

(2) INFORMATION FOR SEQ ID NO: 6:

(i) SEQUENCE CHARACTERISTICS:

(A) LENGTH: 64 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Oligo lid2"
Flask 5: 93% A; 2,33% C; 2,33% G og 2,33 % T.
Flask 6: 93% C; 2,33% A; 2,33% G og 2,33 % T.
Flask 7: 93% G; 2,33% A; 2,33% C og 2,33 % T.
Flask 8: 93% T; 2,33% A; 2,33% C og 2,33 % G.

(xi) SEQUENCE DESCRIPTION: SEQ ID NO: 6:

```
CATTTAT886 888655(C/G)(A/C/G/T)(A/C/G/T)7 55(C/G)88(A/C)5758
8(C/G)76(7/8)58665 788688(8/7)587 75ACGAG(A/T)GC CACG    64
```

2) INFORMATION FOR SEQ ID NO: 7:

(i) SEQUENCE CHARACTERISTICS:

(A) LENGTH: 105 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer 4"

(xi) SEQUENCE DESCRIPTION: SEQ ID NO: 7:

```
GCTCCTCATG GTGGATCCCC AGTTGTGTAT ATAGAGGATT
GAGGAAGGAA GAGAAGTGTG GATAGAGGTA AATTGAGTTG
GAAACTCCAA GCATGGCATC CTTGC                    105
```

(2) INFORMATION FOR SEQ ID NO: 8:

(i) SEQUENCE CHARACTERISTICS:

(A) LENGTH: 54 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer 8479"

(xi) SEQUENCE DESCRIPTION: SEQ ID NO: 8:

```
GCGTGGACGG CCTTGGCTAG CCCTATTCGT CCTCGACCGG TCTCGCAGGA TCTG    54
```

(2) INFORMATION FOR SEQ ID NO: 9:

(i) SEQUENCE CHARACTERISTICS:

(A) LENGTH: 30 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer 7887"

(xi) SEQUENCE DESCRIPTION: SEQ ID NO: 9:

GAATGACTTG GTTGAGTACT CACCAGTCAC 30

(2) INFORMATION FOR SEQ ID NO: 10:

(i) SEQUENCE CHARACTERISTICS:

(A) LENGTH: 46 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer 8932"

(xi) SEQUENCE DESCRIPTION: SEQ ID NO: 10:

GAACTGGATA GGAAATTTGA AGTTCCTGTT GAAAGAAATA AATGAC 46

(2) INFORMATION FOR SEQ ID NO: 11:

(i) SEQUENCE CHARACTERISTICS:

(A) LENGTH: 57 base pairs
(B) TYPE: nucleic acid
(C) STRANDEDNESS: single
(D) TOPOLOGY: linear

(ii) MOLECULE TYPE: other nucleic acid

(A) DESCRTPTION: /desc = "Oligo 1"
Flask 5: 80% A; 6.66% C; 6.66% G og 6.66 % T.
Flask 6: 80% C; 6.66% A; 6.66% G og 6.66 % T.
Flask 7: 80% G; 6.66% A; 6.66% C og 6.66 % T.
Flask 8: 80% T; 6.66% A; 6.66% C og 6.66 % G.

(xi) SEQUENCE DESCRIPTION: SEQ ID NO: 11:

GCGTGGACGG CCTTGGCC86(T/A) 66(A/T) 58(T/A) 67(T/A) 66(T/A)
575 66(T/A) GAGGTC TCG CAGGATC TG 57

(2) INFORMATION FOR SEQ ID NO: 12:

(i) SEQUENCE CHARACTERISTICS:

(A) LENGTH: 18 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer 2056"

(xi) SEQUENCE DESCRIPTION: SEQ ID NO: 12:

GCACGTAATG TTTGTACC 18

(2) INFORMATION FOR SEQ ID NO: 13:

(i) SEQUENCE CHARACTERISTICS:

(A) LENGTH: 18 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer 4699"

(xi) SEQUENCE DESCRIPTION: SEQ ID NO: 13:

CGGTACCCGG GGATCCAC 18

(2) INFORMATION FOR SEQ ID NO: 14:

(i) SEQUENCE CHARACTERISTICS:

(A) LENGTH: 30 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Forward Primer 1"

(xi) SEQUENCE DESCRIPTION: SEQ ID NO: 14:

GAGCTCGAGG AATTCTTACA AACCTTCAAC 30

(2) INFORMATION FOR SEQ ID NO: 15:

(i) SEQUENCE CHARACTERISTICS:

(A) LENGTH: 47 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Reverse Primer 2"

(xi) SEQUENCE DESCRIPTION: SEQ ID NO: 15:

TTAATTAAGG TACCTGAATT TAAATGGTGA AGAGATAGAT ATCCAAG 47

(2) INFORMATION FOR SEQ ID NO: 16:

(i) SEQUENCE CHARACTERISTICS:

(A) LENGTH: 51 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Forward Primer 3"

(xi) SEQUENCE DESCRIPTION: SEQ ID NO: 16:

TCACCATTTA AATTCAGGTA CCTTAATTAA ATTCCTTGTT GGAAGCGTCG A 51

(2) INFORMATION FOR SEQ ID NO: 17:

(i) SEQUENCE CHARACTERISTICS:

(A) LENGTH: 42 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Reverse Primer 4"

(xi) SEQUENCE DESCRIPTION: SEQ ID NO: 17:

TGGTATGCAT AAGCTTGAAT TCAGGTAAAC AAGATATAAT TT 42

(2) INFORMATION FOR SEQ ID NO: 18:

(i) SEQUENCE CHARACTERISTICS:

(A) LENGTH: 32 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "HLIP-A Primer 5"

(xi) SEQUENCE DESCRIPTION: SEQ ID NO: 18:

CCCATTTAAA TATGAGGAGC TCCCTTGTGC TG 32

(2) INFORMATION FOR SEQ ID NO: 19:

(i) SEQUENCE CHARACTERISTICS:

(A) LENGTH: 32 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "HLIP-B Primer 6"

(xi) SEQUENCE DESCRIPTION: SEQ ID NO: 19:

CCCTTAATTA ACTAAAGACA TGTCCCAATT AA 32

(2) INFORMATION FOR SEQ ID NO: 20:

(i) SEQUENCE CHARACTERISTICS:

(A) LENGTH: 6 amino acids
(B) TYPE: amino acid
(D) TOPOLOGY: linear

(ii) MOLECULE TYPE: peptide addition
(xi) SEQUENCE DESCRIPTION: SEQ ID NO: 20:

Arg-Pro-Val-Ser-Gln-Asp 6
5

(2) INFORMATION FOR SEQ ID NO: 21:

(i) SEQUENCE CHARACTERISTICS:

(A) LENGTH: 5 amino acids
(B) TYPE: amino acid
(D) TOPOLOGY: linear

(ii) MOLECULE TYPE: peptide addition
(xi) SEQUENCE DESCRIPTION: SEQ ID NO: 21:

Ser-Pro-Ile-Arg-Met 5
5

(2) INFORMATION FOR SEQ ID NO: 22:

(i) SEQUENCE CHARACTERISTICS:

(A) LENGTH: 6 amino acids
(B) TYPE: amino acid
(D) TOPOLOGY: linear

(ii) MOLECULE TYPE: peptide addition
(xi) SEQUENCE DESCRIPTION: SEQ ID NO: 22:

Ser-Pro-Ile-Arg-Ala-Arg 6
5

(2) INFORMATION FOR SEQ ID NO: 23:

(i) SEQUENCE CHARACTERISTICS:

(A) LENGTH: 6 amino acids
(B) TYPE: amino acid
(D) TOPOLOGY: linear

(ii) MOLECULE TYPE: peptide addition
(xi) SEQUENCE DESCRIPTION: SEQ ID NO: 23:

Ser-Pro-Ile-Arg-Pro-Arg 6
5

(2) INFORMATION FOR SEQ ID NO: 24:

(i) SEQUENCE CHARACTERISTICS:

(A) LENGTH: 6 amino acids
(B) TYPE: amino acid
(D) TOPOLOGY: linear

(ii) MOLECULE TYPE: peptide addition
(xi) SEQUENCE DESCRIPTION: SEQ ID NO: 24:

Ser-Pro-Ile-Arg-Glu-Arg 6
5

(2) INFORMATION FOR SEQ ID NO: 25:

(i) SEQUENCE CHARACTERISTICS:

(A) LENGTH: 5 amino acids
(B) TYPE: amino acid
(D) TOPOLOGY: linear

(ii) MOLECULE TYPE: peptide addition
(xi) SEQUENCE DESCRIPTION: SEQ ID NO: 25:

Ser-Pro-Ile-Arg-Lys 5
5

(2) INFORMATION FOR SEQ ID NO: 26:

(i) SEQUENCE CHARACTERISTICS:

(A) LENGTH: 5 amino acids
(B) TYPE: amino acid
(D) TOPOLOGY: linear

(ii) MOLECULE TYPE: peptide addition

(xi) SEQUENCE DESCRIPTION: SEQ ID NO: 26:

Ser-Pro-Ile-Lys-Lys 5
5

(2) INFORMATION FOR SEQ ID NO: 27:

(i) SEQUENCE CHARACTERISTICS:

(A) LENGTH: 6 amino acids
(B) TYPE: amino acid
(D) TOPOLOGY: linear

(ii) MOLECULE TYPE: peptide addition
(xi) SEQUENCE DESCRIPTION: SEQ ID NO: 27:

Ser-Pro-Ile-Arg-Arg-Pro 6
5

(2) INFORMATION FOR SEQ ID NO: 28:

(i) SEQUENCE CHARACTERISTICS:

(A) LENGTH: 5 amino acids
(B) TYPE: amino acid
(D) TOPOLOGY: linear

(ii) MOLECULE TYPE: peptide addition
(xi) SEQUENCE DESCRIPTION: SEQ ID NO: 28:

Ser-Pro-Pro-Arg-Arg 5
5

(2) INFORMATION FOR SEQ ID NO: 29:

(i) SEQUENCE CHARACTERISTICS:

(A) LENGTH: 5 amino acids
(B) TYPE: amino acid
(D) TOPOLOGY: linear

(ii) MOLECULE TYPE: peptide addition
(xi) SEQUENCE DESCRIPTION: SEQ ID NO: 29:

Ser-Pro-Iso-Pro-Arg 5
5

(2) INFORMATION FOR SEQ ID NO: 30:

(i) SEQUENCE CHARACTERISTICS:

(A) LENGTH: 5 amino acids
(B) TYPE: amino acid
(D) TOPOLOGY: linear

(ii) MOLECULE TYPE: peptide addition
(xi) SEQUENCE DESCRIPTION: SEQ ID NO: 30:

Ser-Pro-Arg-Pro-Arg 5
5

(2) INFORMATION FOR SEQ ID NO: 31:

(i) SEQUENCE CHARACTERISTICS:

(A) LENGTH: 4 amino acids
(B) TYPE: amino acid
(D) TOPOLOGY: linear

(ii) MOLECULE TYPE: peptide addition
(xi) SEQUENCE DESCRIPTION: SEQ ID NO: 31:

Ser-Pro-Ile-Arg 4

(2) INFORMATION FOR SEQ ID NO: 32:

(i) SEQUENCE CHARACTERISTICS:

(A) LENGTH: 5 amino acids
(B) TYPE: amino acid
(D) TOPOLOGY: linear

(ii) MOLECULE TYPE: peptide addition
(xi) SEQUENCE DESCRIPTION: SEQ ID NO: 32:

Ser-Pro-Ile-Arg-Arg 5
5

(2) INFORMATION FOR SEQ ID NO: 33:

(i) SEQUENCE CHARACTERISTICS:

(A) LENGTH: 4 amino acids
(B) TYPE: amino acid
(D) TOPOLOGY: linear

(ii) MOLECULE TYPE: peptide addition
(xi) SEQUENCE DESCRIPTION: SEQ ID NO: 33:

Ser-Cys-Ile-Arg-Arg 5
5

(2) INFORMATION FOR SEQ ID NO: 34:

(i) SEQUENCE CHARACTERISTICS:

(A) LENGTH: 7 amino acids
(B) TYPE: amino acid
(D) TOPOLOGY: linear

(ii) MOLECULE TYPE: peptide addition
(xi) SEQUENCE DESCRIPTION: SEQ ID NO: 34:

Ser-Pro-Ile-Arg-Pro-Arg-Pro 7
5

(2) INFORMATION FOR SEQ ID NO: 35:

(i) SEQUENCE CHARACTERISTICS:

(A) LENGTH: 7 amino acids
(B) TYPE: amino acid
(D) TOPOLOGY: linear

(ii) MOLECULE TYPE: peptide addition
(xi) SEQUENCE DESCRIPTION: SEQ ID NO: 35:

Ser-Cys-Ile-Arg-Pro-Arg-Pro 7
5

(2) INFORMATION FOR SEQ ID NO: 36:

(i) SEQUENCE CHARACTERISTICS:

(A) LENGTH: 7 amino acids
(B) TYPE: amino acid
(D) TOPOLOGY: linear

(ii) MOLECULE TYPE: peptide addition
(xi) SEQUENCE DESCRIPTION: SEQ ID NO: 36:

Ser-Pro-Arg-Arg-Pro-Arg-Thr 7
5

(2) INFORMATION FOR SEQ ID NO: 37:

(i) SEQUENCE CHARACTERISTICS:

(A) LENGTH: 7 amino acids
(B) TYPE: amino acid
(D) TOPOLOGY: linear

(ii) MOLECULE TYPE: peptide addition
(xi) SEQUENCE DESCRIPTION: SEQ ID NO: 37:

Ser-Pro-Phe-Arg-Pro-Lys-Leu 7
5

(2) INFORMATION FOR SEQ ID NO: 38:

(i) SEQUENCE CHARACTERISTICS:

(A) LENGTH: 6 amino acids
(B) TYPE: amino acid

(D) TOPOLOGY: linear

(ii) MOLECULE TYPE: peptide addition
(xi) SEQUENCE DESCRIPTION: SEQ ID NO: 38:

```
Ser-Pro-Pro-Arg-Arg-Pro                                    6
                    5
```

(2) INFORMATION FOR SEQ ID NO: 39:

(i) SEQUENCE CHARACTERISTICS:

(A) LENGTH: 6 amino acids
(B) TYPE: amino acid
(D) TOPOLOGY: linear

(ii) MOLECULE TYPE: peptide addition
(xi) SEQUENCE DESCRIPTION: SEQ ID NO: 39:

```
Ser-Pro-Ile-Arg-Arg-Glu                                    6
                    5
```

(2) INFORMATION FOR SEQ ID NO: 40:

(i) SEQUENCE CHARACTERISTICS:

(A) LENGTH: 6 amino acids
(B) TYPE: amino acid
(D) TOPOLOGY: linear

(ii) MOLECULE TYPE: peptide addition
(xi) SEQUENCE DESCRIPTION: SEQ ID NO: 40:

```
Ser-Pro-Pro-Arg-Pro-Pro                                    6
                    5
```

(2) INFORMATION FOR SEQ ID NO: 41:

(i) SEQUENCE CHARACTERISTICS:

(A) LENGTH: 6 amino acids
(B) TYPE: amino acid
(D) TOPOLOGY: linear

(ii) MOLECULE TYPE: peptide addition
(xi) SEQUENCE DESCRIPTION: SEQ ID NO: 41:

```
Ser-Pro-Pro-Arg-Pro-Arg                                    6
                    5
```

(2) INFORMATION FOR SEQ ID NO: 42:

(i) SEQUENCE CHARACTERISTICS:

(A) LENGTH: 6 amino acids
(B) TYPE: amino acid
(D) TOPOLOGY: linear

(ii) MOLECULE TYPE: peptide addition
(xi) SEQUENCE DESCRIPTION: SEQ ID NO: 42:

Ser-Pro-Pro-Trp-Trp-Pro 6
5

(2) INFORMATION FOR SEQ ID NO: 43:

(i) SEQUENCE CHARACTERISTICS:

(A) LENGTH: 6 amino acids
(B) TYPE: amino acid
(D) TOPOLOGY: linear

(ii) MOLECULE TYPE: peptide addition
(xi) SEQUENCE DESCRIPTION: SEQ ID NO: 43:

Ser-Pro-Pro-Trp-Arg-Pro 6
5

(2) INFORMATION FOR SEQ ID NO: 44:

(i) SEQUENCE CHARACTERISTICS:

(A) LENGTH: 6 amino acids
(B) TYPE: amino acid
(D) TOPOLOGY: linear

(ii) MOLECULE TYPE: peptide addition
(xi) SEQUENCE DESCRIPTION: SEQ ID NO: 44:

Ser-Pro-Pro-Arg-Trp-Pro 6
5

(2) INFORMATION FOR SEQ ID NO: 45:

(i) SEQUENCE CHARACTERISTICS:

(A) LENGTH: 6 amino acids
(B) TYPE: amino acid
(D) TOPOLOGY: linear

(ii) MOLECULE TYPE: peptide addition
(xi) SEQUENCE DESCRIPTION: SEQ ID NO: 45:

Ser-Pro-Pro-Arg-Trp-Pro 6
5

(2) INFORMATION FOR SEQ ID NO: 46:

(i) SEQUENCE CHARACTERISTICS:

(A) LENGTH: 6 amino acids
(B) TYPE: amino acid
(D) TOPOLOGY: linear

(ii) MOLECULE TYPE: peptide addition
(xi) SEQUENCE DESCRIPTION: SEQ ID NO: 46:

```
Ser-His-Trp-Arg-Arg-Trp                                        6
                  5
```

(2) INFORMATION FOR SEQ ID NO: 47:

(i) SEQUENCE CHARACTERISTICS:

(A) LENGTH: 5 amino acids
(B) TYPE: amino acid
(D) TOPOLOGY: linear

(ii) MOLECULE TYPE: peptide addition
(xi) SEQUENCE DESCRIPTION: SEQ ID NO: 47:

```
Ser-His-Trp-Arg-Lys                                            5
                  5
```

(2) INFORMATION FOR SEQ ID NO: 48:

(i) SEQUENCE CHARACTERISTICS:

(A) LENGTH: 5 amino acids
(B) TYPE: amino acid
(D) TOPOLOGY: linear

(ii) MOLECULE TYPE: peptide addition
(xi) SEQUENCE DESCRIPTION: SEQ ID NO: 48:

```
Ser-His-Trp-Arg-Arg                                            5
                  5
```

(2) INFORMATION FOR SEQ ID NO: 49:

(i) SEQUENCE CHARACTERISTICS:

(A) LENGTH: 7 amino acids
(B) TYPE: amino acid
(D) TOPOLOGY: linear

(ii) MOLECULE TYPE: peptide addition
(xi) SEQUENCE DESCRIPTION: SEQ ID NO: 49:

```
Thr-Ala-Ile-Arg-Pro-Arg-Lys                                    7
                  5
```

(2) INFORMATION FOR SEQ ID NO: 50:

(i) SEQUENCE CHARACTERISTICS:

(A) LENGTH: 7 amino acids
(B) TYPE: amino acid
(D) TOPOLOGY: linear

(ii) MOLECULE TYPE: peptide addition
(xi) SEQUENCE DESCRIPTION: SEQ ID NO: 50:

```
Ser-Thr-Arg-Arg-Pro-Arg-Pro                    7
                5
```

(2) INFORMATION FOR SEQ ID NO: 51:

(i) SEQUENCE CHARACTERISTICS:

(A) LENGTH: 7 amino acids
(B) TYPE: amino acid
(D) TOPOLOGY: linear

(ii) MOLECULE TYPE: peptide addition
(xi) SEQUENCE DESCRIPTION: SEQ ID NO: 51:

```
Gly-Pro-Ile-Arg-Pro-Arg-Pro                    7
                5
```

(2) INFORMATION FOR SEQ ID NO: 52:

(i) SEQUENCE CHARACTERISTICS:

(A) LENGTH: 7 amino acids
(B) TYPE: amino acid
(D) TOPOLOGY: linear

(ii) MOLECULE TYPE: peptide addition
(xi) SEQUENCE DESCRIPTION: SEQ ID NO: 52:

```
Leu-Pro-Phe-Arg-Glu-Arg-Pro                    7
                5
```

(2) INFORMATION FOR SEQ ID NO: 53:

(i) SEQUENCE CHARACTERISTICS:

(A) LENGTH: 7 amino acids
(B) TYPE: amino acid
(D) TOPOLOGY: linear

(ii) MOLECULE TYPE: peptide addition
(xi) SEQUENCE DESCRIPTION: SEQ ID NO: 53:

Ser-Arg-Ser-Arg-His-Asp-Ala 7
5

(2) INFORMATION FOR SEQ ID NO: 54:

(i) SEQUENCE CHARACTERISTICS:

(A) LENGTH: 7 amino acids
(B) TYPE: amino acid
(D) TOPOLOGY: linear

(ii) MOLECULE TYPE: peptide addition
(xi) SEQUENCE DESCRIPTION: SEQ ID NO: 54:

Ile-Pro-Ile-Arg-Pro-Arg-Arg 7
5

(2) INFORMATION FOR SEQ ID NO: 55:

(i) SEQUENCE CHARACTERISTICS:

(A) LENGTH: 7 amino acids
(B) TYPE: amino acid
(D) TOPOLOGY: linear

(ii) MOLECULE TYPE: peptide addition
(xi) SEQUENCE DESCRIPTION: SEQ ID NO: 55:

Ser-Thr-Arg-Arg-Pro-Arg-Pro 7
5

(2) INFORMATION FOR SEQ ID NO: 56:

(i) SEQUENCE CHARACTERISTICS:

(A) LENGTH: 7 amino acids
(B) TYPE: amino acid
(D) TOPOLOGY: linear

(ii) MOLECULE TYPE: peptide addition
(xi) SEQUENCE DESCRIPTION: SEQ ID NO: 56:

Thr-Ala-Ile-Arg-Pro-Arg-Lys 7
5

(2) INFORMATION FOR SEQ ID NO: 57:

(i) SEQUENCE CHARACTERISTICS:

(A) LENGTH: 6 amino acids
(B) TYPE: amino acid
(D) TOPOLOGY: linear

(ii) MOLECULE TYPE: peptide addition

(xi) SEQUENCE DESCRIPTION: SEQ ID NO: 57:

Trp-Arg-Trp-Arg-Trp-Arg 6
5

(2) INFORMATION FOR SEQ ID NO: 58:

(i) SEQUENCE CHARACTERISTICS:

(A) LENGTH: 5 amino acids
(B) TYPE: amino acid
(D) TOPOLOGY: linear

(ii) MOLECULE TYPE: peptide addition
(xi) SEQUENCE DESCRIPTION: SEQ ID NO: 58:

Glu-Pro-Ile-Arg-Arg 5
5

(2) INFORMATION FOR SEQ ID NO: 59:

(i) SEQUENCE CHARACTERISTICS:

(A) LENGTH: 5 amino acids
(B) TYPE: amino acid
(D) TOPOLOGY: linear

(ii) MOLECULE TYPE: peptide addition
(xi) SEQUENCE DESCRIPTION: SEQ ID NO: 59:

Ser-His-Trp-Glu-Glu 5
5

(2) INFORMATION FOR SEQ ID NO: 60:

(i) SEQUENCE CHARACTERISTICS:

(A) LENGTH: 8 amino acids
(B) TYPE: amino acid
(D) TOPOLOGY: linear

(ii) MOLECULE TYPE: peptide addition
(xi) SEQUENCE DESCRIPTION: SEQ ID NO: 60:

Arg-Pro-Arg-Pro-Arg-Pro-Arg-Pro 8
5

(2) INFORMATION FOR SEQ ID NO: 61:

(i) SEQUENCE CHARACTERISTICS:

(A) LENGTH: 11 amino acids
(B) TYPE: amino acid
(D) TOPOLOGY: linear

(ii) MOLECULE TYPE: peptide addition
(xi) SEQUENCE DESCRIPTION: SEQ ID NO: 61:

Ser-Ser-Thr-Arg-Arg-Ala-Ser-Pro-Ile-Lys-Lys 11
5 10

(2) INFORMATION FOR SEQ ID NO: 62:

(i) SEQUENCE CHARACTERISTICS:

(A) LENGTH: 11 amino acids
(B) TYPE: amino acid
(D) TOPOLOGY: linear

(ii) MOLECULE TYPE: peptide addition
(xi) SEQUENCE DESCRIPTION: SEQ ID NO: 62:

Ala-Trp-Trp-Pro-Ser-Pro-Ile-Arg-Pro-Arg-Pro 11
5 10

(2) INFORMATION FOR SEQ ID NO: 63:

(i) SEQUENCE CHARACTERISTICS:

(A) LENGTH: 12 amino acids
(B) TYPE: amino acid
(D) TOPOLOGY: linear

(ii) MOLECULE TYPE: peptide addition
(xi) SEQUENCE DESCRIPTION: SEQ ID NO: 63:

Ala-Pro-Pro-Pro-Arg-Pro-Arg-Pro-Arg-Pro-Arg-Pro 12
5 10

(2) INFORMATION FOR SEQ ID NO: 64:

(i) SEQUENCE CHARACTERISTICS:

(A) LENGTH: 12 amino acids
(B) TYPE: amino acid
(D) TOPOLOGY: linear

(ii) MOLECULE TYPE: peptide addition
(xi) SEQUENCE DESCRIPTION: SEQ ID NO: 64:

Ala-Pro-Pro-Pro-Arg-Thr-Arg-Pro-Arg-Pro-Arg-Ser 12
5 10

(2) INFORMATION FOR SEQ ID NO: 65:

(i) SEQUENCE CHARACTERISTICS:

(A) LENGTH: 8 amino acids
(B) TYPE: amino acid
(D) TOPOLOGY: linear

(ii) MOLECULE TYPE: peptide addition
(xi) SEQUENCE DESCRIPTION: SEQ ID NO: 65:

Ser-Pro-Lys-Arg-Lys-Pro-Arg-Pro 8
5

(2) INFORMATION FOR SEQ ID NO: 66:

(i) SEQUENCE CHARACTERISTICS:

(A) LENGTH: 9 amino acids
(B) TYPE: amino acid
(D) TOPOLOGY: linear

(ii) MOLECULE TYPE: peptide addition
(xi) SEQUENCE DESCRIPTION: SEQ ID NO: 66:

Ser-Gln-Arg-Ile-Lys-Gln-Arg-Ile-Lys 9
5

(2) INFORMATION FOR SEQ ID NO: 67:

(i) SEQUENCE CHARACTERISTICS:

(A) LENGTH: 8 amino acids
(B) TYPE: amino acid
(D) TOPOLOGY: linear

(ii) MOLECULE TYPE: peptide addition
(xi) SEQUENCE DESCRIPTION: SEQ ID NO: 67:

Ser-Pro-Pro-Pro-Arg-Pro-Arg-Pro 8
5

(2) INFORMATION FOR SEQ ID NO: 68:

(i) SEQUENCE CHARACTERISTICS:

(A) LENGTH: 10 amino acids
(B) TYPE: amino acid
(D) TOPOLOGY: linear

(ii) MOLECULE TYPE: peptide addition
(xi) SEQUENCE DESCRIPTION: SEQ ID NO: 68:

Ser-Pro-Ile-Arg-Pro-Arg-Pro-Arg-Pro-Arg 10
5 10

(2) INFORMATION FOR SEQ ID NO: 69:

(i) SEQUENCE CHARACTERISTICS:

(A) LENGTH: 9 amino acids
(B) TYPE: amino acid
(D) TOPOLOGY: linear

(ii) MOLECULE TYPE: peptide addition
(xi) SEQUENCE DESCRIPTION: SEQ ID NO: 69:

```
Ser-Pro-Ile-Arg-Lys-Ala-Trp-Trp-Pro                  9
                5
```

(2) INFORMATION FOR SEQ ID NO: 70:

(i) SEQUENCE CHARACTERISTICS:

(A) LENGTH: 12 amino acids
(B) TYPE: amino acid
(D) TOPOLOGY: linear

(ii) MOLECULE TYPE: peptide addition
(xi) SEQUENCE DESCRIPTION: SEQ ID NO: 70:

```
Ala-Pro-Pro-Pro-Lys-Ala-Ser-Pro-Arg-Gln-Arg-Pro      12
```

(2) INFORMATION FOR SEQ ID NO:71:

(i) SEQUENCE CHARACTERISTICS:

(A) LENGTH: 15 amino acids
(B) TYPE: amino acid
(D) TOPOLOGY: linear

(ii) MOLECULE TYPE: peptide addition
(xi) SEQUENCE DESCRIPTION: SEQ ID NO: 71:

```
Ser-Pro-Ile-Arg-Pro-Arg-Pro-Ser-Pro-Ile-Arg-Pro-Arg-Pro-Arg   15
                5                  10                  15
```

(2) INFORMATION FOR SEQ ID NO: 72:

(i) SEQUENCE CHARACTERISTICS:

(A) LENGTH: 8 amino acids
(B) TYPE: amino acid
(D) TOPOLOGY: linear

(ii) MOLECULE TYPE: peptide addition
(xi) SEQUENCE DESCRIPTION: SEQ ID NO: 72:

```
Ser-Pro-Pro-Arg-Trp-Pro-Arg-Arg                      8
                5
```

(2) INFORMATION FOR SEQ ID NO: 73:

(i) SEQUENCE CHARACTERISTICS:

(A) LENGTH: 8 amino acids
(B) TYPE: amino acid
(D) TOPOLOGY: linear

(ii) MOLECULE TYPE: peptide addition
(xi) SEQUENCE DESCRIPTION: SEQ ID NO: 73:

Ser-Pro-Pro-Arg-Trp-Pro-Arg-Trp 8
5

(2) INFORMATION FOR SEQ ID NO: 74:

(i) SEQUENCE CHARACTERISTICS:

(A) LENGTH: 8 amino acids
(B) TYPE: amino acid
(D) TOPOLOGY: linear

(ii) MOLECULE TYPE: peptide addition
(xi) SEQUENCE DESCRIPTION: SEQ ID NO: 74:

Ser-Pro-Pro-Arg-Trp-Pro-Trp-Arg 8
5

(2) INFORMATION FOR SEQ ID NO: 75:

(i) SEQUENCE CHARACTERISTICS:

(A) LENGTH: 8 amino acids
(B) TYPE: amino acid
(D) TOPOLOGY: linear

(ii) MOLECULE TYPE: peptide addition
(xi) SEQUENCE DESCRIPTION: SEQ ID NO: 75:

Ser-Pro-Pro-Trp-Arg-Pro-Arg-Arg 8
5

(2) INFORMATION FOR SEQ ID NO: 76:

(i) SEQUENCE CHARACTERISTICS:

(A) LENGTH: 8 amino acids
(B) TYPE: amino acid
(D) TOPOLOGY: linear

(ii) MOLECULE TYPE: peptide addition
(xi) SEQUENCE DESCRIPTION: SEQ ID NO: 76:

Ser-Pro-Pro-Trp-Trp-Pro-Arg-Trp 8
5

(2) INFORMATION FOR SEQ ID NO: 77:

(i) SEQUENCE CHARACTERISTICS:

(A) LENGTH: 8 amino acids
(B) TYPE: amino acid
(D) TOPOLOGY: linear

(ii) MOLECULE TYPE: peptide addition
(xi) SEQUENCE DESCRIPTION: SEQ ID NO: 77:

Ser-Pro-Pro-Trp-Trp-Pro-Trp-Arg 8
5

(2) INFORMATION FOR SEQ ID NO: 78:

(i) SEQUENCE CHARACTERISTICS:

(A) LENGTH: 8 amino acids
(B) TYPE: amino acid
(D) TOPOLOGY: linear

(ii) MOLECULE TYPE: peptide addition
(xi) SEQUENCE DESCRIPTION: SEQ ID NO: 78:

Ser-Pro-Pro-Trp-Trp-Pro-Trp-Trp 8
5

(2) INFORMATION FOR SEQ ID NO: 79:

(i) SEQUENCE CHARACTERISTICS:

(A) LENGTH: 8 amino acids
(B) TYPE: amino acid
(D) TOPOLOGY: linear

(ii) MOLECULE TYPE: peptide addition
(xi) SEQUENCE DESCRIPTION: SEQ ID NO: 79:

Ser-Pro-Pro-Trp-Pro-Arg-Pro-Arg-Pro 8
5

(2) INFORMATION FOR SEQ ID NO: 80:

(i) SEQUENCE CHARACTERISTICS:

(A) LENGTH: 7 amino acids
(B) TYPE: amino acid
(D) TOPOLOGY: linear

(ii) MOLECULE TYPE: peptide addition
(xi) SEQUENCE DESCRIPTION: SEQ ID NO: 80:

Ser -Ser- Lys-Gln- Asp- Tyr- Arg 7
5

(2) INFORMATION FOR SEQ ID NO: 81:

(i) SEQUENCE CHARACTERISTICS:

(A) LENGTH: 42 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer TiK 57"

(xi) SEQUENCE DESCRIPTION: SEQ ID No. 81:

CTTGGCTAGC CCTATACGTA GATCATCCAC ACAAGATTAT CG 42

(2) INFORMATION FOR SEQ ID NO: 82:

(i) SEQUENCE CHARACTERISTICS:

(A) LENGTH: 32 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer TiK 58"

(xi) SEQUENCE DESCRIPTION: SEQ ID No. 82:

CTTGGCTAGC TCCACACAAG ATTATCGTAT TG 32

(2) INFORMATION FOR SEQ ID NO: 83:

(i) SEQUENCE CHARACTERISTICS:

(A) LENGTH: 32 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer TiK 59"

(xi) SEQUENCE DESCRIPTION: SEQ ID No. 83:

GCCCTCTAGA CTATAAACAG AGACCAGTGT TC 32

(2) INFORMATION FOR SEQ ID NO: 84:

(i) SEQUENCE CHARACTERISTICS:

(A) LENGTH: 83 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer TiK 60"
( B) 5: 90% A and 3.33% of each C, G, T.
6: 90% C and 3.33% of each A, G, T.
7: 90% G and 3.33% of each A, C, T.
8: 90% T and 3.33% of each A, C, G.

(xi) SEQUENCE DESCRIPTION: SEQ ID No. 84:

GTAGTTTTTC GTGGT5CA57 (C/G)TCA58(5/8)67(C/G)
556(7/8)(6/7)(C/G)58(5/8)7 6(T/G)75658(5/8)78
(T/G)88878(T/G)66(G/C) 78(C/G)55885TC CACCTGTTAA TGG 83

(2) INFORMATION FOR SEQ ID NO: 85:

(i) SEQUENCE CHARACTERISTICS:

(A) LENGTH: 17 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer TiK 61"

(xi) SEQUENCE DESCRIPTION: SEQ ID No. 85:

AGAACAGCTG TTGCACC 17

(2) INFORMATION FOR SEQ ID NO: 86:

(i) SEQUENCE CHARACTERISTICS:

(A) LENGTH: 19 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer TiK 62"

(xi) SEQUENCE DESCRIPTION: SEQ ID No. 86:

CCGGGGA TCCACCATG 19

(2) INFORMATION FOR SEQ ID NO: 87:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer TiK 64"

(xi) SEQUENCE DESCRIPTION: SEQ ID No. 87:

GCCCTCTAGA CTATAAACAG 20

(2) INFORMATION FOR SEQ ID NO: 88:

(i) SEQUENCE CHARACTERISTICS:

(A) LENGTH: 17 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer TiK 66"

(xi) SEQUENCE DESCRIPTION: SEQ ID No. 88:

CTGCAGAACT GTCATTC 17

(2) INFORMATION FOR SEQ ID NO: 89:

(i) SEQUENCE CHARACTERISTICS:

(A) LENGTH: 16 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer TiK 72"

(xi) SEQUENCE DESCRIPTION: SEQ ID No. 89:

TTGAGCTTGT ACCACG 16

(2) INFORMATION FOR SEQ ID NO: 90:

(i) SEQUENCE CHARACTERISTICS:

(A) LENGTH: 36 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer TiK 74"

(xi) SEQUENCE DESCRIPTION: SEQ ID No. 90:

```
CCGGGGATCC ACCATGAGAT TTCCTTCTAT TTTTAC                    36
```

(2) INFORMATION FOR SEQ ID NO: 91:

(i) SEQUENCE CHARACTERISTICS:

(A) LENGTH: 31 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer TiK 75"

(xi) SEQUENCE DESCRIPTION: SEQ ID No. 91:

```
TGGAGCTAGC TCTTTTATCC AAAGAAACAC C                         31
```

(2) INFORMATION FOR SEQ ID NO: 92:

(i) SEQUENCE CHARACTERISTICS:

(A) LENGTH: 85 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "Primer TiK 76"
(B) 5: 90% G and 3.33% of each C, G, T.
6: 90% A and 3.33% of each A, G, T.
7: 90% T and 3.33% of each A, C, T.
8: 90% C and 3.33% of each A, C, G.

(xi) SEQUENCE DESCRIPTION: SEQ ID No. 92:

CAGCCAATGC ATACTGC655 6885776778 8755755765
575565875T 88786775T5 57577GCATC CAATTTGCAA
ATTAC 85

(2) INFORMATION FOR SEQ ID NO: 93:

(i) SEQUENCE CHARACTERISTICS:

(A) LENGTH: 1115 base pairs
(B) TYPE: nucleic acid
(C) STRANDEDNESS: single
(D) TOPOLOGY: linear

(ii) MOLECULE TYPE: cDNA
(vi) ORIGINAL SOURCE:

(B) STRAIN: Absidia reflexa ATTC 44896

(xi) SEQUENCE DESCRIPTION: SEQ ID NO: 93:

AAAGGCATTC TCATTTTGTA GTCTTATTGC TAGCAGTATT CATCTGCATG TGCTCTGTAT 60
CGGGTGTGCC ACTGCAAATT GATCCACGCG ATGACAAGAG CTATGTTCCT GAACAATATC 120
CTTTGAAGGT GAATGGTCCT TTGCCAGAAG GTGTAAGCGT GATCCAAGGC TATTGTGAAA 180
ACTGTACCAT GTATCCTGAA AAAAATAGTG TATCGGCATT CTCGTCATCA TCCACACAAG 240
ATTATCGTAT TGCAAGCGAG GCAGAGATTA AGGCACACAC ATTTTACACA GCATTGTCAG 300
CCAATGCATA CTGCAGAACT GTCATTCCTG GTGGTCGATG GAGCTGTCCC CACTGTGGTG 360
TTGCATCCAA TTTGCAAATT ACCAAGACTT TCAGCACCTT AATCACTGAT ACTAATGTCT 420
TGGTGGCTGT TGGCGAAAAG GAGAAGACCA TCTATGTAGT TTTTCGTGGT ACAAGCTCAA 480
TTCGCAACGC CATTGCTGAC ATTGTTTTTG TACCAGTGAA TTATCCACCT GTTAATGGAG 540
CCAAAGTACA CAAAGGATTT CTTGATAGCT ATAACGAAGT CCAGGATAAA CTTGTTGCTG 600
AAGTCAAGGC ACAACTTGAT CGTCATCCAG GATACAAGAT CGTCGTCACT GGACATTCCT 660
TGGGAGGTGC AACAGCTGTT CTCAGTGCAC TTGACCTTTA TCACCATGGC CATGCCAATA 720
TCGAAATCTA TACTCAAGGT CAGCCACGTA TAGGTACTCC AGCATTTGCA AACTATGTGA 780
TAGGCACCAA GATTCCATAC CAACGTCTTG TCCATGAGCG TGACATTGTT CCTCACCTTC 840
CACCTGGTGC ATTTGGTTTC TTGCATGCTG GTGAAGAGTT TTGGATCATG AAAGATAGCT 900
CGTTGCGCGT ATGTCCAAAT GGCATTGAAA CTGACAACTG CAGCAACTCC ATTGTTCCCT 960
TCACTAGTGT CATTGACCAT TTAAGCTATC TTGACATGAA CACTGGTCTC TGTTTATAAT 1020
CTTTAGTATC ATCCACTCCT CCTCTTTAAT GCAATACTTT TTAAGATAAA TCACAAGTAT 1080
ACTTTGTACA AAACCAAAAA AAAAAAAAAA AAAAA 1115

(2) INFORMATION FOR SEQ ID NO: 94:

(i) SEQUENCE CHARACTERISTICS:

(A) LENGTH: 1020 base pairs
(B) TYPE: nucleic acid
(C) STRANDEDNESS: single
(D) TOPOLOGY: circular

(ii) MOLECULE TYPE: other nucleic acid

(A) DESCRIPTION: /desc = "Yeast expression vector pTik05"

(ix) FEATURE:

(A) NAME/KEY: CDS
(B) LOCATION:3..1020

(xi) SEQUENCE DESCRIPTION: SEQ ID NO: 94:

```
CA CAT ACA GGA ATT CAT TCA AGA ATA GTT CAA ACA AGA AGA TTA CAA          47
   His Thr Gly Ile His Ser Arg Ile Val Gln Thr Arg Arg Leu Gln
     1               5                  10                  15

ACT ATC AAT TTC ATA CAC AAT ATA AAC GAC GGT ACC CGG GGA TCC ACC         95
Thr Ile Asn Phe Ile His Asn Ile Asn Asp Gly Thr Arg Gly Ser Thr
                 20                  25                  30

ATG AGG AGC TCC CTT GTG CTG TTC TTT GTC TCT GCG TGG ACG GCC TTG        143
Met Arg Ser Ser Leu Val Leu Phe Phe Val Ser Ala Trp Thr Ala Leu
             35                  40                  45

GCT AGC TCC ACA CAA GAT TAT CGT ATT GCA AGC GAG GCA GAG ATT AAG        191
Ala Ser Ser Thr Gln Asp Tyr Arg Ile Ala Ser Glu Ala Glu Ile Lys
         50                  55                  60
```

```
GCA CAC ACA TTT TAC ACA GCA TTG TCA GCC AAT GCA TAC TGC AGA ACT      239
Ala His Thr Phe Tyr Thr Ala Leu Ser Ala Asn Ala Tyr Cys Arg Thr
    65                  70                  75

GTC ATT CCT GGT GGT CGA TGG AGC TGT CCC CAC TGT GGT GTT GCA TCC      287
Val Ile Pro Gly Gly Arg Trp Ser Cys Pro His Cys Gly Val Ala Ser
 80                  85                  90                  95

AAT TTG CAA ATT ACC AAG ACT TTC AGC ACC TTA ATC ACT GAT ACT AAT      335
Asn Leu Gln Ile Thr Lys Thr Phe Ser Thr Leu Ile Thr Asp Thr Asn
                100                 105                 110

GTC TTG GTG GCT GTT GGC GAA AAG GTT GTT TTT GTA CCA GTG AAT TAT      383
Val Leu Val Ala Val Gly Glu Lys Val Val Phe Val Pro Val Asn Tyr
            115                 120                 125

CCA CCT GTT AAT GGA GCC AAA GTA CAC AAA GGA TTT CTT GAT AGC TAT      431
Pro Pro Val Asn Gly Ala Lys Val His Lys Gly Phe Leu Asp Ser Tyr
        130                 135                 140

AAC GAA GTC CAG GAT AAA CTT GTT GCT GAA GTC AAG GCA CAA CTT GAT      479
Asn Glu Val Gln Asp Lys Leu Val Ala Glu Val Lys Ala Gln Leu Asp
    145                 150                 155

CGT CAT CCA GGA TAC AAG ATC GTC GTC ACT GGA CAT TCC TTG GGA GGT      527
Arg His Pro Gly Tyr Lys Ile Val Val Thr Gly His Ser Leu Gly Gly
160                 165                 170                 175

GCA ACA GCT GTT CTC AGT GCA CTT GAC CTT TAT CAC CAT GGC CAT GCC      575
Ala Thr Ala Val Leu Ser Ala Leu Asp Leu Tyr His His Gly His Ala
                180                 185                 190

AAT ATC GAA ATC TAT ACT CAA GGT CAG CCA CGT ATA GGT ACT CCA GCA      623
Asn Ile Glu Ile Tyr Thr Gln Gly Gln Pro Arg Ile Gly Thr Pro Ala
            195                 200                 205

TTT GCA AAC TAT GTG ATT GGC ACC AAG ATT CCA TAC CAA CGT CTT GTC      671
Phe Ala Asn Tyr Val Ile Gly Thr Lys Ile Pro Tyr Gln Arg Leu Val
        210                 215                 220

CAT GAG CGT GAC ATT GTT CCT CAC CTT CCA CCT GGT GCA TTT GGT TTC      719
His Glu Arg Asp Ile Val Pro His Leu Pro Pro Gly Ala Phe Gly Phe
    225                 230                 235

TTG CAT GCT GGT GAA GAG TTT TGG ATC ATG AAA GAT AGC TCG TTG CGC      767
Leu His Ala Gly Glu Glu Phe Trp Ile Met Lys Asp Ser Ser Leu Arg
240                 245                 250                 255

GTA TGT CCA AAT GGC ATT GAA ACT GAC AAC TGC AGC AAC TCC ATT GTT      815
Val Cys Pro Asn Gly Ile Glu Thr Asp Asn Cys Ser Asn Ser Ile Val
                260                 265                 270

CCC TTC ACT AGT GTC ATT GAC CAT TTA AGC TAT CTT GAC ATG AAC ACT      863
Pro Phe Thr Ser Val Ile Asp His Leu Ser Tyr Leu Asp Met Asn Thr
            275                 280                 285

GGT CTC TGT TTA TAG TCT AGA GGG CCG CAT GAT GTA ATT AGT TAT GTC      911
Gly Leu Cys Leu  *  Ser Arg Gly Pro His Asp Val Ile Ser Tyr Val
        290                 295                 300

ACG CTT ACA TTC ACG CCC TCC CCC CAC ATC CGC TCT AAC CGA AAA GGA      959
Thr Leu Thr Phe Thr Pro Ser Pro His Ile Arg Ser Asn Arg Lys Gly
    305                 310                 315
```

```
AGG AGT TAG ACA ACC TGA AGT CTA GGT CCC TAT TTA TTT TTT TAT AGT        1007
Arg Ser  *  Thr Thr  *  Ser Leu Gly Pro Tyr Leu Phe Phe Tyr Ser
320                 325                 330                 335

TAT GTT AGT ATT  A                                                     1020
Tyr Val Ser Ile
```

(2) INFORMATION FOR SEQ ID NO: 95:

(i) SEQUENCE CHARACTERISTICS:

(A) LENGTH: 339 amino acids
(B) TYPE: amino acid
(D) TOPOLOGY: linear

(ii) MOLECULE TYPE: protein
(xi) SEQUENCE DESCRIPTION: SEQ ID NO: 95:

His Thr Gly Ile His Ser Arg Ile Val Gln Thr Arg Arg Leu Gln Thr
1 5 10 15

Ile Asn Phe Ile His Asn Ile Asn Asp Gly Thr Arg Gly Ser Thr Met
20 25 30

Arg Ser Ser Leu Val Leu Phe Phe Val Ser Ala Trp Thr Ala Leu Ala
35 40 45

Ser Ser Thr Gln Asp Tyr Arg Ile Ala Ser Glu Ala Glu Ile Lys Ala
50 55 60

His Thr Phe Tyr Thr Ala Leu Ser Ala Asn Ala Tyr Cys Arg Thr Val
65 70 75 80

Ile Pro Gly Gly Arg Trp Ser Cys Pro His Cys Gly Val Ala Ser Asn
85 90 95

Leu Gln Ile Thr Lys Thr Phe Ser Thr Leu Ile Thr Asp Thr Asn Val
100 105 110

Leu Val Ala Val Gly Glu Lys Val Val Phe Val Pro Val Asn Tyr Pro
115 120 125

Pro Val Asn Gly Ala Lys Val His Lys Gly Phe Leu Asp Ser Tyr Asn
130 135 140

Glu Val Gln Asp Lys Leu Val Ala Glu Val Lys Ala Gln Leu Asp Arg
145 150 155 160

His Pro Gly Tyr Lys Ile Val Val Thr Gly His Ser Leu Gly Gly Ala
165 170 175

Thr Ala Val Leu Ser Ala Leu Asp Leu Tyr His His Gly His Ala Asn
180 185 190

Ile Glu Ile Tyr Thr Gln Gly Gln Pro Arg Ile Gly Thr Pro Ala Phe
195 200 205

Ala Asn Tyr Val Ile Gly Thr Lys Ile Pro Tyr Gln Arg Leu Val His
210 215 220

Glu Arg Asp Ile Val Pro His Leu Pro Pro Gly Ala Phe Gly Phe Leu
225 230 235 240

His Ala Gly Glu Glu Phe Trp Ile Met Lys Asp Ser Ser Leu Arg Val

```
                    245                 250                 255

        Cys Pro Asn Gly Ile Glu Thr Asp Asn Cys Ser Asn Ser Ile Val Pro
                    260                 265                 270

        Phe Thr Ser Val Ile Asp His Leu Ser Tyr Leu Asp Met Asn Thr Gly
                275                 280                 285

        Leu Cys Leu  *  Ser Arg Gly Pro His Asp Val Ile Ser Tyr Val Thr
            290                 295                 300

        Leu Thr Phe Thr Pro Ser Pro His Ile Arg Ser Asn Arg Lys Gly Arg
        305                 310                 315                 320

        Ser  *  Thr Thr  *  Ser Leu Gly Pro Tyr Leu Phe Phe Tyr Ser Tyr
                        325                 330                 335

        Val Ser Ile
```

(2) INFORMATION FOR SEQ ID NO: 96:

(i) SEQUENCE CHARACTERISTICS:

(A) LENGTH: 255 base pairs
(B) TYPE: nucleic acid
(C) STRANDEDNESS: single
(D) TOPOLOGY: linear

(ii) MOLECULE TYPE: DNA (genomic)
(vi) ORIGINAL SOURCE:

(A) ORGANISM: MF$\alpha$1 signal sequence (mating factor)
(B) STRAIN: yeast strain YPH499

(ix) FEATURE:

(A) NAME/KEY: sig_peptide
(B) LOCATION:1..255

(ix) FEATURE:

(A) NAME/KEY: CDS
(B) LOCATION:1..255

(xi) SEQUENCE DESCRIPTION: SEQ ID NO: 96:

```
ATG AGA TTT CCT TCT ATT TTT ACT GCT GTT TTA TTC GCT GCT TCC TCC        48
Met Arg Phe Pro Ser Ile Phe Thr Ala Val Leu Phe Ala Ala Ser Ser
  1               5                  10                  15

GCT TTA GCT GCT CCA GTC AAC ACT ACC ACT GAA GAT GAA ACG GCT CAA        96
Ala Leu Ala Ala Pro Val Asn Thr Thr Thr Glu Asp Glu Thr Ala Gln
             20                  25                  30

ATT CCA GCT GAA GCT GTC ATC GGT TAC CTT GAT TTA GAA GGT GAT TTC       144
Ile Pro Ala Glu Ala Val Ile Gly Tyr Leu Asp Leu Glu Gly Asp Phe
         35                  40                  45

GAT GTT GCT GTT TTG CCA TTT TCC AAC TCC ACC AAT AAC GGT TTA TTG       192
Asp Val Ala Val Leu Pro Phe Ser Asn Ser Thr Asn Asn Gly Leu Leu
     50                  55                  60

TTT ATC AAT ACT ACT ATT GCC TCC ATT GCT GCT AAA GAA GAA GGT GTT       240
Phe Ile Asn Thr Thr Ile Ala Ser Ile Ala Ala Lys Glu Glu Gly Val
 65                  70                  75                  80

TCT TTG GAT AAA AGA                                                   255
Ser Leu Asp Lys Arg
                 85
```

(2) INFORMATION FOR SEQ ID NO: 97:

(i) SEQUENCE CHARACTERISTICS:

(A) LENGTH: 85 amino acids
(B) TYPE: amino acid
(D) TOPOLOGY: linear

(ii) MOLECULE TYPE: protein
(xi) SEQUENCE DESCRIPTION: SEQ ID NO: 97:

```
Met Arg Phe Pro Ser Ile Phe Thr Ala Val Leu Phe Ala Ala Ser Ser
  1               5                  10                  15

Ala Leu Ala Ala Pro Val Asn Thr Thr Thr Glu Asp Glu Thr Ala Gln
             20                  25                  30

Ile Pro Ala Glu Ala Val Ile Gly Tyr Leu Asp Leu Glu Gly Asp Phe
         35                  40                  45

Asp Val Ala Val Leu Pro Phe Ser Asn Ser Thr Asn Asn Gly Leu Leu
     50                  55                  60

Phe Ile Asn Thr Thr Ile Ala Ser Ile Ala Ala Lys Glu Glu Gly Val
 65                  70                  75                  80

Ser Leu Asp Lys Arg
                 85
```

(2) INFORMATION FOR SEQ ID NO: 98:

(i) SEQUENCE CHARACTERISTICS:

(A) LENGTH: amino acids
(B) TYPE: amino acid
(D) TOPOLOGY: linear

(ii) MOLECULE TYPE: protein
(xi) SEQUENCE DESCRIPTION: SEQ ID NO: 98:

MRFYSVVSLLAVSICTYGVSGVPVQIGPRDKSYVPEQ YPLKMNGPLPEGVSVIQGYCENCTMYPEENS-
VTALSSSKQDYRTASETEIQAHTFYTALSANAYCRNVIPGGRWSCPHCDVTSNLKITKTFSTLITDTNVAVAV
GEKEKTIYIVFRGTNSIRNAIADIVFVPVDYPPVDGAKVHKGFLDSYNEVQDQLVAEVKKQLDNHPGYKIVVA
GHSLGGATAVLCALDLYHHGHHNIEIYTQGQPRVGTPAFAKYVIGTKIPYQRLVNERDIVPHLPPGAFGFLHA
GEEFWIMKDSSLRVCPNGIETDDCSNSIVPFTSVIDHLSYLDMNTGLCL*

(2) INFORMATION FOR SEQ ID NO: 99:

(i) SEQUENCE CHARACTERISTICS:

(A) LENGTH: 864 base pairs
(B) TYPE: nucleic acid
(C) STRANDEDNESS: single
(D) TOPOLOGY: linear

(ii) MOLECULE TYPE: DNA (genomic)
(vi) ORIGINAL SOURCE:

(B) STRAIN: Pseudomonas sp.

(ix) FEATURE:

(A) NAME/KEY: mat_peptide
(B) LOCATION:1..864

(ix) FEATURE:

(A) NAME/KEY: CDS
(B) LOCATION:1..864

(xi) SEQUENCE DESCRIPTION: SEQ ID NO: 99:

```
TTC GGC TCC TCG AAC TAC ACC AAG ACC CAG TAC CCG ATC GTC CTG ACC      48
Phe Gly Ser Ser Asn Tyr Thr Lys Thr Gln Tyr Pro Ile Val Leu Thr
  1               5                  10                  15

CAC GGC ATG CTC GGT TTC GAC AGC CTG CTT GGA GTC GAC TAC TGG TAC      96
His Gly Met Leu Gly Phe Asp Ser Leu Leu Gly Val Asp Tyr Trp Tyr
             20                  25                  30

GGC ATT CCC TCA GCC CTG CGT AAA GAC GGC GCC ACC GTC TAC GTC ACC     144
Gly Ile Pro Ser Ala Leu Arg Lys Asp Gly Ala Thr Val Tyr Val Thr
         35                  40                  45

GAA GTC AGC CAG CTC GAC ACC TCC GAA GCC CGA GGT GAG CAA CTG CTG     192
Glu Val Ser Gln Leu Asp Thr Ser Glu Ala Arg Gly Glu Gln Leu Leu
     50                  55                  60

ACC CAA GTC GAG GAA ATC GTG GCC ATC AGC GGC AAG CCC AAG GTC AAC     240
Thr Gln Val Glu Glu Ile Val Ala Ile Ser Gly Lys Pro Lys Val Asn
 65                  70                  75                  80

CTG TTC GGC CAC AGC CAT GGC GGG CCT ACC ATC CGC TAC GTT GCC GCC     288
Leu Phe Gly His Ser His Gly Gly Pro Thr Ile Arg Tyr Val Ala Ala
                 85                  90                  95

GTG CGC CCG GAT CTG GTC GCC TCG GTC ACC AGC ATT GGC GCG CCG CAC     336
Val Arg Pro Asp Leu Val Ala Ser Val Thr Ser Ile Gly Ala Pro His
            100                 105                 110

AAG GGT TCG GCC ACC GCC GAC TTC ATC CGC CAG GTG CCG GAA GGA TCG     384
Lys Gly Ser Ala Thr Ala Asp Phe Ile Arg Gln Val Pro Glu Gly Ser
        115                 120                 125

GCC AGC GAA GCG ATT CTG GCC GGG ATC GTC AAT GGT CTG GGT GCG CTG     432
Ala Ser Glu Ala Ile Leu Ala Gly Ile Val Asn Gly Leu Gly Ala Leu
    130                 135                 140

ATC AAC TTC CTT TCC GGC AGC AGT TCG GAC ACC CCA CAG AAC TCG CTG     480
Ile Asn Phe Leu Ser Gly Ser Ser Ser Asp Thr Pro Gln Asn Ser Leu
145                 150                 155                 160

GGC ACG CTG GAG TCA CTG AAC TCC GAA GGC GCC GCA CGG TTT AAC GCC     528
Gly Thr Leu Glu Ser Leu Asn Ser Glu Gly Ala Ala Arg Phe Asn Ala
                165                 170                 175

CGC TTC CCC CAG GGG GTA CCA ACC AGC GCC TGC GGC GAG GGC GAT TAC     576
Arg Phe Pro Gln Gly Val Pro Thr Ser Ala Cys Gly Glu Gly Asp Tyr
            180                 185                 190
```

```
GTG GTC AAT GGC GTG CGC TAT TAC TCC TGG AGG GGC ACC AGC CCG CTG      624
Val Val Asn Gly Val Arg Tyr Tyr Ser Trp Arg Gly Thr Ser Pro Leu
        195                 200                 205

ACC AAC GTA CTC GAC CCC TCC GAC CTG CTG CTC GGC GCC ACC TCC CTG      672
Thr Asn Val Leu Asp Pro Ser Asp Leu Leu Leu Gly Ala Thr Ser Leu
    210                 215                 220

ACC TTC GGT TTC GAG GCC AAC GAT GGT CTG GTC GGA CGC TGC AGC TCC      720
Thr Phe Gly Phe Glu Ala Asn Asp Gly Leu Val Gly Arg Cys Ser Ser
225                 230                 235                 240

CGG CTG GGT ATG GTG ATC CGC GAC AAC TAC CGG ATG AAC CAC CTG GAC      768
Arg Leu Gly Met Val Ile Arg Asp Asn Tyr Arg Met Asn His Leu Asp
                245                 250                 255

GAG GTG AAC CAG ACC TTC GGG CTG ACC AGC ATC TTC GAG ACC AGC CCG      816
Glu Val Asn Gln Thr Phe Gly Leu Thr Ser Ile Phe Glu Thr Ser Pro
            260                 265                 270

GTA TCG GTC TAT CGC CAG CAA GCC AAT CGC CTG AAG AAC GCC GGG CTC      864
Val Ser Val Tyr Arg Gln Gln Ala Asn Arg Leu Lys Asn Ala Gly Leu
        275                 280                 285
```

(2) INFORMATION FOR SEQ ID NO: 100:

(i) SEQUENCE CHARACTERISTICS:

(A) LENGTH: 288 amino acids
(B) TYPE: amino acid
(D) TOPOLOGY: linear

(ii) MOLECULE TYPE: protein
(xi) SEQUENCE DESCRIPTION: SEQ ID NO: 100:

Phe Gly Ser Ser Asn Tyr Thr Lys Thr Gln Tyr Pro Ile Val Leu Thr
1 5 10 15

His Gly Met Leu Gly Phe Asp Ser Leu Leu Gly Val Asp Tyr Trp Tyr
20 25 30

Gly Ile Pro Ser Ala Leu Arg Lys Asp Gly Ala Thr Val Tyr Val Thr
35 40 45

Glu Val Ser Gln Leu Asp Thr Ser Glu Ala Arg Gly Glu Gln Leu Leu
50 55 60

Thr Gln Val Glu Glu Ile Val Ala Ile Ser Gly Lys Pro Lys Val Asn
65 70 75 80

Leu Phe Gly His Ser His Gly Gly Pro Thr Ile Arg Tyr Val Ala Ala
85 90 95

Val Arg Pro Asp Leu Val Ala Ser Val Thr Ser Ile Gly Ala Pro His
100 105 110

Lys Gly Ser Ala Thr Ala Asp Phe Ile Arg Gln Val Pro Glu Gly Ser
115 120 125

Ala Ser Glu Ala Ile Leu Ala Gly Ile Val Asn Gly Leu Gly Ala Leu
130 135 140

Ile Asn Phe Leu Ser Gly Ser Ser Ser Asp Thr Pro Gln Asn Ser Leu
145 150 155 160

Gly Thr Leu Glu Ser Leu Asn Ser Glu Gly Ala Ala Arg Phe Asn Ala
165 170 175

Arg Phe Pro Gln Gly Val Pro Thr Ser Ala Cys Gly Glu Gly Asp Tyr
180 185 190

Val Val Asn Gly Val Arg Tyr Tyr Ser Trp Arg Gly Thr Ser Pro Leu
195 200 205

Thr Asn Val Leu Asp Pro Ser Asp Leu Leu Leu Gly Ala Thr Ser Leu
210 215 220

Thr Phe Gly Phe Glu Ala Asn Asp Gly Leu Val Gly Arg Cys Ser Ser
225 230 235 240

Arg Leu Gly Met Val Ile Arg Asp Asn Tyr Arg Met Asn His Leu Asp
245 250 255

Glu Val Asn Gln Thr Phe Gly Leu Thr Ser Ile Phe Glu Thr Ser Pro
260 265 270

Val Ser Val Tyr Arg Gln Gln Ala Asn Arg Leu Lys Asn Ala Gly Leu
275 280 285